# EUROPEAN PATENT APPLICATION

(11) **EP 2 742 949 A1**
(43) Date of publication of application: **18.06.2014**
(21) Application number: 13191686.8
(22) Date of filing: 15.11.2010
(51) Int. Cl.: A61K 38/37

(54) **Factor VIII B cell epitope variants having reduced immunogenicity**

(30) Priority: 13.11.2009 US 261296 P; 03.12.2009 US 266471 P
(62) Divisional of application: 10830867.7
(71) Applicant: Puget Sound Blood Center, Seattle, Washington 98104-1256 (US); James, Eddie Arthur, Monroe, WA 98272 (US)
(72) Inventor: Pratt, Kathleen, Seattle, WA 98104 (US); Ettinger, Ruth, Seattle, WA 98104 (US)
(74) Representative: Atkinson, Jennifer

(57) **Abstract**

Provided herein are methods and compositions for preventing or reducing an initial immune response to factor VIII in patients suffering from hemophilia A, and for reducing the intensity of the immune response in patients having pre-formed inhibitor antibodies against factor VIII.

## Description

### CROSS REFERENCE TO RELATED APPLICATIONS

This application claims the benefit of U.S. Provisional Application No. 61/261,296, filed November 13, 2009, and U.S. Provisional Application No. 61/266,471, filed December 3, 2009, the entire disclosures of which are hereby incorporated by reference in their entirety for all purposes.

### STATEMENT REGARDING FEDERALLY SPONSORED RESEARCH OR DEVELOPMENT

This invention was made with government support under NIH 1RC2HL101851-01 awarded by NIH/NHLBI. The government has certain rights in the invention.

### BACKGROUND

Factor VIII (FVIII) is a protein found in blood plasma which acts as a cofactor in the cascade of reactions leading to blood coagulation. A deficiency in the amount of FVIII activity in the blood results in the clotting disorder known as hemophilia A, which is primarily a congenital condition but can also be acquired in rare cases. Hemophilia A is currently treated with therapeutic preparations of FVIII derived from human plasma or manufactured using recombinant DNA technology. FVIII can be administered in response to a bleeding episode (on-demand therapy) and/or at frequent, regular intervals to prevent uncontrolled bleeding (prophylaxis).

Up to 30% of patients with severe hemophilia A (FVIII activity < 1%) develop inhibitory antibodies to FVIII as a consequence of treatment with therapeutic preparations of FVIII (Lusher et al., J Thromb Haemost; 2:574-583 (2004); Scharrer et al., Haemophilia; 5:145-154 (1999)). Frequently, the inhibitors are persistent and of sufficiently high titer that infusion of FVIII concentrates is ineffective for controlling bleeding episodes. Inhibitor formation therefore represents a major obstacle in treating patients with hemophilia A. In patients with high titer inhibitors, acute bleeding can sometimes be controlled by infusion of bypass clotting factors, including activated prothrombin complex concentrates and/or recombinant human factor VIIa. Bypass factors are considerably more expensive than standard FVIII concentrates, and their use in long-term prophylaxis regimens is limited due to their thrombogenic potential and unreliable hemostatic profile (Hay et al., Br J Haematol; 133:591-605 (2006); Paisley et al., Haemophilia; 9:405-417 (2003)). As a result, patients with persistent high titer inhibitors have a markedly reduced quality of life due to frequent joint bleeds and the early progression of arthropathies (Morfini et al., Haemophilia; 13:606-612 (2007)).

Accordingly, there is a need in the art for safe, effective, and/or low cost treatments for hemophilia patients with inhibitors to FVIII. There is also a need for less immunogenic/antigenic hemophilia treatments, which would reduce and/or prevent the incidence of inhibitor development.

### SUMMARY

Disclosed herein is a modified Factor VIII polypeptide comprising at least one amino acid modification in an unmodified Factor VIII polypeptide, wherein the at least one amino acid modification is at a position corresponding to positions 2173-2332 of the C2 domain of the amino acid sequence set forth in SEQ ID NO:1, and wherein the at least one amino acid modification is at a position corresponding to position 2220, 2196, 2198, 2199, 2200, 2215, 2273, 2231, or 2272 of the amino acid sequence set forth in SEQ ID NO:1.

In some embodiments, the at least one additional amino acid modification is at a position corresponding to position 2220, 2196, 2198, 2199, 2200, or 2215 of the amino acid sequence set forth in SEQ ID NO:1. In some embodiments, the at least one additional amino acid modification is an amino acid substitution at a position corresponding to position 2220, 2196, 2198, 2199, 2200, or 2215 of the amino acid sequence set forth in SEQ ID NO:1, selected from the group consisting of R2220A, R2220Q, F2196A, N2198A, M2199A, L2200A, and R2215A. In some embodiments, the at least one additional amino acid modification is an amino acid substitution at a position corresponding to position 2220, 2196, 2198, 2199, 2200, or 2215 of the amino acid sequence set forth in SEQ ID NO:1, selected from the group consisting of F2196R, N2198R, M2199R, L2200R, and R2215R.

In some embodiments, the at least one additional amino acid modification is at a position corresponding to position 2220 of the amino acid sequence set forth in SEQ ID NO:1. In some embodiments, the at least one additional amino acid modification is at a position corresponding to position 2196 of the amino acid sequence set forth in SEQ ID NO:1. In some embodiments, the at least one additional amino acid modification is at a position corresponding to position 2198 of the amino acid sequence set forth in SEQ ID NO:1. In some embodiments, the at least one additional amino acid modification is at a position corresponding to position 2199 of the amino acid sequence set forth in SEQ ID NO:1. In some embodiments, the at least one additional amino acid modification is at a position corresponding to position 2200 of the amino acid sequence set forth in SEQ ID NO:1. In some embodiments, the at least one additional amino acid modification is at a position corresponding to position 2215 of the amino acid sequence set forth in SEQ ID NO:1. In some embodiments, the at least one additional amino acid modification is at a position corresponding to position 2273 of the amino acid sequence set forth in SEQ ID NO:1. In some embodiments, the at least one additional amino acid modification is at a position corresponding to position 2272 of the amino acid sequence set forth in SEQ ID NO:1. In some embodiments, the at least one additional amino acid modification is at a position corresponding to position 2231 of the amino acid sequence set forth in SEQ ID NO:1.

In some embodiments, the at least one amino acid modification is an amino acid deletion. In some embodiments, the at least one amino acid modification is an amino acid addition. In some embodiments, the at least one amino acid modification is an amino acid substitution. In some embodiments, the at least one amino acid modification is a covalent chemical modification.

In some embodiments, the at least one amino acid modification is a modification in a B cell epitope. In some embodiments, the modified Factor VIII polypeptide retains an activity of the unmodified Factor VIII polypeptide. In some embodiments, the modified Factor VIII polypeptide exhibits reduced immunogenicity/antigenicity upon administration to a subject compared to the unmodified Factor VIII polypeptide.

In some embodiments, the unmodified Factor VIII polypeptide comprises an amino acid sequence that has 40%, 50%, 60%, 70%, 80%, 90%, 91%, 92%, 93%, 94%, 95%, 96%, 97%, 98%, or 99% sequence identity to the amino acid sequence set forth in SEQ ID NO:1, excluding amino acid modification(s).

In some embodiments, the modified Factor VIII polypeptide is a human polypeptide. In some embodiments, the modified Factor VIII polypeptide is a non-human polypeptide.

In some embodiments, the modified Factor VIII polypeptide comprises at least 2, 3, 4, 5, 6, 7, 8, 9, 10, 11, 12, 13, 14, 15, 16, 17, 18, 19, or 20 amino acid modifications. In some embodiments, the modified Factor VIII polypeptide comprises 6 amino acid modifications. In some embodiments, the modified Factor VIII polypeptide comprises a single amino acid modification.

In some embodiments, the modified Factor VIII polypeptide further comprises at least one additional amino acid modification. In some embodiments, the at least one additional amino acid modification is a modification in a T cell epitope. In some embodiments, the at least one additional amino acid modification is at a position corresponding to positions 2173-2332 of the C2 domain of the amino acid sequence set forth in SEQ ID NO:1 or positions 373-740 of the A2 domain of the amino acid sequence set forth in SEQ ID NO:1. In some embodiments, the at least one additional amino acid modification is at a position corresponding to positions 2194-2213 of the amino acid sequence set forth in SEQ ID NO:1. In some embodiments, the at least one additional amino acid modification is at a position corresponding to positions 2202-2221 of the amino acid sequence set forth in SEQ ID NO:1. In some embodiments, the at least one additional amino acid modification is at a position corresponding to positions 2194-2205 of the amino acid sequence set forth in SEQ ID NO:1. In some embodiments, the at least one additional amino acid modification is at a position corresponding to positions 2196-2204 of the amino acid sequence set forth in SEQ ID NO:1. In some embodiments, the at least one additional amino acid modification is at a position corresponding to position F2196, M2199, A2201, or S2204 of the amino acid sequence set forth in SEQ ID NO:1.

Also disclosed herein is a pharmaceutical composition comprising a modified Factor VIII polypeptide as described herein and a pharmaceutically acceptable excipient.

Also disclosed herein is a nucleic acid molecule encoding a modified Factor VIII polypeptide disclosed herein. Also disclosed herein is a recombinant expression vector comprising a nucleic acid molecule disclosed herein. Also disclosed herein is a host cell transformed with the recombinant expression vector.

Also disclosed herein is a method of making a modified Factor VIII polypeptide disclosed herin, comprising: providing a host cell comprising a nucleic acid sequence that encodes the modified Factor VIII polypeptide; and maintaining the host cell under conditions in which the modified Factor VIII polypeptide is expressed.

Also disclosed herein is a method for reducing or preventing a condition associated with an immune response to Factor VIII, comprising administering to a subject in need thereof an effective amount of a modified Factor VIII polypeptide disclosed herein.

In some embodiments, the condition is the formation of an inhibitor antibody against Factor VIII. In some embodiments, the immune response is an initial immune response. In some embodiments, the subject is a naïve subject (e.g., not previously infused with FVIII). In some embodiments, the subject has been infused with Factor VIII but has not developed an inhibitor antibody response requiring additional treatment for bleeding in addition to or instead of Factor VIII infusions.

Also disclosed herein is a method for treating or reducing a condition associated with an immune response to Factor VIII, comprising administering to a subject in need thereof an effective amount of the modified Factor VIII polypeptide disclosed herein.

In some embodiments, the condition is the presence of an inhibitor antibody against Factor VIII. In some embodiments, the condition is the presence of a pre-formed inhibitor antibody against Factor VIII. In some embodiments, the method reduces the intensity of the condition.

Aspects of the invention may be provided in the following numbered clauses:
1. A modified Factor VIII polypeptide comprising at least one amino acid modification in an unmodified Factor VIII polypeptide, wherein the at least one amino acid modification is at a position corresponding to positions 2173-2332 of the C2 domain of the amino acid sequence set forth in SEQ ID NO: 1, and wherein the at least one amino acid modification is at a position corresponding to position 2220, 2196, 2198, 2199, 2200, 2215, 2273, 2231, or 2272 of the amino acid sequence set forth in SEQ ID NO: 1.
2. The modified Factor VIII polypeptide of clause 1, wherein the at least one additional amino acid modification is at a position corresponding to position 2220, 2196, 2198, 2199, 2200, or 2215 of the amino acid sequence set forth in SEQ ID NO: 1.
3. The modified Factor VIII polypeptide of clause 1, wherein the at least one additional amino acid modification is an amino acid substitution at a position corresponding to position 2220, 2196, 2198, 2199, 2200, or 2215 of the amino acid sequence set forth in SEQ ID NO: 1, selected from the group consisting of R2220A, R2220Q, F2196A, N2198A, M2199A, L2200A, and R2215A.
4. The modified Factor VIII polypeptide of clause 1, wherein the at least one additional amino acid modification is an amino acid substitution at a position corresponding to position 2220, 2196, 2198, 2199, 2200, or 2215 of the amino acid sequence set forth in SEQ ID NO: 1, selected from the group consisting of F2196R, N2198R, M2199R, L2200R, and R2215R.
5. The modified Factor VIII polypeptide of clause 1, wherein the at least one additional amino acid modification is at a position corresponding to position 2220 of the amino acid sequence set forth in SEQ ID NO: 1.
6. The modified Factor VIII polypeptide of clause 1, wherein the at least one additional amino acid modification is at a position corresponding to position 2196 of the amino acid sequence set forth in SEQ ID NO: 1.
7. The modified Factor VIII polypeptide of clause 1, wherein the at least one additional amino acid modification is at a position corresponding to position 2198 of the amino acid sequence set forth in SEQ ID NO: 1.
8. The modified Factor VIII polypeptide of clause 1, wherein the at least one additional amino acid modification is at a position corresponding to position 2199 of the amino acid sequence set forth in SEQ ID NO: 1.
9. The modified Factor VIII polypeptide of clause 1, wherein the at least one additional amino acid modification is at a position corresponding to position 2200 of the amino acid sequence set forth in SEQ ID NO: 1.
10. The modified Factor VIII polypeptide of clause 1, wherein the at least one additional amino acid modification is at a position corresponding to position 2215 of the amino acid sequence set forth in SEQ ID NO: 1.
11. The modified Factor VIII polypeptide of clause 1, wherein the at least one additional amino acid modification is at a position corresponding to position 2273 of the amino acid sequence set forth in SEQ ID NO: 1.
12. The modified Factor VIII polypeptide of clause 1, wherein the at least one additional amino acid modification is at a position corresponding to position 2272 of the amino acid sequence set forth in SEQ ID NO: 1.
13. The modified Factor VIII polypeptide of clause 1, wherein the at least one additional amino acid modification is at a position corresponding to position 2231 of the amino acid sequence set forth in SEQ ID NO: 1.
14. The modified Factor VIII polypeptide of clause 1, wherein the at least one amino acid modification is an amino acid deletion.
15. The modified Factor VIII polypeptide of clause 1, wherein the at least one amino acid modification is an amino acid addition.
16. The modified Factor VIII polypeptide of clause 1, wherein the at least one amino acid modification is an amino acid substitution.
17. The modified Factor VIII polypeptide of clause 1, wherein the at least one amino acid modification is a covalent chemical modification.
18. The modified Factor VIII polypeptide of clause 1, wherein the at least one amino acid modification is a modification in a B cell epitope.
19. The modified Factor VIII polypeptide of clause 1, wherein the modified Factor VIII polypeptide retains an activity of the unmodified Factor VIII polypeptide.
20. The modified Factor VIII polypeptide of clause 1, wherein the modified Factor VIII polypeptide exhibits reduced immunogenicity/antigenicity upon administration to a subject compared to the unmodified Factor VIII polypeptide.
21. The modified Factor VIII polypeptide of clause 1, wherein the unmodified Factor VIII polypeptide comprises an amino acid sequence that has 40%, 50%, 60%>, 70%>, 80%>, 90%, 91%, 92%, 93%, 94%, 95%, 96%, 97%, 98%, or 99% sequence identity to the amino acid sequence set forth in SEQ ID NO: 1, excluding amino acid modification(s).
22. The modified Factor VIII polypeptide of clause 1, wherein the modified Factor VIII polypeptide is a human polypeptide.
23. The modified Factor VIII polypeptide of clause 1, wherein the modified Factor VIII polypeptide is a non-human polypeptide.
24. The modified Factor VIII polypeptide of clause 1, wherein the modified Factor VIII polypeptide comprises at least 2, 3, 4, 5, 6, 7, 8, 9, 10, 11, 12, 13, 14, 15, 16, 17, 18, 19, or 20 amino acid modifications.
25. The modified Factor VIII polypeptide of clause 1, wherein the modified Factor VIII polypeptide comprises 6 amino acid modifications.
26. The modified Factor VIII polypeptide of clause 1, wherein the modified Factor VIII polypeptide comprises a single amino acid modification.
27. The modified Factor VIII polypeptide of clause 1, wherein the modified Factor VIII polypeptide further comprises at least one additional amino acid modification.
28. The modified Factor VIII polypeptide of clause 27, wherein the at least one additional amino acid modification is a modification in a T cell epitope.
29. The modified Factor VIII polypeptide of clause 27, wherein the at least one additional amino acid modification is at a position corresponding to positions 2173-2332 of the C2 domain of the amino acid sequence set forth in SEQ ID NO: 1 or positions 373-740 of the A2 domain of the amino acid sequence set forth in SEQ ID NO:1.
30. The modified Factor VIII polypeptide of clause 27, wherein the at least one additional amino acid modification is at a position corresponding to positions 2194-2213 of the amino acid sequence set forth in SEQ ID NO: 1.
31. The modified Factor VIII polypeptide of clause 27, wherein the at least one additional amino acid modification is at a position corresponding to positions 2202-2221 of the amino acid sequence set forth in SEQ ID NO: 1.
32. The modified Factor VIII polypeptide of clause 27, wherein the at least one additional amino acid modification is at a position corresponding to positions 2194-2205 of the amino acid sequence set forth in SEQ ID NO: 1.
33. The modified Factor VIII polypeptide of clause 27, wherein the at least one additional amino acid modification is at a position corresponding to positions 2196-2204 of the amino acid sequence set forth in SEQ ID NO: 1.
34. The modified Factor VIII polypeptide of clause 27, wherein the at least one additional amino acid modification is at a position corresponding to position F2196, M2199, A2201, or S2204 of the amino acid sequence set forth in SEQ ID NO: 1.
35. A pharmaceutical composition comprising a modified Factor VIII polypeptide according to any one of clauses 1-34, and a pharmaceutically acceptable excipient.
36. A nucleic acid molecule encoding the modified Factor VIII polypeptide according to any one of clauses 1-34.
37. A recombinant expression vector comprising a nucleic acid molecule according to clause 36.
38. A host cell transformed with the recombinant expression vector according to clause 37.
39. A method of making the modified Factor VIII polypeptide of clause 1, comprising: providing a host cell comprising a nucleic acid sequence that encodes the modified Factor VIII polypeptide; and maintaining the host cell under conditions in which the modified Factor VIII polypeptide is expressed.
40. A method for reducing or preventing a condition associated with an immune response to Factor VIII, comprising administering to a subject in need thereof an effective amount of the modified Factor VIII polypeptide of clause 1.
41. The method of clause 40, wherein the condition is the formation of an inhibitor antibody against Factor VIII.
42. The method of clause 40, wherein the immune response is an initial immune response.
43. The method of clause 40, wherein the subject is a naive subject.
44. The method of clause 40, wherein the subject has been infused with Factor VIII but has not developed an inhibitor antibody response requiring additional treatment for bleeding in addition to or instead of Factor VIII infusions.
45. A method for treating or reducing a condition associated with an immune response to Factor VIII, comprising administering to a subject in need thereof an effective amount of the modified Factor VIII polypeptide of clause 1.
46. The method of clause 45, wherein the condition is the presence of an inhibitor antibody against Factor VIII.
47. The method of clause 45, wherein the condition is the presence of a pre-formed inhibitor antibody against Factor VIII.
48. The method of clause 45, wherein the method reduces the intensity of the condition.

### BRIEF DESCRIPTION OF THE SEVERAL VIEWS OF THE DRAWINGS

These and other features, aspects, and advantages of the present invention will become better understood with regard to the following description, and accompanying drawings, where:

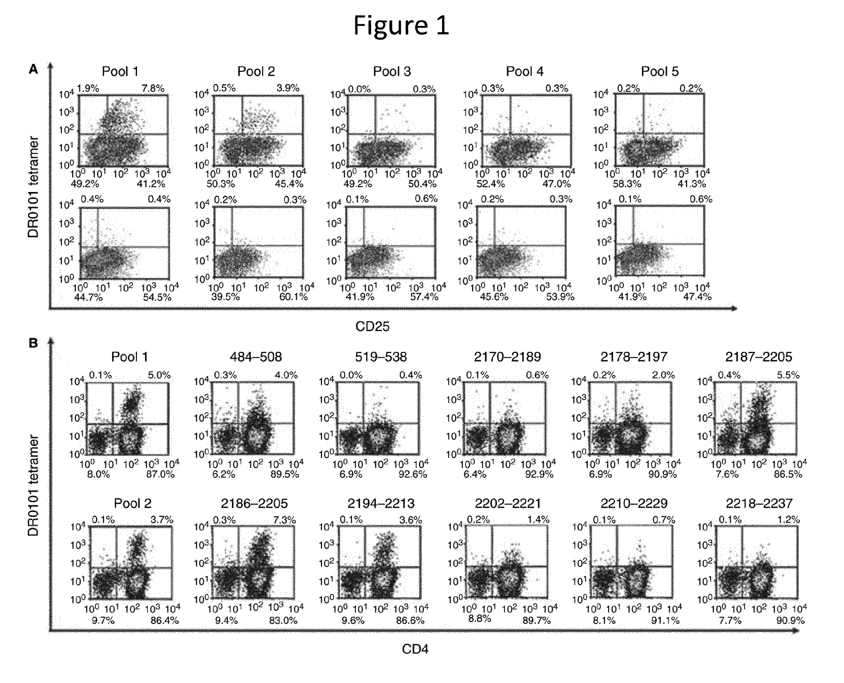

**Figure 1****.** T-cell epitope mapping. (A) Tetramer staining of peptide-stimulated CD4 T cells obtained at 19 weeks following initial inhibitor detection in mild HA inhibitor subject 17 A (upper) and from an HLA-matched non-HA control (lower). Equivalent results were observed when staining with tetramers and fluorescent anti-CD4 rather than anti-CD25 antibody (not shown). (B) Decoding with individual peptide-loaded tetramers. All results were confirmed by subsequent staining.

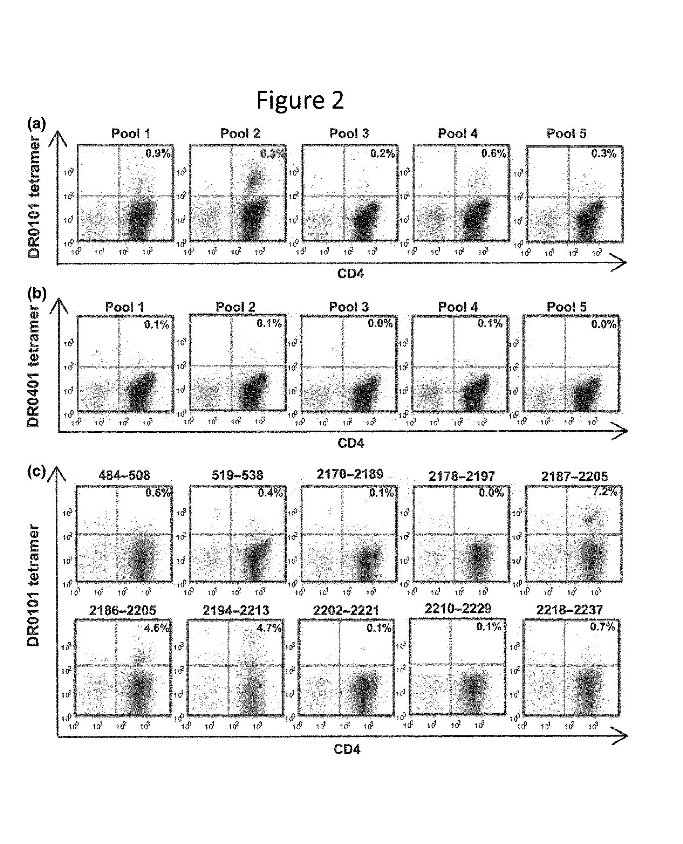

**Figure 2****.** These assays utilized CD4+ T cells from a mild HA subject (brother of subject 17A) who did not have a clinically significant inhibitor, but whose T cells were stained by tetramers loaded with the same peptide recognized by T cells from his brother. CD4+ T cells were stimulated with pooled 20-mer overlapping peptides spanning the FVIII C2 domain sequence. 18 days later, the cells were incubated with phycoerythrin (PE)-labeled DR0101 tetramers loaded with FVIII C2 peptide pools (a) and antibodies. Decoding of positive CD4+ responses to DR0101 tetramers loaded with peptide pools 1 and 2 was carried out w22 days after stimulation of total CD4+ cells (top row) or CD4+CD25+-depleted CD4+ cells (bottom row) respectively. (c). Decoding of DR0101-restricted responses to peptide pool 1 using tetramers loaded with individual peptides comprising pool 1 is shown in the top row. Decoding of DR0101-restricted responses to peptide pool 2 is shown in the bottom row.

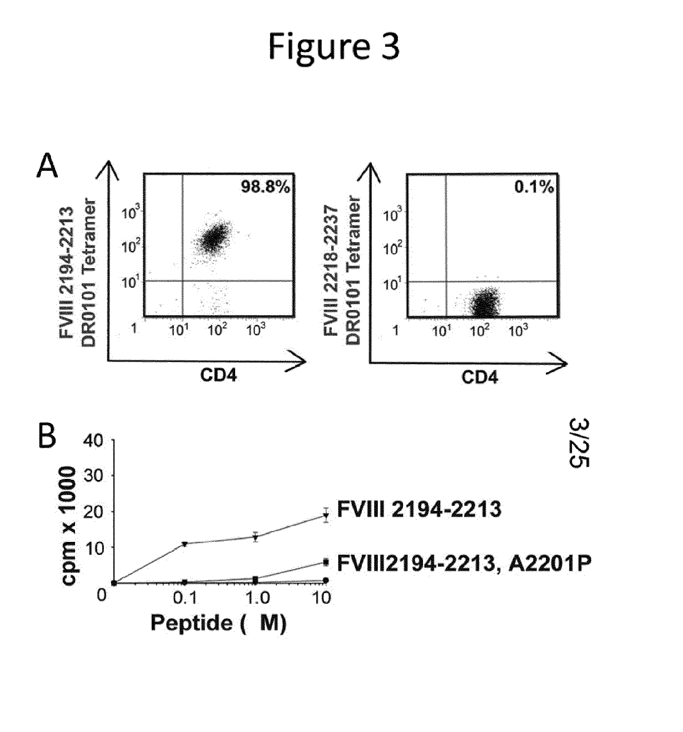

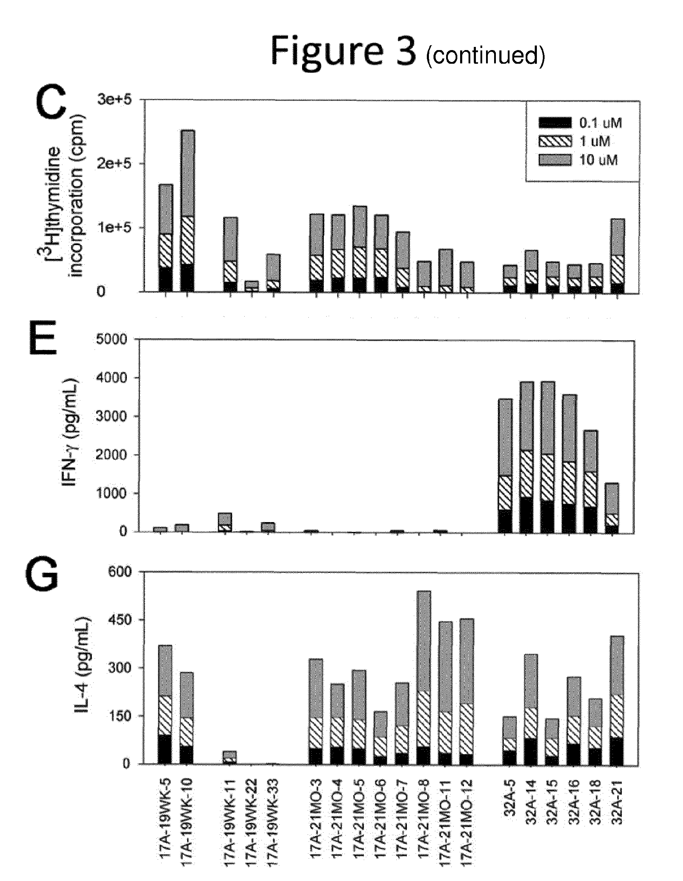

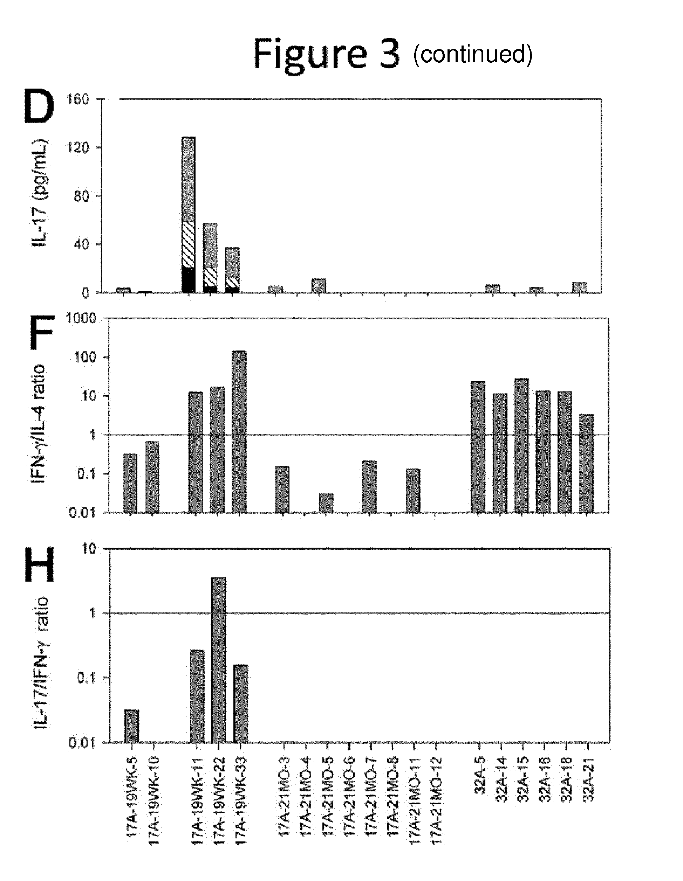

**Figure 3****.** Proliferation and cytokine secretion of T-cell clones isolated from subjects 17A 19 weeks and 21 months after inhibitor development and from 32A at one time point. (A) Left panel: Expanded FVIII-specific T-cell clones stain positive for CD4+ and for the relevant MHC Class II tetramers loaded with the correct FVIII-derived peptide. Right panel: The same DR0101 tetramer loaded with an irrelevant peptide as a control is not recognized by the same subject's T cells. (B) This representative clone proliferates strongly in response to the wild-type peptide (corresponding to infused FVIII) but not in response to a peptide with the hemophilic sequence corresponding to the subject's endogenous FVIII protein. (C) Resting T-cell clones were stimulated with FVIII 2194-2213 (0.1, 1.0 and 10 µM) presented on irradiated PBMCs from an unrelated DRB1*-0101 donor. Proliferation was measured by addition of 3H-thymidine at 48 hours, and cells were harvested 18 hours later. Cell supernatants were collected after 48 hours to measure IL-17 (D), interferon-gamma (E), IL-4 (G), by ELISA. The baseline proliferation and cytokine levels of cells stimulated with buffer as a negative control was subtracted. Results are presented as stacked bar graphs (C-H). The total amounts of cytokines secreted after stimulation with FVIII 2194-2213 at the 3 concentrations indicated in panel C were summed, and ratios of these total levels were calculated. In all panels, clones are grouped according to their cytokine secretion profiles.

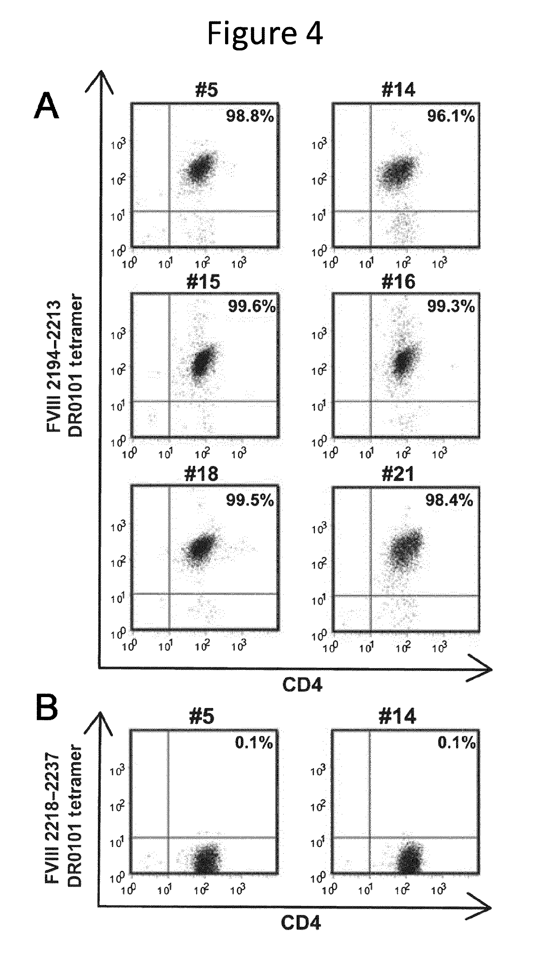

**Figure 4****.** These assays utilized CD4+ T cells from a mild HA subject (32A) who did not have a clinically significant inhibitor, but whose T cells were nevertheless stained by tetramers loaded with the same peptide recognized by T cells from his brother, subject 17A. (a) Tetramer staining of T-cell clones: T-cell clones 5, 14, 15, 16, 18 and 21 were stimulated with HLA-mismatched PBMCs and PHA. 14 days later, the clones were incubated with PE-labeled DR0101 tetramers loaded with peptide FVIII 2194-2213 and FITC-labeled anti-human CD4 IgG. (b) Two of the clones were incubated with DR0101 tetramers loaded with an irrelevant peptide (FVIII 2218-2237) as a negative control.

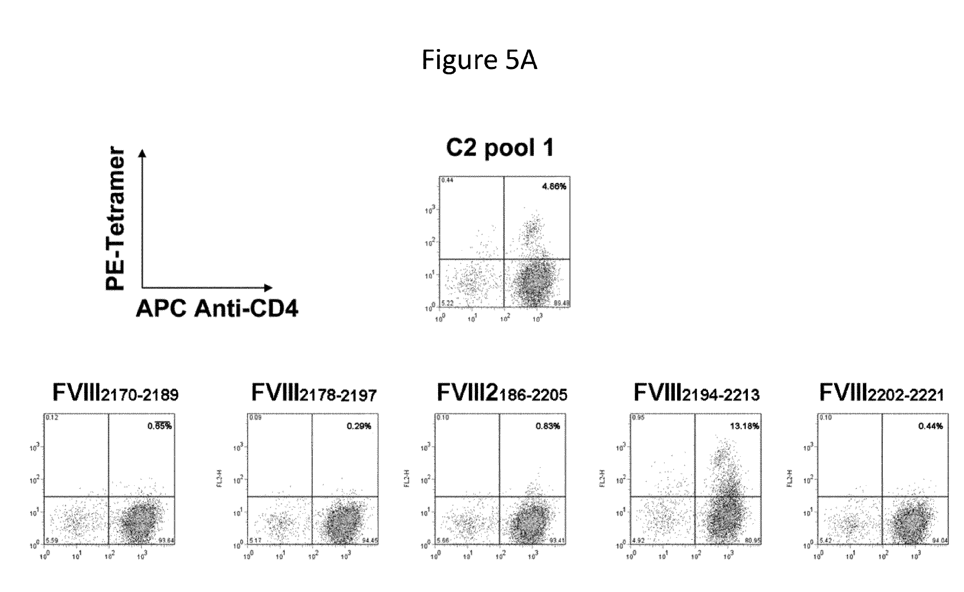

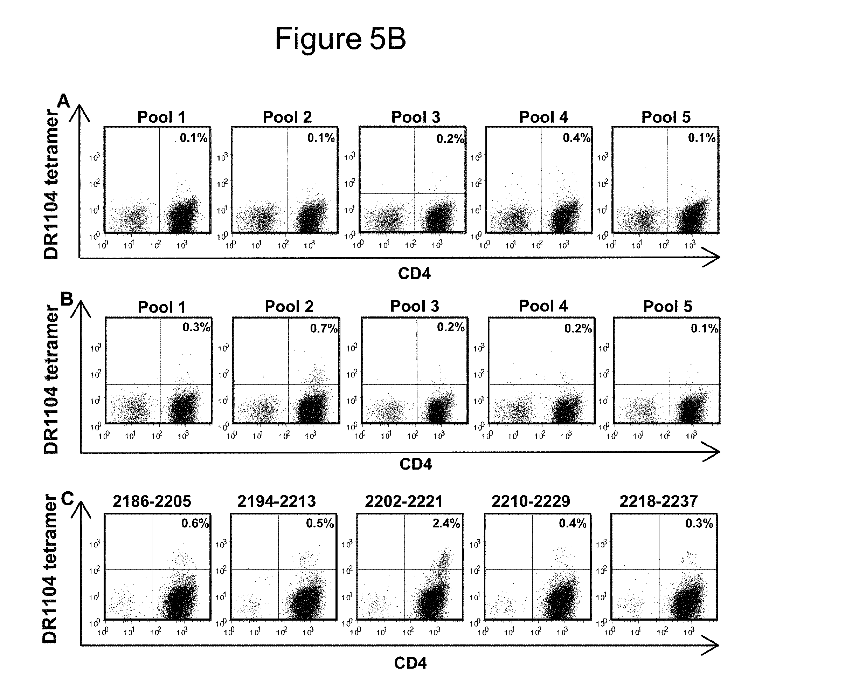

**Figure 5****.** Tetramer staining of CD4+ T cells was carried out for a severe HA inhibitor subject (subject 56A) who was DRB1*0101, following a protocol similar to that described for Figures 1 and 2. Staining results showed an HLA-DRB1*0101-restricted response to the same region recognized by T cells from the mild HA DRB1*0101 subjects, FVIII 2194-2213. Figure 5B is the tetramer staining of the uncle's CD4+ cells.

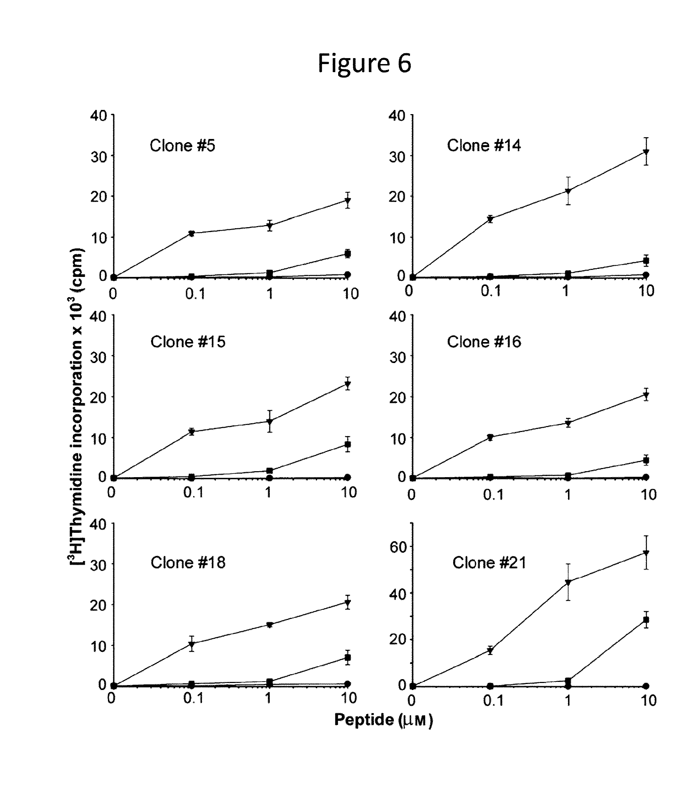

**Figure 6****.** Antigen-specific proliferation of T-cell clones from haemophilia A subject 32A. Resting T-cell clones 5, 14, 15, 16, 18, and 21 were stimulated with PBMCs from a healthy DRB1*0101 donor plus wild-type peptide FVIII 2194-2213 (triangle symbols) or haemophilic peptide FVIII 2194-2213, 2201P (square symbols), or irrelevant peptide FVIII 519-538 (circle symbols) at 0, 0.1, 1.0 and 10 µM final concentration. 3H-thymidine uptake was measured. Data show mean ± SD of triplicate determinations.

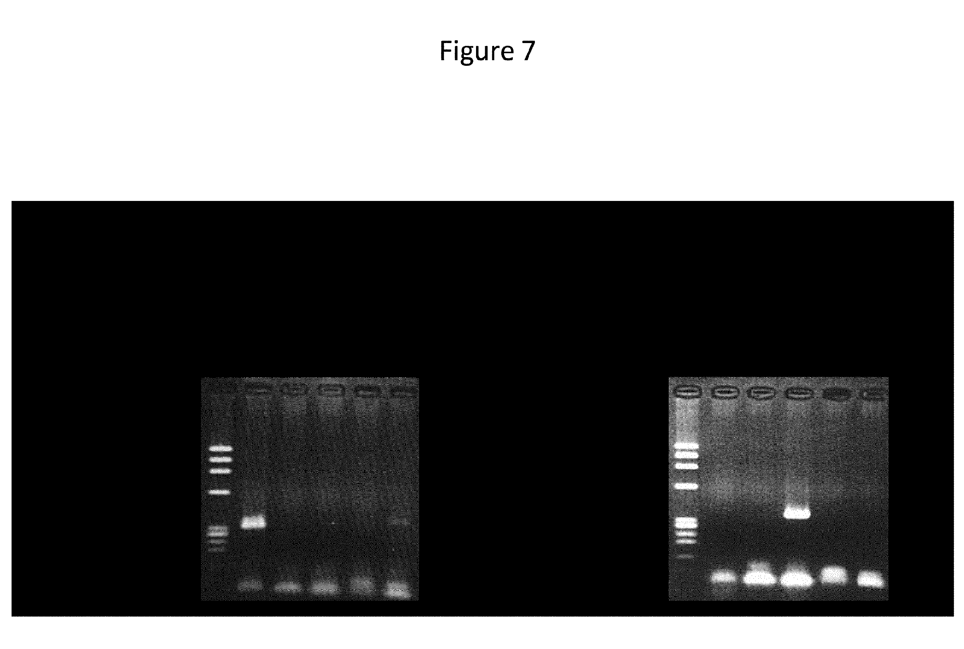

**Figure 7****.** Multiplex PCR was carried out to test whether expanded T-cell clones were actually clonal. Primers sets designed to amplify the human TCRBV region were utilized to carry out PCR reactions that were run on 5 lanes of an agarose gel. Representative results for samples from mild HA subjects 17A and 32A are shown. The single product bands yielded a single DNA sequence in each case (data not shown). The single product band and single sequence confirmed that these were indeed clonal T-cell lines.

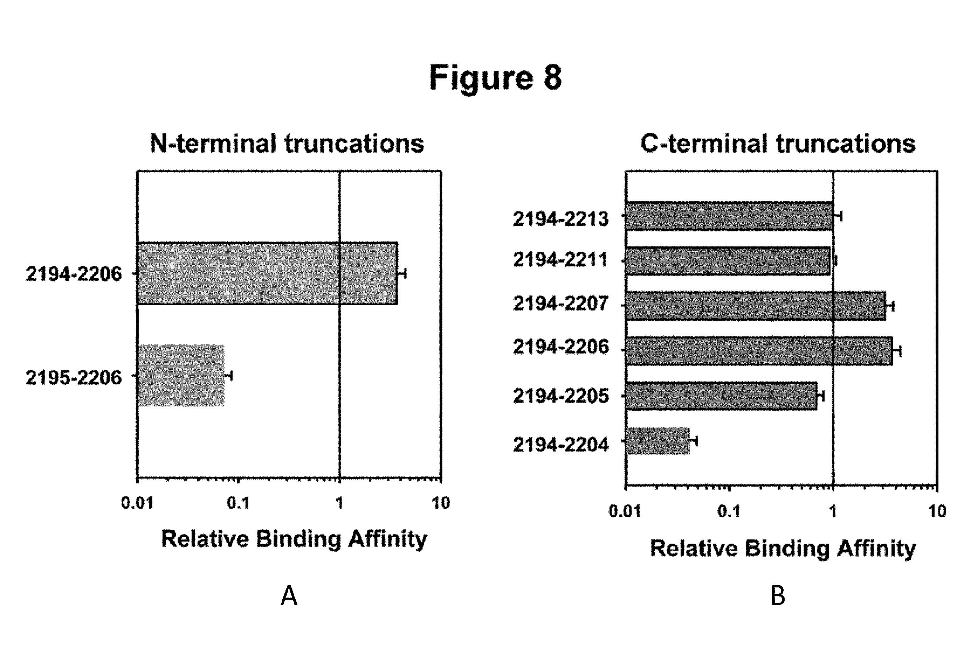

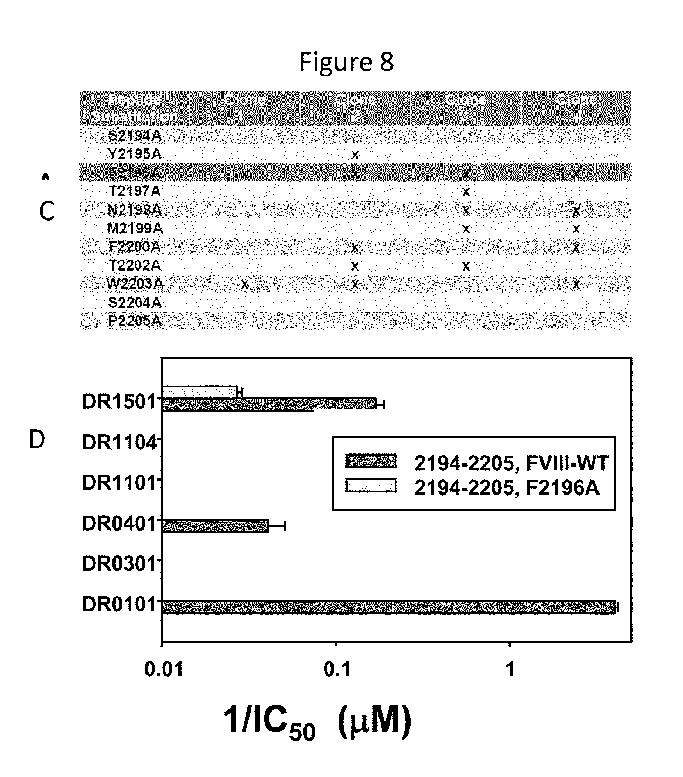

**Figure 8****.** Peptide binding affinities. 20-mer and truncated FVIII peptides and negative control peptide OspA 163-175, 165A were incubated in competition with HA 306-318 peptide for binding to DR0101. Residual bound HA 306-318 was detected by fluorescence of europium-labeled streptavidin. Removal of residue 2194 at the N-terminus or 2205 at the C-terminus significantly reduced the binding affinity for recombinant DR0101, indicating that the minimal DRAB1*0101-restricted epitope includes FVIII residues 2194-2205.

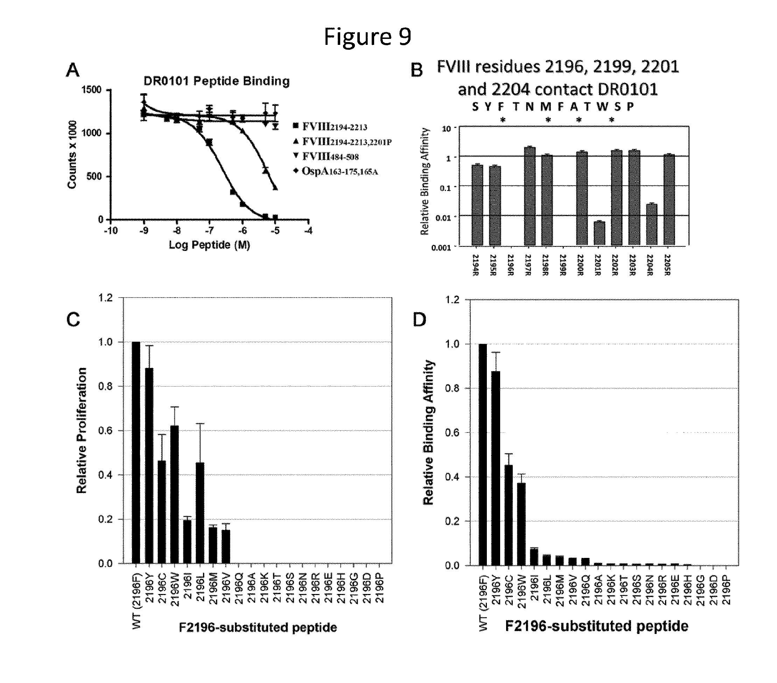

**Figure 9****.** (A). Peptide binding affinities. FVIII peptides and negative control peptide OspA 163-175, 165A were incubated in competition with HA 306-318 peptide for binding to DR0101. Residual bound HA 306-318 was detected by fluorescence of europium-labeled streptavidin. Standard errors of triplicate measurements are indicated (James EA et al., J Thromb Haemostas 5:2399-2402, 2007 Suppl. Figure) (B) Binding of synthetic FVIII peptides with systematic single arginine substitutions to recombinant DR0101 1 protein was measured using the same protocol. Reduced affinity at positions 2196, 2199, 2201, and 2204 indicate that wild-type residues at these positions confer significant binding affinity for this FVIII region to DR0101. (C) Systematic substitution of every possible naturally occurring amino acid at position 2196 was evaluated for effect on proliferation, relative to the wild-type peptide (relative proliferation = ratio of mutant/native proliferation in a 3H-thymidine incorporation assay as described above.). (D) Systematic substitution of every possible naturally occurring amino acid at position 2196 was evaluated for effect on binding affinity for DR0101, using the assay described in (A). Relative binding affinity compared to the wild-type peptide is indicated.

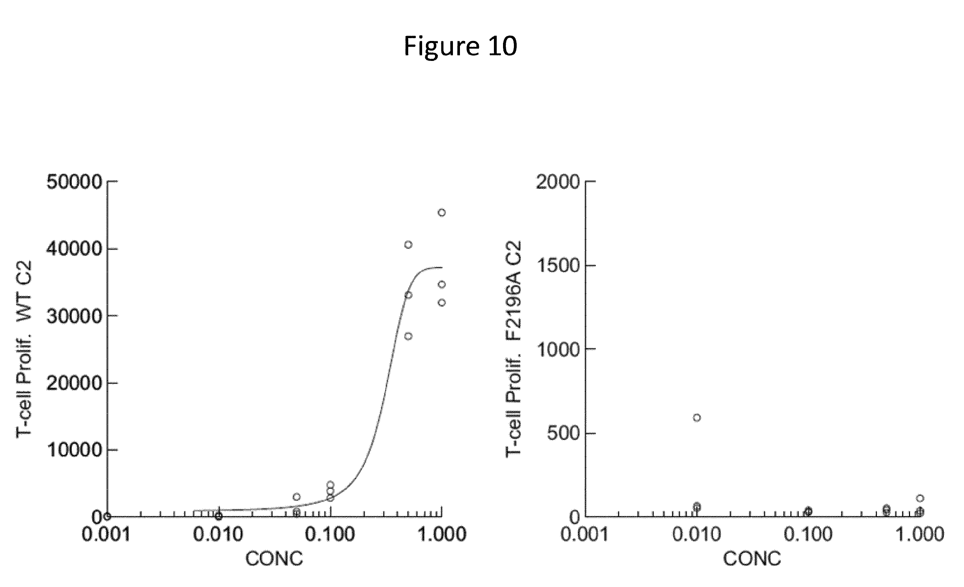

**Figure 10****.** Recombinant FVIII C2 proteins with wild-type sequence, as well as with the substitution F2196A, were generated in an *E*. *coli* system and purified using a protocol to remove endotoxin then filter-sterilized. T-cell clones from subject 17A were then stimulated with both wild-type and mutant protein. Representative results are shown. The clones did not proliferate significantly above background when stimulated with the F2196A protein, indicating that this epitope modification reduced the immunogenicity of the FVIII-C2 protein.

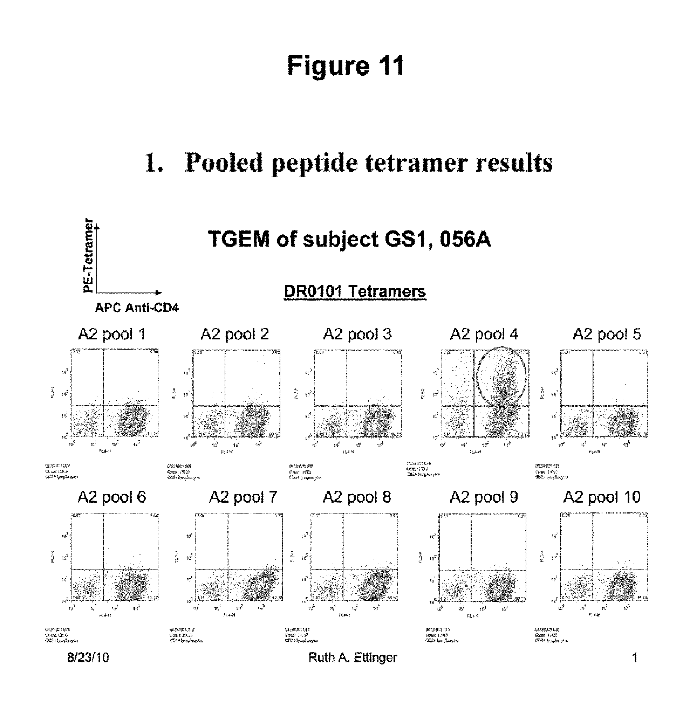

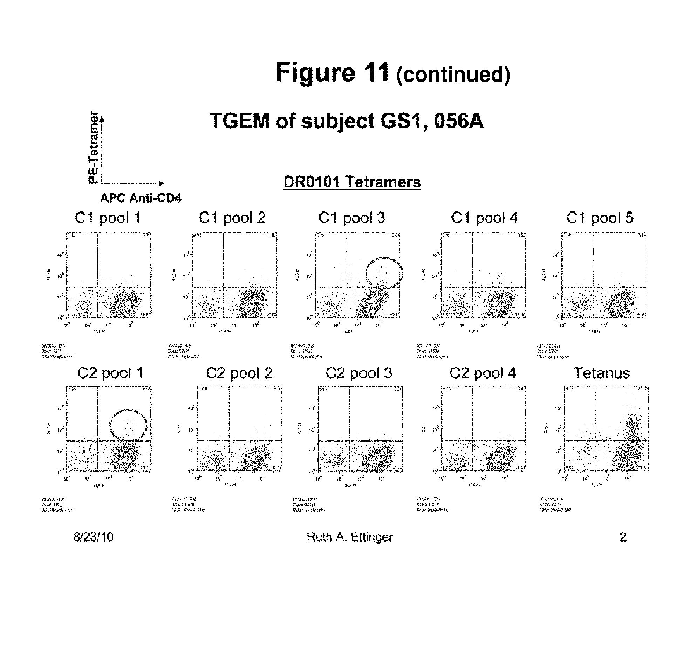

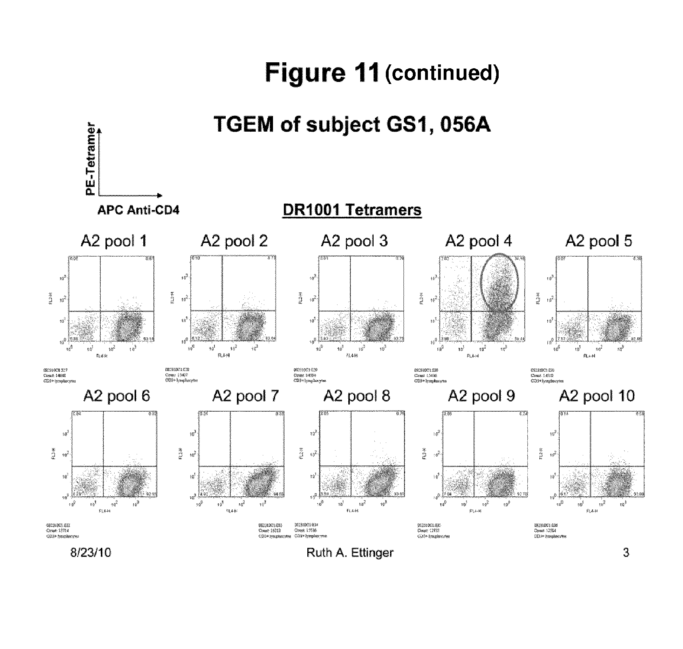

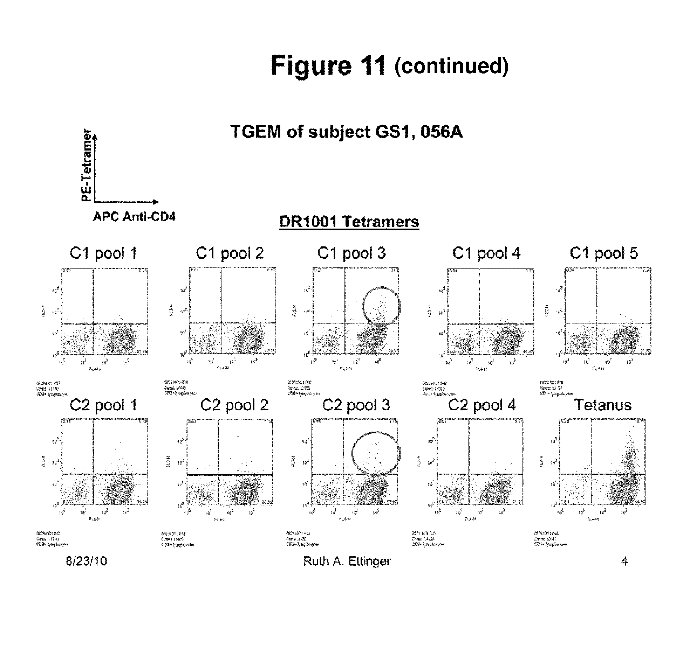

**Figure 11****.** Tetramer staining of CD4+ T cells from severe HA inhibitor subject 56A, who is DR0101, 1001, with pooled peptides.

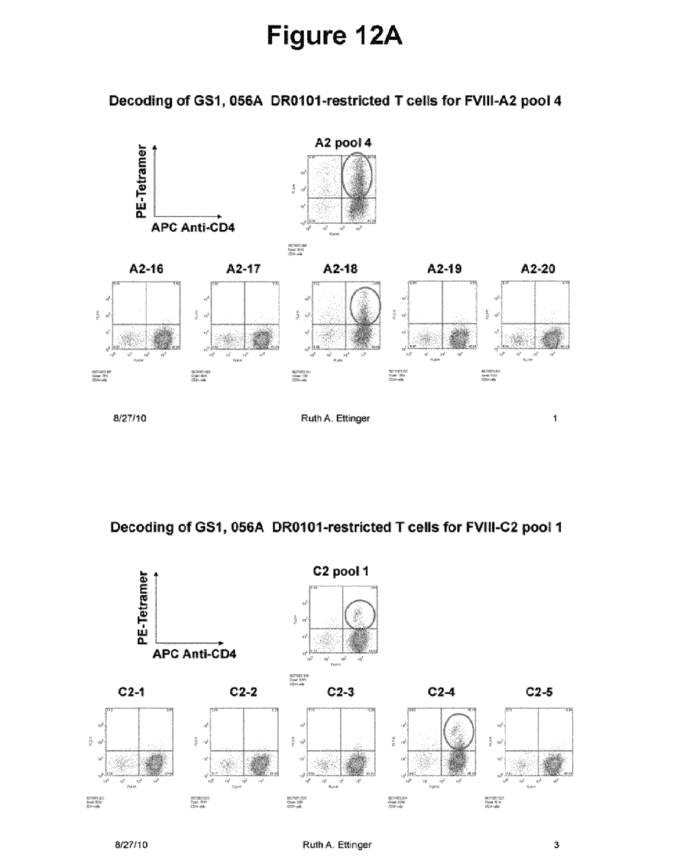

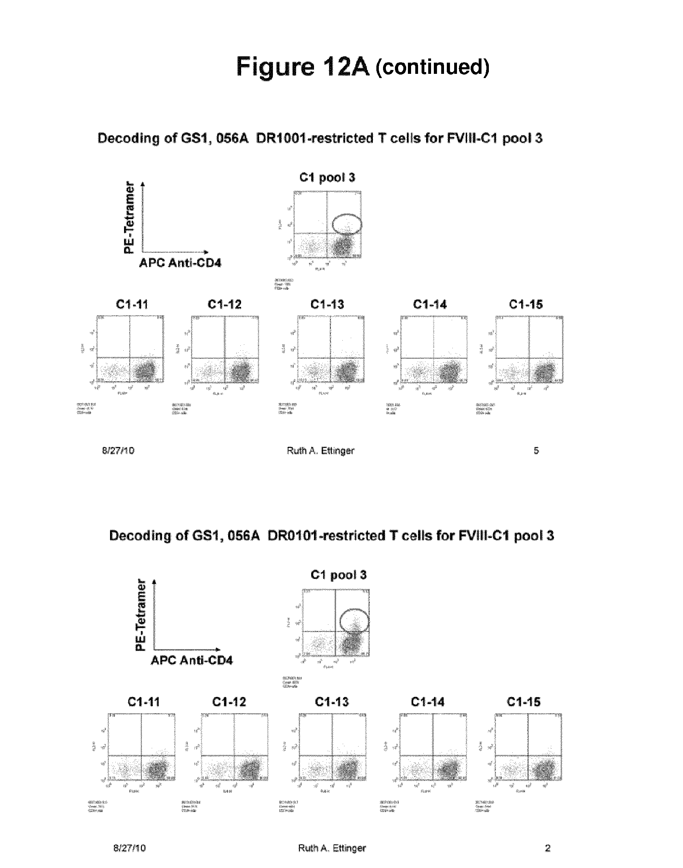

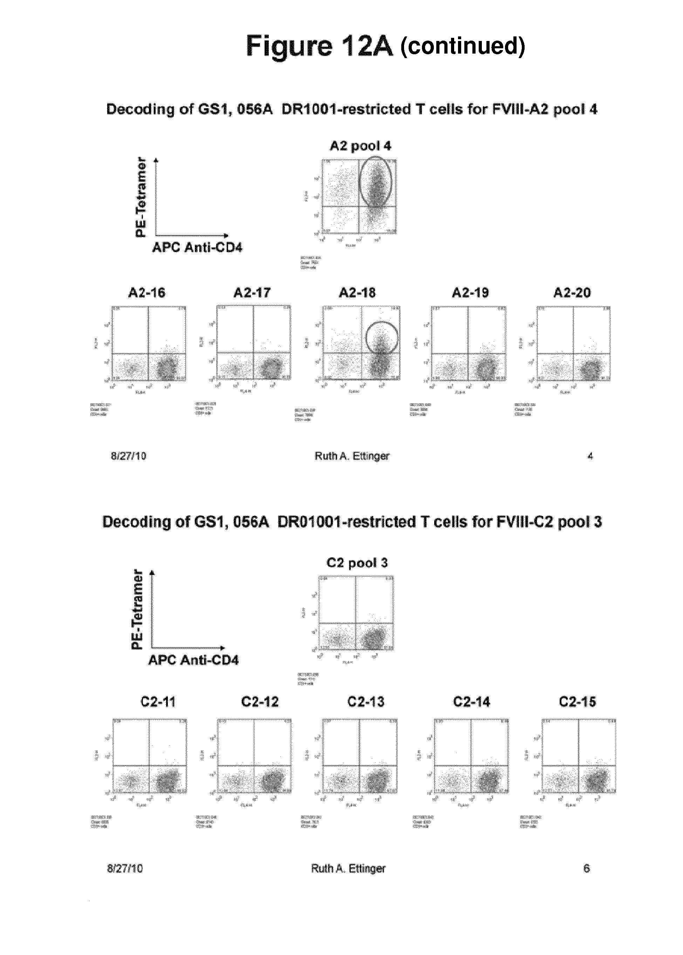

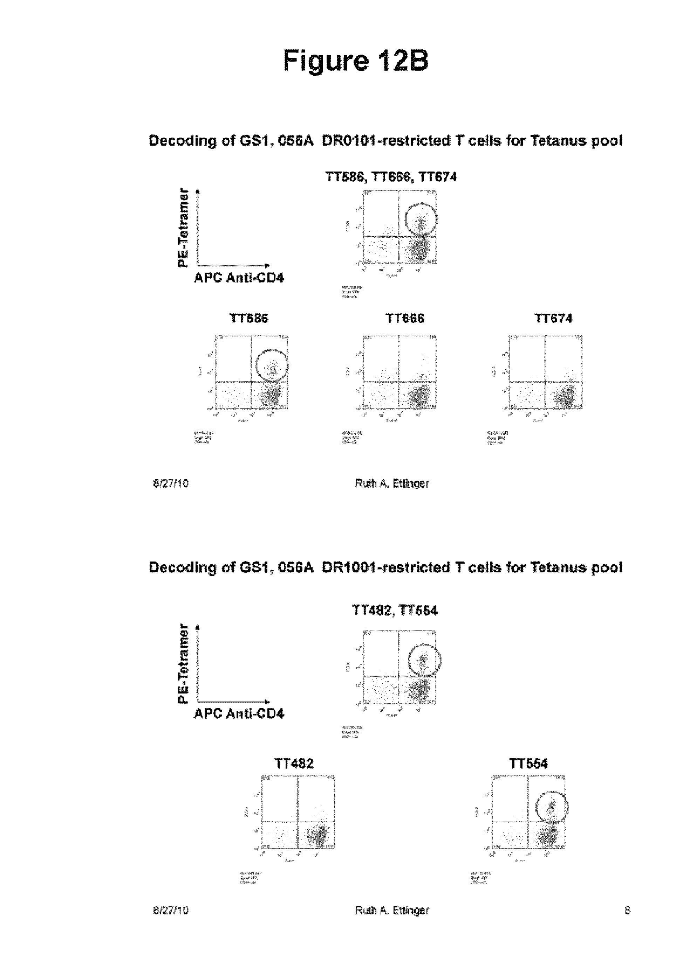

**Figure 12****.** (A) Tetramer staining of CD4+ T cells from severe HA inhibitor subject 56A, who is DR0101, 1001, with individual peptides comprising the peptides pools in Figure 11. (B) Tetanus toxoid peptide staining provides a positive control for staining of DR0101-and DR1001- restricted T-cell responses.

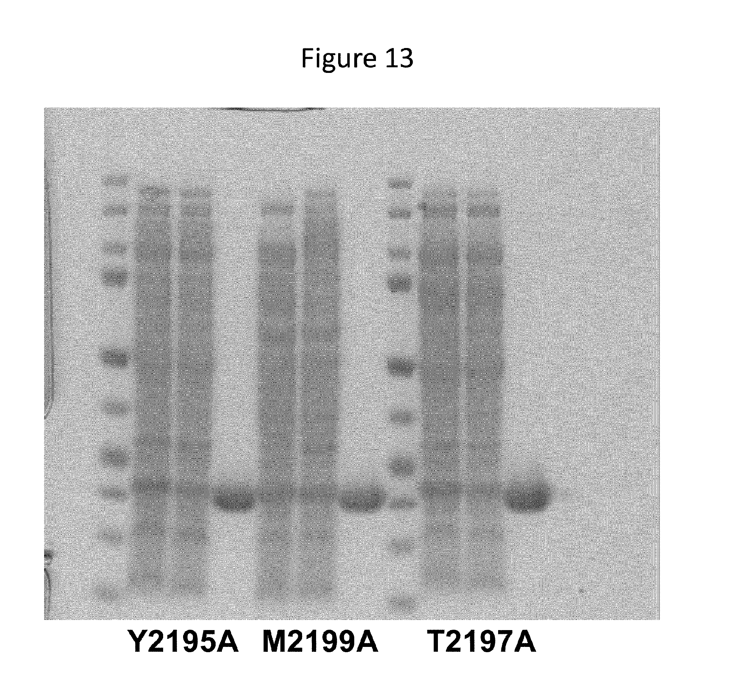

**Figure 13****.** Representative SDS-PAGE gels (15%) showing purification of FVIII-C2 mutant proteins from an *E*. *coli* expression system. The final preparations are free of endotoxin and sterile, so are appropriate for SPRn and for cell stimulation assays.

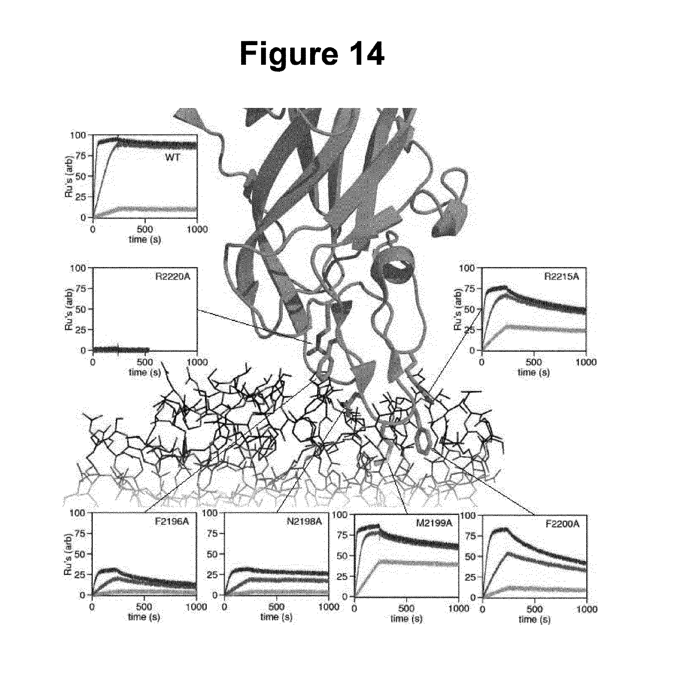

**Figure 14****.** The crystal structure of the FVIII-C2 complex with the BO2C11 Fab is shown with FVIII-C2 in ribbon representation and with relevant side chains shown explicitly, while the BO2C11 surface is shown in stick representation. Sensorgrams corresponding to mutant proteins that bound BO2C11 with affinities fourfold or more lower than that of WT-C2 are shown; black lines map each sensorgram to the relevant wild-type FVIII residue. The sensorgrams record the mass of the C2 protein that becomes attached to the Fab-coated chip. The signals are measured in Resonance Units (RUs), which are in arbitrary units.

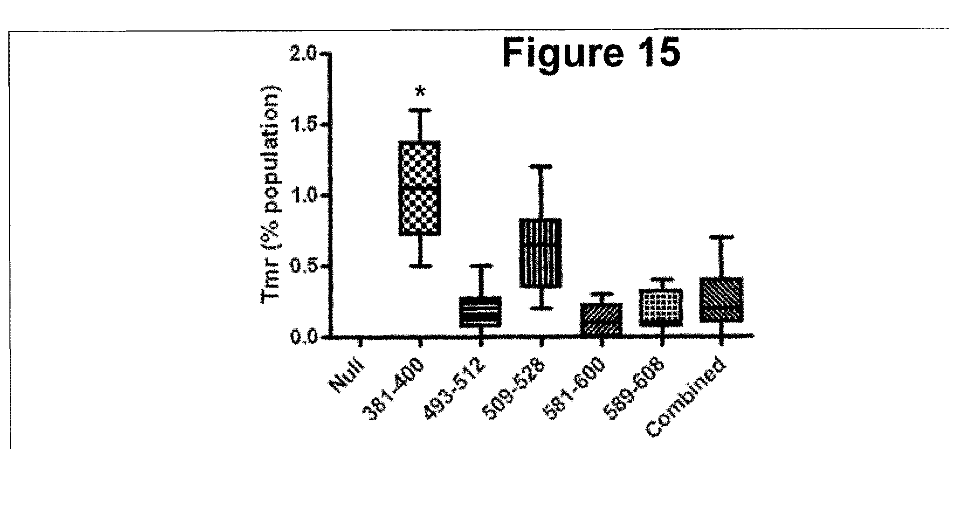

**Figure 15****.** Example of calculating significance of cutoffs used to designate positive staining by tetramers (significantly above background staining). To define an objective criterion for positive tetramer staining, CD4+ T cells from six non-hemophilic DR1101 donors were "sham" stimulated using DMSO for two weeks and subsequently stained using a panel of DR1101 tetramers. One tetramer (FVIII 381-400) gave significantly higher background staining, indicating a peptide-specific effect, while all others had a statistically similar background, allowing calculation of a mean background level. Our criteria for positive staining was designated as the mean background staining plus 3 times the standard error of the mean: 1.53% for FVIII 381-400 and 0.46% for all other specificities

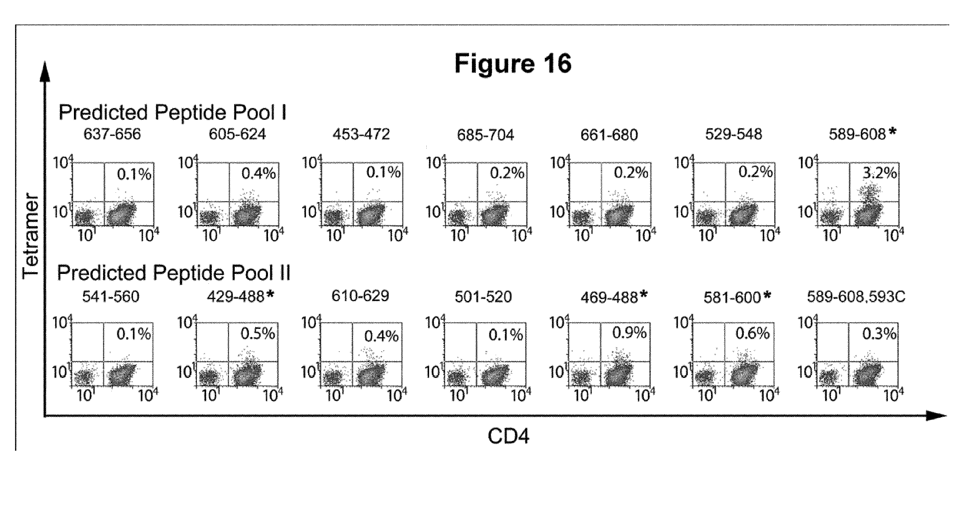

**Figure 16****.** T-cell epitopes recognized by subject 1D. CD4+ cells were stimulated using two pools of seven FVIII peptides each with predicted *HLA-DRB1*1101-*restricted epitopes. Peptides that elicited a tetramer-positive CD4+ population (greater than three times the standard error of the mean above background) are indicated by asterisks. These included FVIII₄₂₉₋₄₄₈, FVIII₄₆₉₋₄₈₈, FVIII₅₈₁₋₆₀₀, and FVIII₅₈₁₋₆₀₀.

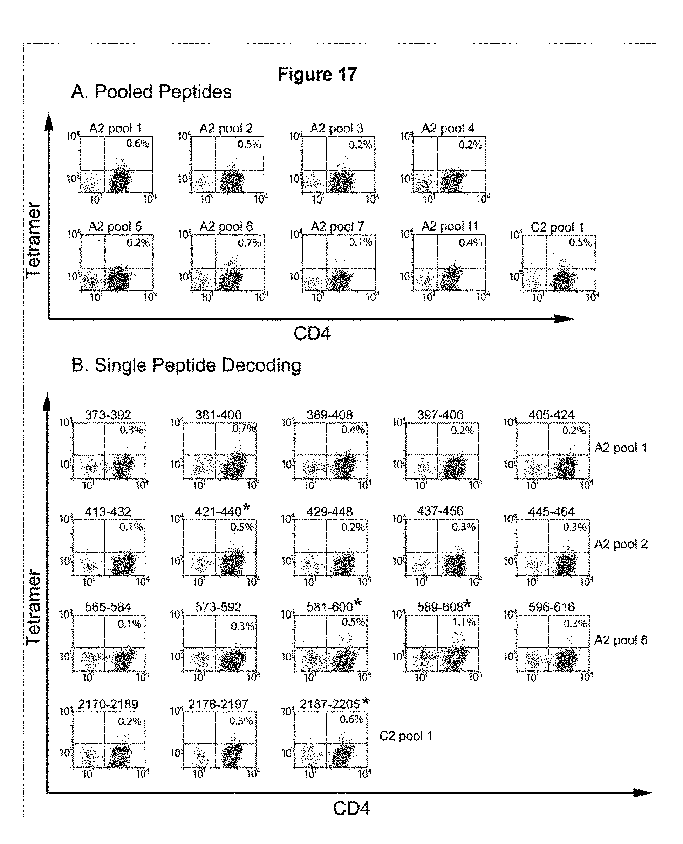

**Figure 17****.** T-cell epitopes recognized by mild HA subject 41A (R593C missense mutation). (A) CD4+ cells were stimulated for two weeks with pooled, overlapping peptides spanning the FVIII A2, C1, and C2 domains. Positive and representative negative tetramer staining results are shown (fluorescent labeling greater than three times the standard error of the mean above background was considered positive). (B) Decoding by staining the same cells with HLA-DR1101 tetramers loaded with individual peptides. Peptides that elicited a tetramer-positive CD4+ population are indicated by asterisks. These included FVIII₄₂₁₋₄₄₀, FVIII₅₈₁₋₆₀₀, FVIII₅₈₁₋₆₀₀ and FVIII₂₁₈₇₋₂₂₀₅ (note that the tetramer loaded with FVIII₃₈₁₋₄₀₀ had an uncharacteristically high background, suggesting possible nonspecific binding to CD4+ cells).

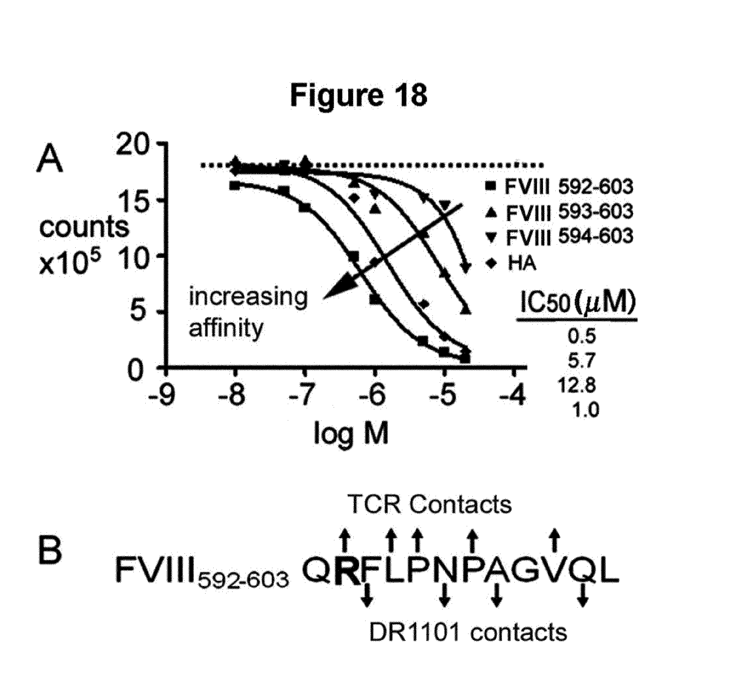

**Figure 18****.** Defining the minimal DR1101-restricted epitope within FVIII₅₈₉₋₆₀₈. (A) *In vitro* binding of truncated peptides FVIII₅₉₂₋₆₀₃, FVIII₅₉₃₋₆₀₃ and FVIII₅₉₄₋₆₀₃ and the influenza HA₃₀₆₋₃₁₈ control to HLA-DR1101 protein (arrow indicates increasing affinity). (B) Schematic of the core HLA-DR1101 binding region within FVIII₅₉₂₋₆₀₃, based on experimental results and the published DR1101 binding motif. Arrows indicate DR1101 contact residues (pointing downward) and possible T-cell receptor contact residues (pointing upward).

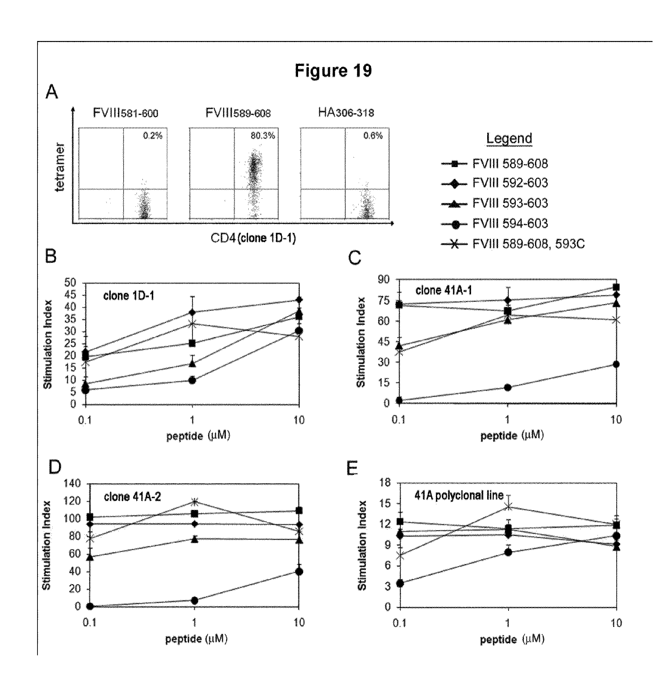

**Figure 19****.** Tetramer staining and proliferation of T-cell clones and a polyclonal T-cell line. (A). Staining of clone 1D-1 using tetramers loaded with FVIII₅₈₁₋₆₀₀, FVIII₅₈₉₋₆₀₈, or the control influenza HA₃₀₆₋₃₁₈ peptide. (B-E) Clones from subject 1D (clone 1D-1, B), subject 41A (clones 41A-1 and 41A-2, C-D) and a polyclonal T-cell line from subject 41A (41A Line, E) were stimulated with FVIII₅₈₉₋₆₀₈, FVIII₅₉₂₋₆₀₃, FVIII₅₉₃₋₆₀₃, FVIII₅₉₄₋₆₀₃, and the hemophilic FVIII_{589-608,593C} peptide at 0, 0.1, 1.0, and 10 µM. [³H]thymidine uptake was measured in triplicate wells. Data are expressed as stimulation index values ± standard deviation (SI ± SD), where SI = measured counts / baseline counts.

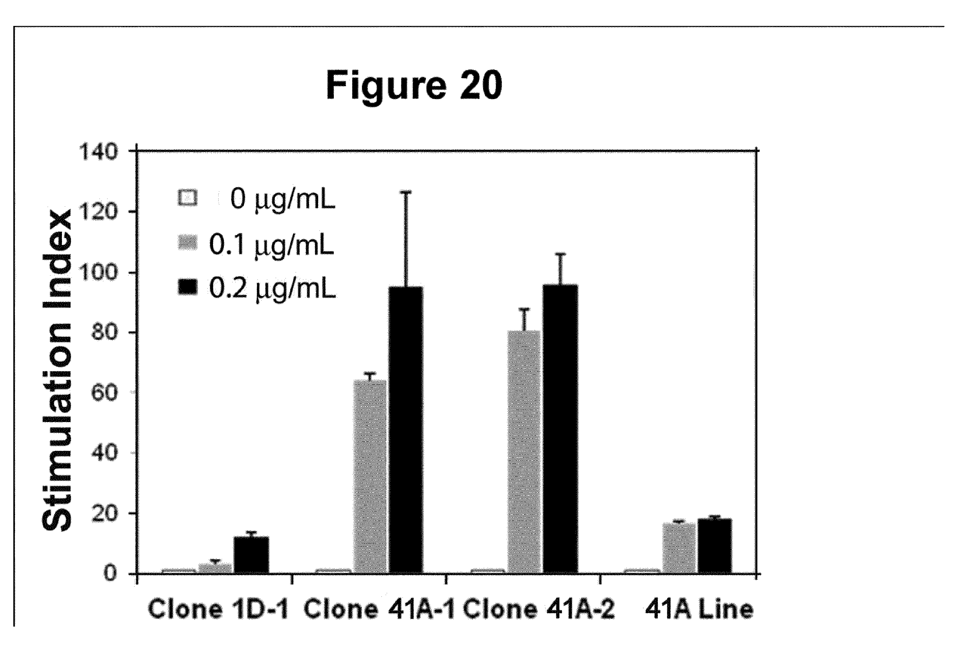

**Figure 20****.** Proliferation of T-cell clones and polyclonal line in response to FVIII. Clones 1D-1, 41A-1 and 41A-2 and a polyclonal T-cell line from subject 41A were stimulated with 0, 0.1, or 0.2 µg/mL of FVIII protein. [³H]thymidine uptake was measured in triplicate wells (data expressed as SI ± SD).

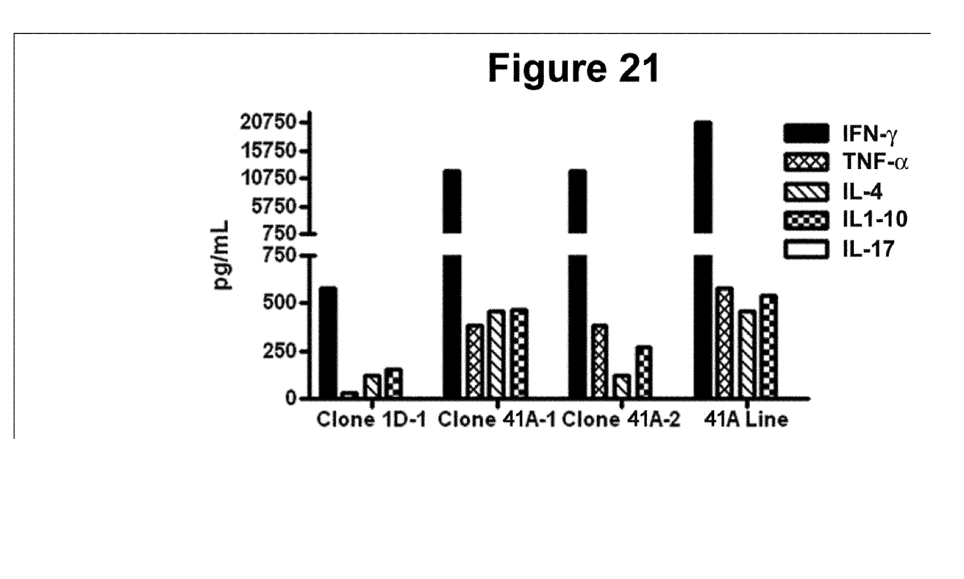

**Figure 21****.** Cytokine secretion by T-cell clones and polyclonal line. Clones from subject 1D and 41A and a polyclonal T-cell line from subject 41A were stimulated with various concentrations of FVIII₅₈₉₋₆₀₈ peptide for 48hr. Supernatants were collected and analyzed by ELISA to quantify interferon-γ, TNF-α , IL-4, IL-10 and IL-17 secretion. Cytokines elicited at peptide concentrations of 10 µg/mL are shown, representing averages from triplicate wells.

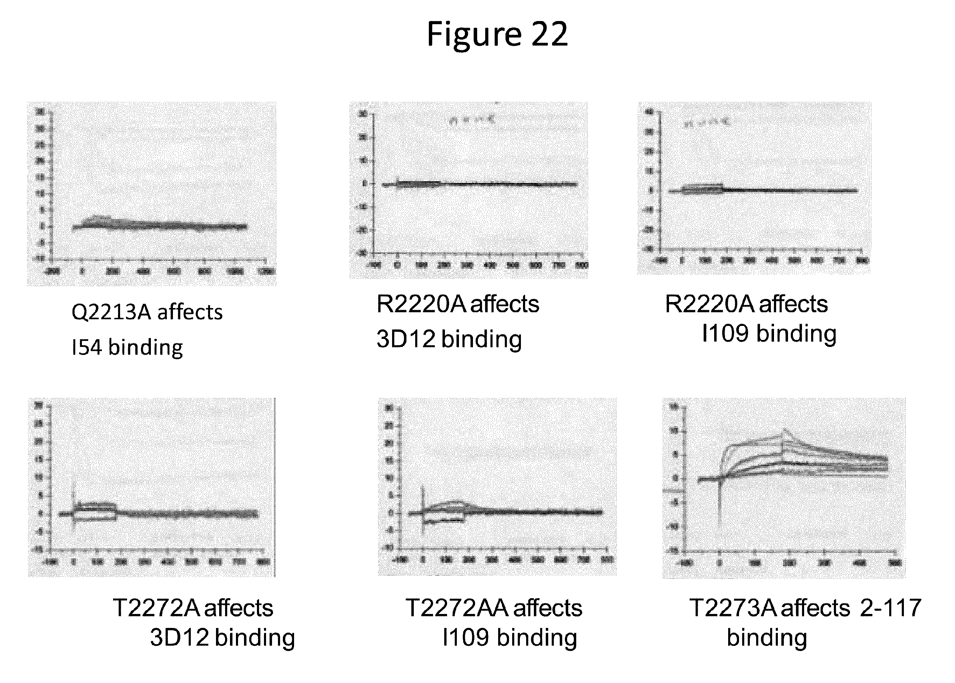

**Figure 22****.** Testing ofB cell Epitope 5 from Table B, shown below.

### DETAILED DESCRIPTION

Terms used in the claims and specification are defined as set forth below unless otherwise specified. In the case of direct conflict with a term used in a parent provisional patent application, the term used in the instant specification shall control.

As used herein, a "Factor VIII" (FVIII) refers to any factor VIII polypeptide or nucleotide, including but not limited to, a recombinantly produced polypeptide, a synthetically produced polypeptide and a factor VIII polypeptide extracted or isolated from cells or tissues including, but not limited to, liver and blood. Factor VIII includes related polypeptides from different species including, but not limited to animals of human and non-human origin. Human factor VIII includes factor VIII, allelic variant isoforms, synthetic molecules from nucleic acids, protein isolated from human tissue and cells, and modified forms thereof. Exemplary unmodified human factor VIII polypeptides include, but are not limited to, unmodified and wild-type native factor VIII polypeptide and the unmodified and wild-type precursor factor VIII polypeptide. The factor VIII polypeptides provided herein can be modified, such as by amino acid addition, amino acid substitution, amino acid deletion, or chemical modification or post-translational modification. Such modifications include, but are not limited to, covalent modifications, pegylation, albumination, glycosylation, farnysylation, carboxylation, hydroxylation, phosphorylation, and other polypeptide modifications known in the art.

Factor VIII includes factor VIII from any species, including human and non-human species. Factor VIII of non-human origin include, but are not limited to, murine, canine, feline, leporine, avian, bovine, ovine, porcine, equine, piscine, ranine, and other primate factor VIII.

Human and non-human factor VIII polypeptides include factor VIII polypeptides, allelic variant isoforms, tissue-specific isoforms and allelic variants thereof, synthetic molecules prepared by translation of nucleic acids, proteins isolated from human and non-human tissue and cells, chimeric factor VIII polypeptides and modified forms thereof. Human and non-human factor VIII also include fragments or portions of factor VIII that are of sufficient length or include appropriate regions to retain at least one activity of the full-length mature polypeptide. Human and non-human factor VIII polypeptides also can include factor VIII polypeptides that are of sufficient length to inhibit one or more activities of a full-length mature factor VIII polypeptide.

As used herein, an "active portion or fragment of a factor VIII polypeptide" refers to a portion of a human or non-human factor VIII polypeptide that includes at least one modification provided herein and exhibits an activity, such as one or more activities of a full-length factor VIII polypeptide or possesses another activity. Activity can be any percentage of activity (more or less) of the full-length polypeptide, including but not limited to, 1% of the activity, 2%, 3%, 4%, 5%, 10%, 20%, 30%, 40%, 50%, 60%, 70%, 80%, 90%, 95%, 96%, 97%, 98%, 99%, 100%, 200%, 300%, 400%, 500%, or more activity compared to the full polypeptide. Assays to determine function or activity of modified forms of factor VIII include those known to those of skill in the art, and exemplary assays are included herein. Activity also includes activities possessed by a fragment or modified form that are not possessed by the full length polypeptide or unmodified polypeptide.

As used herein, "native factor VIII" refers to a factor VIII polypeptide encoded by a naturally occurring factor VIII gene that is present in an organism in nature, including a human or other animal. Included among native factor VIII polypeptides are the encoded precursor polypeptide, fragments thereof, and processed forms thereof, such as any pre- or post-translationally processed or modified form thereof.

As used herein, "unmodified protein," "unmodified polypeptide," "unmodified target protein," "unmodified factor VIII" and grammatical variations thereof refer to a starting polypeptide that is selected for modification as provided herein. The starting target polypeptide can be a naturally-occurring, wild-type form of a polypeptide. In addition, the starting target polypeptide can be altered or mutated, such that it differs from a native wild type isoform but is nonetheless referred to herein as a starting unmodified target protein relative to the subsequently modified polypeptides produced herein. Thus, existing proteins known in the art that have been modified to have a desired increase or decrease in a particular activity or property compared to an unmodified reference protein can be selected and used as the starting unmodified target protein. For example, a protein that has been modified from its native form by one or more single amino acid changes and possesses either an increase or decrease in a desired property, such as a change in an amino acid residue or residues to alter glycosylation, or to alter half-life, etc., can be a target protein, referred to herein as unmodified, for further modification of either the same or a different property.

Existing proteins known in the art that previously have been modified to have a desired alteration, such as an increase or decrease, in a particular biological activity or property compared to an unmodified or reference protein can be selected and used as provided herein for identification of structurally homologous loci on other structurally homologous target proteins. For example, a protein that has been modified by one or more single amino acid changes and possesses either an increase or decrease in a desired property or activity, such as for example reduced immunogenicity/antigenicity, can be utilized with the methods provided herein to identify on structurally homologous target proteins, corresponding structurally homologous loci that can be replaced with suitable replacing amino acids and tested for either an increase or decrease in the desired activity.

As used herein, an "activity" or a "functional activity" of a factor VIII polypeptide refers to any activity exhibited by a factor VIII polypeptide. Activities of a factor VIII polypeptide can be tested *in vitro and*/*or in vivo* and include, but are not limited to, coagulation activity, anticoagulation activity, enzymatic activity, and peptidase activity. Activity can be assessed in vitro or in vivo using recognized assays. The results of such assays that indicate that a polypeptide exhibits an activity can be correlated to activity of the polypeptide in vivo, in which in vivo activity can be referred to as biological activity. Activity can be any level of percentage of activity of the polypeptide, including but not limited to, 1% of the activity, 2%, 3%, 4%, 5%, 10%, 20%, 30%, 40%, 50%, 60%, 70%, 80%, 90%, 95%, 96%, 97%, 98%, 99%, 100%, 200%, 300%, 400%, 500%, or more of activity compared to the full polypeptide. Assays to determine functionality or activity of modified forms of factor VIII are known to those of skill in the art.

As used herein, "exhibits at least one activity" or "retains at least one activity" refers to the activity exhibited by a modified factor VIII polypeptide as compared to an unmodified factor VIII polypeptide of the same form and under the same conditions. For example, a modified factor VIII polypeptide is compared with an unmodified factor VIII polypeptide, under the same experimental conditions, where the only difference between the two polypeptides is the modification under study. Generally, a modified factor VIII polypeptide that retains an activity of an unmodified factor VIII polypeptide either improves or maintains the requisite biological activity of an unmodified factor VIII polypeptide. In some instances, a modified factor VIII polypeptide can retain an activity that is increased compared to an unmodified factor VIII polypeptide. In some cases, a modified factor VIII polypeptide can retain an activity that is decreased compared to an unmodified factor VIII polypeptide. Activity of a modified factor VIII polypeptide can be any level of percentage of activity of the unmodified polypeptide, including but not limited to, 1% of the activity, 2%, 3%, 4%, 5%, 10%, 20%, 30%, 40%, 50%, 60%, 70%, 80%, 90%, 95%, 96%, 97%, 98%, 99%, 100%, 200%, 300%, 400%, 500%, or more activity compared to the unmodified polypeptide. For example, a modified factor VIII polypeptide retains at least about or 1%, 2%, 3%, 4%, 5%, 6%, 7%, 8%, 9%, 10%, 20%, 30%, 40%, 50%, 60%, 70%, 80%, 90%, 95%, 96%, 97%, 98% or at least 99% of the activity of the wild-type factor VIII polypeptide. In other embodiments, the change in activity is at least about 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more times greater than unmodified factor VIII.

As used herein, a "property" of a factor VIII polypeptide refers to any property exhibited by a factor VIII polypeptide. Changes in properties can alter an "activity" of the polypeptide. One example of a property of a modified factor VIII polypeptide is reduced immunogenicity/antigenicity.

As used herein, "factor VIII-associated disease or disorder" refers to any disease or disorder in which treatment with a factor VIII (e.g., modified factor VIII) ameliorates any symptom or manifestation of the disease or disorder. Exemplary factor VIII-associated diseases and disorders include, but are not limited to, hemorrhagic disorders, such as hemophilia. Accordingly, a disease or condition that is treated by administration of factor VIII includes any disease or condition for which factor VIII (e.g., modified factor VIII) is employed for treatment, including, but not limited to, hemorrhagic disorders, such as hemophilia.

As used herein, "hemophilia" refers to a bleeding disorder caused by or involving a deficiency in blood clotting factors. Hemophilia can be the result, for example, of absence, reduced expression, or reduced function of a clotting factor. The most common type of hemophilia is hemophilia A, which results from a deficiency in factor VIII. The second most common type of hemophilia is hemophilia B, which results from a deficiency in factor IX. Another, more rare form of hemophilia is hemophilia C, which results from a deficiency in factor XI. As used herein, "congenital hemophilia" refers to types of hemophilia that are inherited. Congenital hemophilia results from mutation, deletion, insertion, or other modification of a clotting factor gene in which the production of the clotting factor is absent, reduced, or non-functional. For example, hereditary mutations in clotting factor genes, such as factor VIII and factor IX result in the congenital hemophilias, Hemophilia A and B, respectively.

As used herein, "subject" to be treated includes humans and human or non-human animals. Mammals include, primates, such as humans, chimpanzees, gorillas and monkeys; domesticated animals, such as dogs, horses, cats, pigs, goats, cows, and rodents, such as mice, rats, hamsters and gerbils. As used herein, a patient is a human subject.

An "epitope" is a set of amino acids on a protein that are involved in an immunological response, such as antibody binding, class II binding, or T-cell activation. "Epitope" includes T cell epitopes and B cell epitopes.

An "epitope area" is defined as the amino acids situated close to the epitope sequence amino acids. Preferably, the amino acids of an epitope area are located <5 angstrom (ANG) from the epitope sequence. Hence, an epitope area also includes the corresponding epitope sequence itself. Modifications of amino acids of the "epitope area" can, in some embodiments, affect the immunogenic function of the corresponding epitope.

By the term "epitope sequence" is meant the amino acid residues of a parent protein, which have been identified to belong to an epitope by the methods of the present invention.

As used herein, "variant," "factor VIII variant," "modified factor VIII polypeptides" and "modified factor VIII" refers to a factor VIII that has one or more mutations or modifications (e.g., chemical conjugations, additions, substitutions, deletions) compared to an unmodified factor VIII. The one or more mutations can be one or amino acid replacements, insertions or deletions and any combination thereof. Typically, a modified factor VIII has one or more modifications in its primary sequence compared to an unmodified factor VIII polypeptide. For example, a modified factor VIII provided herein can have 1, 2, 3, 4, 5, 6, 7, 8, 9, 10, 11, 12, 13, 14, 15, 16, 17, 18, 19, 20, or more mutations compared to an unmodified factor VIII. Modifications that confer a property (such as, reduced immunogenicity/antigenicity) by virtue of a change in a primary amino acid sequence do not always require a change in post-translational modification of the modified polypeptide to confer the property. Any length polypeptide is contemplated as long as the resulting polypeptide exhibits at least one factor VIII activity associated with a native factor VIII polypeptide or inhibits at least one factor VIII activity associated with a native factor VIII polypeptide.

As used herein, a "single amino acid replacement" refers to the replacement of one amino acid by another amino acid. The replacement can be by a natural amino acid or non-natural amino acids. When one amino acid is replaced by another amino acid in a protein, the total number of amino acids in the protein is unchanged.

As used herein, the phrase "only one amino acid replacement occurs on each target protein" refers to the modification of a target protein, such that it differs from the unmodified form of the target protein by a single amino acid change. For example, in one embodiment, mutagenesis is performed by the replacement of a single amino acid residue at only one target position on the protein backbone, such that each individual mutant generated is the single product of each single mutagenesis reaction. The single amino acid replacement mutagenesis reactions are repeated for each of the replacing amino acids selected at each of the target positions. Thus, a plurality of mutant protein molecules are produced, whereby each mutant protein contains a single amino acid replacement at only one of the target positions.

As used herein, "at a position or positions corresponding to an amino acid position" or "at a position or positions corresponding to position or positions" of a protein or grammatical variations thereof, refers to amino acid positions that are determined to correspond to one another based on sequence and/or structural alignments with a specified reference protein. For example, in a position corresponding to an amino acid position of human factor VIII can be determined empirically by aligning the sequence of amino acids of human factor VIII with a particular factor VIII polypeptide of interest. Corresponding positions can be determined by such alignment by one of skill in the art using manual alignments or by using the numerous alignment programs available (for example, BLASTP). Corresponding positions also can be based on structural alignments, for example by using computer simulated alignments of protein structure. Recitation that amino acids of a polypeptide correspond to amino acids in a disclosed sequence refers to amino acids identified upon alignment of the polypeptide with the disclosed sequence to maximize identity or homology (where conserved amino acids are aligned) using a standard alignment algorithm, such as the GAP algorithm.

As used herein, "at a position corresponding to" refers to a position of interest (i.e., base number or residue number) in a nucleic acid molecule or protein relative to the position in another reference nucleic acid molecule or protein. The position of interest to the position in another reference protein can be in, for example, a precursor protein, an allelic variant, a heterologous protein, an amino acid sequence from the same protein of another species, etc. Corresponding positions can be determined by comparing and aligning sequences to maximize the number of matching nucleotides or residues, for example, such that identity between the sequences is greater than 95%, 96%, 97%, 98% or 99% or more. The position of interest is then given the number assigned in the reference nucleic acid molecule.

As used herein, the terms "homology" and "identity" are used interchangeably, but homology for proteins can include conservative amino acid changes. In general to identify corresponding positions the sequences of amino acids are aligned so that the highest order match is obtained (see, such as: Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carillo et al. (1988) SIAM J Applied Math 48:1073).

As use herein, "sequence identity" refers to the number of identical amino acids (or nucleotide bases) in a comparison between a test and a reference polypeptide or polynucleotide. Homologous polypeptides refer to a pre-determined number of identical or homologous amino acid residues. Homology includes conservative amino acid substitutions as well identical residues. Sequence identity can be determined by standard alignment algorithm programs used with default gap penalties established by each supplier. Homologous nucleic acid molecules refer to a pre-determined number of identical or homologous nucleotides. Homology includes substitutions that do not change the encoded amino acid (i.e., "silent substitutions") as well identical residues. Substantially homologous nucleic acid molecules hybridize typically at moderate stringency or at high stringency all along the length of the nucleic acid or along at least about 70%, 80%, or 90% of the full-length nucleic acid molecule of interest. Also contemplated are nucleic acid molecules that contain degenerate codons in place of codons in the hybridizing nucleic acid molecule. (For determination of homology of proteins, conservative amino acids can be aligned as well as identical amino acids; in this case, percentage of identity and percentage homology vary). Whether any two nucleic acid molecules have nucleotide sequences (or any two polypeptides have amino acid sequences) that are at least 80%, 85%, 90%, 95%, 96%, 97%, 98% or 99% "identical" can be determined using known computer algorithms such as the "FASTA" program, using for example, the default parameters as in Pearson et al. (1988) Proc. Natl. Acad. Sci. USA 85: 2444 (other programs include the GCG program package (Devereux, J., et al. (1984) Nucleic Acids Research 12(I): 387), BLASTP, BLASTN, FASTA (Atschul, S. F., et al. (1990) J. Molec. Biol. 215:403; Guide to Huge Computers, Martin J. Bishop, ed., Academic Press, San Diego (1994), and Carillo et al. (1988) SIAM J Applied Math 48: 1073). For example, the BLAST function of the National Center for Biotechnology Information database can be used to determine identity. Other commercially or publicly available programs include, DNAStar "MegAlign" program (Madison, Wis.) and the University of Wisconsin Genetics Computer Group (UWG) "Gap" program (Madison Wis.). Percent homology or identity of proteins and/or nucleic acid molecules can be determined, for example, by comparing sequence information using a GAP computer program (such as, Needleman et al. (1970) J. Mol. Biol. 48: 443, as revised by Smith and Waterman (1981) Adv. Appl. Math. 2: 482. Briefly, a GAP program defines similarity as the number of aligned symbols (i.e., nucleotides or amino acids) which are similar, divided by the total number of symbols in the shorter of the two sequences. Default parameters for the GAP program can include: (1) a unary comparison matrix (containing a value of 1 for identities and 0 for non identities) and the weighted comparison matrix of Gribskov et al. (1986) Nucl. Acids Res. 14: 6745, as described by Schwartz and Dayhoff, eds. (1979) Atlas of Protein Sequence and Structure, National Biomedical Research Foundation, pp. 353-358; (2) a penalty of 3.0 for each gap and an additional 0.10 penalty for each symbol in each gap; and (3) no penalty for end gaps.

Therefore, as used herein, the term "identity" represents a comparison between a test and a reference polypeptide or polynucleotide. In one non-limiting example, "at least 90% identical to" refers to percent identities from 90 to 100% relative to the reference polypeptides. Identity at a level of 90% or more is indicative of the fact that, assuming for exemplification purposes a test and reference polynucleotide length of 100 amino acids are compared, no more than 10% (i.e., 10 out of 100) of amino acids in the test polypeptide differs from that of the reference polypeptides. Similar comparisons can be made between a test and reference polynucleotides. Such differences can be represented as point mutations randomly distributed over the entire length of an amino acid sequence or they can be clustered in one or more locations of varying length up to the maximum allowable, such as, 10/100 amino acid difference (approximately 90% identity). Differences are defined as nucleic acid or amino acid substitutions, insertions or deletions. At the level of homologies or identities above about 85-90%, the result should be independent of the program and gap parameters set; such high levels of identity can be assessed readily, often without relying on software.

As used herein, the phrase "sequence-related proteins" refers to proteins that have at least 50%, at least 60%, at least 70%, at least 80%, at least 90%, or at least 95% amino acid sequence identity or homology with each other.

As used herein, families of non-related proteins or "sequence-non-related proteins" refer to proteins having less than 50%, less than 40%, less than 30%, less than 20% amino acid identity, or homology with each other.

As used herein, it also is understood that the terms "substantially identical" or "similar" varies with the context as understood by those skilled in the relevant art.

As used herein, "a naked polypeptide chain" refers to a polypeptide that is not post-translationally modified or otherwise chemically modified, but contains only covalently linked amino acids.

As used herein, the amino acids that occur in the various sequences of amino acids provided herein are identified according to their known, three-letter or one-letter abbreviations. The nucleotides which occur in the various nucleic acid fragments are designated with the standard single-letter designations used routinely in the art. As used herein, an "amino acid" is an organic compound containing an amino group and a carboxylic acid group. A polypeptide comprises two or more amino acids. For purposes herein, amino acids include the twenty naturally-occurring amino acids, non-natural amino acids, and amino acid analogs (i.e., amino acids wherein the α-carbon has a side chain). As used herein, the abbreviations for any protective groups, amino acids and other compounds are, unless indicated otherwise, in accord with their common usage, recognized abbreviations, or the IUPAC-IUB Commission on Biochemical Nomenclature (1972) Biochem. 11:1726). Each naturally occurring L-amino acid is identified by the standard three letter code (or single letter code) or the standard three letter code (or single letter code) with the prefactor VIII "L-;" the prefactor VIII "D-" indicates that the stereoisomeric form of the amino acid is D.

As used herein, "amino acid residue" refers to an amino acid formed upon chemical digestion (hydrolysis) of a polypeptide at its peptide linkages. The amino acid residues described herein are presumed to be in the "L" isomeric form. Residues in the "D" isomeric form, which are so designated, can be substituted for any L-amino acid residue as long as the desired functional property is retained by the polypeptide. "NH2" refers to the free amino group present at the amino terminus of a polypeptide. "COOH" refers to the free carboxy group present at the carboxyl terminus of a polypeptide. In keeping with standard polypeptide nomenclature described in (1969) J. Biol. Chem., 243: 3552-3559, and adopted 37.C.F.R. 1.821-1.822.

All amino acid residue sequences represented herein by formulae have a left to right orientation in the conventional direction of amino-terminus to carboxyl-terminus. In addition, the phrase "amino acid residue" is broadly defined to include the amino acids listed herein and modified and unusual amino acids, such as those referred to in 37 C.F.R. 1.821-1.822, and incorporated herein by reference. Furthermore, a dash at the beginning or end of an amino acid residue sequence indicates a peptide bond to a further sequence of one or more amino acid residues, to an amino-terminal group such as NH2 or to a carboxyl-terminal group such as COOH.

As used herein, "naturally occurring amino acids" refer to the 20 L-amino acids that occur in polypeptides.

As used herein, the term "non-natural amino acid" refers to an organic compound that has a structure similar to a natural amino acid but has been modified structurally to mimic the structure and reactivity of a natural amino acid. Non-naturally occurring amino acids thus include, for example, amino acids or analogs of amino acids other than the 20 naturally occurring amino acids and include, but are not limited to, the D-stereoisomers of amino acids. Exemplary non-natural amino acids are described herein and are known to those of skill in the art.

As used herein, nucleic acids include DNA, RNA, and analogs thereof, including protein nucleic acids (PNA) and mixtures thereof. Nucleic acids can be single- or double-stranded. When referring to probes or primers (optionally labeled with a detectable label, such as, a fluorescent or a radiolabel), single-stranded molecules are contemplated. Such molecules are typically of a length such that they are statistically unique of low copy number (typically less than 5, generally less than 3) for probing or priming a library. Generally a probe or primer contains at least 10, 15, 20, 25, or 30 contiguous nucleic acid bases of sequence complementary to, or identical to, a gene of interest. Probes and primers can be 5, 6, 7, 8, 9, 10, or more, 20 or more, 30 or more, 50 or more, 100, or more nucleic acids long.

As used herein, heterologous or foreign nucleic acid, such as DNA and RNA, are used interchangeably and refer to DNA or RNA that does not occur naturally as part of the genome in which it occurs or is found at a locus or loci in a genome that differs from that in which it occurs in nature. Heterologous nucleic acid includes nucleic acid not endogenous to the cell into which it is introduced, but that has been obtained from another cell or prepared synthetically. Generally, although not necessarily, such nucleic acid encodes RNA and proteins that are not normally produced by the cell in which it is expressed. Heterologous DNA herein encompasses any DNA or RNA that one of skill in the art recognizes or considers as heterologous or foreign to the cell or locus in or at which it is expressed. Heterologous DNA and RNA also can encode RNA or proteins that mediate or alter expression of endogenous DNA by affecting transcription, translation, or other regulatable biochemical processes. Examples of heterologous nucleic acid include, but are not limited to, nucleic acid that encodes traceable marker proteins (such as, a protein that confers drug resistance), nucleic acid that encodes therapeutically effective substances (such as, anti-cancer agents), enzymes and hormones, and DNA that encodes other types of proteins (such as, antibodies). Hence, herein heterologous DNA or foreign DNA includes a DNA molecule not present in the exact orientation and position as the counterpart DNA molecule found in the genome. It also can refer to a DNA molecule from another organism or species (i.e., exogenous).

As used herein, "isolated with reference to a nucleic acid molecule or polypeptide or other biomolecule" means that the nucleic acid or polypeptide has separated from the genetic environment from which the polypeptide or nucleic acid were obtained. It also can mean altered from the natural state. For example, a polynucleotide or a polypeptide naturally present in a living animal is not "isolated," but the same polynucleotide or polypeptide separated from the coexisting materials of its natural state is "isolated," as the term is employed herein. Thus, a polypeptide or polynucleotide produced and/or contained within a recombinant host cell is considered isolated. Also intended as an "isolated polypeptide" or an "isolated polynucleotide" are polypeptides or polynucleotides that have been partially or substantially purified from a recombinant host cell or from a native source. For example, a recombinantly produced version of a compound can be substantially purified by the one-step method described in Smith et al. (1988) Gene, 67:31-40. The terms isolated and purified can be used interchangeably.

Thus, by "isolated" it is meant that the nucleic acid is free of coding sequences of those genes that, in the naturally-occurring genome of the organism (if any), immediately flank the gene encoding the nucleic acid of interest. Isolated DNA can be single-stranded or double-stranded, and can be genomic DNA, cDNA, recombinant hybrid DNA or synthetic DNA. It can be identical to a starting DNA sequence or can differ from such sequence by the deletion, addition, or substitution of one or more nucleotides.

"Purified" preparations made from biological cells or hosts mean at least the purity of a cell extracts containing the indicated DNA or protein including a crude extract of the DNA or protein of interest. For example, in the case of a protein, a purified preparation can be obtained following an individual technique or a series of preparative or biochemical techniques, and the DNA or protein of interest can be present at various degrees of purity in these preparations. The procedures can include, but are not limited to, ammonium sulfate fractionation, gel filtration, ion exchange chromatography, affinity chromatography, density gradient centrifugation, and electrophoresis.

A preparation of DNA or protein that is "substantially pure" or "isolated" refers to a preparation substantially free from naturally-occurring materials with which such DNA or protein is normally associated in nature and generally contains 5% or less of the other contaminants.

A cell extract that contains the DNA or protein of interest refers to a homogenate preparation or cell-free preparation obtained from cells that express the protein or contain the DNA of interest. The term "cell extract" is intended to include culture medium, especially spent culture medium from which the cells have been removed.

As used herein, "recombinant" refers to any progeny formed as the result of genetic engineering.

As used herein, the phrase "operatively linked" with reference to a nucleic acid molecule generally means the sequences or segments have been covalently joined into one piece of DNA, whether in single- or double-stranded form, whereby control or regulatory sequences on one segment control or permit expression or replication or other such control of other segments. The two segments are not necessarily contiguous. For gene expression, a DNA sequence and a regulatory sequence(s) are connected in such a way to control or permit gene expression when the appropriate molecular, such as, transcriptional activator proteins, are bound to the regulatory sequence(s).

As used herein, "production by recombinant means by using recombinant DNA methods" means the use of the well-known methods of molecular biology for expressing proteins encoded by cloned DNA, including cloning expression of genes and methods.

The term "ameliorating" refers to any therapeutically beneficial result in the treatment of a disease state, including prophylaxis, lessening in the severity or progression, remission, or cure thereof.

The term *"in situ"* refers to processes that occur in a living cell growing separate from a living organism, e.g., growing in tissue culture.

The term *"in vivo"* refers to processes that occur in a living organism.

The term "sufficient amount" means an amount sufficient to produce a desired effect.

The term "therapeutically effective amount" is an amount that is effective to ameliorate a symptom of a disease. A therapeutically effective amount can be a "prophylactically effective amount" as prophylaxis can be considered therapy.

It must be noted that, as used in the specification and the appended claims, the singular forms "a," "an" and "the" include plural referents unless the context clearly dictates otherwise.

### Factor VIII

Factor VIII (FVIII) exists naturally and in therapeutic preparations as a heterogeneous distribution of polypeptides arising from a single gene product (e.g., Andersson et al., Proc. Natl. Acad. Sci. USA, 83, 2979-2983 (1986), herein incorporated by reference). "Factor VIII" or "FVIII" refers to all such polypeptides, whether derived from blood plasma or produced through the use of recombinant DNA techniques or by other means.

FVIII is secreted as an approximately 300 kDa single chain glycoprotein having the following domain organization NH₂-A1-A2-B-A3-C1-C2-COOH, where each "domain" comprises a structural unit encoded by a continuous sequence of amino acids. FVIII isolated from plasma comprises two subunits, known as the heavy chain and light chain. The FVIII heavy chain comprises the A1, A2, and B domains, and the FVIII light chain comprises the A3, C1, and C2 domains. The B domain has no known biological function in clot formation and can be wholly or partially removed without significantly altering FVIII function.

FVIII generally circulates complexed with another plasma protein, von Willebrand factor (vWF), which is present in a large molar excess (∼50:1) to FVIII in plasma and protects FVIII from premature degradation by plasma proteases. FVIII is proteolytically activated primarily by thrombin (factor IIa), which cleaves the heavy chain between the A1 and A2 domains and dissociates FVIII from von Willebrand factor (vWF) to form factor VIIIa (FVIIIa), which is the active form of FVIII having coagulant activity. FVIIIa acts as a cofactor of activated Factor IX, which accelerates the activation of Factor X, which converts prothrombin into thrombin, which converts fibrinogen into fibrin, which induces clotting.

The human FVIII gene has been isolated and expressed in mammalian cells, as reported by various authors, including Wood et al. in Nature (1984) 312: 330-337 and the amino-acid sequence was deduced from cDNA. U.S. Pat. No. 4,965,199 discloses a recombinant DNA method for producing FVIII in mammalian host cells and purification of human FVIII. The human FVIII detailed structure has been extensively investigated. The cDNA nucleotide sequence encoding human FVIII and predicted amino-acid sequence have been disclosed for instance in U.S. Pat. No. 5,663,060, herein incorporated by reference. In some embodiments, FVIII is a nucleotide sequence encoding human FVIII and the corresponding amino acid sequence are shown in GenBank accession number NM_000132.2, herein incorporated by reference. In some embodiments, FVIII is a nucleotide sequence encoding human FVIII and the corresponding amino acid sequence are shown in GenBank accession number NM_000132.3, herein incorporated by reference. In some embodiments, FVIII is a nucleotide sequence encoding human FVIII with Asp1241 (e.g., Kogenate™) and the corresponding amino acid sequence. In some embodiments, FVIII is a nucleotide sequence encoding human FVIII with Glu1241 (e.g., Recombinate™) and the corresponding amino acid sequence.

### Compositions

The present disclosure relates generally to methods and compositions for ameliorating or preventing the adverse effects of "inhibitor" antibodies in hemophilia patients. One aspect focuses on the mechanisms and structural determinants involved in initiating an inhibitor response. Inhibitor formation is T-cell dependent and involves recognition of specific epitopes on FVIII by antigen-specific T-cells. Factor VIII polypeptides are processed by antigen-presenting cells, which display factor VIII polypeptides to antigen-specific T-cells via cell surface HLA class II complexes. Antigen-specific T-cells recognize and bind certain peptide-HLA II complexes, leading to T-cell activation and downstream stimulation of an antibody response. Disclosed herein are several T-cell epitopes identified using T-cells isolated from hemophilia A patients with inhibitors and characterization of the minimum structural features required for association with HLA II molecules and recognition by T-cells.

Contemplated herein are modified factor VIII polypeptides that differ from unmodified or wild-type factor VIII polypeptides with respect to a property or an activity. Modified factor VIII polypeptides provided herein can have reduced immunogenicity/antigenicity as compared to unmodified factor VIII polypeptides.

Provided herein are methods for reducing the immunogenicity/antigenicity of a factor VIII polypeptide. Provided herein are methods of modifying factor VIII polypeptides to reduce its immunogenicity/antigenicity. Provided herein are modified factor VIII polypeptides in which the primary amino acid sequence is modified to confer reduced immunogenicity/antigenicity. Among the amino acid modifications provided herein are such modifications including replacement of amino acids in the primary sequence of the factor VIII polypeptide in order to reduce the immunogenicity/antigenicity of the factor VIII polypeptide. Further modifications of the factor VIII polypeptide can be included, such as, but not limited to, addition of carbohydrate, phosphate, sulfur, hydroxyl, carboxyl, and polyethylene glycol (PEG) moieties. Thus, the modified factor VIII polypeptides provided herein can be modified, for example, by glycosylation, phosphorylation, sulfation, hydroxylation, carboxylation, and/or PEGylation. Such modifications can be performed in vivo or in vitro.

Provided herein are modified factor VIII polypeptides that display reduced immunogenicity/antigenicity. The reduced immunogenicity/antigenicity of the modified factor VIII polypeptide can be decreased by an amount that is at least about or 1%, 2%, 3%, 4%, 5%, 6%, 7%, 8%, 9%, 10%, 20%, 30%, 40%, 50%, 60%, 70%, 80%, 90%, 100%, 200%, 300%, 400%, 500%, or more compared to the immunogenicity/antigenicity of the unmodified factor VIII polypeptide. In some examples, the reduced immunogenicity/antigenicity of the modified factor VIII polypeptide can be decreased by an amount that is at least 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, or more times when compared to the immunogenicity/antigenicity of the unmodified factor VIII polypeptide. Hence, the modified factor VIII polypeptides provided herein offer factor VIII with advantages including a decrease in the frequency of injections needed to maintain a sufficient drug level in serum, thus leading to, for example, higher comfort and acceptance by subjects, lower doses necessary to achieve comparable biological effects and attenuation of secondary effects.

Provided herein are modified factor VIII polypeptides containing modifications that alter any one or more of the properties of factor VIII that contribute to reduced immunogenicity/antigenicity. Reduced immunogenicity/antigenicity can be accomplished by amino acid replacement. Generally, modified factor VIII polypeptides retain one or more activities of an unmodified factor VIII polypeptide. For example, the modified factor VIII polypeptides provided herein exhibit at least one activity that is substantially unchanged (less than 1%, 5% or 10% changed) compared to the unmodified or wild-type factor VIII. In other examples, the activity of a modified factor VIII polypeptide is increased or is decreased as compared to an unmodified factor VIII polypeptide. In another embodiment, the modified factor VIII polypeptides provided herein can inhibit an activity of the unmodified and/or wild-type native factor VIII polypeptide. Activity includes, for example, but not limited to blood coagulation, platelet binding, cofactor binding and protease activity. Activity can be assessed in vitro or in vivo and can be compared to the unmodified factor VIII polypeptide.

Modified factor VIII polypeptides provided herein can be modified at one or more amino acid positions corresponding to amino acid positions of an unmodified factor VIII polypeptide, for example, a factor VIII polypeptide having an amino acid sequence set forth in SEQ ID NO:1. *See* Table A. SEQ ID NO:2 is one embodiment of a modified factor VIII polypeptide, where X is any amino acid and at least one X is a modified amino acid. *See* Table A. Modified factor VIII polypeptides provided herein include human factor VIII (hFactor VIII) variants. A hfactor VIII polypeptide can be of any human tissue or cell-type origin. Modified factor VIII polypeptides provided herein also include variants of factor VIII of non-human origin. Modified factor VIII polypeptides also include polypeptides that are hybrids of different factor VIII polypeptides and also synthetic factor VIII polypeptides prepared recombinantly or synthesized or constructed by other methods known in the art based upon known polypeptides.

**Table A**

| **Description** | **Sequence** | **SEQ ID NO** |
|---|---|---|
| Factor VIII Polypeptide | MQIELSTCFFLCLLRFCFSATRRYYLGAVELSWDYMQSDLGELP VDARFPPRVPKSFPFNTSVVYKKTLFVEFTDHLFNIAKPRPPWMGLLGPTIQAEVYDT VVITLKNMASHPVSLHAVGVSYWKASEGAEYDDQTSQREKEDDKVFPGGSHTYVWQVL | 1 |
| (NM_00013 2.2) | KENGPMASDPLCLTYSYLSHVDLVKDLNSGLIGALLVCREGSLAKEKTQTLHKFILLF AVFDEGKSWHSETKNSLMQDRDAASARAWPKMHTVNGYVNRSLPGLIGCHRKSVYWHV IGMGTTPEVHSIFLEGHTFLVRNHRQASLEISPITFLTAQTLLMDLGQFLLFCHISSH QHDGMEAYVKVDSCPEEPQLRMKNNEEAEDYDDDLTDSEMDVVRFDDDNSPSFIQIRS VAKKHPKTWVHYIAAEEEDWDYAPLVLAPDDRSYKSQYLNNGPQRIGRKYKKVRFMAY TDETFKTREAIQHESGILGPLLYGEVGDTLLIIFKNQASRPYNIYPHGITDVRPLYSR RLPKGVKHLKDFPILPGEIFKYKWTVTVEDGPTKSDPRCLTRYYSSFVNMERDLASGL IGPLLICYKESVDQRGNQIMSDKRNVILFSVFDENRSWYLTENIQRFLPNPAGVQLED PEFQASNIMHSINGYVFDSLQLSVCLHEVAYWYILSIGAQTDFLSVFFSGYTFKHKMV YEDTLTLFPFSGETVFMSMENPGLWILGCHNSDFRNRGMTALLKVSSCDKNTGDYYED SYEDISAYLLSKNNAIEPRSFSQNSRHPSTRQKQFNATTIPENDIEKTDPWFAHRTPM PKIQNVSSSDLLMLLRQSPTPHGLSLSDLQEAKYETFSDDPSPGAIDSNNSLSEMTHF RPQLHHSGDMVFTPESGLQLRLNEKLGTTAATELKKLDFKVSSTSNNLISTIPSDNLA AGTDNTSSLGPPSMPVHYDSQLDTTLFGKKSSPLTESGGPLSLSEENNDSKLLESGLM NSQESSWGKNVSSTESGRLFKGKRAHGPALLTKDNALFKVSISLLKTNKTSNNSATNR KTHIDGPSLLIENSPSVWQNILESDTEFKKVTPLIHDRMLMDKNATALRLNHMSNKTT SSKNMEMVQQKKEGPIPPDAQNPDMSFFKMLFLPESARWIQRTHGKNSLNSGQGPSPK QLVSLGPEKSVEGQNFLSEKNKVVVGKGEFTKDVGLKEMVFPSSRNLFLTNLDNLHEN NTHNQEKKIQEEIEKKETLIQENVVLPQIHTVTGTKNFMKNLFLLSTRQNVEGSYDGA YAPVLQDFRSLNDSTNRTKKHTAHFSKKGEEENLEGLGNQTKQIVEKYACTTRISPNT SQQNFVTQRSKRALKQFRLPLEETELEKRIIVDDTSTQWSKNMKHLTPSTLTQIDYNE KEKGAITQSPLSDCLTRSHSIPQANRSPLPIAKVSSFPSIRPIYLTRVLFQDNSSHLP AASYRKKDSGVQESSHFLQGAKKNNLSLAILTLEMTGDQREVGSLGTSATNSVTYKKV ENTVLPKPDLPKTSGKVELLPKVHIYQKDLFPTETSNGSPGHLDLVEGSLLQGTEGAI KWNEANRPGKVPFLRVATESSAKTPSKLLDPLAWDNHYGTQIPKEEWKSQEKSPEKTA FKKKDTILSLNACESNHAIAAINEGQNKPEIEVTWAKQGRTERLCSQNPPVLKRHQRE ITRTTLQSDQEEIDYDDTISVEMKKEDFDIYDEDENQSPRSFQKKTRHYFIAAVERLW DYGMSSSPHVLRNRAQSGSVPQFKKVVFQEFTDGSFTQPLYRGELNEHLGLLGPYIRA EVEDNIMVTFRNQASRPYSFYSSLISYEEDQRQGAEPRKNFVKPNETKTYFWKVQHHM APTKDEFDCKAWAYFSDVDLEKDVHSGLIGPLLVCHTNTLNPAHGRQVTVQEFALFFT IFDETKSWYFTENMERNCRAPCNIQMEDPTFKENYRFHAINGYIMDTLPGLVMAQDQR IRWYLLSMGSNENIHSIHFSGHVFTVRKKEEYKMALYNLYPGVFETVEMLPSKAGIWR VECLIGEHLHAGMSTLFLVYSNKCQTPLGMASGHIRDFQITASGQYGQWAPKLARLHY SGSINAWSTKEPFSWIKVDLLAPMIIHGIKTQGARQKFSSLYISQFIIMYSLDGKKWQ TYRGNSTGTLMVFFGNVDSSGIKHNIFNPPIIARYIRLHPTHYSIRSTLRMELMGCDL NSCSMPLGMESKAISDAQITASSYFTNMFATWSPSKARLHLQGRSNAWRPQVNNPKEW LQVDFQKTMKVTGVTTQGVKSLLTSMYVKEFLISSSQDGHQWTLFFQNGKVKVFQGNQ DSFTPVVNSLDPPLLTRYLRIHPQSWVHQIALRMEVLGCEAQDLY | |
| Modified Factor VIII Polypeptide; X is any amino acid and at least one X is a modified amino acid | MQIELSTCFFLCLLRFCFSATRRYYLGAVELSWDYMQSDLGELP VDARFPPRVPKSFPFNTSVVYKKTLFVEFTDHLFNIAKPRPPWMGLLGPTIQAEVYDT VVITLKNMASHPVSLHAVGVSYWKASEGAEYDDQTSQREKEDDKVFPGGSHTYVWQVL KENGPMASDPLCLTYSYLSHVDLVKDLNSGLIGALLVCREGSLAKEKTQTLHKFILLF AVFDEGKSWHSETKNSLMQDRDAASARAWPKMHTVNGYVNRSLPGLIGCHRKSVYWHV IGMGTTPEVHSIFLEGHTFLVRNHRQASLEISPITFLTAQTLLMDLGQFLLFCHISSH QHDGMEAYVKVDSCPEEPQLRMKNNEEAEDYDDDLTDSEMDVVRFDDDNSPSFIQIRS VAKKHPKTWVHYIAAEEEDWDYAPLVLAPDDRSYKSQYLNNGPQRIGRKYKKVRFMAY TDETFKTREAIQHESGILGPLLYGEVGDTLLIIFKNQASRPYNIYPHGITDVRPLYSR RLPKGVKHLKDFPILPGEIFKYKWTVTVEDGPTKSDPRCLTRYYSSFVNMERDLASGL IGPLLICYKESVDQRGNQIMSDKRNVILFSVFDENRSWYLTXXXXXXXXXXXXXXXX XXFQASNIMHSINGYVFDSLQLSVCLHEVAYWYILSIGAQTDFLSVFFSGYTFKHKMV YEDTLTLFPFSGETVFMSMENPGLWILGCHNSDFRNRGMTALLKVSSCDKNTGDYYED SYEDISAYLLSKNNAIEPRSFSQNSRHPSTRQKQFNATTIPENDIEKTDPWFAHRTPM PKIQNVSSSDLLMLLRQSPTPHGLSLSDLQEAKYETFSDDPSPGAIDSNNSLSEMTHF RPQLHHSGDMVFTPESGLQLRLNEKLGTTAATELKKLDFKVSSTSNNLISTIPSDNLA AGTDNTSSLGPPSMPVHYDSQLDTTLFGKKSSPLTESGGPLSLSEENNDSKLLESGLM NSQESSWGKNVSSTESGRLFKGKRAHGPALLTKDNALFKVSISLLKTNKTSNNSATNR KTHIDGPSLLIENSPSVWQNILESDTEFKKVTPLIHDRMLMDKNATALRLNHMSNKTT SSKNMEMVQQKKEGPIPPDAQNPDMSFFKMLFLPESARWIQRTHGKNSLNSGQGPSPK | 2 |
| | QLVSLGPEKSVEGQNFLSEKNKVVVGKGEFTKDVGLKEMVFPSSRNLFLTNLDNLHEN NTHNQEKKIQEEIEKKETLIQENVVLPQIHTVTGTKNFMKNLFLLSTRQNVEGSYDGA YAPVLQDFRSLNDSTNRTKKHTAHFSKKGEEENLEGLGNQTKQIVEKYACTTRISPNT SQQNFVTQRSKRALKQFRLPLEETELEKRIIVDDTSTQWSKNMKHLTPSTLTQIDYNE KEKGAITQSPLSDCLTRSHSIPQANRSPLPIAKVSSFPSIRPIYLTRVLFQDNSSHLP AASYRKKDSGVQESSHFLQGAKKNNLSLAILTLEMTGDQREVGSLGTSATNSVTYKKV ENTVLPKPDLPKTSGKVELLPKVHIYQKDLFPTETSNGSPGHLDLVEGSLLQGTEGAI KWNEANRPGKVPFLRVATESSAKTPSKLLDPLAWDNHYGTQIPKEEWKSQEKSPEKTA FKKKDTILSLNACESNHAIAAINEGQNKPEIEVTWAKQGRTERLCSQNPPVLKRHQRE ITRTTLQSDQEEIDYDDTISVEMKKEDFDIYDEDENQSPRSFQKKTRHYFIAAVERLW DYGMSSSPHVLRNRAQSGSVPQFKKVVFQEFTDGSFTQPLYRGELNEHLGLLGPYIRA EVEDNIMVTFRNQASRPYSFYSSLISYEEDQRQGAEPRKNFVKPNETKTYFWKVQHHM APTKDEFDCKAWAYFSDVDLEKDVHSGLIGPLLVCHTNTLNPAHGRQVTVQEFALFFT IFDETKSWYFTENMERNCRAPCNIQMEDPTFKENYRFHAINGYIMDTLPGLVMAQDQR IRWYLLSMGSNENIHSIHFSGHVFTVRKKEEYKMALYNLYPGVFETVEMLPSKAGIWR VECLIGEHLHAGMSTLFLVYSNKCQTPLGMASGHIRDFQITASGQYGQWAPKLARLHY SGSINAWSTKEPFSWIKVDLLAPMIIHGIKTQGARQKFSSLYISQFIIMYSLDGKKWQ TYRGNSTGTLMVFFGNVDSSGIKHNIFNPPIIARYIRLHPTHYSIRSTLRMELMGCDL NSCSMPLGMESKAISDAQITASXXXXXXXXXXXXXXXXXXXXXXXXXXXXXQVNNPKEW LQVDFQKTMKVTGVTTQGVKSLLTSMYVKEFLISSSQDGHQWTLFFQNGKVKVFQGNQ DSFTPVVNSLDPPLLTRYLRIHPQSWVHQIALRMEVLGCEAQDLY | |

Also among the variants provided herein are modified factor VIII polypeptides with two or more modifications compared to native or wild-type factor VIII. Modified factor VIII polypeptides include those with 2, 3, 4, 5, 6, 7, 8, 9, 10, 11, 12, 13, 14, 15, 16, 17, 18, 19, 20 or more modified positions.

Typically, modifications include replacement (substitution), addition, deletion or a combination thereof, of amino acid residues as described herein. Generally, the modification results in reduced immunogenicity/antigenicity without losing at least one activity, of an unmodified factor VIII polypeptide. Exemplary epitopes for amino acid modification corresponding to amino acid positions of a mature factor VIII polypeptide (e.g., SEQ ID NO:1) that can contribute to reducing immunogenicity/antigenicity are set forth in Table B.

**Table B**

| **Epitope** | **Type** | **FVIII Domain** | **FVIII Residues** | **Minimal** | **Haplotype** | **Target Residues** |
|---|---|---|---|---|---|---|
| 1 | T-cell (A2201P mild hemophilia ) | C2 (2173-2332) | 2194-2213 (SYFTNMFATW SPSKARLHLQ) (SEQ ID NO:3) | S2194-P2205 (SYFTNMF ATWSP) (SEQ ID NO:4) | DR-0101 | F2196, M2199, A2201, S2204 |
| 2 | T-cell (A2201P mild hemophilia ) | C2 (2173-2332) | 2202-2221 (TWSPSKARLHL QGRSNAWRP) (SEQ ID NO:5) | | DR-1104 | |
| 3 | T-cell (R593C mild hemophilia ) | A2 (373-740) | 589-608 (ENIQRFLPNPA GVQLEDPE) (SEQ ID NO:6) | 594-602 (FLPNPAG VQ) (SEQ ID NO:7) | DR-1101 | R593, F594, N597, A599, Q602 |
| 4 | B-cell (IgG4 antibody BO2C11) | C2 (2173-2332) | | | | R2220A , R2220Q F2196A , N2198A , M2199 A, L2200A , R2215A |
| 5 | B-cell | C2 (2173-2332) | | | | L2273A , R2220A , Q2213A , T2272A |

A modified factor VIII polypeptide exhibiting a modified immunogenicity/antigenicity may be produced by changing an identified epitope area of an unmodified factor VIII polypeptide by, e.g., genetically engineering a mutation in a epitope sequence encoding the unmodified factor VIII polypeptide.

An epitope in a factor VIII polypeptide may be changed by substituting at least one amino acid of the epitope area. In an embodiment at least one amino acid deemed important for HLA-class II receptor (e.g., DR) contact is modified. In an embodiment at least one amino acid deemed important for TCR contact is modified. In an embodiment at least one amino acid deemed important for antibody contact is modified. In an embodiment at least one amino acid deemed important for class II or TCR contact is modified and at least one amino acid deemed important for antibody contact is modified. The change will often be substituting to an amino acid of different size, hydrophilicity, and/or polarity, such as a small amino acid versus a large amino acid, a hydrophilic amino acid versus a hydrophobic amino acid, a polar amino acid versus a non-polar amino acid and a basic versus an acidic amino acid.

Other changes may be the addition/insertion or deletion of at least one amino acid of the epitope sequence, e.g., deleting an amino acid important for class II or TCR recognition and activation and/or antibody binding. Furthermore, an epitope area may be changed by substituting some amino acids, and deleting/adding one ore more others.

When one uses protein engineering to alter or eliminate epitopes, it is possible that new epitopes are created, or existing epitopes are duplicated. To reduce this risk, one can map the planned mutations at a given position on the 3-dimensional structure of the protein of interest, and control the emerging amino acid constellation against a database of known epitope patterns, to rule out those possible replacement amino acids, which are predicted to result in creation or duplication of epitopes. Thus, risk mutations can be identified and eliminated by this procedure, thereby reducing the risk of making mutations that lead to increased rather than decreased immunogenicity/antigenicity.

A modified factor VIII polypeptide exhibiting a modified immunogenicity/antigenicity may be produced by chemically modifying (e.g., via conjugation) the identified epitope area of the unmodified factor VIII polypeptide. For example, the factor VIII polypeptide can be incubated with an active or activated polymer and subsequently separated from the unreacted polymer. This can be done in solution followed by purification or it can conveniently be done using the immobilized protein variants, which can easily be exposed to different reaction environments and washes.

Thus, modified factor VIII polypeptides of the invention can be modified within one or more epitopes described herein via, e.g., amino acid additions, substitutions, or deletions. In addition, modification can include chemical conjugation to one or more epitopes described herein. In some embodiments, a modification is made in a T cell epitope. In some embodiments, a modificiation is made in a B cell epitope. In some embodiments, a modification is made in both a T cell epitope and a B cell epitope.

### Methods of Making Factor VIII Polypeptides

The factor VIII polypeptides of this invention largely may be made in transformed host cells using recombinant DNA techniques. To do so, a recombinant DNA molecule coding for the peptide is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidate method. Also, a combination of these techniques could be used.

The invention also includes a vector capable of expressing the peptides in an appropriate host and/or cell. The vector comprises the DNA molecule that codes for the peptides operatively linked to appropriate expression control sequences. Methods of affecting this operative linking, either before or after the DNA molecule is inserted into the vector, are well known. Expression control sequences include promoters, activators, enhancers, operators, ribosomal nuclease domains, start signals, stop signals, cap signals, polyadenylation signals, and other signals involved with the control of transcription or translation.

The resulting vector having the DNA molecule thereon is used to transform an appropriate host and/or cell. This transformation may be performed using methods well known in the art.

Any of a large number of available and well-known host cells may be used in the practice of this invention. The selection of a particular host is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all hosts may be equally effective for the expression of a particular DNA sequence. Within these general guidelines, useful microbial hosts include bacteria (such as *E. coli* sp.), yeast (such as *Saccharomyces* sp.) and other fungi, insects, plants, mammalian (including human) cells in culture, or other hosts known in the art.

Next, the transformed host is cultured and purified. Host cells may be cultured under conventional fermentation conditions so that the desired compounds are expressed. Such fermentation conditions are well known in the art. Finally, the peptides are purified from culture by methods well known in the art.

The compounds may also be made by synthetic methods. For example, solid phase synthesis techniques may be used. Suitable techniques are well known in the art, and include those described in Merrifield (1973), Chem. Polypeptides, pp. 335-61 (Katsoyannis and Panayotis eds.); Merrifield (1963), J. Am. Chem. Soc. 85: 2149; Davis et al. (1985), Biochem. Intl. 10: 394-414; Stewart and Young (1969), Solid Phase Peptide Synthesis; U.S. Pat. No. 3,941,763; Finn et al. (1976), The Proteins (3rd ed.) 2: 105-253; and Erickson et al. (1976), The Proteins (3rd ed.) 2: 257-527. Solid phase synthesis is the preferred technique of making individual peptides since it is the most cost-effective method of making small peptides. Compounds that contain derivatized peptides or which contain non-peptide groups may be synthesized by well-known organic chemistry techniques.

### Pharmaceutical Compositions and Therapeutic Methods of Use

In some embodiments, a modified factor VIII polypeptide is administered to a subj ect in need thereof to reduce or prevent a condition associated with an immune response to factor VIII. In some embodiments, a modified factor VIII polypeptide is administered to a subject in need thereof to treat or reduce a condition associated with an immune response to factor VIII.

In certain embodiments, a factor VIII polypeptide is administered alone. In certain embodiments, a factor VIII polypeptide is administered prior to the administration of at least one other therapeutic agent. In certain embodiments, a factor VIII polypeptide is administered concurrent with the administration of at least one other therapeutic agent. In certain embodiments, a factor VIII polypeptide is administered subsequent to the administration of at least one other therapeutic agent. In other embodiments, a factor VIII polypeptide is administered prior to the administration of at least one other therapeutic agent. As will be appreciated by one of skill in the art, in some embodiments, the factor VIII polypeptide is combined with the other agent/compound. In some embodiments, the factor VIII polypeptide and other agent are administered concurrently. In some embodiments, the factor VIII polypeptide and other agent are not administered simultaneously; with the factor VIII polypeptide being administered before or after the agent is administered. In some embodiments, the subject receives both the factor VIII polypeptide and the other agent during a same period of prevention, occurrence of a disorder, and/or period of treatment.

Pharmaceutical compositions of the invention can be administered in combination therapy, i.e., combined with other agents. In certain embodiments, the combination therapy comprises nuclease molcule, in combination with at least one other agent. Agents include, but are not limited to, in vitro synthetically prepared chemical compositions, antibodies, antigen binding regions, and combinations and conjugates thereof. In certain embodiments, an agent can act as an agonist, antagonist, allosteric modulator, or toxin.

In certain embodiments, the invention provides for pharmaceutical compositions comprising a factor VIII polypeptide together with a pharmaceutically acceptable diluent, carrier, solubilizer, emulsifier, preservative and/or adjuvant.

In certain embodiments, the invention provides for pharmaceutical compositions comprising a factor VIII polypeptide and a therapeutically effective amount of at least one additional therapeutic agent, together with a pharmaceutically acceptable diluent, carrier, solubilizer, emulsifier, preservative and/or adjuvant.

In certain embodiments, acceptable formulation materials preferably are nontoxic to recipients at the dosages and concentrations employed. In some embodiments, the formulation material(s) are for s.c. and/or I.V. administration. In certain embodiments, the pharmaceutical composition can contain formulation materials for modifying, maintaining or preserving, for example, the pH, osmolarity, viscosity, clarity, color, isotonicity, odor, sterility, stability, rate of dissolution or release, adsorption or penetration of the composition. In certain embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. (Remington's Pharmaceutical Sciences, 18th Edition, A. R. Gennaro, ed., Mack Publishing Company (1995). In some embodiments, the formulation comprises PBS; 20 mM NaOAC, pH 5.2, 50 mM NaCl; and/or 10 mM NAOAC, pH 5.2, 9% Sucrose.

In certain embodiments, a factor VIII polypeptide and/or a therapeutic molecule is linked to a half-life extending vehicle known in the art. Such vehicles include, but are not limited to, polyethylene glycol, glycogen (e.g., glycosylation of the factor VIII polypeptide), and dextran. Such vehicles are described, e.g., in U.S. application Ser. No. 09/428,082, now U.S. Pat. No. 6,660,843 and published PCT Application No. WO 99/25044, which are hereby incorporated by reference for any purpose.

In certain embodiments, the optimal pharmaceutical composition will be determined by one skilled in the art depending upon, for example, the intended route of administration, delivery format and desired dosage. See, for example, Remington's Pharmaceutical Sciences, supra. In certain embodiments, such compositions may influence the physical state, stability, rate of in vivo release and rate of in vivo clearance of the antibodies of the invention.

In certain embodiments, the primary vehicle or carrier in a pharmaceutical composition can be either aqueous or non-aqueous in nature. For example, in certain embodiments, a suitable vehicle or carrier can be water for injection, physiological saline solution or artificial cerebrospinal fluid, possibly supplemented with other materials common in compositions for parenteral administration. In some embodiments, the saline comprises isotonic phosphate-buffered saline. In certain embodiments, neutral buffered saline or saline mixed with serum albumin are further exemplary vehicles. In certain embodiments, pharmaceutical compositions comprise Tris buffer of about pH 7.0-8.5, or acetate buffer of about pH 4.0-5.5, which can further include sorbitol or a suitable substitute therefore. In certain embodiments, a composition comprising a factor VIII polypeptide, with or without at least one additional therapeutic agents, can be prepared for storage by mixing the selected composition having the desired degree of purity with optional formulation agents (Remington's Pharmaceutical Sciences, supra) in the form of a lyophilized cake or an aqueous solution. Further, in certain embodiments, a composition comprising a factor VIII polypeptide, with or without at least one additional therapeutic agent, can be formulated as a lyophilizate using appropriate excipients such as sucrose.

In certain embodiments, the pharmaceutical composition can be selected for parenteral delivery. In certain embodiments, the compositions can be selected for inhalation or for delivery through the digestive tract, such as orally. The preparation of such pharmaceutically acceptable compositions is within the ability of one skilled in the art.

In certain embodiments, the formulation components are present in concentrations that are acceptable to the site of administration. In certain embodiments, buffers are used to maintain the composition at physiological pH or at a slightly lower pH, typically within a pH range of from about 5 to about 8.

In certain embodiments, when parenteral administration is contemplated, a therapeutic composition can be in the form of a pyrogen-free, parenterally acceptable aqueous solution comprising a desired factor VIII polypeptide, with or without additional therapeutic agents, in a pharmaceutically acceptable vehicle. In certain embodiments, a vehicle for parenteral injection is sterile distilled water in which a factor VIII polypeptide, with or without at least one additional therapeutic agent, is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that can provide for the controlled or sustained release of the product which can then be delivered via a depot injection. In certain embodiments, hyaluronic acid can also be used, and can have the effect of promoting sustained duration in the circulation. In certain embodiments, implantable drug delivery devices can be used to introduce the desired molecule.

In certain embodiments, a pharmaceutical composition can be formulated for inhalation. In certain embodiments, a factor VIII polypeptide, with or without at least one additional therapeutic agent, can be formulated as a dry powder for inhalation. In certain embodiments, an inhalation solution comprising a factor VIII polypeptide, with or without at least one additional therapeutic agent, can be formulated with a propellant for aerosol delivery. In certain embodiments, solutions can be nebulized. Pulmonary administration is further described in PCT application no. PCT/US94/001875, which describes pulmonary delivery of chemically modified proteins.

In certain embodiments, it is contemplated that formulations can be administered orally. In certain embodiments, a factor VIII polypeptide, with or without at least one additional therapeutic agents, that is administered in this fashion can be formulated with or without those carriers customarily used in the compounding of solid dosage forms such as tablets and capsules. In certain embodiments, a capsule can be designed to release the active portion of the formulation at the point in the gastrointestinal tract when bioavailability is maximized and pre-systemic degradation is minimized. In certain embodiments, at least one additional agent can be included to facilitate absorption of a factor VIII polypeptide and/or any additional therapeutic agents. In certain embodiments, diluents, flavorings, low melting point waxes, vegetable oils, lubricants, suspending agents, tablet disintegrating agents, and binders can also be employed.

In certain embodiments, a pharmaceutical composition can involve an effective quantity of a factor VIII polypeptide, with or without at least one additional therapeutic agents, in a mixture with non-toxic excipients which are suitable for the manufacture of tablets. In certain embodiments, by dissolving the tablets in sterile water, or another appropriate vehicle, solutions can be prepared in unit-dose form. In certain embodiments, suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions will be evident to those skilled in the art, including formulations involving a factor VIII polypeptide, with or without at least one additional therapeutic agent(s), in sustained- or controlled-delivery formulations. In certain embodiments, techniques for formulating a variety of other sustained- or controlled-delivery means, such as liposome carriers, bio-erodible microparticles or porous beads and depot injections, are also known to those skilled in the art. See for example, PCT Application No. PCT/US93/00829 which describes the controlled release of porous polymeric microparticles for the delivery of pharmaceutical compositions. In certain embodiments, sustained-release preparations can include semipermeable polymer matrices in the form of shaped articles, e.g. films, or microcapsules. Sustained release matrices can include polyesters, hydrogels, polylactides (U.S. Pat. No. 3,773,919 and EP 058,481), copolymers of L-glutamic acid and gamma ethyl-L-glutamate (Sidman et al., Biopolymers, 22:547-556 (1983)), poly (2-hydroxyethyl-methacrylate) (Langer et al., J. Biomed. Mater. Res., 15:167-277 (1981) and Langer, Chem. Tech., 12:98-105 (1982)), ethylene vinyl acetate (Langer et al., supra) or poly-D(-)-3-hydroxybutyric acid (EP 133,988). In certain embodiments, sustained release compositions can also include liposomes, which can be prepared by any of several methods known in the art. See, e.g., Eppstein et al., Proc. Natl. Acad. Sci. USA, 82:3688-3692 (1985); EP 036,676; EP 088,046 and EP 143,949.

The pharmaceutical composition to be used for *in vivo* administration typically is sterile. In certain embodiments, this can be accomplished by filtration through sterile filtration membranes. In certain embodiments, where the composition is lyophilized, sterilization using this method can be conducted either prior to or following lyophilization and reconstitution. In certain embodiments, the composition for parenteral administration can be stored in lyophilized form or in a solution. In certain embodiments, parenteral compositions generally are placed into a container having a sterile access port, for example, an intravenous solution bag or vial having a stopper pierceable by a hypodermic injection needle.

In certain embodiments, once the pharmaceutical composition has been formulated, it can be stored in sterile vials as a solution, suspension, gel, emulsion, solid, or as a dehydrated or lyophilized powder. In certain embodiments, such formulations can be stored either in a ready-to-use form or in a form (e.g., lyophilized) that is reconstituted prior to administration.

In certain embodiments, kits are provided for producing a single-dose administration unit. In certain embodiments, the kit can contain both a first container having a dried protein and a second container having an aqueous formulation. In certain embodiments, kits containing single and multi-chambered pre-filled syringes (e.g., liquid syringes and lyosyringes) are included.

In certain embodiments, the effective amount of a pharmaceutical composition comprising a factor VIII polypeptide, with or without at least one additional therapeutic agent, to be employed therapeutically will depend, for example, upon the therapeutic context and objectives. One skilled in the art will appreciate that the appropriate dosage levels for treatment, according to certain embodiments, will thus vary depending, in part, upon the molecule delivered, the indication for which a factor VIII polypeptide, with or without at least one additional therapeutic agent, is being used, the route of administration, and the size (body weight, body surface or organ size) and/or condition (the age and general health) of the patient. In certain embodiments, the clinician can titer the dosage and modify the route of administration to obtain the optimal therapeutic effect. In certain embodiments, a typical dosage can range from about 0.1 µg/kg to up to about 100 mg/kg or more, depending on the factors mentioned above. In certain embodiments, the dosage can range from 0.1 µg/kg up to about 100 mg/kg; or 1 µg/kg up to about 100 mg/kg; or 5 µg/kg up to about 100 mg/kg.

In certain embodiments, the frequency of dosing will take into account the pharmacokinetic parameters of a factor VIII polypeptide and/or any additional therapeutic agents in the formulation used. In certain embodiments, a clinician will administer the composition until a dosage is reached that achieves the desired effect. In certain embodiments, the composition can therefore be administered as a single dose or as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. In certain embodiments, appropriate dosages can be ascertained through use of appropriate dose-response data.

In certain embodiments, the route of administration of the pharmaceutical composition is in accord with known methods, e.g. orally, through injection by intravenous, intraperitoneal, intracerebral (intra-parenchymal), intracerebroventricular, intramuscular, subcutaneously, intra-ocular, intraarterial, intraportal, or intralesional routes; by sustained release systems or by implantation devices. In certain embodiments, the compositions can be administered by bolus injection or continuously by infusion, or by implantation device.

In certain embodiments, the composition can be administered locally via implantation of a membrane, sponge or another appropriate material onto which the desired molecule has been absorbed or encapsulated. In certain embodiments, where an implantation device is used, the device can be implanted into any suitable tissue or organ, and delivery of the desired molecule can be via diffusion, timed-release bolus, or continuous administration.

In certain embodiments, it can be desirable to use a pharmaceutical composition comprising a factor VIII polypeptide, with or without at least one additional therapeutic agent, in an ex vivo manner. In such instances, cells, tissues and/or organs that have been removed from the patient are exposed to a pharmaceutical composition comprising a factor VIII polypeptide, with or without at least one additional therapeutic agent, after which the cells, tissues and/or organs are subsequently implanted back into the patient.

In certain embodiments, a factor VIII polypeptide and/or any additional therapeutic agents can be delivered by implanting certain cells that have been genetically engineered, using methods such as those described herein, to express and secrete the polypeptides. In certain embodiments, such cells can be animal or human cells, and can be autologous, heterologous, or xenogeneic. In certain embodiments, the cells can be immortalized. In certain embodiments, in order to decrease the chance of an immunological response, the cells can be encapsulated to avoid infiltration of surrounding tissues. In certain embodiments, the encapsulation materials are typically biocompatible, semi-permeable polymeric enclosures or membranes that allow the release of the protein product(s) but prevent the destruction of the cells by the patient's immune system or by other detrimental factors from the surrounding tissues.

The modified factor VIII polypeptides and nucleic acid molecules provided herein can be used for treatment of any condition for which unmodified factor VIII is employed. Modified factor VIII polypeptides have therapeutic activity alone or in combination with other agents. The modified factor VIII polypeptides provided herein are designed to retain therapeutic activity but exhibit modified properties, particularly reduced immunogenicity/antigenicity. Such modified properties, for example, can improve the therapeutic effectiveness of the polypeptides and/or can provide for additional routes of administration.

In particular, modified factor VIII polypeptides, are intended for use in therapeutic methods in which factor VIII has been used for treatment. Such methods include, but are not limited to, methods of treatment of diseases and disorders, such as, but not limited to, hemophilias. Modified factor VIII polypeptides also can be used in the treatment of additional bleeding diseases and disorders where deemed efficacious by one of skill in the art.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### EXAMPLE 1

### Materials and Methods

Human subjects. Blood samples from hemophilic brothers with the FVIII missense substitution A2201P, who shared the *HLA-DRA-DRBI *0101* allele, were obtained following written, informed consent according to a protocol approved by the University of Washington Human Subjects Review Committee. One of the brothers developed a high-titer inhibitor (peak titer of 250 BU/ml) after receiving intensive Factor VIII (FVIII) treatment to support tonsillectomy/adenectomy. Samples were also obtained from an uncle with mild hemophilia A due to the missense substitution A2201P, and who was HLA-DRB1*0901, 1104. Tetramer were available to analyze his DRB1*1104-restricted T-cell responses to FVIII. This subject had never been infused with FVIII, unlike his two nephews.

**T-cell clones.** T-cell clones were obtained from blood samples from both brothers by staining CD4+ cells with fluorescent DR0101 tetramers that were loaded with peptide FVIII₂₁₉₄₋₂₂₁₃, followed by single-cell sorting and expansion. Clones were expanded by stimulation with irradiated peripheral blood mononuclear cells (PBMCs) from an HLA-mismatched individual plus phytohemagglutinin (Remel, Lenexa, KS) in the presence of human IL-2 (Hemagen Diagnostics, Inc., Columbia, MD). Clonality was confirmed by tetramer staining, multiplex PCR and sequencing of the VDJ region in the PCR products.

**FVIII peptides.** FVIII₂₁₉₄₋₂₂₁₃ peptide (sequence: SYFTNMFATWSPSKARLHLQ (SEQ ID NO:3)) and peptides truncated and with sequence modifications of this region were obtained from commercial vendors (Mimotopes, Clayton Victoria, Australia; Global Peptide Inc., Ft. Collins, CO; Synpep, Dublin, CA; Anaspec, San Jose, CA). Molecular weights were confirmed by mass spectrometry.

**FVIII C2 domain proteins.** Sequence modifications were introduced into pET16b/wild-type C2 plasmid containing a His tag using QuikChange II site-directed mutagenesis kit (Stratagene, La Jolla, CA). Origami™ B(DE3)pLysS competent cells (Novagen EMD4Biosciences) were transformed with wild-type C2 and sequence modified plasmid. Protein expression was induced with IPTG at 16°C. Cells were disrupted and C2-His tag labeled proteins were purified using a Ni-charged column (Novagen EMD4Biosciences). Endotoxins were removed with a wash step containing 0.1 % Triton X-114⁵. C2 proteins were eluted with 20 mM Tris-HCl, 0.5 M NaCl, 1 M imidazole, pH 7.9. Eluted proteins were dialyzed into 1X D-PBS containing 5% glycerol. Purity was determined by electrophoresis on 4-20% Tris-glycine gels (Invitrogen) in Laemmli's buffer containing dithiothreitol followed by Bio-Safe Coomassie Blue staining (Bio-Rad, Hercules, CA) and ImageQuant 350 digital imaging (GE Healthcare). Endotoxin levels were tested with ToxinSensor™ Chromogenic LAL endotoxin assay kit (GenScript Corporation, Piscataway, NJ). Sterility was assessed by inoculating LB agar plates and incubating at 37°C for 3 days.

**Antigen presentation.** FVIII peptides and FVIII C2 domain proteins were added to irradiated PBMCs from a *DRB1*0101* donor. Peptides were added at final concentrations of 100, 50, 10, 5, 1, 0.1, and 0.01 µM. Proteins were added at final concentrations of 1, 0.5, 0.1, 0.05, 0.01, 0.005, and 0.001 µM. After a 4-hour incubation at 37°C, FVIII₂₁₉₄₋₂₂₁₃ specific T-cell clones restricted by DR0101 were added to each well. At 48 hours, 50 µl supernatant was removed from each well for cytokine analysis and replaced with [³H]thymidine (1 µCi/well) in T-cell medium. Cells were harvested after 14-16 h of further incubation and [³H] thymidine uptake was measured. Levels of IFN-γ, IL-4, and IL-17A in cell supernatants were measured with standard sandwich ELISAs. EC₅₀ values (concentration at which half-maximal levels occur) were determined with Systat (Systat Software, Inc., San Jose, CA) using a three parameter sigmoid model.

**HLA-DR epitope prediction.** Predicted binding of FVIII peptides to HLA-DR was evaluated using ProPred⁶, a software which uses both quantitative peptide binding profiles and pocket information derived from MHC class II structures to construct matrices for 51 HLA-DR alleles.

**HLA-DR peptide binding.** Binding affinities of FVIII peptides were determined by competition assay. Recombinant HLA-DRB1*0101 (DR0101), DRB1*0301 (DR0301), DRB1*0401 (DR0401), DRB1*1101 (DR1101), DRB1*1104 (DR1104), or DRB1*1501 (DR1501) proteins were incubated with 0.05, 0.1, 0.5, 1, 5, 10, and 50 µM of FVIII peptides plus biotinylated reference peptides and immobilized in wells coated with anti-DR antibody (L243) as described⁷. The reference peptides used were: 0.02 µM HA₃₀₆₋₃₁₈ (DR0101), 0.1 µM HA₃₀₆₋₃₁₈ (DR0401), 0.2 µM HA₃₀₆₋₃₁₈ (DR1101), 0.02 µM Myo₁₃₇₋₁₄₈ (DR0301), 0.2 µM HSV-2 VP16₃₄₋₄₄ (DR1104), and 0.03 µM MBP₈₄₋₁₀₂ (DR1501). After washing, biotinylated peptide was labeled using europium-conjugated streptavidin (Perkin Elmer) and quantified using a Victor² D fluorometer (Perkin Elmer). Sigmoidal binding curves were simulated and IC₅₀ values (concentration displacing 50% reference peptide) calculated using SigmaPlot (Systat Software, Inc., San Jose, CA).

### Results

An immunodominant HLA-DRB1*0101-restricted FVIII T-cell epitope was previously identified in a mild hemophilia A inhibitor subject **(****Figure 1****)** and his brother **(****Figure 2****)** using tetramer guided epitope mapping¹⁻². Their CD4+ T cells recognized overlapping synthetic peptides with sequences corresponding to FVIII residues 2186-2205, 2187-2205 and 2194-2213 **(****Figures 1-3****).** T-cell clones obtained by single-cell sorting of tetramer-positive cells from both mild HA brothers followed by expansion with IL-2 in cell culture showed, strong, unambiguous staining by DR0101 tetramers loaded with peptide FVIII-2194-2213 **(****Figures 3-4****).** A strong HLA-DRB1*0101-restricted response to the same epitope was also seen in an unrelated severe hemophilia A inhibitor subject (subject 56A) who also had the DRB1*0101 allele **(****Figure 5A****).** Interestingly, the uncle of these subjects (subject IV-2 in Ettinger et al., Haemophilia 16:44-55, 2010) showed an HLA-DRB1*1104-restricted response to peptide FVIII 2202-2221, only when CD25+ cells were depleted, and even though he had not ever been infused with FVIII **(****Figure 5B****).** This was interpreted as a "naïve" response to FVIII due to autoreactive T cells that were normally suppressed by CD25+ regulatory T cells. The tetramer staining was well above background levels, indicating that this peptide indeed contained a DRB1*1104-restricted T-cell epitope. The clones from both brothers proliferated strongly in response to the wild-type peptide containing FVIII residues 2194-2213, but not to the hemophilic peptide with the A2201P substitution **(****Figures 3** **and** **6****).** Clonality was confirmed by multiplex PCR; **Figure 7** shows representative results in which a single product was obtained for the TCR-VDJ regions amplified by PCR. All subjects provided written informed consent for the study). The sequence overlap suggested that the T-cell epitope was contained within FVIII residues 2194-2205. This was tested by synthesizing peptides truncated from both the amino and carboxy-terminal ends and measuring binding compared to the full-length peptide of FVIII₂₁₉₄₋₂₂₁₃ **(****Figure 8****).** This experiment demonstrated that the minimal binding epitope is 2194-2205 (sequence: SYFTNMFATWSP (SEQ ID NO:4)).

HLA-DR proteins bind peptides utilizing 4-5 pockets within a groove composed of amino acids from both the alpha and beta chains of HLA-DR. The crystal structure of DR0101 demonstrate 4 major pockets that interact with the peptide at relative positions 1, 4, 6, and 9³. Thus, the anchor positions within FVIII₂₁₉₄₋₂₂₀₅ were determined by testing binding of Arg-substituted and Ala-substituted peptides to DR0101. Sequence modifications were made at each amino acid within FVIII₂₁₉₄₋₂₂₀₅. This experiment showed that binding was abolished or greatly reduced with the following substitutions: F2196R, M2199R, A2201R, S2204R, F2196A, and M2199A **(****Figures 9A-B**). These results suggest that the anchor amino acids are F2196, M2199, A2201, and S2204, which are at relative positions 1, 4, 6, and 9, respectively.

Subsequent experiments tested the effects of substituting each of 19 amino acids (excluding the native phenylalanine) at position F2196. The substitutions were generated in synthetic peptides corresponding to FVIII positions 2194-2213 (synthesized and validated by Mimotopes, Inc.). Fifteen substitutions at position 2196 reduced T-cell proliferation 80% or more, compared to the response to the wild-type sequence, when T-cell clone 32A-18 (from mild HA subject 32A) was cultured with the following peptides: F2196I, F2196M, F2196V, F2196Q, F2196A, F2196K, F2196T, F2196S, F2196N, F2196R, F2196E, F2196H, F2196G, F2196D, F2196P **(****Figure 9C****).** The following 16 substitutions reduced the binding affinity for DR0101 by 80% or more: F2196I, F2196L, F2196M, F2196V, F2196Q, F2196A, F2196K, F2196T, F2196S, F2196N, F2196R, F2196E, F2196H, F2196G, F2196D, and F2196P **(****Figure 9D****).** The 15 substitutions that affected both proliferation and binding to DR0101 can all be used in a modified Factor VIII polypeptide.

T-cell clones were isolated from the brothers recognizing this epitope and have previously been described^{1-2,4}. These clones present four distinct T-helper phenotypes⁴ and come from at least six different progenitors based on *TCRBV* sequencing. The response of clones representing four of the six distinct progenitors to sequence-modified FVIII₂₁₉₄₋₂₂₀₅ was tested. Peptides with Ala-substitutions at each position of the peptide were added to antigen presentation assays and compared with wild-type peptide. The response of the T-cell clones to presentation was monitored by measuring T-cell proliferation using the [³H]-thymidine incorporation assay and cytokine secretion with sandwich ELISAs. These assays identified F2196A as the only Ala sequence modification to which all four clones did not respond to. *See* **Figure 8C****.** Competition binding assays showed that the wild-type peptide, but not the peptide with F2196A substitution, bound effectively to Dr 0101, DR0401 and DR1501 **(****Figure 8D****).**

Subsequently, the F2196A sequence modification was introduced into the C2 domain of the FVIII protein, which is at the carboxy-terminus of the protein. The F2196A sequence modified C2 protein and wild-type C2 with His tags were affinity purified from *E*. *coli* over Ni-columns including a wash step to remove endotoxin⁵. Endotoxin levels in both purified proteins were low at 0.2 EU/ml and comparable with that in the human serum used in T cell cultures. The purified C2 proteins were than tested in the antigen presentation assay as described for the FVIII₂₁₉₄₋₂₂₀₅ peptides. All four clones responded robustly to wild-type C2 with EC₅₀ values (half-maximal concentrations) between 0.058-0.597 µM for the four different clones. No T-cell proliferation was observed in response to F2196A sequence modified C2 protein. *See* **Figure 10****.**

Binding of FVIII₂₁₉₄₋₂₂₀₅ to a few other HLA-DR alleles was tested, using the same assay measuring residual Europium fluorescence due to the residual reference peptide (e.g. as described and referenced in E.A. James et al., manuscript submitted): specifically, DR0301, DR0401, DR1101, DR1104, and DR1501. DR0401 and DR1501 also bind to FVIII₂₁₉₄₋₂₂₀₅ with a lower affinity **(****Figure 8D****).** The ProPred prediction aligorithm using a threshold of 3% (intermediate stringency) testing 51 HLA-DR alleles suggested that 12 HLA-DR alleles will bind to this epitope. To test whether the F2196A sequence modification will eliminate immune responses if presented by other HLA-DR alleles, the binding of FVIII_{2194-2205, 2196A} was tested to the same DR alleles and examined with ProPred. Very weak binding with an IC₅₀ > 50 µM was predicted (data not shown). No binding was predicted by ProPred at the 3% threshold and this remained the case when the threshold was set at the lowest stringency (data not shown).

### EXAMPLE 2

### Introduction

The development of antibodies that interfere with FVIII pro-coagulant activity, often referred to as "inhibitors", can complicate the treatment of hemophilia A. These alloimmune responses, as well as the rare development of autoimmune FVIII inhibitors, are associated with significant morbidity and mortality. The production of anti-FVIII antibodies follows stimulation of helper T cells by epitopes in FVIII. An immunodominant HLA-DRBI*0101-restricted T-cell epitope was recognized by CD4+ T cells from a mild hemophilia A inhibitor subject and from his brother, who had a sub-clinical inhibitor (James et al., J Thromb Haemost 5: 2399-2407, 2007). Their CD4+ T cells recognized overlapping synthetic peptides with sequences corresponding to FVIII residues 2186-2205, 2187-2205 and 2194-2213. Nineteen T-cell clones recognizing this epitope were isolated, with phenotypes representing four distinct T-cell lineages. The promiscuity/immunodominance of an HLA-DRB1*0101-restricted T-cell epitope in FVIII was evaluated, and amino acid substitutions were induced that will prevent presentation of this epitope to the immune system by DR0101 and by other DR alleles.

### Methods

The minimal epitope and MHC Class II (DR0101) "anchor" residues were determined using a competition assay measuring displacement of a labeled peptide having high affinity for recombinant DR0101 by a series of FVIII peptides. Peptide concentrations at which 50% inhibition of the labeled peptide binding occurred (IC₅₀) were obtained by regression analysis. Binding of the peptides to five additional DR alleles was evaluated directly using recombinant proteins; predicted binding of peptides to additional DR alleles was evaluated using the program ProPred. Proliferation and cytokine production by the clones in response to wild-type and modified peptides were measured, and the concentrations at which half-maximal T-cell responses (EC₅₀) to the FVIII peptides occurred were determined. The methods used are similar to those used in Example 5 below.

### Results

Binding of truncated peptides to DR0101 identified FVIII₂₁₉₄₋₂₂₀₅ as the minimal epitope. Binding of FVIII₂₁₉₄₋₂₂₀₅ peptides with single Arg substitutions identified F2196, M2199, A2201 and S2204 as anchor residues at positions 1, 4, 6 and 9, respectively, corresponding to peptide-binding pockets seen in the crystal structure of a DR0101-peptide complex. The relative binding of Ala-substituted peptides confirmed that F2196 and M2199 are anchor residues **(Figure 7B)**. T-cell stimulation requires recognition of peptides by both the Class II receptor and the T-cell receptor (TCR). Sequences of TCR variable regions (TCRBVs) expressed by the clones were identified as TCRBV20-1*01 (3 VDJ combinations), TCRBV6- 6*01, TCRBV5-1*01, and TCRBV6-1*01, indicating at least six different T-cell progenitors recognized this epitope. The clones were next stimulated with peptides having modified epitopes. Strikingly, none proliferated or secreted cytokines when stimulated by FVII₂₁₉₄₋₂₂₀₅, _{F2196A}, which also showed an IC₅₀ > 10 µM when tested for binding to DR0101, DR0301, DR0401, DR1101, DR1104, and DR1501. *See* **Figure 9** and data not shown. Substitutions at other anchor positions affected binding to some but not all of the DR proteins. Predicted binding of the F2196A variant to 51 DR alleles was analyzed using ProPred; none bound at a threshold stringency of 10% (low stringency, thus the predicted epitopes included those with lower calculated affinities). In preparation for directly testing the immunogenicity/antigenicity of additional substitutions, all possible amino acid substitutions at position 2196 were evaluated using ProPred, 13 of 19 possible substitutions were predicted to prevent FVIII₂₁₉₄₋₂₂₀₅ binding to all 51 DR alleles included in the algorithm (with a 3% threshold = intermediate stringency) (data not shown).

### Discussion

MHC class II anchor residues and TCR contact sites for an immunodominant HLA-DRB1*0101-restricted T- cell epitope have been mapped precisely. Both measured and predicted effects of amino acid substitutions indicated that this F2196 is essential for effective presentation of this epitope by multiple DR alleles.

### EXAMPLE 3

Summary: Experiments with samples from a severe HA inhibitor subject who has the HLA-DRB1*0101 allele indicated that a peptide corresponding to FVIII residues 2194-2213 elicited a strong T-cell response. Proliferation and staining of T cells by tetramers loaded with this peptide was essentially the same as that seen for previous mild HA subjects who also carried the HLA-DRB1*0101 allele. Ongoing experiments will follow the same procedures used for the mild HA samples (James et al. and Ettinger et al.) to (1) define the minimal epitope and anchor residues and (2) characterize the phenotypes of the antigen-specific T-cell clones and lines. Similar experiments will be carried out soon using frozen PBMCs from at least 10 other mild, moderate, or severe HA subjects who carry the HLA-DRB1*0101 allele. We expect that these experiments will confirm that the same HLA-DRB1*0101-restricted epitope will be identified in samples from most or all of these subjects.

### DESCRIPTION OF THE EXPERIMENTS AND RESULTS

### PURPOSE

Tetramer guided epitope mapping to identify and determine the total number of T-cell epitopes in FVIII that are recognized by a severe hemophilia A subject with a high-titer inhibitor (peak titer was 2222 BU/ml in 2008). The subject failed immune tolerance induction and currently has a high-titer inhibitor of approximately 20 Bethesda Units (BU)/mL. He has a large gene deletion (exons 7-12) within the F8 gene (genotype determined by Shelley Nakaya at PSBC, confirmed in Sept. 2010, data not shown). The HLA-DRB1 type is 0101, 1001.

### MATERIALS

The HA subject# is GS1,056A

### Cells

GS1, 056A PBMC frozen 4/30/09: 5 vials totaling 45 million cells

GS1, 056A PBMC frozen 6/11/09: 10 vials totaling 95 million cells

**Table 1: Peptides for TGEM**

| Peptide Pool | Concentration | Date |
|---|---|---|
| A2 pool 1 | 10,000 uM | ∼8/07 |
| A2 pool 2 | 10,000 uM | ∼8/07 |
| A2 pool 3 | 10,000 uM | ∼8/07 |
| A2 pool 4 | 10,000 uM | ∼8/07 |
| A2 pool 5 | 10,000 uM | ∼8/07 |
| A2 pool 6 | 10,000 uM | ∼8/07 |
| A2 pool 7 | 10,000 uM | ∼8/07 |
| A2 pool 8 | 10,000 uM | ∼8/07 |
| A2 pool 9 | 10,000 uM | ∼8/07 |
| A2 pool 10 | 10,000 uM | ∼8/07 |
| C1 pool 1 | 10 mg/ml | 5/1/07 |
| C1 pool 2 | 10 mg/ml | 5/1/07 |
| C1 pool 3 | 10 mg/ml | 5/1/07 |
| C1 pool 4 | 10 mg/ml | 5/1/07 |
| C1 pool 5 | 10 mg/ml | 5/1/07 |
| C2 pool 1 NEW | 10 mg/ml? | 9/09 |
| C2 pool 2 NEW | 10 mg/ml? | 9/09 |
| C2 pool 3 NEW | 10 mg/ml? | 9/09 |
| C2 pool 4 NEW | 10 mg/ml? | 9/09 |
| 0101 1001 TT reference pool | ∼5,000 uM | Prepared 8/9/10 |

**Table 2: 0101 1001 Tetanus toxin reference pool**

| Peptide | DR allele | Concentration |
|---|---|---|
| TT 586-605 | 0101 | 10,000 uM |
| TT 666-685 | 0101 | 10,000 uM |
| TT 674-693 | 0101 | 20 mg/ml |
| TT 482-501 | 0101 | 20 mg/ml |
| TT 554-573 | 0101 | 20 mg/ml |

**Table 3: C2 peptides used to create C2 pools 1-4 NEW.**

| | | | SEQ ID NO |
|---|---|---|---|
| C2-1 NEW | FVIII 2170-2189 | DLNSCSMPLGMESKAISDAQ | 8 |
| | FVIII 2178-2197 | LGMESKAISDAQITASSYFT | 9 |
| | FVIII 2186-2205 | SDAQITASSYFTNMFATWSP | 10 |
| | FVIII 2194-2213 | SYFTNMFATWSPSKARLHLQ | 11 |
| | FVIII 2202-2221 | TWSPSKARLHLQGRSNAWRP | 12 |
| C2-2 NEW | FVIII 2210-2229 | LHLQGRSNAWRPQVNNPKEW | 13 |
| | FVIII 2218-2237 | AWRPQVNNPKEWLQVDFQKT | 14 |
| | FVIII 2226-2245 | PKEWLQVDFQKTMKVTGVTT | 15 |
| | FVIII 2234-2253 | FQKTMKVTGVTTQGVKSLLT | 16 |
| | FVIII 2242-2261 | GVTTQGVKSLLTSMYVKEFL | 17 |
| C2-3 NEW | FVIII 2250-2269 | SLLTSMYVKEFLISSSQDGH | 18 |
| | FVIII 2258-2277 | KEFLISSSQDGHQWTLFFQN | 19 |
| | FVIII 2265-2284 | SQDGHQWTLFFQNGKVKVFQ | 20 |
| | FVIII 2273-2292 | LFFQNGKVKVFQGNQDSFTP | 21 |
| | FVIII 2281-2300 | KVFQGNQDSFTPVVNSLDPP | 22 |
| C2-4 NEW | FVIII 2289-2308 | SFTPVVNSLDPPLLTRYLRI | 23 |
| | FVIII 2297-2316 | LDPPLLTRYLRIHPQSWVHQ | 24 |
| | FVIII 2305-2324 | YLRIHPQSWVHQIALRMEVL | 25 |
| | FVIII 2313-2332 | WVHQIALRMEVLGCEAQDLY | 26 |

**Table 4: A2 and C1 peptide pools are the same used in mapping of the T cell responses in the R593C mild HA subjects.**

| Pool | Residue numbers | Peptide sequence | SEQ ID NO |
|---|---|---|---|
| A2-1 | FVIII 373-392 | SVAKKHPKTWVHYIAAEEED | 27 |
| | FVIII 381-400 | TWVHYIAAEEEDWDYAPLVL | 28 |
| | FVIII 389-408 | EEEDWDYAPLVLAPDDRSYK | 29 |
| | FVIII 397-416 | PLVLAPDDRSYKSQYLNNGP | 30 |
| | FVIII 405-424 | RSYKSQYLNNGPQRIGRKYK | 31 |
| A2-2 | FVIII 413-432 | NNGPQRIGRKYKKVRFMAYT | 32 |
| | FVIII 421-440 | RKYKKVRFMAYTDETFKTRE | 33 |
| | FVIII 429-448 | MAYTDETFKTREAIQHESGI | 34 |
| | FVIII 437-456 | KTREAIQHESGILGPLLYGE | 35 |
| | FVIII 445-464 | ESGILGPLLYGEVGDTLLII | 36 |
| A2-3 | FVIII 453-472 | LYGEVGDTLLIIFKNQASRP | 37 |
| | FVIII 461-480 | LLIIFKNQASRPYNIYPHGI | 38 |
| | FVIII 469-488 | ASRPYNIYPHGITDVRPLYS | 39 |
| | FVIII 477-496 | PHGITDVRPLYSRRLPKGVK | 40 |
| | FVIII 485-504 | PLYSRRLPKGVKHLKDFPIL | 41 |
| A2-4 | FVIII 493-512 | KGVKHLKDFPILPGEIFKYK | 42 |
| | FVIII 501-520 | FPILPGEIFKYKWTVTVEDG | 43 |
| | FVIII 509-528 | IFKYKWTVTVEDGPTKSDPR | 44 |
| | FVIII 517-536 | VEDGPTKSDPRCLTRYYSSF | 45 |
| | FVIII 525-544 | DPRCLTRYYSSFVNMERDLA | 46 |
| A2-5 | FVIII 529-548* | LTRYYSSFVNMERDLASGLI | 47 |
| | FVIII 533-552* | YSSFVNMERDLASGLIGPLL | 48 |
| | FVIII 541-560 | RDLASGLIGPLLICYKESVD | 49 |
| | FVIII 549-568 | GPLLICYKESVDQRGNQIMS | 50 |
| | FVIII 557-576 | ESVDQRGNQIMSDKRNVILF | 51 |
| A2-6 | FVIII 565-584 | QIMSDKRNVILFSVFDENRS | 52 |
| | FVIII 573-592 | VILFSVFDENRSWYLTENIQ | 53 |
| | FVIII 581-600 | ENRSWYLTENIQRFLPNPAG | 54 |
| | FVIII 589-608 | ENIQRFLPNPAGVQLEDPEF | 55 |
| | FVIII 597-616 | NPAGVQLEDPEFQASNIMHS | 56 |
| A2-7 | FVIII 605-624 | DPEFQASNIMHSINGYVFDS | 57 |
| | FVIII 613-632 | IMHSINGYVFDSLQLSVCLH | 58 |
| | FVIII 610-619* | ASNIMHSINGYVFDSLQLSV | 59 |
| | FVIII 621-640 | VFDSLQLSVCLHEVAYWYIL | 60 |
| | FVIII 629-648 | VCLHEVAYWYILSIGAQTDF | 61 |
| A2-8 | FVIII 637-656* | LHEVAYWYILSIGAQTDFLS | 62 |
| | FVIII 645-664 | WYILSIGAQTDFLSVFFSGY | 63 |
| | FVIII 653-672 | QTDFLSVFFSGYTFKHKMVY | 64 |
| | FVIII 661-680 | FSGYTFKHKMVYEDTLTLFP | 65 |
| | FVIII 669-688 | KMVYEDTLTLFPFSGETVFM | 66 |
| A2-9 | FVIII 677-696 | TLFPFSGETVFMSMENPGLW | 67 |
| | FVIII 685-704 | TVFMSMENPGLWILGCHNSD | 68 |
| | FVIII 672-691 | PFSGETVFMSMENPGLWILG | 69 |
| | FVIII 685-704 | PGLWILGCHNSDFRNRGMTA | 70 |
| | FVIII 693-712 | HNSDFRNRGMTALLKVSSCD | 71 |
| A2-10 | FVIII 693-710* | HNSDFRNRGMTALLKVSS | 72 |
| | FVIII 701-720 | GMTALLKVSSCDKNTGDYYE | 73 |
| | FVIII 709-728 | SSCDKNTGDYYEDSYEDISA | 74 |
| | FVIII 712-731* | DKNTGDYYEDSYEDISAYLL | 75 |
| | FVIII 717-740 | DYYEDSYEDISAYLLSKNNAIEP R | 76 |
| C1-1 | FVIII 2004-2023 | EHLHAGMSTLFLVYSNKCQT | 77 |
| | FVIII 2001-2020 | LIGEHLHAGMSTLFLVYSNK | 78 |
| | FVIII 2012-2031 | TLFLVYSNKCQTPLGMASGH | 79 |
| | FVIII 2020-2039 | KCQTPLGMASGHIRDFQITA | 80 |
| | FVIII 2022-2041 | QTPLGMASGHIRDFQITASG | 81 |
| C1-2 | FVIII 2028-2147 | ASGHIRDFQITASGQYGQWA | 82 |
| | FVIII 2036-2055 | QITASGQYGQWAPKLARLHY | 83 |
| | FVIII 2044-2063 | GQWAPKLARLHYSGSINAWS | 84 |
| | FVIII 2052-2071 | RLHYSGSINAWSTKEPFSWI | 85 |
| | FVIII 2060-2079 | NAWSTKEPFSWIKVDLLAPM | 86 |
| C1-3 | FVIII 2068-2087 | FSWIKVDLLAPMIIHGIKTQ | 87 |
| | FVIII 2076-2095 | LAPMIIHGIKTQGARQKFSS | 88 |
| | FVIII 2084-2103 | IKTQGARQKFSSLYISQFII | 89 |
| | FVIII 2092-2111 | KFSSLYISQFIIMYSLDGKK | 90 |
| | FVIII 2100-2119 | QFIIMYSLDGKKWQTYRGNS | 91 |
| C1-4 | FVIII 2108-2127 | DGKKWQTYRGNSTGTLMVFF | 92 |
| | FVIII 2116-2135 | RGNSTGTLMVFFGNVDSSGI | 93 |
| | FVIII 2124-2143 | MVFFGNVDSSGIKHNIFNPP | 94 |
| | FVIII 2132-2151 | SSGIKHNIFNPPIIARYIRL | 95 |
| | FVIII 2140-2159 | FNPPIIARYIRLHPTHYSIR | 96 |
| C1-5 | FVIII 2148-2167 | YIRLHPTHYSIRSTLRMELM | 97 |
| | FVIII 2154-2173 | THYSIRSTLRMELMGCDLNS | 98 |

**Table 5: Tetramers for TGEM**

| **0101 Tetramers** | **1001 Tetramers** |
|---|---|
| A2 pool 1 (9/8/09) | A2 pool 1 (9/8/09) |
| A2 pool 2 (9/8/09) | A2 pool 2 (9/8/09) |
| A2 pool 3 (9/8/09) | A2 pool 3 (9/8/09) |
| A2 pool 4 (9/8/09) | A2 pool 4 (9/8/09) |
| A2 pool 5 (9/8/09) | A2 pool 5 (9/8/09) |
| A2 pool 6 (9/8/09) | A2 pool 6 (9/8/09) |
| A2 pool 7 (9/8/09) | A2 pool 7 (9/8/09) |
| A2 pool 8 (9/8/09) | A2 pool 8 (9/8/09) |
| A2 pool 9 (9/8/09) | A2 pool 9 (9/8/09) |
| A2 pool 10 (9/8/09) | A2 pool 10 (9/8/09) |
| C1 pool 1 (9/8/09) | C1 pool 1 (9/8/09) |
| C1 pool 2 (9/8/09) | C1 pool 2 (9/8/09) |
| C1 pool 3 (9/8/09) | C1 pool 3 (9/8/09) |
| C1 pool 4 (9/8/09) | C1 pool 4 (9/8/09) |
| C1 pool 5 (9/8/09) | C1 pool 5 (9/8/09) |
| C2 pool 1 NEW (9/8/09) | C2 pool 1 NEW (9/8/09) |
| C2 pool 2 NEW (9/8/09) | C2 pool 2 NEW (9/8/09) |
| C2 pool 3 NEW (9/8/09) | C2 pool 3 NEW (9/8/09) |
| C2 pool 4 NEW (9/8/09) | C2 pool 4 NEW (9/8/09) |
| TT586 (4/20/10) | TT482 (4/20/10) |
| TT666 (4/20/10) | TT554 (4/20/10) |
| TT674 (4/20/10) | |

### OTHER REAGENTS:

CD4 T cell isolation kit II human, 1x1 ml, 1x2ml (Miltenyi Biotec, 130-091-155), stored at 4C, Lot #5090721100.

0.4 % Trypan blue (Sigma, T8154-100ML), stored at room temperature. Lot #106K2402

Human Interleukin-2, purified, 50 ml (Hemagen, Product No. 906011, Lot #6011081), stored as aliquots (1 ml or 2 ml) at -20°C.

FITC conjugated anti-human CD4 (L3T4), Clone: RPA-T4 (EBioscience, Cat#11-0049-71, Lot #E031818, 20 ul/test = 1 ug), stored at 4C

PE conjugated anti-human CD4 (L3T4) Clone: RPA-T4 (eBioscience, Cat #12-0049-71, Lot #E016770, 20 ul/test, 0.5 ml)

PerCP mouse anti-human CD3 (BD Pharmingen, Cat #552851, Lot #73100, Exp 2010-08-31, 50 tests, 1.0 ml)

APC conjugated anti-human CD4 (L3T4) Clone: RPA-T4 (eBioscience, Cat #17-0049-73, Lot #E019142, 20 ul/test, 2.0 ml)

FITC conjugated anti-human CD25 (IL-2 receptor) Clone: BC96 (eBioscience, Cat #11-0259-73, Lot #E016414, 20 ul/test, 2.0 ml). Lot #E016414 for decoding stain.

### BUFFERS AND MEDIA:

Running buffer (1X DPBS-2 mM EDTA-0.5% BSA).

15% human serum T cell media, prepared fresh.
1. Media components: a. ImmunO human serum, type AB, off clot, sterile filtered male (MP Biomedicals, 82319) at a final concentration of 15%. Lot #4738K; b. RPMI 1640 with 25 mM HEPES (Invitrogen, 22400-089); c. 200 mM L-glutamine (Invitrogen, 25030-081) at a final concentration of 2 mM; d. pencillin/streptomycin (Invitrogen, 15070-063) at a final concentration of 50 U/ml pencillin and 50 ug/ml streptomycin
2. Thawed 20-25 ml aliquot of human serum (MP Biomedicals, 82319) at 37°C.
3. At the time the media was to be prepared, warm all media components in a 37C water bath.
4. Filtered aliquot of human serum through a 0.8 micron filter (Nalgene, 115 ml, Cat.# 380-0080) and measured the volume recovered with a serological pipet. Vol = 22 ml
5. Calculated volume of media components based on the volume of serum. Media should contain 15% human serum, 1% 200 mM L-glutamine, 1% pencillin/streptomycin, and the remainder is RPMI 1640 with 25 mM HEPES (147 ml: 22 ml human serum, 1.5 ml P/S, 1.5 ml L-glutamine, 122 ml RPMI 1640).
6. To the filter unit of a 250 ml bottle (Nalgene NYL filter unit, 250 ml, 50 mm diameter membrane, 0.2 micron pore size, Cat. # 153-0020) added the components and filter. Swirl the bottle to mix the ingredients.
7. Store at 4°C in the dark

FACS wash buffer (1X DPBS-1% FBS-0.1% NaN₃)

### PROCEDURE:

### 1. Thawing of cryopreserved PBMCs from subject GS1, 056A

a. Sterilized hood. b. Warmed RPMI, 5% FBS in RPMI, and 15% human serum T cell media to 37C. c. Prepared 50:50 FBS:RPMI mix. d. Removed frozen vial of cells from cryotank. e. Transferred vials to 37°C waterbath to thaw then transferred to hood. f. Transferred the vial contents to an empty 15 ml conical tube. Added the 2 ml 50% FBS in RPMI with HEPES dropwise while gently mixing the thawed cells. Added 7 ml RPMI medium dropwise. g. Centrifuged at 1200 rpm, 8 min, room temperature, low brake to pellet cells. h. Aspirated the supernatant. i. Resuspended each cell pellets in 6 ml 5% FBS in RPMI with HEPES and transferred each to one 50 ml conical tube. The 15 ml conical tubes were rinsed with 2 ml 5% FBS in RPMI. j. Centrifuged at 1200 rpm, 10 min, room temperature, low brake to pellet the cells. k. Aspirated the supernatant. 1. Resuspended cells in each 50 ml conical tube in 7.25 ml 15% human serum T cell media. Mixed together and then divided evenly between the 2 tubes. m. Counted cells with hemocytometer.

### 2. Labeling cells for CD4 isolation

a. Placed on ice: aliquot of running buffer, CD4 no-touch isolation kit. b. Cells were pelleted by centrifugation at 1200 rpm, 10 min, 6C, low brake in the Beckman Coulter Allegra 6KR centrifuge. c. Resuspended each 10 x 10⁶ cells in 40 µl running buffer. The maximum cell number per prep is 10⁸ cells, thus I will treat each pellet separately (80.9 x 10⁶ cells/10 x 10⁶ cells/ml = 8.09 x 40 ul = 323.6 ul (x2)). d. Added 10 µl of CD4 isolation antibody cocktail for each 10 x 10⁶ cells (8.09 x 10 ul = 80.9 ul (x2)). e. Mixed by swirling. f. Incubated for 10 min at 4C by placing the tube in the refrigerator. g. Added 30 µl running buffer for each 10 x 10⁶ cells (8.09 x 30 ul = 242.7 ul (x2)). h. Added 20 µl anti-biotin microbeads for each 10 x 10⁶ cells (8.09 x 20 ul = 161.8 ul (x2)). i. Incubated for 15 min at 4C by placing the tube in the refrigerator. j. Added 2 ml running buffer for each 10 x 10⁶ cells (8.09 x 2 ml = 16 ml). k. Centrifuged at 1200 rpm, 10 min, 6C, low brake in the Beckman Coulter Allegra 6KR centrifuge. 1. Aspirated cell supernatant. m. Resuspended cells in 2.5 ml running buffer using a serological pipet.

### 3. Separation of CD4+ and CD4- cells using EasySep magnet

a. Transferred the cells to a 5 ml polypropylene round bottom tube. b. Inserted tube into the EasySep block. c. Incubated at room temperature, 15 min. d. Decanted supernatant into a 15 ml conical tube. Left the last drop behind with the CD4- cells. Pooled both samples into the same 15 ml conical tube (Decanted cells = CD4+; Cells stuck to tube = CD4-). e. CD4- cells: Resuspended in 2.5 ml T cell media and transferred to a conical tube. Rinsed the tube with another 2.5 ml T cell media for a total of 5 ml. Took a 10 µl aliquot for cell counting. f. CD4+ cells: Determined volume and took a 10 µl aliquot for counting. g. Counted cells. Need 3.0 million CD4- cells/well and 1.7 million CD4+ cells/well. Mixed 10 µl of cell sample with 10 µl 0.4% trypan blue.

### 4. Adhered CD4- cells to plate and resuspended CD4+ cells in T cell media

a. Centrifuged CD4- and CD4+cells at 1200 rpm, 10 min, 23C, low brake in the Beckman Coulter Allegra 6KR centrifuge. b. Aspirated supernatants. c. Resuspended CD4-cells at a concentration of 10 x 10⁶ cells/ml and CD4+ cells at 3.4 x 10⁶ cells/ml in T cell media. d. Aliquoted 300 µl CD4- cells (3 million cells) to wells in a 48-well plate. Aliquoted to 15 wells on 3 plates. e. Incubated at 37C, 5% CO₂, for 1 hour.

**Table 6**

| Cell Fraction | Total Cell # (x 10⁶ cells) | Concentration (x 10⁶ cells/ml) | Volume (ml) | # of wells |
|---|---|---|---|---|
| CD4- | 68.7 | 10 | 6.87 | 22.9 |
| CD4+ | 26.0 | 3.4 | 7.65 | 15.3 |

### 5. Washed CD4- adherent cells

a. Filled each well with T cell media and used a transfer pipette to wash away all non-adherent cells. Pipeted up and down 16 times with transfer pipette in a circle around the well. b. Added 200 ul fresh T cell media after washing to prevent the well from drying out.

### 6. Added T-cell, peptide, and media to adherent CD4- cells

a. Added total CD4+ cells (1.7 million) in 500 µl volume to the well containing the adherent cells. b. Added 1 µl pooled peptides at ∼5,000 uM concentration (original protocol was 10 mg/ml for 20-mers which is close to 5,000 uM. c. Added 300 µl T cell media to bring volume to 1 ml. d. Incubated at 37C 5% CO₂.

**Table 7**

| Well # | Peptide Pool | Concentration | Date |
|---|---|---|---|
| 1 | A2 pool 1 and 2 | 10,000 uM | ∼8/07 |
| 2 | A2 pool 3 and 4 | 10,000 uM | ∼8/07 |
| 3 | A2 pool 5 and 6 | 10,000 uM | ∼8/07 |
| 4 | A2 pool 7 and 8 | 10,000 uM | -8/07 |
| 5 | A2 pool 9 and 10 | 10,000 uM | -8/07 |
| 6 | C1 pool 1 | 10 mg/ml | 5/1/07 |
| 7 | C1 pool 2 | 10 mg/ml | 5/1/07 |
| 8 | C1 pool 3 | 10 mg/ml | 5/1/07 |
| 9 | C1 pool 4 | 10 mg/ml | 5/1/07 |
| 10 | C1 pool 5 | 10 mg/ml | 5/1/07 |
| 11 | C2 pool 1 NEW | 10 mg/ml? | 9/09 |
| 12 | C2 pool 2 NEW | 10 mg/ml? | 9/09 |
| 13 | C2 pool 3 NEW | 10 mg/ml? | 9/09 |
| 14 | C2 pool 4 NEW | 10 mg/ml? | 9/09 |
| 15 | 0101 1001 TT reference | ∼5,000 uM | 8/9/10 |

### 7. Froze remaining CD4- cells in 7% DMSO. 23.7 million cells were frozen.

A. Cells were pelleted by centrifugation at 1200 rpm, 10 min, 6C, low brake in the Beckman Coulter Allegra 6KR centrifuge. B. Aspirated cell supernatant. C. Resuspended in 1000 ul cold FBS. D. Prepared 14% DMSO/FBS (210 ul DMSO and 1290 ul FBS) and chilled. E. Generated labels and chilled vials. F. Added freezing media dropwise to cells. G. Aliquoted 1 ml to 2 vials (11.8 million/vial). H. Placed in freezing container O/N at -80C. I. Transferred to liquid nitrogen storage the next day.

### 8. Added IL-2

a. Observed cells. Cells looked healthy in all wells. b. Added 50 µl of warmed IL-2 to each well. Pipetted into the supernatant at the top of the well and didn't mix the cells.

### 9. Expansion of the cells with IL-2 as described (e.g. Ettinger et al., 2009)

### 10. Day 14: staining with pooled tetramers

### Harvest cells for tetramer staining

a. Removed media from the well until the remaining volume was approximately 0.5 ml. b. Resuspended the cells in each well. c. Transferred 75µl of cells from each well to a labeled FACS tube according to the experimental plan below. d. Used tetanus texoid stimulated cells for compensation stains: unstained cells, CD4-FITC, CD4-PE, CD3-PerCP, and CD4-APC. e. The media removed was added back after the cell aliquots were taken.

### Tetramer staining

a. Added 1.5 µl PE-labeled tetramers (final concentration 10 µg/ml. b. Mixed by shaking the rack containing the FACS tubes. c. Incubated the cells with tetramer for 1 hr in the 37C 5% CO₂ incubator.

### Antibody staining

a. Incubated tubes in the refrigerator for >5 min. b. Made an antibody cocktail consisting of 3.75 µl anti-CD4-APC, 3.75 µl anti-CD3-PerCP, 3.75 µl anti-CD25-FITC per sample. 3.75 ul x 50 = 187.5 ul. c. Added 3.75 µl control antibodies to 75 µl control cells (1 - unstained; 2- anti-CD4-FITC; 3-anti-CD4-PE; 4- anti-CD3-PerCP; 5- anti-CD4-APC). d. Added 11.25 µl antibody cocktail to each sample. e. Incubated all samples at 4C (put in the refrigerator) for 20 min in the dark.

### Washed samples

a. Added 2 ml cold FACS wash buffer to each tube. b. Centrifuged at 1200 rpm, 10 min, 4C, low brake in the Beckman Coulter Allegra 6KR centrifuge. c. Decanted the supernatant. d. Resuspended in 200 µl FACS wash buffer. e. Stored tubes in a covered ice container for FACS analysis.

**Table 8**

| Sample # | Cells (75 ul) | PE-Tetramers (1.5 ul)* | Antibody | FACS File | Events |
|---|---|---|---|---|---|
| Unstained | TT | | none | 082310C1.002 | 10000 |
| FITC | TT | | 3.75 ul CD4-FITC | 082310C1.003 | 10000 |
| PE | TT | | 3.75 ul CD4-PE | 082310C1.004 | 10000 |
| PerCP | TT | | 3.75 ul C D3-PerCP | 082310C1.005 | 10000 |
| APC | TT | | 3.75 ul CD4-APC | 082310C1.006 | 10000 |
| 1 | A2 pool 1 & 2 | DR0101 A2 pool 1 | 11.25 ul Ab cocktail | 082310C1.007 | 25000 |
| 2 | A2 pool 1 & 2 | DR0101 A2 pool 2 | 11.25 ul Ab cocktail | 082310C1.008 | 25000 |
| 3 | A2 pool 3 & 4 | DR0101 A2 pool 3 | 11.25 ul Ab cocktail | 082310C1.009 | 25000 |
| 4 | A2 pool 3 & 4 | DR0101 A2 pool 4 | 11.25 ul Ab cocktail | 082310C1.010 | 25000 |
| 5 | A2 pool 5 & 6 | DR0101 A2 pool 5 | 11.25 ul Ab cocktail | 082310C1.011 | 25000 |
| 6 | A2 pool 5 & 6 | DR0101 A2 pool 6 | 11.25 ul Ab cocktail | 082310C1.012 | 25000 |
| 7 | A2 pool 7 & 8 | DR0101 A2 pool 7 | 11.25 ul Ab cocktail | 082310C1.0131 | 25000 |
| 8 | A2 pool 7 & 8 | DR0101 A2 pool 8 | 11.25 ul Ab cocktail | 082310C1.014 | 25000 |
| 9 | A2 pool 9 & 10 | DR0101 A2 pool 9 | 11.25 ul Ab cocktail | 082310C1.015 | 25000 |
| 10 | A2 pool 9 & 10 | DR0101 A2 pool 10 | 11.25 ul Ab cocktail | 082310C1.016 | 25000 |
| 11 | C1 pool 1 | DR0101 C1 pool 1 | 11.25 ul Ab cocktail | 082310C1.017 | 25000 |
| 12 | C1 pool 2 | DR0101 C1 pool 2 | 11.25 ul Ab cocktail | 082310C1.018 | 25000 |
| 13 | C1 pool 3 | DR0101 C1 pool 3 | 11.25 ul Ab cocktail | 082310C1.019 | 25000 |
| 14 | C1 pool 4 | DR0101 C1 pool 4 | 11.25 ul Ab cocktail | 082310C1.020 | 25000 |
| 15 | C1 pool 5 | DR0101 C1 pool 5 | 11.25 ul Ab cocktail | 082310C1.021 | 25000 |
| 16 | C2 pool 1 | DR0101 C2 pool 1 | 11.25 ul Ab cocktail | 082310C1.022 | 25000 |
| 17 | C2 pool 2 | DR0101 C2 pool 2 | 11.25 ul Ab cocktail | 082310C1.023 | 25000 |
| 18 | C2 pool 3 | DR0101 C2 pool 3 | 11.25 ul Ab cocktail | 082310C1.024 | 25000 |
| 19 | C2 pool 4 | DR0101 C2 pool 4 | 11.25 ul Ab cocktail | 082310C1.025 | 25000 |
| 20 | TT | DR0101 TT586, TT666, TT674 | 11.25 ul Ab cocktail | 082310C1.026 | 25000 |
| 21 | A2 pool 1 & 2 | DR1001 A2 pool 1 | 11.25 ul Ab cocktail | 082310C1.027 | 25000 |
| 22 | A2 pool 1 & 2 | DR1001 A2 pool 2 | 11.25 ul Ab cocktail | 082310C1.028 | 25000 |
| 23 | A2 pool 3 & 4 | DR1001 A2 pool 3 | 11.25 ul Ab cocktail | 082310C1.029 | 25000 |
| 24 | A2 pool 3 & 4 | DR1001 A2 pool 4 | 11.25 ul Ab cocktail | 082310C1.030 | 25000 |
| 25 | A2 pool 5 & 6 | DR1001 A2 pool 5 | 11.25 ul Ab cocktail | 082310C1.031 | 25000 |
| 26 | A2 pool 5 & 6 | DR1001 A2 pool 6 | 11.25 ul Ab cocktail | 082310C1.032 | 25000 |
| 27 | A2 pool 7 & 8 | DR1001 A2 pool 7 | 11.25 ul Ab cocktail | 082310C1.033 | 25000 |
| 28 | A2 pool 7 & 8 | DR1001 A2 pool 8 | 11.25 ul Ab cocktail | 082310C1.034 | 25000 |
| 29 | A2 pool 9 & 10 | DR1001 A2 pool 9 | 11.25 ul Ab cocktail | 082310C1.035 | 25000 |
| 30 | A2 pool 9 & 10 | DR1001 A2 pool 10 | 11.25 ul Ab cocktai | 082310C1.036 | 25000 |
| 31 | C1 pool 1 | DR1001 C1 pool 1 | 11.25 ul Ab cocktail | 082310C1.037 | 25000 |
| 32 | C1 pool 2 | DR1001 C1 pool 2 | 11.25 ul Ab cocktail | 082310C1.038 | 25000 |
| 33 | C1 pool 3 | DR1001 C1 pool 3 | 11.25 ul Ab cocktail | 082310C1.039 | 25000 |
| 34 | C1 pool 4 | DR1001 C1 pool 4 | 11.25 ul Ab cocktail | 082310C1.040 | 25000 |
| 35 | C1 pool 5 | DR1001 C1 pool 5 | 11.25 ul Ab cocktail | 082310C1.041 | 25000 |
| 36 | C2 pool 1 | DR1001 C2 pool 1 | 11.25 ul Ab cocktail | 082310C1.042 | 25000 |
| 37 | C2 pool 2 | DR1001 C2 pool 2 | 11.25 ul Ab cocktail | 082310C1.043 | 25000 |
| 38 | C2 pool 3 | 3.0 ul DR1001 C2 pool 3 | 11.25 ul Ab cocktail | 082310C1.044 | 25000 |
| 39 | C2 pool 4 | DR1001 C2 pool 4 | 11.25 ul Ab cocktail | 082310C1.045 | 25000 |
| 40 | TT | DR1001 TT482, TT554 | 11.25 ul Ab cocktail | 082310C1.046 | 25000 |

| | | | | | |
|---|---|---|---|---|---|
| 3.0 ul DR1001 C2 pool 3 tetramer was used because a precipitate was observed in the bottom of the tube. The supernatant was still pink. | | | | | |

Performed FACS analysis on the FACSCaliber in the PSBC Flow Lab

### 11. Expansion of the cells (continued)

After finished the flow analysis, fed the cells in order to keep them in culture until decoding could be completed. All wells were given 500 ul of T cell media containing a 1:10 dilution of IL-2.

### RESULTS of pooled tetramer staining

The FACS data were analyzed using FlowJo. Positive pools are noted in the table below. Positive pools presumably contain at least one peptide with an HLA-restricted FVIII T-cell epitope.

**Table 9**

| DR Tetramer | Pool | Peptide | FVIII Residues |
|---|---|---|---|
| DR0101 | A2-4 | A2-16 | FVIII 493-512 |
| | | A2-17 | FVIII 501-520 |
| | | A2-18 | FVIII 508-527 |
| | | A2-19 | FVIII 517-536 |
| | | A2-20 | FVIII 525-544 |
| DR0101 | C1-3 | C1-11 | FVIII 2068-2087 |
| | | C1-12 | FVIII 2076-2095 |
| | | C1-13 | FVIII 2084-2103 |
| | | C1-14 | FVIII 2092-2111 |
| | | C1-15 | FVIII 2100-2119 |
| DR0101 | C2-1 NEW | C2-1 | FVIII 2170-2189 |
| | | C2-2 | FVIII 2178-2197 |
| | | C2-3B | FVIII 2186-2205 |
| | | C2-4 | FVIII 2194-2213 |
| | | C2-5 | FVIII 2202-2221 |
| | | | |
| DR1001 | A2-4 | A2-16 | FVIII 493-512 |
| | | A2-17 | FVIII 501-520 |
| | | A2-18 | FVIII 509-528 |
| | | A2-19 | FVIII 517-536 |
| | | A2-20 | FVIII 525-544 |
| DR1001 | C1-3 | C1-11 | FVIII 2068-2087 |
| | | C1-12 | FVIII 2076-2095 |
| | | C1-13 | FVIII 2084-2103 |
| | | C1-14 | FVIII 2092-2111 |
| | | C1-15 | FVIII 2100-2119 |
| DR1001 | C2-3 NEW | C2-11 | FVIII 2250-2269 |
| | | C2-12 | FVIII 2258-2277 |
| | | C2-13 | FVIII 2265-2284 |
| | | C2-14 | FVIII 2273-2292 |
| | | C2-15 | FVIII 2281-2300 |

### PROCEDURE (continued)

### 12. Expansion of the cells by adding 1:10 dilution of IL-2

### 13. Day 18: Individual tetramer staining to decode pools

### Harvest cells for tetramer staining

a. The media volume was adjusted depending on the volume of cells needed for the experiment and the number of wells available. b. Resuspended the cells in each well. c. Transferred 75µl of cells from each well to a labeled FACS tube according to the experimental plan below. d. Used tetanus texoid stimulated cells for compensation stains: unstained cells, CD4-FITC, CD4-PE, CD3-PerCP, and CD4-APC. e. Added back the media to the cells.

### Tetramer staining

a. Added 1.5 µl PE-labeled tetramers (final concentration 10 µg/ml). b. Mixed by shaking the rack containing the FACS tubes. c. Incubated the cells with tetramer for 1 hr in the 37C 5% CO₂ incubator.

### Antibody staining

a. Incubated tubes in the refrigerator for >5 min. b. Made an antibody cocktail consisting of 3.75 µl anti-CD4-APC, 3.75 µl anti-CD3-PerCP, 3.75 µl anti-CD25-FITC per sample. 3.75 ul x 55 = 206.25 ul (For the CD25-FITC Ab, I opened a new vial (Lot #E016414) - taking 100 ul from this lot.) c. Added 3.75 µl control antibodies to 75 µl control cells (1 - unstained; 2- anti-CD4-FITC; 3- anti-CD4-PE; 4- anti-CD3-PerCP; 5- anti-CD4-APC). d. Added 11.25 µl antibody cocktail to each sample. e. Incubated all samples at 4C (put in the refrigerator) for 20 min in the dark.

### Washed samples

a. Added 2 ml cold FACS wash buffer to each tube. b. Centrifuged at 1200 rpm, 10 min, 4C, low brake in the Beckman Coulter Allegra 6KR centrifuge. c. Decanted the supernatant. d. Resuspended in 250 µl FACS wash buffer. e. Stored tubes in a covered ice container for FACS analysis.

Performed FACS analysis on the FACSCaliber in the PSBC Flow Lab

### RESULTS of staining with individual peptide loaded tetramers

The FACS data was analyzed using FlowJo.

**Table 10: Summary of results of individual peptide loaded tetramer staining**

| DR Tetramer | Pool | Peptide | FVIII Residues | Positive |
|---|---|---|---|---|
| DR0101 | A2-4 | **Pool** | | **yes** |
| | | A2-16 | FVIII 493-512 | no |
| | | A2-17 | FVIII 501-520 | no |
| | | **A2-18** | **FVIII 508-527** | **yes** |
| | | A2-19 | FVIII 517-536 | no |
| | | A2-20 | FVIII 525-544 | no |
| DR0101 | C1-3 | **Pool** | | **yes** |
| | | C1-11 | FVIII 2068-2087 | no |
| | | C1-12 | FVIII 2076-2095 | no |
| | | C1-13 | FVIII 2084-2103 | no |
| | | C1-14 | FVIII 2092-2111 | no |
| | | C1-15 | FVIII 2100-2119 | no |
| DR0101 | C2-1 NEW | **Pool** | | **yes** |
| | | C2-1 | FVIII 2170-2189 | no |
| | | C2-2 | FVIII 2178-2197 | no |
| | | **C2-3B** | **FVIII 2186-2205** | **weak** |
| | | C2-4 | FVIII 2194-2213 | yes |
| | | C2-5 | FVIII 2202-2221 | no |
| | | | | |
| DR1001 | A2-4 | **Pool** | | **yes** |
| | | A2-16 | FVIII 493-512 | no |
| | | A2-17 | FVIII 501-520 | no |
| | | **A2-18** | **FVIII 509-528** | **yes** |
| | | A2-19 | FVIII 517-536 | no |
| | | A2-20 | FVIII 525-544 | no |
| DR1001 | C1-3 | **Pool** | | **yes** |
| | | C1-11 | FVIII 2068-2087 | no |
| | | C1-12 | FVIII 2076-2095 | no |
| | | C1-13 | FVIII 2084-2103 | no |
| | | C1-14 | FVIII 2092-2111 | no |
| | | C1-15 | FVIII 2100-2119 | no |
| DR1001 | C2-3 NEW | Pool | | no |
| | | C2-11 | FVIII 2250-2269 | no |
| | | C2-12 | FVIII 2258-2277 | no |
| | | C2-13 | FVIII 2265-2284 | no |
| | | C2-14 | FVIII 2273-2292 | no |
| | | C2-15 | FVIII 2281-2300 | no |

### DISCUSSION

The results showed that there are a limited number of epitopes provoking strong T cell responses for this severe hemophilia subject with a very high titer inhibitor. *See* **Table 10** and **Figures 11-12A****.** Responses to tetanus toxin provided a positive control for tetramer staining **(****Figure 12B****).** The T cell epitopes identified in this subject were: DR0101-FVIII 508-527, DR0101-FVIII 2194-2213, and DR1001-FVIII 508-527. (Note: the staining of DR0101 and DR1001 by FVIII 508-527 may have been nonspecific.)

There were a few other weak positives indicating genuine T-cell epitopes. These possible weak positives included epitopes that were not revealed by decoding the C1-3 pool (DR0101-restricted), C1-3 pool (DR1001-restricted), and C2-3 pool (DR1001-restricted). There were also a few other possible positives among the pooled tetramer results. These samples showed tetramer+ CD4+ staining between 0.5-1 %. These were: For DR0101: A2 pool 1, A2 pool 2, A2 pool 6, and C1 pool 4. For DR1001: A2 pool 1, A2 pool 2, A2 pool 8, and C2 pool 1.

DR0101-restricted responses to peptide FVIII 2194-2213 indicated a DRB1*0101-restricted response to the same T cell epitope that we identified previously in mild hemophilia A subjects (James et al., 2007; Ettinger et al., 2009; Ettinger et al., 2010).

DR0101-restricted and DR1001-restricted FVIII 508-527 is a newly identified T cell epitope. Very strong staining was observed both with the A2-4 pool and for the A2-18 peptide (FVIII 508-527) loaded on both DR0101 and DR1001.

### Example 4

The most prevalent complication encountered when hemophilia A patients receive infusions of factor VIII (FVIII) is the development of antibodies that neutralize the pro-coagulant function of FVIII. These antibodies, commonly referred to as "inhibitors", develop in approximately 25-30% of severe hemophilia A patients^{1,2}. They can also occur in individuals with mild or moderate hemophilia A³ and in nonhemophilic individuals who develop immunity to their own FVIII⁴. The resulting bleeding disorders are difficult and extremely expensive to treat. There is a compelling need for improved therapies to reduce the incidence of inhibitors and to provide effective alternative treatments when they do occur. Development of anti-FVIII antibodies depends on the involvement of T cells^{5,6}; FVIII-activated T cells stimulate B cells, which then secrete anti-FVIII IgG. Antibodies that bind to functionally important regions, e.g. surfaces where thrombin or activated factor X bind to FVIII and activate it proteolytically, or where activated FVIII (FVIIIa) attaches to platelet membranes, von Willebrand factor (VWF) or components of the intrinsic factor X activating complex, constitute a subset of anti-FVIII IgGs that inhibit its cofactor activity. Identification of specific amino acids essential for formation of FVIII-IgG complexes, as well as those involved in B- and T-cell signaling, will increase our understanding of molecular mechanisms underlying these immune reactions and indicate sites that could be modified to produce less immunologically reactive FVIII proteins.

In attempting to evaluate the feasibility of modifying specific residues in FVIII to evade inhibitory antibodies, it would be helpful to know (a) how many amino acids contribute significantly to antigen-antibody complex formation, and (b) their relative contributions to the total binding energy. The human monoclonal antibody B2C11 is an IgG4κ purified from the supernatant of an EBV-immortalized human B-cell line derived from an inhibitor subject's blood⁷; this inhibitory IgG bound to FVIII with an association rate constant *kₐ* ∼ 7.4 x 10⁵ *M*⁻*¹ s*⁻¹, and dissociation rate constants *k_{d}* ≤ 1 x 10⁻⁵ *s⁻¹*, yielding a *K_{D} = k_{d}*/*kₐ* = 1.4 x 10⁻¹¹ *M*⁻*¹ s⁻¹*. B02C11 binds to the FVIII C2 domain, interfering with its attachment to activated phospholipid membranes and to VWF⁷. A 2.0 *Å* resolution crystal structure of the BO2C11 Fab fragment bound to the FVIII C2 domain⁸ identifies all intermolecular contacts between this antibody and FVIII.

The interface buries approximately 1200 *Å*² of each molecular surface and includes extensive hydrophobic interactions, as well as a network of hydrogen and ionic bonds. In order to determine which interactions were most responsible for the strong binding affinity, as well as the relative importance of hydrophobic versus ionic and polar interactions, a series of 50 recombinant C2 proteins was generated, each with a single surface residue changed to alanine and/or to another residue, including hemophilic substitutions S2173I, A2201P, V2223M, P2300L and R2307Q⁹. Effects on binding to the BO2C11 Fab fragment were evaluated by surface plasmon resonance (SPR). Substitutions that markedly altered the BO2C11-C2 affinity were investigated further by SPR experiments carried out at several temperatures followed by van't Hoff analysis to estimate the relative thermodynamic contributions of side chains within the epitope.

### Materials and Methods:

*Reagents:* BugBuster Extraction reagent, *E. coli* strain OrigamiB(DE3)pLysS, Benzonase Nuclease and rLysozyme Solution were from Novagen (San Diego, CA). Quikchange kits were from Stratagene (La Jolla, CA). Concentrations of protein solutions with A₂₈₀ < 0.2 were determined using the DC protein microplate assay kit from BioRad (Hercules, CA). 1-ethyl-3-(3-dimethylaminopropyl) carbodiimide HCl (EDC), N-hydroxysuccinimide (NHS), and ethanolamine and CM5 sensor chips, HBS-EP+ running buffer, and glycine (pH 1.5 and 2.0) regeneration solutions were from Biacore Life Sciences (Piscataway, NJ).

*Antibodies:* BO2C11 was purified from the supernatant of a human hybridoma cell line as described⁷. Its Fab was produced by papain digestion and stored at -80°C; purity was judged to be ≥95% by SDS-polyacrylamide gel electrophoresis. Murine anti-FVIII C2 domain monoclonal antibodies ESH4 and ESH8 were from American Diagnostica (Stamford, CT), while monoclonal antibodies 2-77, 2-117, 3D12,154 and I109¹⁰ were kindly provided by Dr. Pete Lollar.

*Recombinant proteins:* FVIII C2 proteins were produced in *E. coli.* The wild-type C2 (WT-C2) sequence, consisting of residues 2170-2332, was amplified from a puc18-C2 plasmid¹¹ using PCR primers introducing a 5' NdeI restriction site and a 3' BamHI restriction site: forward: 5'-GGCGCGCATATGGATTTAAATAGTTGCAGCATG (SEQ ID NO:99); reverse: 3'-GGCGCGGGATCCCTAGTAGAGGTCCTGTGC (SEQ ID NO:100). The PCR product was digested with NdeI and BamHI (New England Biolabs, Ipswich, MA) and subcloned into expression vector pet16b(+) (Novagen, San Diego, CA), linearized by digestion with the same enzymes, to make pET16b-WTC2. Pet16b introduces an N-terminal extension of 10 His residues. Mutations to introduce single amino acid substitutions were designed after calculating solvent exposures of all amino acid residues from the FVIII C2 domain crystal structure¹² using the program Stride¹³. Fifty C2 constructs with a single surface-exposed residue changed to alanine and/or another residue were generated **(Table 11)** using the QuikChange protocol (Stratagene, La Jolla, CA); mutagenesis primers are in **Tables 13-14.** The pet16b-C2 plasmids were purified by minipreps (Qiagen, Valencia, CA) and all C2 sequences verified by DNA sequencing. The host strain *E*. *coli* OrigamiB(DE3)pLysS (Novagen) was transfected by adding 20 µl of a log phase culture grown in Luria Broth (LB) to 1 µl of each pet16-C2 plasmid (miniprep DNA diluted 1:5 in distilled water), incubating this mixture for 30 s at 42 °C followed by 2 min on ice; 80 µl SOC medium was added and cultures were shaken at 37°C for 1 hr and plated on LB/agar plates containing 75 µg/mL carbenicillin, 34 µg/mL chloramphenicol. The plates were incubated at 37°C overnight, then five colonies were picked for each mutant and ten-mL cultures grown overnight in LB plus carbenicillin (75 µg/mL) and chloramphenicol (34 µg/mL). Three mL of each culture was added to 150 mL LB and shaken at 37°C to log-phase growth, 150 ul 1M IPTG was added, and the culture was shaken for 15-20 minutes at 37°C, then at 8°C or 16°C overnight. Cells were pelleted at 4000g for 20 minutes, the supernatant discarded, and 4 mL Bug Buster extraction reagent plus 4 µl benzonase (250 U/µl), 0.133 µl rLysozyme (300kU/µl) and 2.5% v/v glycerol was added. Cells were carefully resuspended and stirred for 20 minutes at room temperature, then centrifuged at 16,000 x g for 30 minutes at 4°C and the supernatant was applied to a His-Bind purification column (Novagen). The eluate was dialyzed against 4L 20 mM Tris HCl, 150 mM NaCl, 2.5% v/v glycerol, pH 7.4 two times, then against 4L 10 mM HEPES, 150 mM NaCl and 2.5% v/v glycerol, pH 7.4. Sodium azide was added to 0.015% (w/v). Several mutant proteins tended to precipitate, so all samples were spun in a benchtop centrifuge following dialysis for 2 min at 13,000 rpm, and soluble protein in the supernatant was diluted to ∼0.1 mg/mL and stored at 4°C. Protein concentrations were determined by Absorbance at 280 nm, using a calculated extinction coefficient of ε^{280nm,0.1%} = 1.8¹².

Expression and refolding protocols were optimized for several mutant proteins as follows: (1) after IPTG induction, cell cultures were grown at 4°C overnight and glycerol was not added to the extraction reagent; the His-Bind column eluate was dialyzed against 1L 20 mM Tris HCl, 500mM NaCl and 0.3% N-lauroylsarcosine pH 7.9 for 2-3 hours. The buffer was diluted sequentially by adding 500ml 20 mM Tris HCl, 60mM NaCl and 0.3% N-Lauroylsarcosine pH 7.9 every 2-3 hours to 4L. The sample was then dialyzed twice against 4L 20 mM Tris HCl, 60mM NaCl and 0.3% N-lauroylsarcosine pH7.9. Samples were assessed for possible aggregation by size-exclusion chromatography on a Superdex 75 column (GE); each C2 protein eluted as a single major UV peak (>88% of total area under peaks); retention times were compared to those of typical standards run on the same column (aprotinin: 6.5 kDa, cytochrome c: 12.4 kDa, and carbonic anhydrase: 29 kDa), and each eluted at a position consistent with the C2 monomer size. Larger molecular-weight peaks each comprised < 1% of the total area under peaks in the chromatogram. SDS-PAGE of C2 proteins purified on the nickel column indicated they were ≥ 90% pure (Figure 13) so SPR analysis was carried out without further purification. Human factor VIII was from outdated therapeutic vials of Kogenate FS manufactured by Bayer HealthCare. (Lot #: 27NON51, Exp: Jan. 20^{th}, 2007). FVIII concentrations were determined by microplate assay using the BioRad DC protein assay kit according to the manufacturer's instructions.

*Surface plasmon resonance:* All SPR measurements were carried out on a Biacore T-100 instrument (Biacore Life Sciences). The B02C11 Fab fragment was immobilized covalently on a CM5 chip by amine derivatization, following the manufacturer's suggested protocol. The BO2C11 Fab fragment (3.9 mg/mL) was diluted 200-fold in 10 mM sodium acetate pH 5.0. The Fab was immobilized by amine coupling, using 1-Ethyl-3-(3-dimethylaminopropyl)carbodiimide-HCl and N-hydroxysuccinimide (Biacore) to activate the CM5 surface, then injecting the protein serially in Running Buffer HBS-EP+ until 300 ± 5 resonance units (RUs) corresponding to immobilized protein were recorded, at which point 35 µl ethanolamine was injected to quench the reactive sites on the chip. This immobilization level yielded Rmax values between 30 and 100 RU (most were between 50-75 RU). These relatively low Rmax values circumvented potential complications associated with mass transport limitation, which were a concern due to the high association rate of the B02C11-C2 complex. A reference flow cell was created by activating and then immediately deactivating the surface without exposing it to the BO2C11 Fab. C2 mutant proteins at 0.4 to 50 nM were injected for at least 120 seconds, and dissociation was monitored for 300-900 seconds to determine the association and dissociation rate constants, respectively. All experiments were carried out at 25°C. Regeneration of the BO2C11 surface was accomplished by injecting 10mM glycine-HCl, pH 2.0 for 30-60 seconds. Resonance signals after regeneration injections were monitored to ensure complete dissociation of the C2 protein before initiating the next experiment. The sensorgrams were subtracted from the reference flow cell signal, subsequently subtracted from a blank run signal, then subjected to a curve fit analysis using Biacore Evaluation Software version 2.0.1, using a 1:1 binding model. C2 mutant proteins with a dissociation constant (*k_{d}*) at least four times higher than the average *k_{d}* for WT-C2 were further evaluated by SPR runs at several temperatures to determine the relative contributions of enthalpy and entropy to the binding free energy.

*Van't Hoff analysis:* Thermodynamic analysis was carried out using the Biacore T100 Evaluation Software, version 2.0.1. On and off rates for C2 -BO2C11 Fab binding were obtained by fitting sensorgrams to a theoretical 1:1 binding curve, and the resulting *kₐ* and *k_{d}* constants were converted to dissociation equilibrium constants, *K_{D} = k_{d}*/*kₐ*.¹⁴ SPR measurements were executed from 10 to 40°C in increments of 5°C for mutant C2 proteins that had dissociation constants greater than 4X that of the WT-C2 at 25°C and for several other mutants. Van't Hoff plots were generated by plotting In *K_{D}* versus 1/*T*, and thermodynamic values were obtained utilizing the relationship: In *K_{D}* = (*ΔH_{A}^{o}*/*RT*) *-* (*ΔS_{A}^{o}*/*R*), where *ΔH_{A}^{o}* and *ΔS_{A}^{o}* are enthalpy and entropy, respectively, at standard conditions (25°C and 1 atm), R is the gas constant and T is the temperature in kelvin. Linear data fitting allowed calculation of standard enthalpies from the slopes (*ΔH_{A}^{o}*/*R*) and standard entropies were obtained from the y-intercepts (*-ΔS_{A}^{o}*/*R*)¹⁵*.* The Gibbs free energy (*ΔG_{A}^{o} = ΔH_{A}^{o}- TΔS_{A}^{o}*) was calculated for each experiment, and the wild-type *ΔG_{A}^{o}* was subtracted from the *ΔG_{A}^{o}* for each mutant protein (*ΔΔG_{A}^{o}* = *ΔG_{A}^{o}* (mutant) - *ΔG_{A}^{o}* (wild-type)); the resulting *ΔΔG_{A}^{o}* values estimate the energetic cost of the corresponding amino acid substitutions. Substitutions that resulted in increased free energy or enthalpy relative to the values of these parameters for WT-C2 *(i. e. ΔΔG_{A}^{o}* or *ΔΔH_{A}^{o}*> 0) reflected a loss of binding energy or enthalpy, respectively, when the side chain was altered, while substitutions that decreased the entropy (*Δ*(*TΔS_{A}^{o}*) < 0) indicated that binding of the mutant protein to the BO2C11 Fab was less entropically driven than wild-type C2 binding. To test the structural integrity of the mutant proteins, SPR runs were carried out to determine the kinetics of their binding to a series of monoclonal antibodies that recognize distinct epitopes on the FVIII C2 domain: ESH4, ESH8, 2-77, 2-117, 3D12, 154, and I109, which were each immobilized to CM5 chips as described above.

### Results

*C2 proteins and SPR conditions:* The 50 proteins listed in **Table 11** were produced from *E. coli* with purity estimated by SDS-PAGE to be ≥ 90% **(****Figure 13****),** although the yields of several were lowered due to decreased secretion and/or partial precipitation after elution from the nickel column. Four additional constructs were not secreted at detectable levels (not shown). Proteins that tended to precipitate were stored at concentrations below 0.2 mg/mL and their concentrations were verified by a BioRad DC microplate assay immediately before dilution for SPR runs. Several mutant proteins displayed anomalous *kₐ* values, which could indicate the presence of impurities that bound to the antibody nonspecifically, or else they might indicate slight conformational changes in C2 affecting the antigen-antibody association. FVIII binding to the reference cell was not detected. Occasionally, the sensorgram corresponding to the highest concentration was omitted from the analysis due to apparent non-specific binding, but in these cases the binding curves at lower C2 concentrations were adequate to calculate reliable rate constants. Although these possible sources of error in measuring kinetic rates should be small for a series of similar, purified proteins, the dissociation constants were selected as the most reliable metric to follow in comparing effects of amino acid substitutions. All C2 proteins bound to at least three additional anti-C2 monoclonal antibodies with kinetics highly similar to those for WT-C2 binding (not shown), indicating that structural changes resulting from the single amino acid substitutions were not global.

*Epitope Mapping based on Dissociation Rate Constants (k_{d})*: The B02C11-FVIII complex has a very slow dissociation rate constant *k_{d}* ≤ 1 x 10⁻⁵ *s-¹,⁷* and a slow dissociation rate was obtained for the C2-Fab complex, as expected (*k_{d}* = 8.5 x 10⁻⁵ *s⁻¹*). Five SPR runs were carried out to determine average kinetic constants for WT-C2 binding to the BO2C11 Fab. The kinetic constants were the same (within a factor of 2) for association periods of 120 or 300 seconds and for dissociation periods of 900 or 1800 seconds. The average *kₐ* and *k_{d}* were 9.4 x 10⁶ *M⁻¹s⁻¹* and 8.5 x 10⁻⁵ *s⁻¹,* respectively. Amino acid substitutions R2220A and R2220Q resulted in proteins with virtually no binding to the immobilized Fab. Curve fits for these mutants revealed a maximum resonance signal (Rmax) ≤12% of the Rmax for WT-C2. In contrast, all other mutant proteins had Rmax levels comparable to that of WT-C2, indicating they were capable of binding BO2C11. However, dissociation constants for some mutants were considerably higher than the *k_{d}* for WT-C2, indicating the substitutions had a pronounced effect on binding **(Table 11**). Some heterogeneity in the association rate *k*ₐ was seen in fitting the experimental data and calculated isotherms for several C2 mutants, indicating possible conformational effects or nonspecific binding of minor contaminants.

*Van't Hoff thermodynamic analysis:* Thermodynamic studies of the C2 mutants R2220A and R2220Q could not be carried out because these proteins showed virtually no binding to B02C11. Because the off rate for WT-C2 binding to the B02C11 Fab is extremely slow, faster protein dissociations, reflected by higher *k*_{d} values, indicated structural alterations at the B-cell epitope; these were apparent upon visual inspection of the sensorgrams **(****Figure 14****).** No mutant proteins had a significantly altered *k*ₐ with a wild-type *k*_{d}, although in principle this type of pattern could occur. In order to determine the temperature dependence of the *K_{D}* values and analyze the contributions of particular residues to binding, SPR measurements were carried out from 10-40°C in 5°C increments for the five mutant C2 proteins that showed dissociation constants greater than 4X that of the WT-C2 at 25°C: C2-F2196A, C2-N2198A, C2-M2199A, C2-L2200A and C2-R2215A. All of these substitutions destabilized the Fab-C2 complex, with F2200A being the most severe (*ΔΔG_{A}^{o}*= 13 kJ/mol). Binding of each mutant protein to at least three of the antibodies ESH4, ESH8, 2-77, 2-117, 3D12,154, and I109 was essentially indistinguishable from binding of WT-C2 (data not shown), indicating the substitutions did not cause global structural perturbations.

### Discussion

The development of an immune ("inhibitor") response in hemophilia A patients treated with Factor VIII remains difficult to circumvent. Inhibitors can also occur in nonhemophilic individuals who develop an autoimmune response to their endogenous FVIII¹⁶. Immune tolerance induction and "bypass" therapies such as administration of pro-coagulant concentrates like FEIBA or activated factor VII (NovoSeven) can be extremely expensive to administer, and they yield inconsistent results¹⁷. FVIII is a highly immunogenic molecule, as evidenced by the development of anti-FVIII antibodies in both humans^{1,18} and animals¹⁹⁻²¹, even following infusion with therapeutic levels of FVIII (∼1 nM) with no adjuvant. FVIII consists of three A domains that are homologous to the copper-binding protein ceruloplasmin²², a B domain with no close homologues identified, and two C domains that are members of the discoidin family²³, arranged as follows: Al-A2-B-A3-Cl-C2²⁴. Although antibodies may bind to any region of FVIII, antibodies against the C2 domain, which contributes to attachment of FVIII to VWF, and of FVIIIa to activated platelets, thrombin, activated factor IX and factor X, are commonly found in inhibitor patients. Antibodies from inhibitor patients and from hemophilic mice have been shown to block FVIII interactions with VWF and/or phospholipid²⁵⁻³⁰. Thirty anti-C2 murine monoclonal antibodies with epitopes mapping to several distinct C2 surfaces (by ELISA and functional assays) were characterized recently by the Lollar laboratory¹⁰. Relevance of these epitopes to those recognized by human inhibitor subjects was demonstrated by ELISA assays showing competition of the human IgG samples with the monoclonal antibodies³¹ These studies clearly indicate that there are distinct B-cell epitopes on the C2 domain having clinical relevance. Epitopes have also been mapped to specific regions on the FVIII surface by other methods including ELISA assays, analysis of hybrid or domain-deleted FVIII proteins, competitive inhibition by synthetic peptides, immunoblotting and immunoprecipitation, mass spectrometry, luminex assays and phage display³²⁻⁴¹.

One approach to developing alternative therapies for inhibitor patients is to design recombinant versions of FVIII that are less immunogenic (less likely to stimulate T cells) or less antigenic (containing fewer B-cell epitopes, i.e. surfaces that bind to anti-FVIII IgG). Proteins with reduced antigenicity will by definition bind to inhibitory IgGs with lower affinity and therefore could be useful in attempting to achieve hemostasis in patients with an established inhibitor. To design such proteins, common inhibitor epitopes must be characterized by determining which amino acid residues are essential to form high-affinity antigen-antibody complexes. The present study evaluates an antigenic site on FVIII recognized by a human-derived inhibitory monoclonal IgG, BO2C11. A crystal structure of the FVIII C2 domain bound to the BO2C11 Fab fragment provides the most detailed characterization to date of a human inhibitor epitope⁸. Although this structure clearly shows which FVIII residues interact with the antibody, the contributions of particular residues to the overall affinity must be determined experimentally.

In this study, each of the 15 C2 side chains at the C2-Fab interface⁸, which buries 1200 *Å*² of each protein surface, was substituted to alanine and in some cases to another amino acid **(Table 12).** Contributions of individual residues to *kₐ* and *k_{d}* constants, and hence to the overall affinity, were estimated by SPR. Substitutions to alanine remove all interactions of the IgG with atoms beyond the beta carbon of a particular side chain. Therefore, thermodynamic analysis of alanine substitutions that affect kinetic rates can reasonably estimate the binding energy contributed by particular side chains. Substitutions at only six sites decreased the affinity for BO2C11 significantly compared to WT-C2. R2220A and R2220Q completely abrogated binding, while F2196A, N2198A, M2199A, L2200A and R2215A displayed markedly higher k_{d} values compared to WT-C2. Altered *kₐ* or *k_{d}* constants may reflect loss of a critical interaction between the substituted amino acid side chain and the antibody, or they may indicate that the substitution caused misfolding of the mutant protein. In order to confirm that altered binding kinetics were not due to major structural perturbations, binding of all C2 mutant proteins to six inhibitory anti-FVIII C2 monoclonal antibodies was evaluated by SPR. All bound to IgGs having distinct epitopes that did not overlap that of B02C11¹⁰ with affinities similar to that of WT-C2, indicating that these substitutions did not cause global misfolding.

Van't Hoff analysis allowed quantitation of energetic consequences of amino acid substitutions. C2-R2220A and C2-R2220Q could not be evaluated thermodynamically because these substitutions abrogated binding. Therefore, R2220 is considered to contribute the most binding energy, even though that energy could not be quantitated using methods described here. Although a relative order of energetic contributions was established for the other residues (F2200 > F2196 = R2215 > N2198 > M2199) the *ΔΔG_{A}^{o}* values for these substitutions were similar (approx. 11 ± 3 kJ/mol). Four of these five mutant proteins exhibited standard enthalpy values (-15 ± 3 kJ/mol) similar to that of WT-C2 (-14 kJ/mol), indicating clearly that the decreased affinity was due to an increase in the entropy of the system (mutant C2 + Fab + solvent) **(Table 12),** e.g., by allowing greater flexibility of protein side chains or backbone, or by changing the solvent exposure of hydrophobic residues and/or the ordering of water molecules. The substitution R2215A had a dramatic effect on the binding enthalpy, as expected, reflecting the salt bond between C2-R2215 and BO2C11-D52. The sum of individual *ΔΔG_{A}^{o}* values for these five alanine substitutions was calculated and compared to the measured *ΔG_{A}^{o}* (-68 ± 1 kJ/mol) for WT-C2 binding to see how well the summed contributions accounted for the overall binding energy. The sum indicated that these residues contributed approximately -53 kJ/mol, consistent with the importance of R2220, whose contribution was clearly significant but could not be measured.

Interestingly, only one of two beta-hairpin turns in C2 that comprise part of the C2-Fab interface contributes appreciably to the binding energy **(****Figure 14****).** Substitutions at L2251 and L2252 in the second hairpin turn had surprisingly little effect on the off-rate and affinity, despite its extensive contact with the antibody. The solvent accessibilities of all 15 C2 residues at the BO2C11 interface were calculated and compared to their accessibility in the crystal structure of the uncomplexed FVIII C2 protein¹². The number of crystallographically well-defined waters in the two structures may indicate entropic contributions of solvent ordering and release. The C2 crystal structure includes 46 water molecules within van der Waals distance of the C2-BO2C11 interface, and 37 water molecules were built into density at this interface in the C2-BO2C11 co-crystal structure, suggesting that release of ∼9 water molecules could contribute to the increased entropy that the SPR experiments indicate drives formation of the antigen-antibody complex.

Because the FVIII C2 domain is a beta-sandwich structure, the BO2C11 epitope is discontinuous **(****Figure 14****).** Substitutions of several amino acids in contact with the antibody had little if any effect on the k_{d} or overall affinity. Although somewhat counter-intuitive, this phenomenon has been noted previously, leading to the concept of structural versus functional epitopes⁴². Structural epitopes consist of amino acids that are buried in the interface, whereas functional epitopes are the subset of interfacial residues that contribute significantly to the binding affinity. In a seminal paper, Clackson and Wells⁴³ systematically substituted all residues on both hormone and receptor at the interface between human growth hormone (hGH) and the extracellular domain of its receptor, hGHbp. Interestingly, residues at both molecular surfaces that contributed the most binding energy formed complementary interactions, thus reinforcing the notion of functional epitopes that exist within larger buried surface areas. Such energetic "hot spots" may be predominantly hydrophobic⁴⁴ or polar⁴⁵.

Mutations at FVIII positions 2196, 2198, 2199, 2200, 2215 and 2220 resulted in diminished binding to B02C11. However, the substitution T2197A did not substantially affect the *k_{d}* **(Table 11).** The T2197 hydroxyl moiety forms a weak hydrogen bond with the Y33 hydroxyl group in BO2C11 (d = 3.4 *Å*)⁸ and is hydrogen bonded to a water molecule in the FVIII C2 domain structure¹². If Y33 in BO2C11 also forms a hydrogen bond with water in free BO2C11, then one might expect the T2197-Y33 interaction to be entropically favored, as two waters would be released. The percentage of solvent-accessible surface area (%ASA), calculated by comparing the area of each residue to values obtained for models of the same residue in a Gly-X-Gly tripeptide, was calculated for all of the C2 residues in C2 and in the BO2C11-C2 complex structures¹³. The change for T2197 (Δ%ASA₂₁₉₇ = 25.5) was smaller than for other residues in this loop (Δ%ASA for F2196, N2198, M2199, F2200 are 39.2, 46.4, 99, and 85, respectively), indicating that steric as well as entropic effects on binding may be less pronounced. Thermodynamic data for T2197A indicate that the substitution decreased binding affinity (*ΔΔG_{A}^{o}*= +4 kJ/mol) with both entropy (*Δ*(*TΔS_{A}^{o}*) = -2 kJ/mol) and enthalpy (*ΔΔH_{A}^{o}*= +2 kJ/mol) contributing to the loss.

The enthalpic and entropic changes associated with the 5 out of 6 substitutions for which significantly altered *k_{d}* values could be measured were quite comparable, and the overall energetic costs ranged from to 8 to 13 kJ/mol. The estimated contribution of these five residues to the binding energy (-53 kJ/mol) did not fully account for the standard Gibbs free energy for WT-C2 binding to B02C11 (-68 kJ/mol). Additional binding energy is clearly contributed by the sixth residue, R2220 and by additional factors, e.g. changes in flexibility and solvation. Interestingly, the substitutions M2199A and Q2270A resulted in association rate constants that were more than double that of WT-C2, indicating decreased activation energy for binding, possibly due to an induced fit between antigen and antibody, or a smaller entropy change upon binding.

Substitutions of other C2 residues in intimate contact with the antibody, notably the adjacent β hairpin consisting of S2250-T2253, did not affect measured *k_{d}* values appreciably. The S2250 hydroxyl group forms a hydrogen bond with the antibody D100 side chain carbonyl (d = 2.5 *Å*) and also interacts with the backbone nitrogen of P101 (d = 3.7 *Å*). Both C2-S2250 and BO2C11-D100 are surface-exposed, so complex formation presumably involves exchange of a hydrogen bond with bulk solvent for an intermolecular bond. The *k*_{d} for C2-52250A increased modestly relative to WT-C2, but this effect was less pronounced than those seen for substitutions in the adjacent hairpin turn. It has been suggested that hydrogen bonds contribute about 2.5 kJ/mol to protein folding when folding causes exchange of a protein-solvent hydrogen bond for one that is sequestered within the protein interior⁴⁶. The *ΔΔG_{A}^{o}* for C2-S2250A was +8 kJ/mol (from van't Hoff analysis), with an enthalpic change of +11 kJ/mol, which may reflect loss of a hydrogen bond between C2-S2250 and B02C11-D 100; solvent shielding by hydrophobic groups in the interface may strengthen this bond in the WT-C2-BO2C11 complex.

L2251 and L2252 are solvent-exposed¹², and they contact BO2C11-V2, BO2C11-Y27 and BO2C11-L32 when the C2-antibody complex is formed⁸. Although the *ΔG_{A}^{o}* values for WT-C2, C2-L2251A and C2-L2252A are similar (-68, -66, and -63 kJ/mol, respectively) the corresponding enthalpic and entropic changes are substantial. The binding enthalpy of the mutants with leucines replaced by alanines (*ΔΔH_{A}^{o}*= -17 and -35 kJ/mol for L2251A and L2252A, respectively, was much more favorable than for WT-C2. This may be due to a strengthening of nearby salt bridges and/or hydrogen bonds. However, the substitutions did not change the *K_{D}* appreciably because the entropy increase upon binding that drives complex formation for WT-C2 was diminished for the mutants (*Δ(TΔS_{A}^{o}*) = -19 and -40 kJ/mol), compensating for the favorable enthalpic effect of the substitutions. The smaller entropic contributions of C2-L2251A and C2-L2252A relative to WT-C2 were not unexpected, because alanine residues cannot present the hydrophobic surface that is a striking feature of the FVIII C2 domain structure¹². Solvent exposure of L2251 and L2252 would be expected to cause ordering of nearby water molecules, which would be released upon antibody binding, driving the binding entropically.

IgG4 antibodies such as BO2C11, which have large complementarity determining regions (CDRs) and therefore are likely to shield extensive surfaces of their targets, are common in anti-FVIII immune responses^{47,48}. Many inhibitor antibodies block FVIII binding to activated membranes and VWF, suggesting that they bind to epitopes overlapping that for BO2C11. Our results for this prototypical inhibitor suggest that a very limited number of amino acid substitutions could produce modified FVIII proteins capable of eluding antibodies that bind to similar epitopes. Clearly, many of the residues that are in intimate contact with the antibody are essentially bystanders, from an energetic standpoint, in the formation of a high-affinity complex. The present study identifies R2215 and R2220 as significant contributors to the binding of the FVIII C2 domain to B02C11.

**Table 11. Mutant and wild-type FVIII-C2 proteins with kinetic and equilibrium constants calculated assuming a 1:1 binding model. The entries in bold are the 16 substitutions at 15 positions at the BO2C11 binding interface ("contacts" defined as C2-BO2C11 interatomic d < 3.8 Å).**

| Substitution | *kₐ* (SE) | *k_{d}* (SE) | *K_{D}* |
|---|---|---|---|
| S21731 | 1.5 x 10⁷ (2.7 x 10⁵) | 1.1 X 10⁻⁴ (2.9 X 10⁻⁶) | 7.3 x 10⁻¹² |
| E2181A | 2.2 x 10⁷ (3.8 x 10⁵) | 1.9 x 10⁻⁴ (2.9 x 10⁻⁶) | 8.6 x 10⁻¹² |
| **F2196A** | **1.3 x 10⁷ (1.2 x 10⁵)** | **2.9 x 10⁻³ (2.0 x 10⁻⁵)** | **2.2 X 10⁻¹⁰** |
| **T2197A** | **4.4 x 10⁶ (1.6 x 10⁴)** | **4.3 x 10⁻⁵ (4.4 x 10⁻⁷)** | **9.8 X 10⁻¹²** |
| **N2198A** | **6.2 x 10⁶ (3.4 x 10⁴)** | **3.5 x 10⁻⁴ (2.3 x 10⁻⁴)** | **5.6 x 10⁻¹¹** |
| **M2199A** | **4.6 x 10⁷ (4.8 x 10⁵)** | **7.6 x 10⁻⁴ (6.9 X 10⁻⁶)** | **1.7 x 10⁻¹¹** |
| **F2200A** | **9.4 x 10⁶ (3.6** x **10⁴)** | **2.3 x 10⁻³ (7.9 x 10⁻⁶)** | **2.4 x 10⁻¹⁰** |
| A2201P | 8.4 x 10⁶ (5.8 x 10⁴) | 1.6 x 10⁻⁴ (1.6 x 10⁻⁶) | 1.9 x 10⁻¹¹ |
| T2202A | 6.0 x 10⁶ (2.9 x 10⁴) | 3.5 x 10⁻⁵ (1.5 x 10⁻⁷) | 5.8 x 10⁻¹² |
| K2207A | 4.6 x 10⁶ (7.0 x 10³) | 5.9 x 10⁻⁵ (1.7 x 10⁻⁷) | 1.3 x 10⁻¹¹ |
| H2211A | 7.1 x 10⁶ (2.3 x 10⁴) | 3.8 x 10⁻⁵ (2.9 x 10⁻⁷) | 5.4 x 10⁻¹² |
| L2212A | 2.3 x 107 (2.3 x 10⁵) | 4.4 x 10⁻⁵ (2.6 x 10⁻⁷) | 1.9 x 10⁻¹² |
| Q2213A | 1.4 x 10⁷ (2.6 x 10⁴) | 6.1 x 10⁻⁵ (3.1 x 10⁻⁷) | 4.4 x 10⁻¹² |
| **R2215A** | **4.1 x 10⁶ (1.2 x 10⁴)** | **6.1 x 10⁻⁴ (1.3 x 10⁻⁶)** | **1.5 x 10⁻¹⁰** |
| **R2220A** | **N/A** | **N/A** | **N/A** |
| **R2220Q** | **N/A** | **N/A** | **N/A** |
| **Q2222A** | **7.4 x 10⁶ (1.7 x 10⁵)** | **3.9 x 10⁻⁵ (2.0 x 10⁻⁷)** | **5.3 x 10⁻¹²** |
| **V2223M** | **5.7 x 10⁶ (1.6 x 10⁵)** | **3.6 x 10⁻⁵ (1.6 x 10⁻⁷)** | **6.3 x 10⁻¹²** |
| N2224A | 3.2 x 10⁷ (3.1 x 10⁵) | 8.6 x 10⁻⁵ (5.8 x 10⁻⁷) | 2.7 x 10⁻¹² |
| N2225A | 1.6 x 10⁷ (1.3 x 10⁵) | 3.9 x 10⁻⁵ (2.2 x 10⁻⁷) | 2.4 x 10⁻¹² |
| K2227A | 1.9 x 10⁷ (8.6 x 10⁴) | 6.4 x 10⁻⁵ (4.0 x 10⁻⁷) | 3.4 x 10⁻¹² |
| K2227Q | 6.8 x 10⁶ (2.9 x 10⁴) | 2.0 x 10⁻⁵ (3.1 x 10⁻⁷) | 2.9 x 10⁻¹² |
| K2249A | 6.1 x 10⁶ (1.8 x 10⁴) | 3.7 x 10⁻⁵ (2.6 x 10⁻⁷) | 6.1 x 10⁻¹² |
| **S2250A** | **4.3 x 10⁶ (1.9 x 10⁴)** | **9.4 x 10⁻⁵ (3.1 x 10⁻⁷)** | **2.2 x 10⁻¹¹** |
| **L2251A** | **8.1 x 10⁶ (2.3 x 10⁵)** | **5.0 x 10⁻⁵ (2.4 x 10⁻⁷)** | **6.2 x 10⁻¹²** |
| **L2252A** | **2.8 x 10⁷ (1.1 x 10⁵)** | **1.7 x 10⁻⁴ (6.3 x 10⁻⁷)** | **6.1 x 10⁻¹²** |
| **T2253A** | **5.3 x 10⁶ (3.4 x 10⁴)** | **5.2 x 10⁻⁵ (1.4 x 10⁻⁷)** | **9.8 x 10⁻¹²** |
| H2269A | 2.2 x 10⁷ (4.7 x 10⁵) | 1.7 x 10⁻⁴ (3.4 x 10⁻⁶) | 7.7 x 10⁻¹² |
| Q2270A | 1.2 x 10⁸ (1.6 x 10⁷) | 1.8 x 10⁻⁴ (1.7 x 10⁻⁵) | 1.5 x 10⁻¹² |
| T2272A | 1.3 x 10⁷ (4.6 x 10⁴) | 5.4 x 10⁻⁵ (2.3 x 10⁻⁷) | 4.2 x 10⁻¹² |
| L2273A | 1.1 x 10⁷ (1.4 x 10⁵) | 1.7 x 10⁻⁴ (2.6 x 10⁻⁶) | 1.5 x 10⁻¹¹ |
| N2277A | 1.2 x 10⁷ (3.0 x 10⁴) | 6.0 x 10⁻⁵ (4.8 x 10⁻⁷) | 5.0 x 10⁻¹² |
| K2279A | 7.2 x 10⁶ (8.2 x 10⁴) | 1.9 x 10⁻⁴ (2.8 x 10⁻⁶) | 2.6 x 10⁻¹¹ |
| P2300L | 1.8 x 10⁷ (1.9 x 10⁵) | 9.5 x 10⁻⁵ (7.1 x 10⁻⁷) | 5.3 x 10⁻¹² |
| L2302A | 2.4 x 10⁷ (1.9 x 10⁵) | 5.7 x 10⁻⁵ (4.7 x 10⁻⁷) | 2.4 x 10⁻¹² |
| R2304H | 1.0 x 10⁷ (2.4 x 10⁵) | 9.2 x 10⁻⁵ (4.5 x 10⁻⁶) | 9.2 x 10⁻¹² |
| R2307Q | 1.9 x 10⁷ (1.4 x 10⁵) | 9.0 x 10⁻⁵ (5.2 x 10⁻⁷) | 4.7 x 10⁻¹² |
| H2309A | 1.5 x 10⁷ (7.0 x 10⁴) | 2.9 x 10⁻⁵ (1.4 x 10⁻⁷) | 1.9 x 10⁻¹² |
| Q2311A | 2.3 x 10⁷ (1.4 x 10⁵) | 6.8 x 10⁻⁵ (3.6 x 10⁻⁷) | 3.0 x 10⁻¹² |
| W2313Y | 8.2 x 10⁶ (1.2 x 10⁵) | 8.7 x 10⁻⁵ (2.8 x 10⁻⁶) | 1.1 x 10⁻¹¹ |
| **H2315A** | **1.5 x 10⁷ (8.8 x 10⁴)** | **7.3 x 10⁻⁵ (2.7 x 10⁻⁷)** | **4.9 x 10⁻¹²** |
| **Q2316A** | **1.3 x 10⁷ (3.5 x 10⁵)** | **3.2 x 10⁻⁴ (6.1 x 10⁻⁷)** | **2.5 x 10⁻¹¹** |
| E2327A | 2.1 x 10⁷ (1.4 x 10⁵) | 5.4 x 10⁻⁵ (3.3 x 10⁻⁷) | 2.6 x 10⁻¹² |
| WT-C2 | 9.4 x 10⁷ (2.7 x 10⁵) | 8.5 x 10⁻⁵ (2.5 x 10⁻⁶) | 9.0 x 10⁻¹² |

**Table 12.**

| | | | | | | | | |
|---|---|---|---|---|---|---|---|---|
| Thermodynamic values from van't Hoff analysis. Data are in kJ/mol unless otherwise specified. Bold-face regions indicate substitutions at *the functional* epitope for BO2C11, i.e. where the substitution caused a greater than fourfold increase in *k_{d}* relative to wild-type C2 binding. The standard error (s.e.) of ΔH and TΔS are based on the s.e. of the slope and intercept, respectively. The s.e. of ΔG was derived from the s.e. of fitted values from the linear regression (van't Hoff analysis), transformed to the ΔG scale (e.g. s.e.(ln(*K_{d}*)\|T=298)*R*T/1000)⁴⁹. The ΔG and ΔΔG errors were all less than one but are reported here as "±1" for consistency with the significant figures of the measured data. The *K_{D}* values from the earlier kinetic runs at 25°C (final column) are included for comparison with *K_{D}* values derived from the *ΔG_{A}^{o}* of SPR runs carried out at several temperatures. | | | | | | | | |

| Mutant | *ΔH_{A}^{o}* | *TΔS_{A}^{o}* | *ΔG_{A}^{o}* | *ΔΔH_{A}^{o}* | *Δ*(*TΔS _{A}^{o}*) | *ΔΔG_{A}^{o}* | *K_{D}^{o}* (M) | *K_{D}* (M) = *k_{d}*/*kₐ* |
|---|---|---|---|---|---|---|---|---|
| WT-C2 | -14 ± 3 | 54 ± 3 | -68 ± 1 | N.A. | N.A. | N.A. | 1.2 x 10⁻¹² | 4.0 x 10⁻¹² |
| **F2196 A** | **-16 ± 1** | **41 ± 1** | **-57 ± 1** | **-2 ± 4** | **-13 ± 4** | **11 ± 1** | **1.0 x 10⁻¹⁰** | **2.2 x 10⁻¹⁰** |
| **N2198 A** | **-13 ± 9** | **45 ± 9** | **-58 ± 1** | **1 ± 10** | **-9 ± 10** | **10 ± 1** | **6.8 x 10⁻¹¹** | **5.6 x 10⁻¹¹** |
| **M2199 A** | **-16 ± 2** | **44±2** | **-60 ± 1** | **-2 ± 4** | **-10 ± 4** | **8 ± 1** | **3.0 x 10⁻¹¹** | **1.7 x 10⁻¹¹** |
| **F2200 A** | **-18 ± 2** | **37 ± 2** | **-55 ± 1** | **-4 ± 4** | **-17 ± 4** | **13 ± 1** | **2.3 x 10⁻¹⁰** | **2.4 x 10⁻¹⁰** |
| **R2215 A** | **-8 ± 5** | **49 ± 5** | **-57 ± 1** | **6±5** | **-5 ± 5** | **11 ± 1** | **1.0 x 10⁻¹⁰** | **1.5 x 10⁻¹⁰** |
| T2197 A | -12 ± 5 | 52 ± 5 | -64 ± 1 | 2 ± 8 | -2 ± 8 | 4 ± 1 | 6.1 x 10⁻¹² | 9.8 x 10⁻¹² |
| S2250 A | -3 ± 5 | 57 ± 5 | -60 ± 1 | 11 ± 5 | 3 ± 5 | 8 ± 1 | 3.0 x 10⁻¹¹ | 2.2 x 10⁻¹¹ |
| L2251 A | -31 ± 10 | 35 ± 10 | -66 ± 1 | -17 ± 11 | -19 ± 11 | 2 ± 1 | 2.7 x 10⁻¹² | 6.2 x 10⁻¹² |
| L2252 A | -49 ± 12 | 14 ± 12 | -63 ± 1 | -35 ± 12 | -40 ± 12 | 5 ± 1 | 9.1 x 10⁻¹² | 6.1 x 10⁻¹² |

**Table 13**

| **Mutation** | **Forward Primer** | **SEQ ID NO:** |
|---|---|---|
| **S21731** | CTCGAGAAAAGAGTGGATTTAAATGCTTGCAGCATGCCATTGGG | 101 |
| **E2181A** | GCATGCCATTGGGAATGGCGAGTAAAGCAATATCAGATGC | 102 |
| **F2196A** | GCACAGATTACTGCTTCATCCTACGCTACCAATATGTTTGCCACC | 103 |
| **T2197A** | GCTTCATCCTACTTTGCCAATATGTTTGCCACCTGG | 104 |
| **N2198A** | CAGATTACTGCTTCATCCTACTTTACCGCTATGTTTGCCACCTGG | 105 |
| **M2199A** | CTTCATCCTACTTTACCAATGCGTTTGCCACCTGGTCTCCTT | 106 |
| **F2200A** | CTTCATCCTACTTTACCAATATGGCTGCCACCTGGTCTCC | 107 |
| **A2201P** | CCTACTTTACCAATATGTGCGCCACCTGGTCTCCTTCAAAAGC | 108 |
| **T2202A** | CCTACTTTACCAATATGTTTGCCGCCTGGTCTCCTTCAAAAGC | 109 |
| **K2207A** | GGTCTCCTTCAGCAGCTCGACTTCACCTCCAAGGG | 110 |
| **H2211A** | CCTTCAAAAGCTCGACTTGCCCTCCAAGGGAGGAGTAATGCC | 111 |
| **L2212A** | CCTTCAAAAGCTCGACTTCACGCCCAAGGGAGGAGTAATGCC | 112 |
| **Q2213A** | CCTTCAAAAGCTCGACTTCACCTCGCAGGGAGGAGTAATGCC | 113 |
| **R2215A** | CGACTTCACCTCCAAGGGGCGAGTAATGCCTGGAGACC | 114 |
| **R2220A** | CCAAGGGAGGAGTAATGCCTGGGCACCTCAGGT | 115 |
| **R2220Q** | GGGAGGAGTAATGCCTGGCAACCTCAGGTGAATAATCC | 116 |
| **Q2222A** | GGAGTAATGCCTGGAGACCTGCGGTGAATAATCCAAAAGAGTGG | 117 |
| **V2223M** | GCCTGGAGACCTCAGATGAATAATCCAAAAGAGTGG | 118 |
| **N2224A** | GGAGTAATGCCTGGAGACCTCAGGTGGCTAATCCAAAAGAGTGGC | 119 |
| **N2225A** | CCTGGAGACCTCAGGTGAATGCTCCAAAAGAGTGGCTGC | 120 |
| **K2227A** | CCTCAGGTGAATAATCCAGCAGAGTGGCTGCAAGTGG | 121 |
| **K2227Q** | GGAGACCTCAGGTGAATAATCCACAAGAGTGGTGCAAGTGG | 122 |
| **K2249A** | GTAACTACTCAGGGAGTAGCATCTCTGCTTACCAGCATGTATGTG | 123 |
| **S2250A** | CAGGGAGTAAAAGCTCTGCTTACCAGCATGTATGTG | 124 |
| **L2251A** | GAGTAAAATCTGCGCTTACCAGCATGTAT | 125 |
| **L2252A** | CTCAGGAGTAAAATCTCTGGCTACCAGCATGTATGTGAAGG | 126 |
| **T2253A** | GGAGTAAAATCTCTGCTTGCCAGCATGTATGTGAAGGAG | 127 |
| **H2269A** | CATCTCCAGCAGTCAAGATGGCGCTCAGTGGACTCTC | 128 |
| **Q2270A** | GCAGTCAAGATGGCCATGCGTGGACTCTCTTTTTTCAGAATGCC | 129 |
| **T2272A** | GGCCATCAGTGGGCTCTCTTTTTTCAGAATGGC | 130 |
| **L2273A** | GATGGCCATCAGTGGACTGCCTTTTTTCAGAATGGCAAAGTAAAG | 131 |
| **N2277A** | CAGTGGACTCTCTTTTTTCAGGCTGGCAAAGTAAAGGTTTTTCAG | 132 |
| **K2279A** | GGACTCTCTTTTTTCAGAATGGCGCAGTAAAGGTTTTTCAGAATGG | 133 |
| **P2300L** | GGTGAACTCTCTAGACCCACTGTTACTGACTCGC | 134 |
| **L2302A** | GACCCACCGTTAGCGACTCGCTACCTTCGAATTCACC | 135 |
| **R2304H** | CCACCGTTACTGACTCACTACCTTCGAATTCACC | 136 |
| **R2307Q** | CTGACTCGCTACCTTCAAATTCACCCCCAGAGTTGG | 137 |
| **H2309A** | CGCTACCTTCGAATTGCCCCCCAGAGTTGGGTGC | 138 |
| **Q2311A** | CCTTCGAATTCACCCCGCGAGTTGGGTGCACCAG | 139 |
| **W2313Y** | CGAATTCACCCCCAGAGTTACGTGCACCAGATTGCCC | 140 |
| **H2315A** | CCAGAGTTGGGTGGCCCAGATTGCCCTGAGGATGG | 141 |
| **Q2316A** | CCCCAGAGTTGGGTGCACGCCATTGCCCTGAGGATGG | 142 |
| **E2327A** | GGTTCTGGGCTGCGCGGCACAGGACC | 143 |

**Table 14**

| **Mutation** | **Reverse Primer** | **SEQ ID NO:** |
|---|---|---|
| **S21731** | CCCAATGGCATGCTGCAAGCATTTAAATCCACTCTTTTCTCGAG | 144 |
| **E2181A** | GCATCTGATATTGCTTTACTCGCCATTCCCAATGGCATGC | 145 |
| **F2196A** | GGTGGCAAACATATTGGTAGCGTAGGATGAAGCAGTAATCTGTGC | 146 |
| **T2197A** | CCAGGTGGCAAACATATTGGCAAAGTAGGATGAAGC | 147 |
| **N2198A** | CCAGGTGGCAAACATAGCGGTAAAGTAGGATGAAGCAGTAATCTG | 148 |
| **M2199A** | AAGGAGACCAGGTGGCAAACGCATTGGTAAAGTAGGATGAAG | 149 |
| **F2200A** | GGAGACCAGGTGGCAGCCATATTGGTAAAGTAGGATGAAG | 150 |
| **A2201P** | GCTTTTGAAGGAGACCAGGTGGCGCACATATTGGTAAAGTAGG | 151 |
| **T2202A** | GCTTTTGAAGGAGACCAGGCGGCAAACATATTGGTAAAGTAGG | 152 |
| **K2207A** | CCCTTGGAGGTGAAGTCGAGCTGCTGAAGGAGACC | 153 |
| **H2211A** | GGCATTACTCCTCCCTTGGAGGGCAAGTCGAGCTTTTGAAGG | 154 |
| **L2212A** | GGCATTACTCCTCCCTTGGGCGTGAAGTCGAGCTTTTGAAGG | 155 |
| **Q2213A** | GGCATTACTCCTCCCTGCGAGGTGAAGTCGAGCTTTTGAAGG | 156 |
| **R2215A** | GGTCTCCAGGCATTACTCGCCCCTTGGAGGTGAAGTCG | 157 |
| **R2220A** | ACCTGAGGTGCCCAGGCATTACTCCTCCCTTGG | 158 |
| **R2220Q** | GGATTATTCACCTGAGGTTGCCAGGCATTACTCCTCCC | 159 |
| **Q2222A** | CCACTCTTTTGGATTATTCACCGCAGGTCTCCAGGCATTACTCC | 160 |
| **V2223M** | CCACTCTTTTGGATTATTCATCTGAGGTCTCCAGGC | 161 |
| **N2224A** | GCCACTCTTTTGGATTAGCCACCTGAGGTCTCCAGGCATTACTCC | 162 |
| **N2225A** | GCAGCCACTCTTTTGGAGCATTCACCTGAGGTCTCCAGG | 163 |
| **K2227A** | CCACTTGCAGCCACTCTGCTGGATTATTCACCTGAGG | 164 |
| **K2227Q** | CCACTTGCACCACTCTTGTGGATTATTCACCTGAGGTCTCC | 165 |
| **K2249A** | CACATACATGCTGGTAAGCAGAGATGCTACTCCCTGAGTAGTTAC | 166 |
| **S2250A** | CACATACATGCTGGTAAGCAGAGCTTTTACTCCCTG | 167 |
| **L2251A** | ATACATGCTGGTAAGCGCAGATTTTACTC | 168 |
| **L2252A** | CCTTCACATACATGCTGGTAGCCAGAGATTTTACTCCTGAG | 169 |
| **T2253A** | CTCCTTCACATACATGCTGGCAAGCAGAGATTTTACTCC | 170 |
| **H2269A** | GAGAGTCCACTGAGCGCCATCTTGACTGCTGGAGATG | 171 |
| **Q2270A** | GGCATTCTGAAAAAAGAGAGTCCACGCATGGCCATCTTGACTGC | 172 |
| **T2272A** | GCCATTCTGAAAAAAGAGAGCCCACTGATGGCC | 173 |
| **L2273A** | CTTTACTTTGCCATTCTGAAAAAAGGCAGTCCACTGATGGCCATC | 174 |
| **N2277A** | CTGAAAAACCTTTACTTTGCCAGCCTGAAAAAAGAGAGTCCACTG | 175 |
| **K2279A** | CCATTCTGAAAAACCTTTACTGCGCCATTCTGAAAAAAGAGAGTCC | 176 |
| **P2300L** | GCGAGTCAGTAACAGTGGGTCTAGAGAGTTCACC | 177 |
| **L2302A** | GGTGAATTCGAAGGTAGCGAGTCGCTAACGGTGGGTC | 178 |
| **R2304H** | GGTGAATTCGAAGGTAGTGAGTCAGTAACGGTGG | 179 |
| **R2307Q** | CCAACTCTGGGGGTGAATTTGAAGGTAGCGAGTCAG | 180 |
| **H2309A** | GCACCCAACTCTGGGGGGCAATTCGAAGGTAGCG | 181 |
| **Q2311A** | CTGGTGCACCCAACTCGCGGGGTGAATTCGAAGG | 189 |
| **W2313Y** | GGGCAATCTGGTGCACGTAACTCTGGGGGTGAATTCG | 190 |
| **H2315A** | CCATCCTCAGGGCAATCTGGGCCACCCAACTCTGG | 191 |
| **Q2316A** | CCATCCTCAGGGCAATGGCGTGCACCCAACTCTGGGG | 192 |
| **E2327A** | GGTCCTGTGCCGCGCAGCCCAGAACC | 193 |

### Example 4 References

1. Ehrenforth S, Kreuz W, Scharrer I, et al. Incidence of development of factor VIII and factor IX inhibitors in haemophiliacs. Lancet. 1992;339:594-598.
2. Lusher JM, Lee CA, Kessler CM, Bedrosian CL. The safety and efficacy of B-domain deleted recombinant factor VIII concentrate in patients with severe haemophilia A. Haemophilia. 2003;9:38-49.
3. Hay CR, Ludlam CA, Colvin BT, et al. Factor VIII inhibitors in mild and moderate-severity haemophilia A. Thromb Haemost. 1998;79:762-766.
4. Collins PW, Hirsch S, Baglin TP, et al. Acquired hemophilia A in the United Kingdom: a 2-year national surveillance study by the United Kingdom Haemophilia Centre Doctors' Organisation. Blood. 2007;109:1870-1877.
5. Bray GL, Kroner BL, Arkin S, et al. Loss of high-responder inhibitors in patients with severe hemophilia A and human immunodeficiency virus type 1 infection: a report from the Multi-Center Hemophilia Cohort Study. Am J Hematol. 1993;42:375-379.
6. Reding MT, Wu H, Krampf M, et al. Sensitization of CD4+ T cells to coagulation factor VIII: response in congenital and acquired hemophilia patients and in healthy subjects. Thromb Haemost. 2000;84:643-652.
7. Jacquemin MG, Desqueper BG, Benhida A, et al. Mechanism and kinetics of factor VIII inactivation: study with an IgG4 monoclonal antibody derived from a hemophilia A patient with inhibitor. Blood. 1998;92:496-506.
8. Spiegel PC, Jr., Jacquemin M, Saint-Remy JM, Stoddard BL, Pratt KP. Structure of a factor VIII C2 domain-immunoglobulin G4kappa Fab complex: identification of an inhibitory antibody epitope on the surface of factor VIII. Blood. 2001;98:13-19.
9. Kemball-Cook G, Tuddenham EG, Wacey AI. The factor VIII Structure and Mutation Resource Site: HAMSTeRS version 4. Nucleic Acids Res. 1998;26:216-219.
10. Meeks SL, Healey JF, Parker ET, Barrow RT, Lollar P. Antihuman factor VIII C2 domain antibodies in hemophilia A mice recognize a functionally complex continuous spectrum of epitopes dominated by inhibitors of factor VIII activation. Blood. 2007;110:4234-4242.
11. Takeshima K, Smith C, Tait J, Fujikawa K. The preparation and phospholipid binding property of the C2 domain of human factor VIII. Thromb Haemost. 2003;89:788-794.
12. Pratt KP, Shen BW, Takeshima K, Davie EW, Fujikawa K, Stoddard BL. Structure of the C2 domain of human factor VIII at 1.5 A resolution. Nature. 1999;402:439-442.
13. Frishman D, Argos P. Knowledge-based protein secondary structure assignment. Proteins. 1995;23:566-579.
14. Deinum J, Gustavsson L, Gyzander E, Kullman-Magnusson M, Edstrom A, Karlsson R. A thermodynamic characterization of the binding of thrombin inhibitors to human thrombin, combining biosensor technology, stopped-flow spectrophotometry, and microcalorimetry. Anal Biochem. 2002;300:152-162.
15. Roos H, Karlsson R, Nilshans H, Persson A. Thermodynamic analysis of protein interactions with biosensor technology. J Mol Recognit. 1998;11:204-210.
16. Mannucci PM. Acquired Disorders of Coagulation. (ed 3rd): Churchill Livingstone 1994.
17. Berntorp E, Shapiro A, Astermark J, et al. Inhibitor treatment in haemophilias A and B: summary statement for the 2006 international consensus conference. Haemophilia. 2006;12 Suppl 6:1-7.
18. Lusher JM, Arkin S, Abildgaard CF, Schwartz RS. Recombinant factor VIII for the treatment of previously untreated patients with hemophilia A. Safety, efficacy, and development of inhibitors. Kogenate Previously Untreated Patient Study Group. N Engl J Med. 1993;328:453-459.
19. Qian J, Borovok M, Bi L, Kazazian HH, Jr., Hoyer LW. Inhibitor antibody development and T cell response to human factor VIII in murine hemophilia A. Thromb Haemost. 1999;81:240-244.
20. Wu H, Reding M, Qian J, et al. Mechanism of the immune response to human factor VIII in murine hemophilia A. Thromb Haemost. 2001;85:125-133.
21. Pratt KP, Qian J, Ellaban E, et al. Immunodominant T-cell epitopes in the factor VIII C2 domain are located within an inhibitory antibody binding site. Thromb Haemost. 2004;92:522-528.
22. Liu M, Murphy ME, Thompson AR. A domain mutations in 65 haemophilia A families and molecular modelling of dysfunctional factor VIII proteins. Br J Haematol. 1998;103:1051-1060.
23. Fuentes-Prior P, Fujikawa K, Pratt KP. New insights into binding interfaces of coagulation factors V and VIII and their homologues; lessons from high resolution crystal structures. Curr Protein Pept Sci. 2002;3:313-339.
24. Thompson AR. Structure and function of the factor VIII gene and protein. Semin Thromb Hemost. 2003;29:11-22.
25. Foster PA, Fulcher CA, Houghten RA, Zimmerman TS. A synthetic factor VIII peptide of eight amino acid residues (1677-1684) contains the binding region of an anti-factor VIII antibody which inhibits the binding of factor VIII to von Willebrand factor. Thromb Haemost. 1990;63:403-406.
26. Scandella D, Gilbert GE, Shima M, et al. Some factor VIII inhibitor antibodies recognize a common epitope corresponding to C2 domain amino acids 2248 through 2312, which overlap a phospholipid-binding site. Blood. 1995;86:1811-1819.
27. Barrow RT, Healey JF, Jacquemin MG, Saint-Remy JM, Lollar P. Antigenicity of putative phospholipid membrane-binding residues in factor VIII. Blood. 2001;97:169-174.
28. Saenko EL, Shima M, Rajalakshmi KJ, Scandella D. A role for the C2 domain of factor VIII in binding to von Willebrand factor. J Biol Chem. 1994;269:11601-11605.
29. Shima M, Yoshioka A, Nakai H, et al. Epitope localization of monoclonal antibodies against factor VIII light chain which inhibit complex formation by factor VIII with von Willebrand factor. Int J Hematol. 1991;54:515-522.
30. Saenko EL, Scandella D. A mechanism for inhibition of factor VIII binding to phospholipid by von Willebrand factor. J Biol Chem. 1995;270:13826-13833.
31. Meeks SL, Healey JF, Parker ET, Barrow RT, Lollar P. Non-classical anti-C2 domain antibodies are present in patients with factor VIII inhibitors. Blood 2008;112:1151-1153.
32. Scandella D, deGraaf Mahoney S, Mattingly M, Roeder D, Timmons L, Fulcher C. Epitope mapping of human factor VIII inhibitor antibodies by deletion analyis of factor VIII fragments expressed in Escherichia coli. Proc Nat Acad Sci USA. 1988;85:6152-6156.
33. Foster PA, Fulcher CA, Houghten RA, Zimmerman TS. A synthetic factor VIII peptide of eight amino acid residues (1677-1684) contains the binding region of an anti-factor VIII antibody which inhibits the binding of factor VIII to von Willebrand factor. Thromb Haemostas 1990;63:403-406.
34. Huang C-C, Shen M-C, Chen J-Y, Hung M-H, Hsu T-C, Lin S-W. Epitope mapping of factor VIII inhibitor antibodies of Chinese origin. Brit J Haematol. 2001;113:915-924.
35. Ansong C, Miles SM, Fay PJ. Epitope mapping of factor VIII A2 domain by affinity-directed mass spectrometry: residues 497-510 and 584-593 comprise a discontinuous epitope for the monoclonal antibody R8B12. J Thromb Haemostas. 2006;4:842-847.
36. Nogami K, Shima M, Criddings JC, Takeyama M, Tanaka I, Yoshioka A. Relationship between the binding sites for von WIllebrand factor, phospholipid, and human factor VIII C2 inhibitor alloantibodies within the factor VIII C2 domain. Int J Hematol 2007;85:317-322.
37. Chaves DG, Velloso-Rodrigues C, Moreau V, et al. Reactivity profiles of anti-factor VIII antibodies with designed synthetic peptides mimicking epitopes of the C2 and al domains. Brit J Haematol 2008;141:708-715.
38. Albert T, Egler C, Jakuschev S, et al. The B-cell epitope of the monoclonal anti-factor VIII antibody ESH8 characterized by peptide array analysis. Thromb Haemostas. 2008;99:634-637.
39. Kessel C, Königs C, Linde R, et al. Humoral immune responsiveness to a defined epitope on factor VIII before and after B cell ablation with rituximab. Mol Immunol 2008;46:8-15.
40. Lavigne-Lissalde G, Rothschild C, Pouplard C, et al. Characteristics, mechanisms of action, and epitope mapping of anti-factor VIII antibodies. Clinic Rev Allerg Immunol. Prepublished on January 27, 2009 as DOI 10.1007/s12016-009-8119-0.
41. Pratt KP, Thompson AR. B-cell and T-cell epitopes in anti-factor VIII immune responses. Clinic Rev Allerg Immunol. 2009; Prepublished on January 27, 2009 as DOI 10.1007/s12016-009-8120-7.
42. Cunningham BC, Wells JA. Comparison of a structural and a functional epitope. J Mol Biol. 1993;234:554-563.
43. Clackson T, Wells JA. A hot spot of binding energy in a hormone-receptor interface. Science. 1995;267:383-386.
44. DeLano WL, Ultsch MH, de Vos AM, Wells JA. Convergent solutions to binding at a protein-protein interface. Science. 2000;287:1279-1283.
45. Pearce KH, Jr., Potts BJ, Presta LG, Bald LN, Fendly BM, Wells JA. Mutational analysis of thrombopoietin for identification of receptor and neutralizing antibody sites. J Biol Chem. 1997;272:20595-20602.
46. Pace CN, Shirley BA, McNutt M, Gajiwala K. Forces contributing to the conformational stability of proteins. FASEB J. 1996;10:75-83.
47. Fulcher CA, de Graaf Mahoney S, Zimmerman TS. FVIII inhibitor IgG subclass and FVIII polypeptide specificity determined by immunoblotting. Blood. 1987;69:1475-1480.
48. Gilles JG, Arnout J, Vermylen J, Saint-Remy JM. Anti-factor VIII antibodies of hemophiliac patients are frequently directed towards nonfunctional determinants and do not exhibit isotypic restriction. Blood. 1993;82:2452-2461.
49. Weisberg S. Applied Linear Regression. New York, NY: Wiley & Sons; 1980. 21.

### Example 5

FVIII-neutralizing antibodies ("inhibitors") develop in some hemophilia A (HA) patients who receive factor VIII (FVIII) infusions, resulting in bleeding complications [1-3]. Inhibitors are observed in 25-35% of severe HA patients but also can occur in mild/moderately severe HA [4, 5]. Inhibitors have been associated with multiple F8 missense genotypes [6], including F8-R593C [7-9]. Multiple lines of evidence, including sequences/subclasses of inhibitory antibodies [10-13], efficacy of anti-CD40L inhibition [14], and the influence of CD4+ cell counts on antibody titers [15], indicate that inhibitor induction, affinity maturation and antibody class switching involve help from CD4+ T cells. Experimental evidence [16-18] has suggested that T-cell responses in mild/moderately severe HA may be directed against epitopes that contain the wild-type FVIII sequence at the hemophilic mutation site. Several studies have also indicated that B-cell epitopes may include the missense site [9, 19-21]. Although T-cell proliferation in response to FVIII protein and peptides has been investigated [22-25], further study is warranted to establish the HLA restriction of T-cell epitopes within FVIII, particularly in the context of specific F8 genotypes. This information could improve estimates of inhibitor risk in defined sub-populations, allowing individualized treatment of high-risk patients by reducing their exposure to wild-type FVIII concentrates, and would motivate the design of less immunogenic versions of FVIII.

In the present study, two unrelated HA subjects with F8-R593C genotype and similar HLA-DR haplotypes were studied to characterize T-cell responses and to identify epitopes within FVIII. *The in vitro* antigenicity of synthetic, overlapping peptides spanning the FVIII-A2, FVIII-C1 and FVIII-C2 domains were evaluated. To test our hypothesis that the hemophilic substitution site coincides with an important T-cell epitope, the binding of peptides containing R593 to various recombinant HLA-DR proteins was evaluated, and the results were correlated with reported inhibitor incidences in F8-R593C patient cohorts. Our findings support a paradigm in which binding and presentation of FVIII epitopes containing the wild-type R593 by several common HLA-DR alleles may influence the relative risk of developing an inhibitor in this HA subpopulation.

### Materials and Methods

### Subjects and blood samples

Samples from two unrelated HA subjects and from eight *HLA-DRB1*1101-*matched healthy controls were used. Subject 1D *(HLA-DRBI *7707* and *DRB1*1302*), from a Dutch cohort of *F8-R593C* patients, had an initial inhibitor titer of 22 Bethesda units (BU)/mL that declined but persisted for years [26]. Prior to inhibitor development, his baseline FVIII clotting activity (FVIII:C) was 20%; this declined to 1% at peak inhibitor titer, indicating that the inhibitor cross-reacted to neutralize his endogenous (hemophilic) FVIII, then increased to 1.4% in subsequent years [26]. He received FVIII to support an operation, which boosted his titer to 2 BU/mL and elicited cross-reactive antibodies against the FVIII A2 domain [9, 27]. Subject 41A *(HLA-DRBI *1101* and *DRB1*1303*), from a cohort of American *F8-R593C* patients, also developed an inhibitor after receiving FVIII infusions to support surgery. His baseline FVIII:C was 26%. In the month before and after peak titer (34 BU/mL) his FVIII:C activity ranged from ∼1-4%, indicating that the initial inhibitor cross-reacted to neutralize his endogenous (hemophilic) FVIII. He was treated with Rituximab and the titer declined. His most recent titer (2007) was undetectable (<0.5 BU/mL). Neither patient underwent immune tolerance induction. Blood samples from both subjects were collected >6 months after their last FVIII infusion. Peripheral blood mononuclear cells (PBMCs) were obtained by Ficoll underlay and either frozen (7% DMSO in serum) or assayed immediately. Research was performed with IRB approval from the University of Washington Human Subjects Committee or the Universiteit van Amsterdam Medical Ethics Committee, with written informed consent.

### FVIII peptides and protein

20-mer peptides (with 12-residue overlaps) with sequences **(Table 17)** spanning the FVIII A2, C1, and C2 domains were synthesized and verified by mass spectrometry (Mimotopes, Clayton Victoria, Australia; Global Peptide Inc., Ft. Collins, CO; Synpep, Dublin, CA; Anaspec, San Jose, CA). Peptides were dissolved at 10-20 mg/mL in DMSO or DMSO/water. Peptide pools contained equal amounts of 3-7 peptides (10 mg/ml total). Recombinant FVIII was obtained from Pharmacia/Upjohn (manufactured by CSL Behring GmbH).

### Peptide-binding predictions and assays

The binding affinities of peptides spanning the FVIII-A2 sequence to the HLA-DR1101 protein were predicted using the ProPred MHC class II binding algorithm (http://www.imtech.res.in/raghava/propred/) [28]. This program predicts affinities of peptide sequences for common HLA-DR molecules that present peptides antigen-presenting cells, by evaluating their ability to fit into the canonical 9-residue peptide binding groove that is a feature of the MHC Class II. Every possible 9-mer sequence within FVIII-A2 was analyzed with the algorithm's threshold value set to list binding scores above 0.8. The predicted set of peptides was further narrowed by excluding sequences with valine at position 1 of the DR1101 1 binding motif (i.e. the fit of the peptide into the groove), since this residue has been shown to bind weakly in this pocket [29]. Peptides with sequences containing R593 or C593 were evaluated regardless of their scores.

Affinities of FVIII peptides for HLA-DR monomers were determined experimentally by competition assays. Recombinant HLA-DR0101, DR0301, DR0401, DR1101, DR1104, or DR1501 proteins were incubated with (1) FVIII peptides at 0.05, 0.1, 0.5, 1, 5, 10, and 50 µM plus (2) biotinylated reference peptides that bound to specific DR proteins with high affinity **(Table 17).** The DR proteins were then immobilized in wells coated with anti-DR capture antibody (L243) [30]. After washing, residual bound biotinylated peptide was labeled using europium-conjugated streptavidin (Perkin Elmer) and quantified using a Victor² D fluorometer (Perkin Elmer). Sigmoidal binding curves were simulated and IC₅₀ values (concentration displacing 50% reference peptide) calculated using SigmaPlot (Systat Software, Inc., San Jose, CA).

### HLA-DR Tetramers

HLA-DR1101 tetramers were generated as described [31]. Briefly, biotinylated recombinant DR1101 protein was incubated with pooled or individual peptides at 37°C for 72hr with n-octyl-ß-D-glucopyranoside and Pefabloc (Sigma-Aldrich, St. Louis, MO) and conjugated using R-phycoerythrin (PE) streptavidin (Biosource, Camarillo, CA). Tetramer quality was confirmed by staining a reference T-cell clone (not shown).

### Isolation and peptide stimulation of primary CD4+ T cells

T-cell isolation was carried out as described [17, 32]. Frozen PBMCs from subject 1D were thawed, washed, and CD4+ T cells were fractionated by no-touch isolation (Miltenyi Biotec, Auburn, CA). For subject 41A and HLA-matched control subjects, CD4+ T cells were fractionated from freshly isolated PBMCs. Three million autologous, CD4-depleted PBMCs were plated into 48-well plates for 1hr and then washed, leaving a layer of residual adherent cells behind as APCs. Two million purified CD4+ responder cells were then plated into these wells. Wells were stimulated with 10 µg/ml pooled peptides in T-cell medium (RPMI 1640 with 10% human serum, 1 mM sodium pyruvate, 50 U/ml penicillin and 50 µg/ml streptomycin), supplemented with 40 U/ml IL-2 (Hemagen, Waltham, MD) on day 7, and maintained with medium and IL-2.

### Tetramer Guided Epitope Mapping (TGEM)

After two weeks, cells were analyzed with DR1101 tetramers as described [32, 33]. For subject 1D and a control subject, 0.75 x 10⁵ cells were incubated with tetramers (labeled with PE) loaded with individual FVIII peptides predicted to bind DR1101 (Table 15) [28] at 37°C for 1hr, then incubated with anti-CD3-PerCP (BD Biosciences, San Jose, CA), anti-CD4-APC (eBioscience, San Diego, CA), and anti-CD25-FITC (eBioscience) at 4°C for 20min, and then analyzed on a FACSCalibur (Becton Dickinson, San Jose, CA). For subject 41A and a second HLA-matched control subject, 0.75 x 10⁵ cells were stained in a similar fashion, using tetramers loaded with peptide pools spanning the A2, C1, and C2 domains of FVIII **(Table 17).** Tetramer-positive responses were decoded using tetramers loaded with individual peptides. To define an objective criterion for positive tetramer staining, CD4+ T cells from six non-hemophilic DR1101 donors were "sham" stimulated using DMSO for two weeks and subsequently stained using a panel of DR1101 tetramers. One tetramer (FVIII 381-400) gave significantly higher background staining, indicating a peptide-specific effect, while all others had a statistically similar background, allowing calculation of a mean background level **(****Figure 15). Figure 15** shows background staining threshold for tetramer reagents. CD4+ cells from six healthy subjects were "mock" stimulated and stained with a panel of DR1101 tetramer reagents. The first five boxes indicate the mean (horizontal line) and 95% confidence boundaries (bars) of the background staining observed for representative single tetramers. Among these FVIII381-400 had significantly higher background (indicated by asterisk). The final box indicates the combined background level, excluding FVIII318-400.) Our criterion for positive staining was designated as the mean background staining plus 3 times the standard error of the mean: 1.53% for FVIII 381-400 and 0.46% for all other specificities. The latter is consistent with the cut-off used in previous published studies [17, 18, 30-33].

### Isolation of T-cell clones and a polyclonal line

For all cultures that demonstrated tetramer-positive staining, FVIII-specific T cells were stained and isolated as described [17] following staining with DR1101-PE tetramers and anti-CD4-FITC (eBioscience). CD4+ tetramer-positive cells were sorted using a FACS Vantage (Becton Dickinson) into 96-well plates containing T-cell medium at one cell per well (to produce clones) or 250 cells per well (to produce a polyclonal line) and expanded by adding 2 µg/ml phytohemagglutinin and 200,000 irradiated PBMCs plus IL-2. Expanded cells were stained with DR1101-PE tetramers and analyzed on a FACSCalibur (Becton Dickinson).

### Antigen-specific T-cell proliferation assay

T-cell proliferation was assessed as described [17, 18]. Briefly, irradiated PBMCs from an HLA-matched (*DRB1*1101*) non-HA donor were plated at 10⁵ cells/well in 100 µl T-cell medium. Peptides (final concentrations 10, 1, 0.1, and 0 µM) and T cells (10⁴ cells/well) were added in 100 µl T-cell medium and plates were incubated at 37°C. Wells were pulsed with [³H]thymidine (1 µCi/well) after 48hr and cells were harvested 18hr later. [³H]thymidine uptake was measured with a scintillation counter, and stimulation indices (SIs) were calculated as the counts per minute (cpm) of peptide- stimulated cultures divided by the cpm with no peptide added.

### Cytokine sandwich ELISAs.

Interferon-γ (IFN-γ), tumor necrosis factor-α (TNF-α), interleukin-4 (IL-4), interleukin-10 (IL-10) and interleukin-17A (IL-17A) were measured in supernatants by ELISA. Plates were coated with 100 µl of 2-4 µg/ml cytokine-specific antibody (anti-IFN-γ MD-1, anti-TNF-α MAbl, anti-IL-4 8D4-8, anti-IL-10 JES3-9D7, and anti-IL-17A eBio64CAP17, eBioscience) in coating buffer (eBioscience) overnight at 4°C, washed in PBS with 0.05% Tween 20, blocked with diluent solution (eBioscience) for 1hr at room temperature and washed again. Cytokine standard (100 µl) (Cell Sciences or eBioscience) or 20-50 µl cell supernatant (plus diluent) was added to each well, and plates were incubated overnight at 4°C and washed. Biotin-labeled antibody (100 µl at 2 µg/ml) (anti-IFN-γ clone 4S.B3, anti-TNF-α MAb11, anti-IL-4 MP4-25D2, anti-IL-10 JES3-12G8, and anti-IL-17 eBio64DEC17, eBioscience) was added and incubated at room temperature for 1hr. Avidin horseradish peroxidase (eBioscience) was added (1:1000 dilution), incubated at room temperature for 30min and washed. Super Aquablue substrate (100 µl) (eBioscience) was then added and A₄₀₅ measured using a Bio-Rad 550 reader (Hercules, CA). Cytokine concentrations were calculated from linear standard curves for each cytokine. Th1/Th2 ratios were calculated as: ([IFN-γ] + [TNF-α])/([IL-4] + [IL-10]).

### Results

### Binding of FVIII peptides to DR1101

The two R593C subjects had the *DRB1*1101* allele in common. An MHC class II binding computer prediction algorithm [28] was used to predict which FVIII-A2 peptides might bind to DR1101. For these predictions a higher score (see **Table 15)** indicates a greater likelihood that the corresponding peptide is capable of binding. Seventeen synthetic peptides corresponding to sequences with the highest predicted binding scores were then tested to empirically determine their *in vitro* affinities for recombinant DR1101 protein. Observed IC₅₀ values ranged from 0.2 µM to >100 µM, the detection limit. As summarized in **Table 15,** 8 of the 17 peptides with predicted binding scores above 0.8 bound to DR1101 with an IC₅₀ under 10µM. Notably, FVIII₅₈₁₋₆₀₀, FVIII₅₈₉₋₆₀₈, and FVIII_{589-608,593C}, all of which contain the missense site, bound to DR1101 1 with reasonable affinity as compared with the influenza HA₃₀₆₋₃₁₈ control peptide **(Table 16),** whereas FVIII_{581-600,593C} did not.

### T-Cell responses to selected peptides

For inhibitor subject 1D, the number of cryo-preserved cells available for study was only sufficient to test responses to a limited number of peptides. Therefore, peptides that contained predicted FVIII-A2 domain epitopes **(Table 15)** were utilized to query his T-cell responses. These were divided into two 7-peptide pools, which were then used to stimulate CD4+ T cells from him and from a control subject. T cells from the inhibitor and control subjects were cultured for 14 days and then stained using DR1101 tetramers loaded with individual peptides. A clear population of CD4+ T cells was stained by tetramers loaded with FVIII₅₈₉₋₆₀₈ **(****Figure 16****),** which bound to DR1101 with high affinity (IC₅₀ = 0.5±0.4µM). Weaker positive staining was observed for FVIII₄₂₉₋₄₄₈, FVIII₄₆₉₋₄₈₈, and FVIII₅₈₁₋₆₀₀, which bound to DR1101 with IC₅₀ values of 0.5±0.4µM, 8.9±8µM, and ∼100µM. Notably, tetramer staining was negative for CD4+ T cells stimulated by the hemophilic peptide FVIII_{589-608,593C}. Attempts to stain T cells from the control subject, using tetramers loaded with each of the 14 peptides containing predicted epitopes **(Table 15)** yielded negative results (not shown).

### Mapping epitopes in the FVIII A2, C1, and C2 domains

CD4+ T cells freshly isolated from subject 41A were stimulated with peptides spanning the FVIII A2, C1 and C2 domains, including two peptides with the R593C substitution **(Table 17).** Cells were cultured and evaluated for responses by staining with fluorescent, peptide-loaded DR1101 tetramers. Representative results are shown in **Figure 17A****.** Tetramer staining was above background for CD4+ cells stimulated with FVIII-A2 peptide pools 1, 2 and 6 and with FVIII-C2 pool 1. Therefore, T cells stimulated with these pools were selected for further analysis (decoding) using tetramers loaded with single peptides that comprised these pools **(****Figure 17B****).** T cells stimulated using peptide pool 6 showed positive staining by tetramers loaded with FVIII₅₈₉₋₆₀₈ and FVIII₅₈₁₋₆₀₀, both of which bound with IC₅₀ values of 0.5±0.4 µM. FVIII-A2 peptide pool 2 and FVIII-C2 peptide pool 1 showed weaker positive staining by tetramers loaded with FVIII₄₂₁₋₄₄₀ and FVIII₂₁₈₇₋₂₂₀₅ respectively. The IC₅₀ values for these peptides were 5.0±18µM, and 12±26µM. The apparent positive staining of A2 peptide pool 1 was due to FVIII₃₈₁₋₄₀₀, which caused high peptide-specific background staining. Tetramer-stained cells were generally CD25+, suggesting they were activated (not shown). Notably, staining with tetramers loaded with FVIII-A2 peptide pool 11, which contains two peptides with the hemophilic R593C substitution, was negative, indicating that neither peptide containing C593 elicited a high-avidity T-cell response. The same peptide-loaded tetramers were used to evaluate T-cell responses for an *HLA-DRB1*1101* control subject. All staining results using T cells from this subject were negative (not shown).

### Isolating T-cell Clones and evaluating additional control subjects

To facilitate further study of FVIII-specific T-cell responses, cells from each positive well were stained again and single-cell sorted to obtain FVIII-specific T-cell clones and lines (as described in Materials and Methods of this example above). Multiple high-affinity FVIII₅₈₉₋₆₀₈-specific T-cell clones and lines were isolated. Sorted cells with other specificities did not expand. To evaluate the disease specificity of the DR1101-restricted T-cell responses observed in these two - inhibitor subjects, T cells from six additional non-HA subjects were stimulated with FVIII peptides and stained with tetramers after two weeks of *in vitro* culture. In all cases, tetramer staining was below the positivity threshold (not shown). Despite the limited number of subjects, the magnitude of FVIII₅₈₉₋₆₀₈-specific tetramer staining observed for hemophilic subjects with inhibitors was significantly higher than for healthy subjects (p=0.045). No other tetramer-positive signals were statistically different for patients and controls.

### Binding of truncated peptides to DR1101

To determine the minimal T-cell epitope within FVIII₅₈₉₋₆₀₈, binding of truncated peptides to recombinant DR1101 was measured in a competition assay **(****Figure 18A****).** While FVIII₅₉₂₋₆₀₃ bound with affinity comparable to FVIII₅₈₉₋₆₀₈, the FVIII₅₉₃₋₆₀₃ and FVIII₅₉₄₋₆₀₃ peptides bound with 10-fold and 25-fold lower affinity, respectively. This suggests that residue F594 occupies position 1 of the canonical, nine-residue peptide-binding groove in HLA-DR1101 **(****Figure 18B****),** consistent with an epitope predicted by the computer program Propred [28].

### T-cell clone proliferation and cytokine secretion

Three antigen-specific T-cell clones and one polyclonal T-cell line were isolated from the same peptide-stimulated cultures used for epitope mapping. Clone 1D-1 was stained by tetramers loaded with FVIII₅₈₉₋₆₀₈ but not with FVIII₅₈₁₋₆₀₀ or an unrelated influenza control peptide, HA₃₀₆₋₃₁₈ **(****Figure 19A****).** T cells isolated from subject 41A gave similar results (not shown), indicating that these cells recognize FVIII₅₈₉₋₆₀₈. Proliferation assays were conducted for these T cells using FVIII₅₈₉₋₆₀₈ and truncated versions of this peptide to determine the functional epitope. In all cases, residue R593 was essential for maximal proliferation **(****Figures 19B-E**). Interestingly, peptides containing either R593 (wild-type sequence) or C593 (hemophilic sequence) elicited similar proliferation. These T cells proliferated well above background in response to wild-type FVIII protein **(****Figure 20****).**

Supernatants harvested 48hr following incubation with FVIII₅₈₉₋₆₀₈ were assayed to determine the cytokines secreted in response to FVIII peptide stimulation. Both the T-cell clones and the polyclonal line secreted robust levels of interferon-γ, significant amounts of TNF-α, IL-4, and IL-10, but no IL-17 **(****Figure 21****).** Th1/Th2 ratios ranged from 1.8 to 31.6. In the absence of peptide stimulation, cytokine secretion was negligible.

### Binding of FVIII peptides to additional HLA-DR proteins

To determine which common HLA-DR proteins [34] can effectively present FVIII peptides containing the wild-type R593, the binding of FVIII₅₈₉₋₆₀₈, FVIII_{589-608,593C}, FVIII₅₈₁₋₆₀₀, and FVIII_{581-600,593C} to DR0101, DR0301, DR0401, DR1101, DR1104 and DR1501 proteins, which represent prevalent HLA-DR haplotypes in the Dutch and American study population, was measured. As summarized in **Table 16,** FVIII₅₈₉₋₆₀₈ and FVIII_{589-608,593C} bound to DR0101, DR1101 and DR1501. FVIII₅₈₁₋₆₀₀ bound to DR1101, DR1104, and DR1501. These alleles are found in 33% of individuals in European and non-indigenous North American populations [34]. This suggests that a substantial fraction of haemophilia A patients with F8-R593C, those with DRB1*01, DRB1*1, or DRB1*15 haplotypes, may be at increased risk of inhibitor formation. Of course, additional alleles that were not tested in the present study may also be associated with increased inhibitor risk as well.

### Discussion

Inhibitory antibodies are the most severe complication affecting HA patients with access to FVIII replacement therapy. However, predicting inhibitor development for individuals remains challenging because risk factors include genetic and environmental components [35-43]. Clinical and experimental evidence suggests that responses to FVIII in mild/moderately severe HA can be triggered by differences between endogenous and infused FVIII and can be potentiated by immune challenges [17, 26]. This study of two unrelated HA subjects with established inhibitors (sharing the *F8-R593C* genotype and *HLA-DRB1*1101* allele) demonstrated robust T-cell responses directed against an epitope that contains the wild-type FVIII sequence at the hemophilic mutation site. Mild HA patients would only be exposed to this epitope upon treatment or prevention of bleeding episodes by infusions with wild-type FVIII concentrates. Our experiments also showed that the *in vitro* binding affinity of the wild-type FVIII peptide containing R593 for DR1101 was stronger than that of several other peptides containing predicted high-affinity epitopes. In fact, there was only a weak correlation (R²=0.14) between the observed IC₅₀ value and predicted binding score. These results indicate the importance of complementing epitope prediction methods with physical peptide-binding measurements and T-cell assays in order to obtain an accurate assessment of immunogenicity/antigenicity. Many FVIII peptides bound to DR1101 with high affinity but did not elicit T-cell responses, suggesting that both the mild HA subjects and nonhemophilic individuals have central tolerance to these sequences. Some of these sequences may, however, elicit immune responses in severe HA subjects with no circulating FVIII protein.

In agreement with previous studies of mild HA subjects [16, 17, 44], the experimental results indicate robust T-cell responses directed against an epitope that contains the wild-type sequence at the hemophilic mutation site. For subject 1D **(****Figure 16****),** analysis with a limited set of peptides revealed a high affinity T-cell response directed against FVIII₅₈₉₋₆₀₈ and weaker responses directed against an overlapping peptide (FVIII₅₈₁₋₆₀₀) and two distinct sequences (FVIII₄₂₉₋₄₄₈ and FVIII₄₆₉₋₄₈₈) which appeared to be of lower affinity. T-cell responses of subject 41A were queried using a much larger panel of overlapping FVIII peptides that spanned the FVIII A2, C1, and C2 domains **(****Figure 17****),** and FVIII₅₈₉₋₆₀₈ again elicited a high affinity response. Weaker, apparently low affinity responses were directed against FVIII₄₂₁₋₄₄₀, FVIII₅₈₁₋₆₀₀ and FVIII₂₁₈₇₋₂₂₀₅. Expanded FVIII₅₈₉₋₆₀₈-specific T cells from both HA subjects proliferated in response to FVIII protein, indicating that this peptide mimics a naturally processed epitope. Although it is still possible that additional T-cell responses to regions of FVIII not tested here, e.g., the A1, A3 or B domains, may also contribute to FVIII immunogenicity/antigenicity, our results suggest that high affinity *HLA-DRB1*1101*-restricted T-cell responses to an epitope within FVIII₅₈₉₋₆₀₈ contributed to inhibitor formation in both of these HA subjects. Among the peptides that elicited positive responses, only FVIII₅₈₉₋₆₀₈ had significantly higher staining for HA subjects (p=0.045) than for healthy control subjects. However, it should be noted that due to the limited number of HA subjects analyzed, there were insufficient data to conclude that responses to FVIII₅₈₉₋₆₀₈ occur only in hemophilic subjects with inhibitors. In fact, in a previous study of brothers who shared the DR0101 haplotype and had mild HA due to the A2201P missense genotype, both subjects had T-cell responses to the same peptide (which included the mutation site) even though they were discordant for inhibitor development [18]. However, T-cell clones isolated from their blood had distinctly different phenotypes, and IgG concentrated from plasma donated by the "non-inhibitor" brother had a measurable Bethesda titer, indicating he in fact had a circulating but sub-clinical inhibitor [18, 44]. Therefore, there is accumulating evidence that T-cell responses such as those characterized here indicate the presence of anti-FVIII antibodies, although actual titers may vary significantly.

T-cell help can drive development and maturation of antibody responses. T cells can also exhibit regulatory phenotypes, including FoxP3 expression, anergy, and IL-10 secretion [45]. Therefore, analysis of tetramer-stained, FVIII-specific T-cell clones and the polyclonal T-cell line included quantification of representative Th1 and Th2 cytokines, IL-10, and IL-17. FVIII-specific T cells from both inhibitor subjects secreted robust levels of interferon-γ and detectable TNF-α, IL-4, and IL-10, with Th1/Th2 ratios suggesting varying degrees of Th1-polarization. This is consistent with previous observations that interferon-γ and IL-4 are both secreted by FVIII-stimulated CD4+ T cells from inhibitor subjects [46]. A recent study using a HA mouse model suggested that Th1-polarization was associated with tolerance [47]. A study of a mild HA subject [44] showed that *HLA-DRB1*0101*-restricted T-cell clones isolated two years after inhibitor formation were strongly Th2-polarized, while clones isolated at earlier time points secreted interferon-γ and IL-17. Another study of human inhibitor responses concluded that Th2-driven inhibitors occur when the anti-FVIII antibody response is intense, whereas Th1 cells may be involved in the long-term maintenance of anti-FVIII antibody synthesis [48]. Additional studies evaluating changes in T-cell phenotypes and responses over time, particularly in subjects matched by disease severity, genetic characteristics including F8 genotype and HLA haplotype, and treatment regime, are needed to determine mechanisms leading to tolerance versus high-titer anti-FVIII antibodies.

Initial T-cell proliferation experiments revealed the existence of an epitope within the FVIII₅₈₉₋₆₀₈ peptide. Although responses of the single clone obtained from subject 1D were not as vigorous as those of the cells isolated from subject 41A, proliferation assays indicated robust responses to FVIII₅₉₂₋₆₀₃ for all three clones and for the polyclonal line. Their proliferation was less pronounced in response to FVIII₅₉₄₋₆₀₃, highlighting the importance of the R593 residue. The experimental results and prediction algorithms both indicated that F594 occupies position 1 in the DR1101 peptide-binding groove, while N597, A599 and Q602 fit into the pockets at positions 4, 6 and 9, and adjacent and intervening side chains project outward to interact with T-cell receptors [49].

Interestingly, all three expanded T-cell clones and the polyclonal line proliferated in response to the hemophilic FVIII_{589-608,593C} peptide, despite the fact that neither primary nor cloned T cells were stained by tetramers loaded with this peptide, suggesting a lower-avidity interaction of T cells with tetramers or antigen-presenting cells when the hemophilic peptide was presented on the DR1101 1 surface. Peptide affinities for DR1101 are determined by the fit of peptide "anchor" residues into specific pockets in the class II binding groove, whereas tetramer staining of cells has the additional requirement that the DR1101-peptide complex be recognized by the T-cell receptor on the surface of the responding T cell. Residue 593 is adjacent to the classic 9-residue class II binding motif, but it clearly contributes to binding affinities. The results imply that although the tetramer loaded with the hemophilic peptide was less effective in staining the T cells (so that labeled cells were below the threshold for a "tetramer-positive" response) this lower-avidity interaction was nevertheless strong enough to stimulate T-cell proliferation. This raises the possibility that T cells initially activated by wild-type FVIII can cross-react with wild-type and hemophilic FVIII. This cross-reactivity at the T-cell level may be analogous with cross-reactivity seen at the B-cell level for both subjects, whose inhibitors neutralized their endogenous FVIII. Cross-maintenance of FVIII₅₈₉₋₆₀₈-specific T cells by the endogenous peptide/protein containing the substitution R593C may also contribute to the persistence of immune responses to FVIII; indeed, inhibitors and epitope-specific T-cell responses to FVIII have been observed in mild HA subjects even years after their last infusion [17, 44].

Peptide affinities for a series of HLA-DR proteins indicated that DR0101, DR1104, and DR1501, but not DR0301 and DR0401, can present FVIII peptides containing R593. This reinforces previous suggestions that while HLA haplotypes are not a general risk factor for inhibitor development, certain combinations of FVIII genotype and HLA haplotype may confer an increased risk [7, 50]. In the American and Dutch cohorts of *F8-R593C* hemophilia subjects (69 total subjects) nine of the ten (90%) inhibitor subjects had *DRB1*01, DRB1*11, or DRB1*15* haplotypes, while 26 of the 59 (44%) subjects without inhibitors had these haplotypes [7 and unpublished data]. These alleles are found in 33% of individuals in European and non-indigenous North American populations [34]. Fisher's exact probability test indicates that this is a significant increase (p-value = 0.0076) in inhibitor risk for subjects with *these* alleles, as compared to all other class II HLA types. However, these results should be replicated using larger populations and accounting for confounding factors such as intensity of treatment [9] and genetic determinants such as IL-10 [36] and TNF-α [38] polymorphisms, before drawing firm conclusions about HLA-associated inhibitor risks.

T-cell responses to FVIII were characterized for two unrelated individuals in this study. Both demonstrated Th1-polarized responses (with accompanying low-level IL-4 secretion) directed against a common *HLA-DRB1*1101*-restricted epitope, supporting the notion that T-cell responses to epitopes that contain the hemophilic substitution site contribute to inhibitor formation in mild/moderately severe HA. These T cell responses may occur whenever epitopes containing the wild-type sequence at a missense site are bound to and presented by particular DR proteins at the surface of an antigen-presenting cell. Knowledge of HLA-restricted T-cell epitopes in FVIII and their binding affinities for HLA-DR and possibly other MHC class II proteins should improve predictions of inhibitor risk. Only certain MHC class II proteins on the surface of antigen-presenting cells will likely be capable of effectively presenting particular FVIII peptides.

**Table 15**

| | **FVIII-A2 Peptides** | **Sequence** | **SEQ ID NO** | **IC₅₀**^{†} | **Predicted DR1101 Binding Score^{‡}** |
|---|---|---|---|---|---|
| 1 | 429-488 | MAYTDETFKTREAIQHESG I | 194 | **8.9±8** | 1.3 |
| 2 | 453-472 | LYGEVGDTLLIIFKNQASR P | 195 | **0.2±0.1** | 2.7 |
| 3 | 469-488 | ASRPYNIYPHGITDVRPLY S | 196 | >100 | 0.8 |
| 4 | 501-520 | FPILPGEIFKYKWTVTVED G | 197 | >100 | 0.9 |
| | | LTRYYSSFVNMERDLASGL | 198 | | |
| 5 | 529-548 | I | | **0.2±0.0 6** | 1.9 |
| 6 | 541-560 | RDLASGLIGPLLICYKESV D | 199 | 25±24 | 1.3 |
| 7 | 581-600 | ENRSWYLTENIQRFLPNPA G | 200 | **0.5±0.4** | 0.8 |
| 8 | 581-600,593C | ENRSWYLTENIQCFLPNPA G | 201 | >100 | 1.5 |
| 9 | 589-608 | ENIQRFLPNPAGVQLEDPE F | 202 | **0.5±0.4** | 1.4 |
| 10 | 589-608,593C | ENIQCFLPNPAGVQLEDPE F | 203 | **1.5±1.7** | 1.4 |
| 11 | 605-624 | DPEFQASNIMHSINGYVFD S | 204 | **8.9±20** | 3.2 |
| 12 | 610-629 | ASNIMHSINGYVFDSLQLS V | 205 | >100 | 1.0 |
| 13 | 637-656 | LHEVAYWYILSIGAQTDFL S | 206 | **0.3±0.4** | 4.3 |
| 14 | 653-672 | FSGYTFKHKMVYEDTLTLF P | 207 | 20±47 | 1.9 |
| 15 | 661-680 | FSGYTFKHKMVYEDTLTLF P | 208 | >20 | 1.9 |
| 16 | 677-696 | TVFMSMENPGLWILGCHNS D | 209 | >100 | 2.0 |
| 17 | 685-704 | TVFMSMENPGLWILGCHNS D | 210 | >100 | 2.0 |
| | FVIII-A2 domain peptides predicted to bind DR1101 with high affinity, using the ProPred algorithm [28]. Peptides subsequently pooled and used to stimulate T cells are in bold font; the three remaining peptides contained predicted MHC Class II binding motifs (the 9-residue sequences predicted to fit into the HLA-DR1101 binding groove, underlined for each peptide) that were also present in one of the other peptides. Binding scores generated by Propred for all peptides are in the far right column (higher scores indicate stronger predicted affinity). Measured IC₅₀ values under 10 are in bold font. | | | | |
| | † IC₅₀ values are shown in µM ± the standard error of the mean. A lower IC₅₀ value indicates stronger binding. IC₅₀>100 indicates no detectable binding in the assay. | | | | |
| | ‡ The binding score reflects expected binding affinity. Higher scores indicate stronger binding. | | | | |

**Table 16: Binding of Peptides to DRB1 Proteins**

| Class II protein | Reference peptide* (IC₅₀ in µM) | IC₅₀^{†} (µm) FVIII₅₈₁₋₆₀₀ | IC₅₀^{†} (µm) FVIII_{581-600,593C} | IC₅₀^{†} (µm) FVIII₅₈₉₋₆₀₈ | IC₅₀^{†} (µm) FVIII_{589-608,593C} |
|---|---|---|---|---|---|
| DR0101 | HA₃₀₆₋₃₁₈(0.26) | 38±30 | 50±3 | 4.2±0.3 | 8.3±0.7 |
| DR0301 | Myo₁₃₇₋₁₄₈ (0.82) | 44±7 | NB^{‡} | 50±4 | NB^{‡} |
| DR0401 | HA₃₀₆₋₃₁₈ (3.1) | 48±7 | NB^{‡} | 38±3 | NB^{‡} |
| DR1101 | HA₃₀₆₋₃₁₈ (5.0) | 1.1±0.1 | NB^{‡} | 1.1±0.1 | 6.3±0.6 |
| DR1104 | VP16₃₄₋₄₄ (3.1) | 9.8±0.8 | NB^{‡} | 59±3 | NB^{‡} |
| DR1501 | MBP₈₄₋₁₀₂ (0.05) | 3.7±0.4 | 56±4 | 4.6±0.4 | 9.8±0.6 |
| * IC₅₀ indicates the strength of interaction between the class II protein and FVIII peptide compared to a reference peptide (sequences shown in Supplementary Table 1). IC₅₀ values for reference peptides are listed in parentheses. Lower numbers indicate stronger interactions. | | | | | |
| † Values shown ± standard error of the mean | | | | | |
| ‡ NB indicates no binding | | | | | |

**Table 17: Peptide Sequences**

| Pool | Residue numbers | Peptide sequence | SEQ ID NO |
|---|---|---|---|
| A2-1 | FVIII 373-392 | SVAKKHPKTWVHYIAAEEED | 211 |
| | FVIII 381-400 | TWVHYIAAEEEDWDYAPLVL | 212 |
| | FVIII 389-408 | EEEDWDYAPLVLAPDDRSYK | 213 |
| | FVIII 397-416 | PLVLAPDDRSYKSQYLNNGP | 214 |
| | FVIII 405-424 | RSYKSQYLNNGPQRIGRKYK | 215 |
| A2-2 | FVIII 413-432 | NNGPQRIGRKYKKVRFMAYT | 216 |
| | FVIII 421-440 | RKYKKVRFMAYTDETFKTRE | 217 |
| | FVIII 429-448 | MAYTDETFKTREAIQHESGI | 218 |
| | FVIII 437-456 | KTREAIQHESGILGPLLYGE | 219 |
| | FVIII 445-464 | ESGILGPLLYGEVGDTLLII | 220 |
| A2-3 | FVIII 453-472 | LYGEVGDTLLIIFKNQASRP | 221 |
| | FVIII 461-480 | LLIIFKNQASRPYNIYPHGI | 222 |
| | FVIII 469-488 | ASRPYNIYPHGITDVRPLYS | 223 |
| | FVIII 477-496 | PHGITDVRPLYSRRLPKGVK | 224 |
| | FVIII 485-504 | PLYSRRLPKGVKHLKDFPIL | 225 |
| A2-4 | FVIII 493-512 | KGVKHLKDFPILPGEIFKYK | 226 |
| | FVIII 501-520 | FPILPGEIFKYKWTVTVEDG | 227 |
| | FVIII 509-528 | IFKYKWTVTVEDGPTKSDPR | 228 |
| | FVIII 517-536 | VEDGPTKSDPRCLTRYYSSF | 229 |
| | FVIII 525-544 | DPRCLTRYYSSFVNMERDLA | 230 |
| A2-5 | FVIII 529-548* | LTRYYSSFVNMERDLASGLI | 231 |
| | FVIII 533-552* | YSSFVNMERDLASGLIGPLL | 232 |
| | FVIII 541-560 | RDLASGLIGPLLICYKESVD | 233 |
| | FVIII 549-568 | GPLLICYKESVDQRGNQIMS | 234 |
| | FVIII 557-576 | ESVDQRGNQIMSDKRNVILF | 235 |
| A2-6 | FVIII 565-584 | QIMSDKRNVILFSVFDENRS | 236 |
| | FVIII 573-592 | VILFSVFDENRSWYLTENIQ | 237 |
| | FVIII 581-600 | ENRSWYLTENIQRFLPNPAG | 238 |
| | FVIII 589-608 | ENIQRFLPNPAGVQLEDPEF | 239 |
| | FVIII 597-616 | NPAGVQLEDPEFQASNIMHS | 240 |
| A2-7 | FVIII 605-624 | DPEFQASNIMHSINGYVFDS | 241 |
| | FVIII 613-632 | IMHSINGYVFDSLQLSVCLH | 242 |
| | FVIII 610-619* | ASNIMHSINGYVFDSLQLSV | 243 |
| | FVIII 621-640 | VFDSLQLSVCLHEVAYWYIL | 244 |
| | FVIII 629-648 | VCLHEVAYWYILSIGAQTDF | 245 |
| A2-8 | FVIII 637-656* | LHEVAYWYILSIGAQTDFLS | 246 |
| | FVIII 645-664 | WYILSIGAQTDFLSVFFSGY | 247 |
| | FVIII 653-672 | QTDFLSVFFSGYTFKHKMVY | 248 |
| | FVIII 661-680 | FSGYTFKHKMVYEDTLTLFP | 249 |
| | FVIII 669-688 | KMVYEDTLTLFPFSGETVFM | 250 |
| A2-9 | FVIII 677-696 | TLFPFSGETVFMSMENPGLW | 251 |
| | FVIII 685-704 | TVFMSMENPGLWILGCHNSD | 252 |
| | FVIII 672-691 | PFSGETVFMSMENPGLWILG | 253 |
| | FVIII 685-704 | PGLWILGCHNSDFRNRGMTA | 254 |
| | FVIII 693-712 | HNSDFRNRGMTALLKVSSCD | 255 |
| A2-10 | FVIII 693-710* | HNSDFRNRGMTALLKVSS | 256 |
| | FVIII 701-720 | GMTALLKVSSCDKNTGDYYE | 257 |
| | FVIII 709-728 | SSCDKNTGDYYEDSYEDISA | 258 |
| | FVIII 712-731* | DKNTGDYYEDSYEDISAYLL | 259 |
| | FVIII 717-740 | DYYEDSYEDISAYLLSKNNA IEPR | 260 |
| A2-11 | FVIII 581-600,593C | ENRSWYLTENIQCFLPNPAG | 261 |
| | FVIII 589-608,593C | ENIQCFLPNPAGVQLEDPEF | 262 |
| C1-1 | FVIII 2004-2023 | EHLHAGMSTLFLVYSNKCQT | 263 |
| | FVIII 2001-2020 | LIGEHLHAGMSTLFLVYSNK | 264 |
| | FVIII 2012-2031 | TLFLVYSNKCQTPLGMASGH | 265 |
| | FVIII 2020-2039 | KCQTPLGMASGHIRDFQITA | 266 |
| | FVIII 2022-2041 | QTPLGMASGHIRDFQITASG | 267 |
| C1-2 | FVIII 2028-2147 | ASGHIRDFQITASGQYGQWA | 268 |
| | FVIII 2036-2055 | QITASGQYGQWAPKLARLHY | 269 |
| | FVIII 2044-2063 | GQWAPKLARLHYSGSINAWS | 270 |
| | FVIII 2052-2071 | RLHYSGSINAWSTKEPFSWI | 271 |
| | FVIII 2060-2079 | NAWSTKEPFSWIKVDLLAPM | 272 |
| C1-3 | FVIII 2068-2087 | FSWIKVDLLAPMIIHGIKTQ | 273 |
| | FVIII 2076-2095 | LAPMIIHGIKTQGARQKFSS | 274 |
| | FVIII 2084-2103 | IKTQGARQKFSSLYISQFII | 275 |
| | FVIII 2092-2111 | KFSSLYISQFIIMYSLDGKK | 276 |
| | FVIII 2100-2119 | QFIIMYSLDGKKWQTYRGNS | 277 |
| C1-4 | FVIII 2108-2127 | DGKKWQTYRGNSTGTLMVFF | 278 |
| | FVIII 2116-2135 | RGNSTGTLMVFFGNVDSSGI | 279 |
| | FVIII 2124-2143 | MVFFGNVDSSGIKHNIFNPP | 280 |
| | FVIII 2132-2151 | SSGIKHNIFNPPIIARYIRL | 281 |
| | FVIII 2140-2159 | FNPPIIARYIRLHPTHYSIR | 282 |
| C1-5 | FVIII 2148-2167 | YIRLHPTHYSIRSTLRMELM | 283 |
| | FVIII 2154-2173 | THYSIRSTLRMELMGCDLNS | 284 |
| C2-1 | FVIII 2170-2189 | DLNSCSMPLGMESKAISDAQ | 285 |
| | FVIII 2178-2197 | LGMESKAISDAQITASSYFT | 286 |
| | FVIII 2187-2205 | DAQITASSYFTNMFATWSP | 287 |
| C2-2 | FVIII 2186-2205 | SDAQITASSYFTNMFATWSP | 288 |
| | FVIII 2194-2213 | SYFTNMFATWSPSKARLHLQ | 289 |
| | FVIII 2202-2221 | TWSPSKARLHLQGRSNAWRP | 290 |
| | FVIII 2210-2229 | LHLQGRSNAWRPQVNNPKEW | 291 |
| | FVIII 2218-2237 | AWRPQVNNPKEWLQVDFQKT | 292 |
| C2-3 | FVIII 2226-2245 | PKEWLQVDFQKTMKVTGVTT | 293 |
| | FVIII 2234-2253 | FQKTMKVTGVTTQGVKSLLT | 294 |
| | FVIII 2242-2261 | GVTTQGVKSLLTSMYVKEFL | 295 |
| | FVIII 2250-2269 | SLLTSMYVKEFLISSSQDGH | 296 |
| | FVIII 2258-2277 | KEFLISSSQDGHQWTLFFQN | 297 |
| C2-4 | FVIII 2265-2284 | SQDGHQWTLFFQNGKVKVFQ | 298 |
| | FVIII 2273-2292 | LFFQNGKVKVFQGNQDSFTP | 299 |
| | FVIII 2281-2300 | KVFQGNQDSFTPVVNSLDPP | 300 |
| | FVIII 2289-2308 | SFTPVVNSLDPPLLTRYLRI | 301 |
| | FVIII 2297-2316 | LDPPLLTRYLRIHPQSWVHQ | 302 |
| C2-5 | FVIII 2305-2324 | YLRIHPQSWVHQIALRMEVL | 303 |
| | FVIII 2313-2332 | WVHQIALRMEVLGCEAQDLY | 304 |
| | FVIII 2313-2327 | WVHQIALRMEVLGCE | 305 |
| | FVIII 2317-2332 | IALRMEVLGCEAQDLY | 306 |
| Refe renc e Pept ides | Influenza HA 306-318^{a} | PKYVKQNTLKLAT | 307 |
| | sw Myoglobin 137-148^{b} | LFRKDIAAKYKE | 308 |
| | HSV-2 VP16 34-44^{c} | PLYATGRLSQA | 309 |
| | Human MBP 84-102^{d} | NPVVHFFKNIVTPRTPPPS | 310 |
| * peptide designed to avoid including a free cysteine | | | |
| ^{a} reference peptide for DR0101, DR0401, and DR1101 | | | |
| ^{b} reference peptide for DR0301 | | | |
| ^{c} reference peptide for DR1104 | | | |
| ^{d} reference peptide for DR1501 | | | |

### Example 5 References

1. Hoyer, LW. Factor VIII Inhibitors. Curr Opin Hematol 1995; 2: 365-71.
2. Bray GL, Gomperts ED, Courter S, Gruppo R, Gordon EM, Manco-Johnson M, Shapiro A, Scheibel E, White G 3rd, Lee M. A multicenter study of recombinant factor VIII (recombinate): safety, efficacy, and inhibitor risk in previously untreated patients with hemophilia A. The Recombinate Study Group. Blood 1994; 83: 2428-35.
3. Kreuz W, Ettingshausen CE, Zyschka A, Oldenburg J, Saguer IM, Ehrenforth S, Klingebiel T. Inhibitor development in previously untreated patients with hemophilia A: a prospective long-term follow-up comparing plasma-derived and recombinant products. Semin Thromb Hemost 2002; 28: 285-90.
4. Hay CR. Factor VIII inhibitors in mild and moderate-severity haemophilia A. Haemophilia 1998; 4: 558-63.
5. d'Oiron R, Pipe SW, Jacquemin M. Mild/moderate haemophilia A: new insights into molecular mechanisms and inhibitor development. Haemophilia 2008; 14 S3: 138-46.
6. Oldenburg J, Pavlova A. Genetic risk factors for inhibitors to factors VIII and IX. Haemophilia 2006; 12 S6: 15-22.
7. Bril WS, MacLean PE, Kaijen PH, van den Brink EN, Lardy NM, Fijnvandraat K, Peters M, Voorberg J. HLA class II genotype and factor VIII inhibitors in mild haemophilia A patients with an Arg593 to Cys mutation. Haemophilia 2004; 10: 509-14.
8. Thompson AR, Murphy ME, Liu M, Saenko EL, Healey JF, Lollar P, Scandella D. Loss of tolerance to exogenous and endogenous factor VIII in a mild hemophilia A patient with an Arg593 to Cys mutation. Blood 1997; 90: 1902-10.
9. Eckhardt CL, Menke LA, van Ommen CH, van der Lee JH, Geskus RB, Kamphuisen PW, Peters M, Fijnvandraat K. Intensive peri-operative use of factor VIII and the Arg593 → Cys mutation are risk J Thromb Haemost factors for inhibitor development in mild/moderate hemophilia A. 2009; 7: 930-37.
10. van Den Brink EN, Turenhout EA, Davies J, Bovenschen N, Fijnvandraat K, Ouwehand WH, Peters M, Voorberg J. Human antibodies with specificity for the C2 domain of factor VIII are derived from VH1 germline genes. Blood 2000; 95: 558-63.
11. Fulcher CA, de Graaf Mahoney S, Zimmerman TS. FVIII inhibitor IgG subclass and FVIII polypeptide specificity determined by immunoblotting. Blood 1987; 69: 1475-80.
12. Shima M. Characterization of factor VIII inhibitors. Int J Hematol 2006; 83: 109-18.
13. Lacroix-Desmazes S, Misra N, Bayry J, Mohanty D, Kaveri SV, Kazatchkine MD. Autoantibodies to factor VIII. Autoimmun Rev 2002; 1: 105-10.
14. Ewenstein BM, Hoots WK, Lusher JM, DiMichele D, White GC 2nd, Adelman B, Nadeau K. Inhibition of CD40 ligand (CD154) in the treatment of factor VIII inhibitors. Haematologica 2000; 85: 35-9.
15. Bray GL, Kroner BL, Arkin S, Aledort LW, Hilgartner MW, Eyster ME, Ragni MV, Goedert JJ. Loss of high-responder inhibitors in patients with severe hemophilia A and human immunodeficiency virus type 1 infection: a report from the Multi-Center Hemophilia Cohort Study. Am JHematol 1993; 42: 375-79.
16. Jacquemin M, Vantomme V, Buhot C, Lavend'homme R, Burny W, Demotte N, Chaux P, Peerlinck K, Vermylen J, Maillere B, van der Bruggen P, Saint-Remy JM. CD4+ T-cell clones specific for wild-type factor VIII: a molecular mechanism responsible for a higher incidence of inhibitor formation in mild/moderate hemophilia A. Blood 2003; 101: 1351-8.
17. James EA, Kwok WW, Ettinger RA, Thompson AR, Pratt KP. T-cell Responses over time in a mild hemophilia A inhibitor subject: epitope identification and transient immunogenicity/antigenicity of the corresponding self-peptide. Thromb Haemost 2007; 5: 2399-407.
18. Ettinger RA, James EA, Kwok WW, Thompson AR, and Pratt KP. HLA-DR-restricted T-cell responses to Factor VIII epitopes in a mild haemophilia A family with missense substitution A2201P. Haemophilia 2010; 16: 44-55.
19. Peerlinck K, Jacquemin MG, Arnout J, Hoylaerts MF, Gilles JG, Lavend'homme R, Johnson KM, Freson K, Scandella D, Saint-Remy JM, Vermylen J. Antifactor VIII antibody inhibiting allogeneic but not autologous factor VIII in patients with mild hemophilia A. Blood 1999; 93: 2267-73.
20. d'Oiron R, Lavergne JM, Lavend'homme R, Benhida A, Bordet JC, Negrier C, Peerlinck K, Vermylen J, Saint-Remy JM, Jacquemin M. Deletion of alanine 2201 in the FVIII C2 domain results in mild hemophilia A by impairing FVIII binding to VWF and phospholipids and destroys a major FVIII antigenic determinant involved in inhibitor development. Blood 2004; 103: 155-7.
21. Jacquemin M, Benhida A, Peerlinck K, Desqueper B, Vander Elst L, Lavend'homme R, d'Oiron R, Schwaab R, Bakkus M, Thielemans K, Gilles JG, Vermylen J, Saint-Remy JM. A human antibody directed to the factor VIII C1 domain inhibits factor VIII cofactor activity and binding to von Willebrand factor. Blood 2000; 95: 156-63.
22. Hu GL, Okita DK, Conti-Fine BM. T cell recognition of the A2 domain of coagulation factor VIII in hemophilia patients and healthy subjects. J Thromb Haemost 2004; 2: 1908-17.
23. Reding MT, Wu H, Krampf M, Okita DK, Diethelm-Okita BM, Key NS, Conti-Fine BM. CD4+ T cell response to factor VIII in hemophilia A, acquired hemophilia and healthy subjects. Thromb Haemost 1999; 82: 509-15.
24. Jones TD, Phillips WJ, Smith BJ, Bamford CA, Nayee PD, Baglin TP, Gaston JS, Baker MP. Identification and removal of a promiscuous CD4+ T cell epitope from the C1 domain of factor VIII. J Thromb Haemost 2005; 3: 991-1000.
25. Reding MT, Wu H, Krampf M, Okita DK, Diethelm-Okita BM, Christie BA, Key NS, Conti-Fine BM. Sensitization of CD4+ T cells to coagulation factor VIII: response in congenital and acquired hemophilia patients and in healthy subjects. Thromb Haemost 2000; 84: 643-52.
26. Fijnvandraat K, Turenhout EA, van den Brink EN, ten Cate JW, van Mourik JA, Peters M, Voorberg J. The missense mutation Arg593 --> Cys is related to antibody formation in a patient with mild hemophilia A. Blood 1997; 89: 4371-77.
27. Bril WS, Turenhout EA, Kaijen PH, van den Brink EN, Koopman MM, Peters M, Voorberg J. Analysis of factor VIII inhibitors in a haemophilia A patient with an Arg593-->Cys mutation using phage display. Br J Haematol 2002; 119: 393-96.
28. Singh H, Raghava GP. ProPred: prediction of HLA-DR binding sites. Bioinformatics 2001; 17: 1236-7.
29. Verreck FA, van de Poel A, Drijfhout JW, Amons R, Coligan JE, Konig F. Natural peptides isolated from Gly86/Val86-containing variants of HLA-DR1, -DR11, -DR13, and -DR52. Immunogenetics 1996; 43: 392-7.
30. James EA, Moustakas AK, Berger D, Huston L, Papadopoulos GK, Kwok WW. Definition of the peptide binding motif within DRB1*1401 restricted epitopes by peptide competition and structural modeling. Mol Immunol 2008; 45: 2651-9.
31. Novak EJ, Liu AW, Nepom GT, Kwok WW. MHC class II tetramers identify peptide-specific human CD4(+) T cells proliferating in response to influenza A antigen. J Clin Invest 1999; 104: R63-7.
32. James EA, Bui J, Berger D, Huston L, Roti M, Kwok WW. Tetramer-guided epitope mapping reveals broad, individualized repertoires of tetanus toxin-specific CD4+ T cells and suggests HLA-based differences in epitope recognition. Int Immunol 2007; 19: 1291-301.
33. Novak EJ, Liu AW, Gebe JA, Falk BA, Nepom GT, Koelle DM, Kwok WW. Tetramer-guided epitope mapping: rapid identification and characterization of immunodominant CD4+ T cell epitopes from complex antigens. J Immunol 2001; 166: 6665-70.
34. Meyer D, Singe RM, Mack SJ, Lancaster A, Nelson MP, Erlich H, Fernandez-Vina M, Thomson G. Single Locus Polymorphism of Classical HLA Genes. In: Hansen JA, ed. Immunobiology of the Human MHC: Proceedings of the 13th International Histocompatibility Workshop and Conference, Vol 1. Seattle, WA: IHWG Press; 2007; 653-704.
35. Oldenburg J, Schröder J, Brackmann HH, Müller-Reible C, Schwaab R, Tuddenham E. Environmental and genetic factors influencing inhibitor development. Semin Hematol 2004; 41 S1: 82-8.
36. Astermark J, Oldenburg J, Pavlova A, Berntorp E, Lefvert AK; MIBS Study Group. Polymorphisms in the IL10 but not in the IL1beta and IL4 genes are associated with inhibitor development in patients with hemophilia A. Blood 2006; 107: 3167-72.
37. Lee CA, Lillicrap D, Astermark J. Inhibitor development in hemophiliacs: the roles of genetic versus environmental factors. Semin Thromb Hemost 2006; 32 S2: 10-14.
38. Astermark J, Oldenburg J, Carlson J, Pavlova A, Kavakli K, Berntorp E, Lefvert AK. Polymorphisms in the TNFA gene and the risk of inhibitor development in patients with hemophilia A. Blood 2006; 108: 3739-45.
39. Repessé Y, Slaoui M, Ferrandiz D, Gautier P, Costa C, Costa JM, Lavergne JM, Borel-Derlon A. Factor VIII (FVIII) gene mutations in 120 patients with hemophilia A: detection of 26 novel mutations and correlation with FVIII inhibitor development. J Thromb Haemost 2007; 5: 1469-76.
40. Pavlova A, Delev D, LaCrois-Desmazes S, Schwaab R, Mende M, Fimmers R, Astermark J, Oldenburg J. Impact of polymorphisms of the major histocompatibility complex class II, interleukin-10, tumor necrosis factor-α and cytotoxic T-lymphocyte antigen-4 genes on inhibitor development in severe hemophilia A. 2009; J Thromb Haemost 7: 2006-15.
41. Gouw SC, van den Berg M. The multifactorial etiology of inhibitor development in hemophilia: Genetics and environment. 2009; Sem Thromb Hemostas 35: 723-34.
42. Astermark J, Altisent C, Batarova A, Diniz MJ, Gringeri A, Holme PA, Karafoulidou A, Lopez-Fernández MF, Reipert BM, Rocino A, Schiavoni M, von Depka M, Windyga J, Fijnvandraat K. Non-genetic risk factors and the development of inhibitors in haemophilia: a comprehensive review and consensus report. Haemophilia 2010 Apr. 14; 1-20.
43. Bafunno V, Santacroce R, CHetta M, D'Andrea G, Pisanelli D, Sessa F, Trota T, Tagariello G, Peyvandi F, Margaglione M. Polymorphisms in genes involved in autoimmune disease and the risk of FVIII inhibitor development in Italian patients with haemophilia A. Haemophilia 2010; 16: 469-73.
44. Ettinger RA, James EA, Kwok WW, Thompson AR, Pratt KP. Lineages of human T-cell clones, including T helper 17 / T helper 1 cells, isolated at different stages of anti-factor VIII immune responses. Blood 2009; 114: 1423-8.
45. Fehérvari Z and Sakaguchi S. CD4+ Tregs and immune control. J Clin Invest 2004; 114: 1209-17.
46. Hu G, Guo D, Key NS, Conti-Fine BM. Cytokine production by CD4+ T cells specific for coagulation factor VIII in healthy subjects and haemophilia A patients. Thromb Haemost 2007; 97: 788-94.
47. Waters B, Qadura M, Burnett E, Chegeni R, Labelle A, Thompson P, Hough C, Lillicrap D. Anti-CD3 prevents factor VIII inhibitor development in hemophilia A mice by a regulatory CD4+CD25+-dependent mechanism and by shifting cytokine production to favor a Th1 response. Blood 2009; 113: 193-203.
48. Reding MT, Lei S, Lei H, Green D, Gill J, Conti-Fine BM. Distribution of Th1- and Th2-induced anti-factor VIII IgG subclasses in congenital and acquired hemophilia patients. Thromb Haemost 2002; 88: 568-75.
49. Hammer J, Valsasnini P, Tolba K, Bolin D, Higelin J, Takacs B, Sinigaglia F. Promiscuous and allele-specific anchors in HLA-DR-binding peptides. Cell 1993; 74: 197-203.
50. White GC 2nd, Kempton CL, Grimsley A, Nielsen B, Roberts HR. Cellular immune responses in hemophilia: why do inhibitors develop in some, but not all hemophiliacs? J Thromb Haemost 2005; 3: 1676-81.

### Example 6

### Introduction

In order to map the B-cell epitopes of monoclonal anti-factor VIII C2 domain inhibitors, forty five surface residues of the C2 domain (Pratt et al., Nature 1999, 402, p. 439) were chosen and changed to alanine or another structurally conservative amino acid.

Competition ELISAs and functional assays were used to classify the antibodies into five groups corresponding to distinct regions on the C2 surface (Meeks et al., Blood 110, 4234-42, 2007). The present study is a high-resolution mapping of the epitope recognized by antibodies (2-77, 2-117, 3D12, 3E6, I109 and I54) using surface plasmon resonance (SPR). The association and dissociation rates for binding of these proteins to the six monoclonal antibodies were determined, in order to determine which mutations affected the binding kinetics for particular antibodies. Altered binding kinetics to one but not all monoclonal antibodies was taken to indicate the corresponding wild-type residues comprised part of the B-cell epitope to that antibody.

### Experimental

Protocols for producing and purifying recombinant FVIII C2 domain proteins were the same as those described for the experiments involving B02C11 epitope mapping.

Briefly, select surface residues of the C2 domain (Pratt et al., Nature 1999, 402, p. 439) were changed to alanine or another structurally conservative amino acid. Recombinant FVIII C2 domain proteins were generated in an E. coli expression system. These poly-His tagged proteins were purified on a nickel column and analyzed by SDS-PAGE (>90% purity).

Epitope mapping of the C2-domain inhibitors was undertaken via the technique of Surface Plasmon Resonance (SPR). Kinetics data was obtained from a Biacore T100 instrument using SPR chips and protocols based on the manufacturer's recommendations.

The antibodies were either attached covalently to a CM5 chip or captured using rat anti-mouse IgG covalently bound to the chip. The length of association and dissociation time between wild-type and mutant proteins was chosen to allow accurate analysis of binding kinetics.

A1:1 binding model was used to determine k_{dissoc} values. Substitutions that resulted in at least a 4-fold increase in k_{dissoc} threw light on those residues which contributed significant binding energy to the mutant-antibody interaction. In other words, these residues were strong candidates for side chains comprising part of the B-cell epitope recognized by the monoclonal antibody being tested.

Kinetics analysis of the mutants' interaction with additional antibodies, e.g. monoclonal antibodies BO2C11(Fab), 2-77, 2-117, 3D12, 3E6, I109, I54 and ESH8, was carried out as an indication of proper folding of the mutant C2 domain proteins. Almost all of the mutant C2 domain proteins that showed altered binding to one monoclonal antibody were found to bind other monoclonal antibodies with similar kinetics to wild-type FVIII-C2 protein (not shown). This result indicates that the mutations did not affect the structure and folding of the mutant FVIII C2 domain proteins.

### Results and Discussion

Four amino acid substitutions abrogated the binding of the corresponding mutant C2 proteins to certain monoclonal antibodies. See Figure 22.

The six representative antibodies studied here have been classified as being one of five of types A, B, C, AB, BC. These correspond to five regions on the C2 surface.
3E6 and I54 are of type A
3D12 is of type B
2-117 is of type C
I109 is of type AB
2-77 is of type BC
ESH8 is of type C

The four amino acid substitutions that abrogated the binding of the corresponding FVIII C2 domain protein to a particular antibody are as follows:

**mutant monoclonal antibody**

| | |
|---|---|
| Q2213A | I54 |
| R2220A | 3D12 |
| R2220A | I109 |
| T2272A | 3D12 |
| T2272A | I109 |
| L2273A | 2-117 |

### Example 7: Administration of a modified factor VIII to a mammal in need thereof.

Mammals (e.g., mice, rats, rodents, humans, guinea pigs) are used in the study. Mammals are administered (e.g., intravenously) one or more modified factor VIIIs described herein or a control. In some instances the modified factor VIII is SEQ ID NO:2. In some instances the modified factor VIII is a factor VIII polypeptide with at least one amino acid modification at a position corresponding to positions 2194-2213, 2194-2205, 2202-2221, or 589-608 of the amino acid sequence set forth in SEQ ID NO:1. In some instances the modified factor VIII is a factor VIII polypeptide with a modification in an epitope or amino acid residue as shown in Table B. In some instances the modified factor VIII is a modified factor VIII polypeptide described in the summary section above. The modified factor VIII can be any of those disclosed herein. Various types of modifications can be used, e.g., additions, delections, substitutions, and/or chemical modifications. In some instances the modified factor VIII is formulated in a pharmaceutically acceptable carrier. In some instances the modified factor VIII is formulated as described in the pharmaceutical compositions section above, e.g., using the same methods and dosages used for administration of an unmodified factor VIII.

Multiple rounds of doses are used where deemed useful. Effects on factor VIII-specific immune responses, inflammatory cytokine levels, and/or conditions associated with hemophilia are monitored in the mammals, e.g., via tetramer analysis, ELISA, and other methods known in the art. Similar studies are performed with different treatment protocols and administration routes (e.g., intramuscular administration, etc.). The effectiveness of a modified factor VIII is demonstrated by measuring the anti-FVIII antibody titer (either absolute titer or neutralizing activity titer, the latter measured in Bethesda units/mL). Effectiveness may also be measured by measuring FVIII half-life, relative affinitiy FVIII binding to von Willebrand factor, phospholipids or platelets, and binding to other serine proteases in the coagulation cascade, or by comparing the factor VIII-specific immune responses, inflammatory cytokine levels, and/or conditions associated with hemophilia in mammals treated with a modified factor VIII disclosed herein to mammals treated with control formulations and/or an unmodified factor VIII.

In an example, a human subject in need of treatment is selected or identified. The subject can be in need of, e.g., reducing, preventing, or treating a condition associated with an immune response to factor VIII and/or a condition associated with hemophilia. The identification of the subject can occur in a clinical setting, or elsewhere, e.g., in the subject's home through the subject's own use of a self-testing kit.

At time zero, a suitable first dose of a modified factor VIII is administered to the subject. The modified factor VIII is formulated as described herein. After a period of time following the first dose, e.g., 7 days, 14 days, and 21 days, the subject's condition is evaluated, e.g., by measuring the anti-FVIII antibody titer (either absolute titer or neutralizing activity titer, the latter measured in Bethesda units/mL). Effectiveness may also be measured by measuring FVIII half-life, relative affinitiy FVIII binding to von Willebrand factor, phospholipids or platelets, and binding to other serine proteases in the coagulation cascade, or by comparing the factor VIII-specific immune responses, inflammatory cytokine levels, and/or conditions associated with hemophilia in mammals treated with a modified factor VIII. Other relevant criteria can also be measured, e.g., ELISPOT. The number and strength of doses are adjusted according to the subject's needs.

After treatment, the subject's anti-FVIII antibody titer (either absolute titer or neutralizing activity titer, the latter measured in Bethesda units/mL), FVIII half-life, relative affinitiy FVIII binding to von Willebrand factor, levels of phospholipids or platelets, binding to other serine proteases in the coagulation cascade, factor VIII-specific immune responses, inflammatory cytokine levels, and/or conditions associated with hemophilia in mammals treated with a modified factor VIII are lowered and/or improved relative to the levels existing prior to the treatment, or relative to the levels measured in a similarly afflicted but untreated/control subject, or relative to the levels measured in a similarly afflicted subject treated with an unmodified factor VIII.

While the invention has been particularly shown and described with reference to a preferred embodiment and various alternate embodiments, it will be understood by persons skilled in the relevant art that various changes in form and details can be made therein without departing from the spirit and scope of the invention.

All references, issued patents and patent applications cited within the body of the instant specification are hereby incorporated by reference in their entirety, for all purposes.

## Claims

1. A modified Factor VIII polypeptide comprising at least one amino acid modification in an unmodified Factor VIII polypeptide, wherein the at least one amino acid modification is at a position corresponding to position 2196 of the C2 domain of the amino acid sequence set forth in SEQ ID NO: 1.

2. The modified Factor VIII polypeptide of claim 1, wherein the at least one amino acid modification is an amino acid substitution of F2196A; or F2196R.

3. The modified Factor VIII polypeptide of claim 1, wherein the at least one amino acid modification is an amino acid deletion; or
wherein the at least one amino acid modification is an amino acid addition; or wherein the at least one amino acid modification is an amino acid substitution; or wherein the at least one amino acid modification is a covalent chemical modification.

4. The modified Factor VIII polypeptide of claim 1, wherein the at least one amino acid modification is a modification in a B cell epitope.

5. The modified Factor VIII polypeptide of claim 1, wherein the modified Factor VIII polypeptide retains an activity of the unmodified Factor VIII polypeptide; or
wherein the modified Factor VIII polypeptide exhibits reduced immunogenicity/antigenicity upon administration to a subject compared to the unmodified Factor VIII polypeptide.

6. The modified Factor VIII polypeptide of claim 1, wherein the unmodified Factor VIII polypeptide comprises an amino acid sequence that has 40%, 50%, 60%, 70%, 80%, 90%, 91%, 92%, 93%, 94%, 95%, 96%, 97%, 98%, or 99% sequence identity to the amino acid sequence set forth in SEQ ID NO: 1, excluding amino acid modification(s).

7. The modified Factor VIII polypeptide of claim 1, wherein the modified Factor VIII polypeptide is a human polypeptide; or
wherein the modified Factor VIII polypeptide is a non-human polypeptide.

8. The modified Factor VIII polypeptide of claim 1, wherein the modified Factor VIII polypeptide comprises at least 2, 3, 4, 5, 6, 7, 8, 9, 10, 11, 12, 13, 14, 15, 16, 17, 18, 19, or 20 amino acid modifications; or
wherein the modified Factor VIII polypeptide comprises 6 amino acid modifications; or
wherein the modified Factor VIII polypeptide comprises a single amino acid modification.

9. The modified Factor VIII polypeptide of claim 1 , wherein the modified Factor VIII polypeptide further comprises at least one additional amino acid modification.

10. The modified Factor VIII polypeptide of claim 9, wherein the at least one additional amino acid modification is a modification in a T cell epitope; or
wherein the at least one additional amino acid modification is at a position corresponding to positions 2194, 2195, or 2197-2213 of the amino acid sequence set forth in SEQ ID NO: 1; or
wherein the at least one additional amino acid modification is at a position corresponding to positions 2194, 2195, or 2197-2205 of the amino acid sequence set forth in SEQ ID NO: 1; or
wherein the at least one additional amino acid modification is at a position corresponding to position M2199, A2201, or S2204 of the amino acid sequence set forth in SEQ ID NO: 1; or
wherein the at least one additional amino acid modification is at a position corresponding to positions 2173-2195 or 2197-2332 of the C2 domain of the amino acid sequence set forth in SEQ ID NO: 1, or positions 373-740 of the A2 domain of the amino acid sequence set forth in SEQ ID NO:1, or positions 598-608 of the A2 domain of the amino acid sequence set forth in SEQ ID NO:1; or
wherein the at least one additional amino acid modification is at a position corresponding to positions 2202-2221 of the amino acid sequence set forth in SEQ ID NO: 1; or
wherein the at least one additional amino acid modification is at a position corresponding to positions 2197-2204 of the amino acid sequence set forth in SEQ ID NO: 1; or
wherein the at least one additional amino acid modification is at a position corresponding to position 2200, 2199, 2215, 2220, 2198, 2273, 2231, or 2272 of the amino acid sequence set forth in SEQ ID NO: 1.

11. A pharmaceutical composition comprising a modified Factor VIII polypeptide according to any one of claims 1-10, and a pharmaceutically acceptable excipient.

12. A nucleic acid molecule encoding the modified Factor VIII polypeptide according to any one of claims 1-10.

13. A recombinant expression vector comprising a nucleic acid molecule according to claim 12.

14. A host cell transformed with the recombinant expression vector according to claim 13.

15. A method of making the modified Factor VIII polypeptide of claim 1, comprising:
providing a host cell comprising a nucleic acid sequence that encodes the modified Factor VIII polypeptide; and maintaining the host cell under conditions in which the modified Factor VIII polypeptide is expressed.

16. The modified Factor VIII polypeptide of claim 1 for use in treating, reducing or preventing a condition associated with an immune response to Factor VIII.


SEQUENCE LISTING

<110> PUGET SOUND BLOOD CENTER

<120> FACTOR VIII B CELL EPITOPE VARIANTS HAVING REDUCED IMMUNOGENICITY

<130> JA63573P.EPPD1

<140> PCT/US2010/056732
<141> 2010-11-15

<150> 61/266,471
<151> 2009-12-03

<150> 61/261,296
<151> 2009-11-13

<160> 312

<170> PatentIn version 3.5

<210> 1
<211> 2351
<212> PRT
<213> Homo sapiens

<400> 1
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Asp
    770                 775                 780

Ile Glu Lys Thr Asp Pro Trp Phe Ala His Arg Thr Pro Met Pro Lys
785                 790                 795                 800

Ile Gln Asn Val Ser Ser Ser Asp Leu Leu Met Leu Leu Arg Gln Ser
                805                 810                 815

Pro Thr Pro His Gly Leu Ser Leu Ser Asp Leu Gln Glu Ala Lys Tyr
            820                 825                 830

Glu Thr Phe Ser Asp Asp Pro Ser Pro Gly Ala Ile Asp Ser Asn Asn
        835                 840                 845

Ser Leu Ser Glu Met Thr His Phe Arg Pro Gln Leu His His Ser Gly
    850                 855                 860

Asp Met Val Phe Thr Pro Glu Ser Gly Leu Gln Leu Arg Leu Asn Glu
865                 870                 875                 880

Lys Leu Gly Thr Thr Ala Ala Thr Glu Leu Lys Lys Leu Asp Phe Lys
                885                 890                 895

Val Ser Ser Thr Ser Asn Asn Leu Ile Ser Thr Ile Pro Ser Asp Asn

            900                 905                 910

Leu Ala Ala Gly Thr Asp Asn Thr Ser Ser Leu Gly Pro Pro Ser Met
        915                 920                 925

Pro Val His Tyr Asp Ser Gln Leu Asp Thr Thr Leu Phe Gly Lys Lys
    930                 935                 940

Ser Ser Pro Leu Thr Glu Ser Gly Gly Pro Leu Ser Leu Ser Glu Glu
945                 950                 955                 960

Asn Asn Asp Ser Lys Leu Leu Glu Ser Gly Leu Met Asn Ser Gln Glu
                965                 970                 975

Ser Ser Trp Gly Lys Asn Val Ser Ser Thr Glu Ser Gly Arg Leu Phe
            980                 985                 990

Lys Gly Lys Arg Ala His Gly Pro Ala Leu Leu Thr Lys Asp Asn Ala
        995                 1000                1005

Leu Phe Lys Val Ser Ile Ser Leu Leu Lys Thr Asn Lys Thr Ser
    1010                1015                1020

Asn Asn Ser Ala Thr Asn Arg Lys Thr His Ile Asp Gly Pro Ser
    1025                1030                1035

Leu Leu Ile Glu Asn Ser Pro Ser Val Trp Gln Asn Ile Leu Glu
    1040                1045                1050

Ser Asp Thr Glu Phe Lys Lys Val Thr Pro Leu Ile His Asp Arg
    1055                1060                1065

Met Leu Met Asp Lys Asn Ala Thr Ala Leu Arg Leu Asn His Met
    1070                1075                1080

Ser Asn Lys Thr Thr Ser Ser Lys Asn Met Glu Met Val Gln Gln
    1085                1090                1095

Lys Lys Glu Gly Pro Ile Pro Pro Asp Ala Gln Asn Pro Asp Met
    1100                1105                1110

Ser Phe Phe Lys Met Leu Phe Leu Pro Glu Ser Ala Arg Trp Ile
    1115                1120                1125

Gln Arg Thr His Gly Lys Asn Ser Leu Asn Ser Gly Gln Gly Pro
    1130                1135                1140

Ser Pro Lys Gln Leu Val Ser Leu Gly Pro Glu Lys Ser Val Glu
    1145                1150                1155

Gly Gln Asn Phe Leu Ser Glu Lys Asn Lys Val Val Val Gly Lys
    1160                1165                1170

Gly Glu Phe Thr Lys Asp Val Gly Leu Lys Glu Met Val Phe Pro
    1175                1180                1185

Ser Ser Arg Asn Leu Phe Leu Thr Asn Leu Asp Asn Leu His Glu
    1190                1195                1200

Asn Asn Thr His Asn Gln Glu Lys Lys Ile Gln Glu Glu Ile Glu
    1205                1210                1215

Lys Lys Glu Thr Leu Ile Gln Glu Asn Val Val Leu Pro Gln Ile
    1220                1225                1230

His Thr Val Thr Gly Thr Lys Asn Phe Met Lys Asn Leu Phe Leu
    1235                1240                1245

Leu Ser Thr Arg Gln Asn Val Glu Gly Ser Tyr Asp Gly Ala Tyr
    1250                1255                1260

Ala Pro Val Leu Gln Asp Phe Arg Ser Leu Asn Asp Ser Thr Asn
    1265                1270                1275

Arg Thr Lys Lys His Thr Ala His Phe Ser Lys Lys Gly Glu Glu
    1280                1285                1290

Glu Asn Leu Glu Gly Leu Gly Asn Gln Thr Lys Gln Ile Val Glu
    1295                1300                1305

Lys Tyr Ala Cys Thr Thr Arg Ile Ser Pro Asn Thr Ser Gln Gln
    1310                1315                1320

Asn Phe Val Thr Gln Arg Ser Lys Arg Ala Leu Lys Gln Phe Arg
    1325                1330                1335

Leu Pro Leu Glu Glu Thr Glu Leu Glu Lys Arg Ile Ile Val Asp
    1340                1345                1350

Asp Thr Ser Thr Gln Trp Ser Lys Asn Met Lys His Leu Thr Pro
    1355                1360                1365

Ser Thr Leu Thr Gln Ile Asp Tyr Asn Glu Lys Glu Lys Gly Ala
    1370                1375                1380

Ile Thr Gln Ser Pro Leu Ser Asp Cys Leu Thr Arg Ser His Ser
    1385                1390                1395

Ile Pro Gln Ala Asn Arg Ser Pro Leu Pro Ile Ala Lys Val Ser
    1400                1405                1410

Ser Phe Pro Ser Ile Arg Pro Ile Tyr Leu Thr Arg Val Leu Phe
    1415                1420                1425

Gln Asp Asn Ser Ser His Leu Pro Ala Ala Ser Tyr Arg Lys Lys
    1430                1435                1440

Asp Ser Gly Val Gln Glu Ser Ser His Phe Leu Gln Gly Ala Lys
    1445                1450                1455

Lys Asn Asn Leu Ser Leu Ala Ile Leu Thr Leu Glu Met Thr Gly
    1460                1465                1470

Asp Gln Arg Glu Val Gly Ser Leu Gly Thr Ser Ala Thr Asn Ser
    1475                1480                1485

Val Thr Tyr Lys Lys Val Glu Asn Thr Val Leu Pro Lys Pro Asp
    1490                1495                1500

Leu Pro Lys Thr Ser Gly Lys Val Glu Leu Leu Pro Lys Val His
    1505                1510                1515

Ile Tyr Gln Lys Asp Leu Phe Pro Thr Glu Thr Ser Asn Gly Ser
    1520                1525                1530

Pro Gly His Leu Asp Leu Val Glu Gly Ser Leu Leu Gln Gly Thr
    1535                1540                1545

Glu Gly Ala Ile Lys Trp Asn Glu Ala Asn Arg Pro Gly Lys Val
    1550                1555                1560

Pro Phe Leu Arg Val Ala Thr Glu Ser Ser Ala Lys Thr Pro Ser
    1565                1570                1575

Lys Leu Leu Asp Pro Leu Ala Trp Asp Asn His Tyr Gly Thr Gln
    1580                1585                1590

Ile Pro Lys Glu Glu Trp Lys Ser Gln Glu Lys Ser Pro Glu Lys
    1595                1600                1605

Thr Ala Phe Lys Lys Lys Asp Thr Ile Leu Ser Leu Asn Ala Cys
    1610                1615                1620

Glu Ser Asn His Ala Ile Ala Ala Ile Asn Glu Gly Gln Asn Lys
    1625                1630                1635

Pro Glu Ile Glu Val Thr Trp Ala Lys Gln Gly Arg Thr Glu Arg
    1640                1645                1650

Leu Cys Ser Gln Asn Pro Pro Val Leu Lys Arg His Gln Arg Glu
    1655                1660                1665

Ile Thr Arg Thr Thr Leu Gln Ser Asp Gln Glu Glu Ile Asp Tyr
    1670                1675                1680

Asp Asp Thr Ile Ser Val Glu Met Lys Lys Glu Asp Phe Asp Ile
    1685                1690                1695

Tyr Asp Glu Asp Glu Asn Gln Ser Pro Arg Ser Phe Gln Lys Lys
    1700                1705                1710

Thr Arg His Tyr Phe Ile Ala Ala Val Glu Arg Leu Trp Asp Tyr
    1715                1720                1725

Gly Met Ser Ser Ser Pro His Val Leu Arg Asn Arg Ala Gln Ser
    1730                1735                1740

Gly Ser Val Pro Gln Phe Lys Lys Val Val Phe Gln Glu Phe Thr
    1745                1750                1755

Asp Gly Ser Phe Thr Gln Pro Leu Tyr Arg Gly Glu Leu Asn Glu
    1760                1765                1770

His Leu Gly Leu Leu Gly Pro Tyr Ile Arg Ala Glu Val Glu Asp
    1775                1780                1785

Asn Ile Met Val Thr Phe Arg Asn Gln Ala Ser Arg Pro Tyr Ser
    1790                1795                1800

Phe Tyr Ser Ser Leu Ile Ser Tyr Glu Glu Asp Gln Arg Gln Gly
    1805                1810                1815

Ala Glu Pro Arg Lys Asn Phe Val Lys Pro Asn Glu Thr Lys Thr
    1820                1825                1830

Tyr Phe Trp Lys Val Gln His His Met Ala Pro Thr Lys Asp Glu
    1835                1840                1845

Phe Asp Cys Lys Ala Trp Ala Tyr Phe Ser Asp Val Asp Leu Glu

1850                1855                1860

Lys Asp Val His Ser Gly Leu Ile Gly Pro Leu Leu Val Cys His
1865                1870                1875

Thr Asn Thr Leu Asn Pro Ala His Gly Arg Gln Val Thr Val Gln
1880                1885                1890

Glu Phe Ala Leu Phe Phe Thr Ile Phe Asp Glu Thr Lys Ser Trp
1895                1900                1905

Tyr Phe Thr Glu Asn Met Glu Arg Asn Cys Arg Ala Pro Cys Asn
1910                1915                1920

Ile Gln Met Glu Asp Pro Thr Phe Lys Glu Asn Tyr Arg Phe His
1925                1930                1935

Ala Ile Asn Gly Tyr Ile Met Asp Thr Leu Pro Gly Leu Val Met
1940                1945                1950

Ala Gln Asp Gln Arg Ile Arg Trp Tyr Leu Leu Ser Met Gly Ser
1955                1960                1965

Asn Glu Asn Ile His Ser Ile His Phe Ser Gly His Val Phe Thr
1970                1975                1980

Val Arg Lys Lys Glu Glu Tyr Lys Met Ala Leu Tyr Asn Leu Tyr
1985                1990                1995

Pro Gly Val Phe Glu Thr Val Glu Met Leu Pro Ser Lys Ala Gly
2000                2005                2010

Ile Trp Arg Val Glu Cys Leu Ile Gly Glu His Leu His Ala Gly
2015                2020                2025

Met Ser Thr Leu Phe Leu Val Tyr Ser Asn Lys Cys Gln Thr Pro
2030                2035                2040

Leu Gly Met Ala Ser Gly His Ile Arg Asp Phe Gln Ile Thr Ala
2045                2050                2055

Ser Gly Gln Tyr Gly Gln Trp Ala Pro Lys Leu Ala Arg Leu His
2060                2065                2070

Tyr Ser Gly Ser Ile Asn Ala Trp Ser Thr Lys Glu Pro Phe Ser
2075                2080                2085

Trp Ile Lys Val Asp Leu Leu Ala Pro Met Ile Ile His Gly Ile
    2090                2095                2100

Lys Thr Gln Gly Ala Arg Gln Lys Phe Ser Ser Leu Tyr Ile Ser
    2105                2110                2115

Gln Phe Ile Ile Met Tyr Ser Leu Asp Gly Lys Lys Trp Gln Thr
    2120                2125                2130

Tyr Arg Gly Asn Ser Thr Gly Thr Leu Met Val Phe Phe Gly Asn
    2135                2140                2145

Val Asp Ser Ser Gly Ile Lys His Asn Ile Phe Asn Pro Pro Ile
    2150                2155                2160

Ile Ala Arg Tyr Ile Arg Leu His Pro Thr His Tyr Ser Ile Arg
    2165                2170                2175

Ser Thr Leu Arg Met Glu Leu Met Gly Cys Asp Leu Asn Ser Cys
    2180                2185                2190

Ser Met Pro Leu Gly Met Glu Ser Lys Ala Ile Ser Asp Ala Gln
    2195                2200                2205

Ile Thr Ala Ser Ser Tyr Phe Thr Asn Met Phe Ala Thr Trp Ser
    2210                2215                2220

Pro Ser Lys Ala Arg Leu His Leu Gln Gly Arg Ser Asn Ala Trp
    2225                2230                2235

Arg Pro Gln Val Asn Asn Pro Lys Glu Trp Leu Gln Val Asp Phe
    2240                2245                2250

Gln Lys Thr Met Lys Val Thr Gly Val Thr Thr Gln Gly Val Lys
    2255                2260                2265

Ser Leu Leu Thr Ser Met Tyr Val Lys Glu Phe Leu Ile Ser Ser
    2270                2275                2280

Ser Gln Asp Gly His Gln Trp Thr Leu Phe Phe Gln Asn Gly Lys
    2285                2290                2295

Val Lys Val Phe Gln Gly Asn Gln Asp Ser Phe Thr Pro Val Val
    2300                2305                2310

Asn Ser Leu Asp Pro Pro Leu Leu Thr Arg Tyr Leu Arg Ile His
    2315                2320                2325

Pro Gln Ser Trp Val His Gln Ile Ala Leu Arg Met Glu Val Leu
    2330                2335                2340

Gly Cys Glu Ala Gln Asp Leu Tyr
    2345                2350

<210> 2
<211> 2351
<212> PRT
<213> Artificial Sequence

<220>
<223> Description of Artificial Sequence: Synthetic
polypeptide

<220>
<221> MOD_RES
<222> (608)..(626)
<223> Any amino acid

<220>
<221> MOD_RES
<222> (2213)..(2240)
<223> Any amino acid

<220>
<223> see specification as filed for detailed description of
substitutions and preferred embodiments

<400> 2
Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser

115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
545                 550                 555                 560

Asp Leu Ala Ser Gly Leu Ile Gly Pro Leu Leu Ile Cys Tyr Lys Glu
                565                 570                 575

Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Xaa
        595                 600                 605

Xaa Xaa Xaa Xaa Xaa Xaa Xaa Xaa Xaa Xaa Xaa Xaa Xaa Xaa Xaa Xaa
    610                 615                 620

Xaa Xaa Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Ser Arg His Pro
        755                 760                 765

Ser Thr Arg Gln Lys Gln Phe Asn Ala Thr Thr Ile Pro Glu Asn Asp
    770                 775                 780

Ile Glu Lys Thr Asp Pro Trp Phe Ala His Arg Thr Pro Met Pro Lys
785                 790                 795                 800

Ile Gln Asn Val Ser Ser Ser Asp Leu Leu Met Leu Leu Arg Gln Ser
                805                 810                 815

Pro Thr Pro His Gly Leu Ser Leu Ser Asp Leu Gln Glu Ala Lys Tyr
            820                 825                 830

Glu Thr Phe Ser Asp Asp Pro Ser Pro Gly Ala Ile Asp Ser Asn Asn
        835                 840                 845

Ser Leu Ser Glu Met Thr His Phe Arg Pro Gln Leu His His Ser Gly
    850                 855                 860

Asp Met Val Phe Thr Pro Glu Ser Gly Leu Gln Leu Arg Leu Asn Glu
865                 870                 875                 880

Lys Leu Gly Thr Thr Ala Ala Thr Glu Leu Lys Lys Leu Asp Phe Lys
                885                 890                 895

Val Ser Ser Thr Ser Asn Asn Leu Ile Ser Thr Ile Pro Ser Asp Asn
            900                 905                 910

Leu Ala Ala Gly Thr Asp Asn Thr Ser Ser Leu Gly Pro Pro Ser Met
        915                 920                 925

Pro Val His Tyr Asp Ser Gln Leu Asp Thr Thr Leu Phe Gly Lys Lys
    930                 935                 940

Ser Ser Pro Leu Thr Glu Ser Gly Gly Pro Leu Ser Leu Ser Glu Glu
945                 950                 955                 960

Asn Asn Asp Ser Lys Leu Leu Glu Ser Gly Leu Met Asn Ser Gln Glu
                965                 970                 975

Ser Ser Trp Gly Lys Asn Val Ser Ser Thr Glu Ser Gly Arg Leu Phe
            980                 985                 990

Lys Gly Lys Arg Ala His Gly Pro Ala Leu Leu Thr Lys Asp Asn Ala
        995                 1000                1005

Leu Phe Lys Val Ser Ile Ser Leu Leu Lys Thr Asn Lys Thr Ser
    1010                1015                1020

Asn Asn Ser Ala Thr Asn Arg Lys Thr His Ile Asp Gly Pro Ser
    1025                1030                1035

Leu Leu Ile Glu Asn Ser Pro Ser Val Trp Gln Asn Ile Leu Glu
    1040                1045                1050

Ser Asp Thr Glu Phe Lys Lys Val Thr Pro Leu Ile His Asp Arg
    1055                1060                1065

Met Leu Met Asp Lys Asn Ala Thr Ala Leu Arg Leu Asn His Met
    1070                1075                1080

Ser Asn Lys Thr Thr Ser Ser Lys Asn Met Glu Met Val Gln Gln
    1085                1090                1095

Lys Lys Glu Gly Pro Ile Pro Pro Asp Ala Gln Asn Pro Asp Met
    1100                1105                1110

Ser Phe Phe Lys Met Leu Phe Leu Pro Glu Ser Ala Arg Trp Ile

1115                 1120                 1125

Gln Arg Thr His Gly Lys Asn Ser Leu Asn Ser Gly Gln Gly Pro
    1130                 1135                 1140

Ser Pro Lys Gln Leu Val Ser Leu Gly Pro Glu Lys Ser Val Glu
    1145                 1150                 1155

Gly Gln Asn Phe Leu Ser Glu Lys Asn Lys Val Val Val Gly Lys
    1160                 1165                 1170

Gly Glu Phe Thr Lys Asp Val Gly Leu Lys Glu Met Val Phe Pro
    1175                 1180                 1185

Ser Ser Arg Asn Leu Phe Leu Thr Asn Leu Asp Asn Leu His Glu
    1190                 1195                 1200

Asn Asn Thr His Asn Gln Glu Lys Lys Ile Gln Glu Glu Ile Glu
    1205                 1210                 1215

Lys Lys Glu Thr Leu Ile Gln Glu Asn Val Val Leu Pro Gln Ile
    1220                 1225                 1230

His Thr Val Thr Gly Thr Lys Asn Phe Met Lys Asn Leu Phe Leu
    1235                 1240                 1245

Leu Ser Thr Arg Gln Asn Val Glu Gly Ser Tyr Asp Gly Ala Tyr
    1250                 1255                 1260

Ala Pro Val Leu Gln Asp Phe Arg Ser Leu Asn Asp Ser Thr Asn
    1265                 1270                 1275

Arg Thr Lys Lys His Thr Ala His Phe Ser Lys Lys Gly Glu Glu
    1280                 1285                 1290

Glu Asn Leu Glu Gly Leu Gly Asn Gln Thr Lys Gln Ile Val Glu
    1295                 1300                 1305

Lys Tyr Ala Cys Thr Thr Arg Ile Ser Pro Asn Thr Ser Gln Gln
    1310                 1315                 1320

Asn Phe Val Thr Gln Arg Ser Lys Arg Ala Leu Lys Gln Phe Arg
    1325                 1330                 1335

Leu Pro Leu Glu Glu Thr Glu Leu Glu Lys Arg Ile Ile Val Asp
    1340                 1345                 1350

Asp Thr Ser Thr Gln Trp Ser Lys Asn Met Lys His Leu Thr Pro
    1355                1360                1365

Ser Thr Leu Thr Gln Ile Asp Tyr Asn Glu Lys Glu Lys Gly Ala
    1370                1375                1380

Ile Thr Gln Ser Pro Leu Ser Asp Cys Leu Thr Arg Ser His Ser
    1385                1390                1395

Ile Pro Gln Ala Asn Arg Ser Pro Leu Pro Ile Ala Lys Val Ser
    1400                1405                1410

Ser Phe Pro Ser Ile Arg Pro Ile Tyr Leu Thr Arg Val Leu Phe
    1415                1420                1425

Gln Asp Asn Ser Ser His Leu Pro Ala Ala Ser Tyr Arg Lys Lys
    1430                1435                1440

Asp Ser Gly Val Gln Glu Ser Ser His Phe Leu Gln Gly Ala Lys
    1445                1450                1455

Lys Asn Asn Leu Ser Leu Ala Ile Leu Thr Leu Glu Met Thr Gly
    1460                1465                1470

Asp Gln Arg Glu Val Gly Ser Leu Gly Thr Ser Ala Thr Asn Ser
    1475                1480                1485

Val Thr Tyr Lys Lys Val Glu Asn Thr Val Leu Pro Lys Pro Asp
    1490                1495                1500

Leu Pro Lys Thr Ser Gly Lys Val Glu Leu Leu Pro Lys Val His
    1505                1510                1515

Ile Tyr Gln Lys Asp Leu Phe Pro Thr Glu Thr Ser Asn Gly Ser
    1520                1525                1530

Pro Gly His Leu Asp Leu Val Glu Gly Ser Leu Leu Gln Gly Thr
    1535                1540                1545

Glu Gly Ala Ile Lys Trp Asn Glu Ala Asn Arg Pro Gly Lys Val
    1550                1555                1560

Pro Phe Leu Arg Val Ala Thr Glu Ser Ser Ala Lys Thr Pro Ser
    1565                1570                1575

Lys Leu Leu Asp Pro Leu Ala Trp Asp Asn His Tyr Gly Thr Gln
    1580                1585                1590

Ile Pro Lys Glu Glu Trp Lys Ser Gln Glu Lys Ser Pro Glu Lys
    1595                1600                1605

Thr Ala Phe Lys Lys Lys Asp Thr Ile Leu Ser Leu Asn Ala Cys
    1610                1615                1620

Glu Ser Asn His Ala Ile Ala Ala Ile Asn Glu Gly Gln Asn Lys
    1625                1630                1635

Pro Glu Ile Glu Val Thr Trp Ala Lys Gln Gly Arg Thr Glu Arg
    1640                1645                1650

Leu Cys Ser Gln Asn Pro Pro Val Leu Lys Arg His Gln Arg Glu
    1655                1660                1665

Ile Thr Arg Thr Thr Leu Gln Ser Asp Gln Glu Glu Ile Asp Tyr
    1670                1675                1680

Asp Asp Thr Ile Ser Val Glu Met Lys Lys Glu Asp Phe Asp Ile
    1685                1690                1695

Tyr Asp Glu Asp Glu Asn Gln Ser Pro Arg Ser Phe Gln Lys Lys
    1700                1705                1710

Thr Arg His Tyr Phe Ile Ala Ala Val Glu Arg Leu Trp Asp Tyr
    1715                1720                1725

Gly Met Ser Ser Ser Pro His Val Leu Arg Asn Arg Ala Gln Ser
    1730                1735                1740

Gly Ser Val Pro Gln Phe Lys Lys Val Val Phe Gln Glu Phe Thr
    1745                1750                1755

Asp Gly Ser Phe Thr Gln Pro Leu Tyr Arg Gly Glu Leu Asn Glu
    1760                1765                1770

His Leu Gly Leu Leu Gly Pro Tyr Ile Arg Ala Glu Val Glu Asp
    1775                1780                1785

Asn Ile Met Val Thr Phe Arg Asn Gln Ala Ser Arg Pro Tyr Ser
    1790                1795                1800

Phe Tyr Ser Ser Leu Ile Ser Tyr Glu Glu Asp Gln Arg Gln Gly
    1805                1810                1815

Ala Glu Pro Arg Lys Asn Phe Val Lys Pro Asn Glu Thr Lys Thr
    1820                1825                1830

Tyr Phe Trp Lys Val Gln His His Met Ala Pro Thr Lys Asp Glu
    1835                1840                1845

Phe Asp Cys Lys Ala Trp Ala Tyr Phe Ser Asp Val Asp Leu Glu
    1850                1855                1860

Lys Asp Val His Ser Gly Leu Ile Gly Pro Leu Leu Val Cys His
    1865                1870                1875

Thr Asn Thr Leu Asn Pro Ala His Gly Arg Gln Val Thr Val Gln
    1880                1885                1890

Glu Phe Ala Leu Phe Phe Thr Ile Phe Asp Glu Thr Lys Ser Trp
    1895                1900                1905

Tyr Phe Thr Glu Asn Met Glu Arg Asn Cys Arg Ala Pro Cys Asn
    1910                1915                1920

Ile Gln Met Glu Asp Pro Thr Phe Lys Glu Asn Tyr Arg Phe His
    1925                1930                1935

Ala Ile Asn Gly Tyr Ile Met Asp Thr Leu Pro Gly Leu Val Met
    1940                1945                1950

Ala Gln Asp Gln Arg Ile Arg Trp Tyr Leu Leu Ser Met Gly Ser
    1955                1960                1965

Asn Glu Asn Ile His Ser Ile His Phe Ser Gly His Val Phe Thr
    1970                1975                1980

Val Arg Lys Lys Glu Glu Tyr Lys Met Ala Leu Tyr Asn Leu Tyr
    1985                1990                1995

Pro Gly Val Phe Glu Thr Val Glu Met Leu Pro Ser Lys Ala Gly
    2000                2005                2010

Ile Trp Arg Val Glu Cys Leu Ile Gly Glu His Leu His Ala Gly
    2015                2020                2025

Met Ser Thr Leu Phe Leu Val Tyr Ser Asn Lys Cys Gln Thr Pro
    2030                2035                2040

Leu Gly Met Ala Ser Gly His Ile Arg Asp Phe Gln Ile Thr Ala
    2045                2050                2055

Ser Gly Gln Tyr Gly Gln Trp Ala Pro Lys Leu Ala Arg Leu His

2060                2065                2070

Tyr Ser Gly Ser Ile Asn Ala Trp Ser Thr Lys Glu Pro Phe Ser
    2075                2080                2085

Trp Ile Lys Val Asp Leu Leu Ala Pro Met Ile Ile His Gly Ile
    2090                2095                2100

Lys Thr Gln Gly Ala Arg Gln Lys Phe Ser Ser Leu Tyr Ile Ser
    2105                2110                2115

Gln Phe Ile Ile Met Tyr Ser Leu Asp Gly Lys Lys Trp Gln Thr
    2120                2125                2130

Tyr Arg Gly Asn Ser Thr Gly Thr Leu Met Val Phe Phe Gly Asn
    2135                2140                2145

Val Asp Ser Ser Gly Ile Lys His Asn Ile Phe Asn Pro Pro Ile
    2150                2155                2160

Ile Ala Arg Tyr Ile Arg Leu His Pro Thr His Tyr Ser Ile Arg
    2165                2170                2175

Ser Thr Leu Arg Met Glu Leu Met Gly Cys Asp Leu Asn Ser Cys
    2180                2185                2190

Ser Met Pro Leu Gly Met Glu Ser Lys Ala Ile Ser Asp Ala Gln
    2195                2200                2205

Ile Thr Ala Ser Xaa Xaa Xaa Xaa Xaa Xaa Xaa Xaa Xaa Xaa Xaa
    2210                2215                2220

Xaa Xaa Xaa Xaa Xaa Xaa Xaa Xaa Xaa Xaa Xaa Xaa Xaa Xaa Xaa
    2225                2230                2235

Xaa Xaa Gln Val Asn Asn Pro Lys Glu Trp Leu Gln Val Asp Phe
    2240                2245                2250

Gln Lys Thr Met Lys Val Thr Gly Val Thr Thr Gln Gly Val Lys
    2255                2260                2265

Ser Leu Leu Thr Ser Met Tyr Val Lys Glu Phe Leu Ile Ser Ser
    2270                2275                2280

Ser Gln Asp Gly His Gln Trp Thr Leu Phe Phe Gln Asn Gly Lys
    2285                2290                2295

Val Lys Val Phe Gln Gly Asn Gln Asp Ser Phe Thr Pro Val Val
    2300                2305                2310

Asn Ser Leu Asp Pro Pro Leu Leu Thr Arg Tyr Leu Arg Ile His
    2315                2320                2325

Pro Gln Ser Trp Val His Gln Ile Ala Leu Arg Met Glu Val Leu
    2330                2335                2340

Gly Cys Glu Ala Gln Asp Leu Tyr
    2345                2350

<210> 3
<211> 20
<212> PRT
<213> Homo sapiens

<400> 3
Ser Tyr Phe Thr Asn Met Phe Ala Thr Trp Ser Pro Ser Lys Ala Arg
1               5                   10                  15

Leu His Leu Gln
            20

<210> 4
<211> 12
<212> PRT
<213> Homo sapiens

<400> 4
Ser Tyr Phe Thr Asn Met Phe Ala Thr Trp Ser Pro
1               5                   10

<210> 5
<211> 20
<212> PRT
<213> Homo sapiens

<400> 5
Thr Trp Ser Pro Ser Lys Ala Arg Leu His Leu Gln Gly Arg Ser Asn
1               5                   10                  15

Ala Trp Arg Pro
            20

<210> 6
<211> 19
<212> PRT
<213> Homo sapiens

<400> 6
Glu Asn Ile Gln Arg Phe Leu Pro Asn Pro Ala Gly Val Gln Leu Glu
1               5                   10                  15

Asp Pro Glu

<210> 7
<211> 9
<212> PRT
<213> Homo sapiens

<400> 7
Phe Leu Pro Asn Pro Ala Gly Val Gln
1               5

<210> 8
<211> 20
<212> PRT
<213> Homo sapiens

<400> 8
Asp Leu Asn Ser Cys Ser Met Pro Leu Gly Met Glu Ser Lys Ala Ile
1               5                   10                  15

Ser Asp Ala Gln
            20

<210> 9
<211> 20
<212> PRT
<213> Homo sapiens

<400> 9
Leu Gly Met Glu Ser Lys Ala Ile Ser Asp Ala Gln Ile Thr Ala Ser
1               5                   10                  15

Ser Tyr Phe Thr
            20

<210> 10
<211> 20
<212> PRT
<213> Homo sapiens

<400> 10
Ser Asp Ala Gln Ile Thr Ala Ser Ser Tyr Phe Thr Asn Met Phe Ala
1               5                   10                  15

Thr Trp Ser Pro
            20

<210> 11
<211> 20
<212> PRT
<213> Homo sapiens

<400> 11

Ser Tyr Phe Thr Asn Met Phe Ala Thr Trp Ser Pro Ser Lys Ala Arg
1               5                   10                  15

Leu His Leu Gln
            20

<210> 12
<211> 20
<212> PRT
<213> Homo sapiens

<400> 12
Thr Trp Ser Pro Ser Lys Ala Arg Leu His Leu Gln Gly Arg Ser Asn
1               5                   10                  15

Ala Trp Arg Pro
            20

<210> 13
<211> 20
<212> PRT
<213> Homo sapiens

<400> 13
Leu His Leu Gln Gly Arg Ser Asn Ala Trp Arg Pro Gln Val Asn Asn
1               5                   10                  15

Pro Lys Glu Trp
            20

<210> 14
<211> 20
<212> PRT
<213> Homo sapiens

<400> 14
Ala Trp Arg Pro Gln Val Asn Asn Pro Lys Glu Trp Leu Gln Val Asp
1               5                   10                  15

Phe Gln Lys Thr
            20

<210> 15
<211> 20
<212> PRT
<213> Homo sapiens

<400> 15
Pro Lys Glu Trp Leu Gln Val Asp Phe Gln Lys Thr Met Lys Val Thr
1               5                   10                  15

Gly Val Thr Thr
            20

<210> 16
<211> 20
<212> PRT
<213> Homo sapiens

<400> 16
Phe Gln Lys Thr Met Lys Val Thr Gly Val Thr Thr Gln Gly Val Lys
1               5                   10                  15

Ser Leu Leu Thr
            20

<210> 17
<211> 20
<212> PRT
<213> Homo sapiens

<400> 17
Gly Val Thr Thr Gln Gly Val Lys Ser Leu Leu Thr Ser Met Tyr Val
1               5                   10                  15

Lys Glu Phe Leu
            20

<210> 18
<211> 20
<212> PRT
<213> Homo sapiens

<400> 18
Ser Leu Leu Thr Ser Met Tyr Val Lys Glu Phe Leu Ile Ser Ser Ser
1               5                   10                  15

Gln Asp Gly His
            20

<210> 19
<211> 20
<212> PRT
<213> Homo sapiens

<400> 19
Lys Glu Phe Leu Ile Ser Ser Ser Gln Asp Gly His Gln Trp Thr Leu
1               5                   10                  15

Phe Phe Gln Asn
            20

<210> 20
<211> 20
<212> PRT
<213> Homo sapiens

<400> 20

Ser Gln Asp Gly His Gln Trp Thr Leu Phe Phe Gln Asn Gly Lys Val
1               5                   10                  15

Lys Val Phe Gln
            20

<210> 21
<211> 20
<212> PRT
<213> Homo sapiens

<400> 21
Leu Phe Phe Gln Asn Gly Lys Val Lys Val Phe Gln Gly Asn Gln Asp
1               5                   10                  15

Ser Phe Thr Pro
            20

<210> 22
<211> 20
<212> PRT
<213> Homo sapiens

<400> 22
Lys Val Phe Gln Gly Asn Gln Asp Ser Phe Thr Pro Val Val Asn Ser
1               5                   10                  15

Leu Asp Pro Pro
            20

<210> 23
<211> 20
<212> PRT
<213> Homo sapiens

<400> 23
Ser Phe Thr Pro Val Val Asn Ser Leu Asp Pro Pro Leu Leu Thr Arg
1               5                   10                  15

Tyr Leu Arg Ile
            20

<210> 24
<211> 20
<212> PRT
<213> Homo sapiens

<400> 24
Leu Asp Pro Pro Leu Leu Thr Arg Tyr Leu Arg Ile His Pro Gln Ser
1               5                   10                  15

Trp Val His Gln
            20

<210> 25
<211> 20
<212> PRT
<213> Homo sapiens

<400> 25
Tyr Leu Arg Ile His Pro Gln Ser Trp Val His Gln Ile Ala Leu Arg
1               5                   10                  15

Met Glu Val Leu
            20

<210> 26
<211> 20
<212> PRT
<213> Homo sapiens

<400> 26
Trp Val His Gln Ile Ala Leu Arg Met Glu Val Leu Gly Cys Glu Ala
1               5                   10                  15

Gln Asp Leu Tyr
            20

<210> 27
<211> 20
<212> PRT
<213> Homo sapiens

<400> 27
Ser Val Ala Lys Lys His Pro Lys Thr Trp Val His Tyr Ile Ala Ala
1               5                   10                  15

Glu Glu Glu Asp
            20

<210> 28
<211> 20
<212> PRT
<213> Homo sapiens

<400> 28
Thr Trp Val His Tyr Ile Ala Ala Glu Glu Glu Asp Trp Asp Tyr Ala
1               5                   10                  15

Pro Leu Val Leu
            20

<210> 29
<211> 20
<212> PRT
<213> Homo sapiens

<400> 29

Glu Glu Glu Asp Trp Asp Tyr Ala Pro Leu Val Leu Ala Pro Asp Asp
1               5                   10                  15

Arg Ser Tyr Lys
            20

<210> 30
<211> 20
<212> PRT
<213> Homo sapiens

<400> 30
Pro Leu Val Leu Ala Pro Asp Asp Arg Ser Tyr Lys Ser Gln Tyr Leu
1               5                   10                  15

Asn Asn Gly Pro
            20

<210> 31
<211> 20
<212> PRT
<213> Homo sapiens

<400> 31
Arg Ser Tyr Lys Ser Gln Tyr Leu Asn Asn Gly Pro Gln Arg Ile Gly
1               5                   10                  15

Arg Lys Tyr Lys
            20

<210> 32
<211> 20
<212> PRT
<213> Homo sapiens

<400> 32
Asn Asn Gly Pro Gln Arg Ile Gly Arg Lys Tyr Lys Lys Val Arg Phe
1               5                   10                  15

Met Ala Tyr Thr
            20

<210> 33
<211> 20
<212> PRT
<213> Homo sapiens

<400> 33
Arg Lys Tyr Lys Lys Val Arg Phe Met Ala Tyr Thr Asp Glu Thr Phe
1               5                   10                  15

Lys Thr Arg Glu
            20

<210> 34
<211> 20
<212> PRT
<213> Homo sapiens

<400> 34
Met Ala Tyr Thr Asp Glu Thr Phe Lys Thr Arg Glu Ala Ile Gln His
1               5                   10                  15

Glu Ser Gly Ile
            20

<210> 35
<211> 20
<212> PRT
<213> Homo sapiens

<400> 35
Lys Thr Arg Glu Ala Ile Gln His Glu Ser Gly Ile Leu Gly Pro Leu
1               5                   10                  15

Leu Tyr Gly Glu
            20

<210> 36
<211> 20
<212> PRT
<213> Homo sapiens

<400> 36
Glu Ser Gly Ile Leu Gly Pro Leu Leu Tyr Gly Glu Val Gly Asp Thr
1               5                   10                  15

Leu Leu Ile Ile
            20

<210> 37
<211> 20
<212> PRT
<213> Homo sapiens

<400> 37
Leu Tyr Gly Glu Val Gly Asp Thr Leu Leu Ile Ile Phe Lys Asn Gln
1               5                   10                  15

Ala Ser Arg Pro
            20

<210> 38
<211> 20
<212> PRT
<213> Homo sapiens

<400> 38

Leu Leu Ile Ile Phe Lys Asn Gln Ala Ser Arg Pro Tyr Asn Ile Tyr
1               5                   10                  15

Pro His Gly Ile
            20

<210> 39
<211> 20
<212> PRT
<213> Homo sapiens

<400> 39
Ala Ser Arg Pro Tyr Asn Ile Tyr Pro His Gly Ile Thr Asp Val Arg
1               5                   10                  15

Pro Leu Tyr Ser
            20

<210> 40
<211> 20
<212> PRT
<213> Homo sapiens

<400> 40
Pro His Gly Ile Thr Asp Val Arg Pro Leu Tyr Ser Arg Arg Leu Pro
1               5                   10                  15

Lys Gly Val Lys
            20

<210> 41
<211> 20
<212> PRT
<213> Homo sapiens

<400> 41
Pro Leu Tyr Ser Arg Arg Leu Pro Lys Gly Val Lys His Leu Lys Asp
1               5                   10                  15

Phe Pro Ile Leu
            20

<210> 42
<211> 20
<212> PRT
<213> Homo sapiens

<400> 42
Lys Gly Val Lys His Leu Lys Asp Phe Pro Ile Leu Pro Gly Glu Ile
1               5                   10                  15

Phe Lys Tyr Lys
            20

<210> 43
<211> 20
<212> PRT
<213> Homo sapiens

<400> 43
Phe Pro Ile Leu Pro Gly Glu Ile Phe Lys Tyr Lys Trp Thr Val Thr
1               5                   10                  15

Val Glu Asp Gly
            20

<210> 44
<211> 20
<212> PRT
<213> Homo sapiens

<400> 44
Ile Phe Lys Tyr Lys Trp Thr Val Thr Val Glu Asp Gly Pro Thr Lys
1               5                   10                  15

Ser Asp Pro Arg
            20

<210> 45
<211> 20
<212> PRT
<213> Homo sapiens

<400> 45
Val Glu Asp Gly Pro Thr Lys Ser Asp Pro Arg Cys Leu Thr Arg Tyr
1               5                   10                  15

Tyr Ser Ser Phe
            20

<210> 46
<211> 20
<212> PRT
<213> Homo sapiens

<400> 46
Asp Pro Arg Cys Leu Thr Arg Tyr Tyr Ser Ser Phe Val Asn Met Glu
1               5                   10                  15

Arg Asp Leu Ala
            20

<210> 47
<211> 20
<212> PRT
<213> Homo sapiens

<400> 47

Leu Thr Arg Tyr Tyr Ser Ser Phe Val Asn Met Glu Arg Asp Leu Ala
1               5                   10                  15

Ser Gly Leu Ile
            20

<210> 48
<211> 20
<212> PRT
<213> Homo sapiens

<400> 48
Tyr Ser Ser Phe Val Asn Met Glu Arg Asp Leu Ala Ser Gly Leu Ile
1               5                   10                  15

Gly Pro Leu Leu
            20

<210> 49
<211> 20
<212> PRT
<213> Homo sapiens

<400> 49
Arg Asp Leu Ala Ser Gly Leu Ile Gly Pro Leu Leu Ile Cys Tyr Lys
1               5                   10                  15

Glu Ser Val Asp
            20

<210> 50
<211> 20
<212> PRT
<213> Homo sapiens

<400> 50
Gly Pro Leu Leu Ile Cys Tyr Lys Glu Ser Val Asp Gln Arg Gly Asn
1               5                   10                  15

Gln Ile Met Ser
            20

<210> 51
<211> 20
<212> PRT
<213> Homo sapiens

<400> 51
Glu Ser Val Asp Gln Arg Gly Asn Gln Ile Met Ser Asp Lys Arg Asn
1               5                   10                  15

Val Ile Leu Phe
            20

<210> 52
<211> 20
<212> PRT
<213> Homo sapiens

<400> 52
Gln Ile Met Ser Asp Lys Arg Asn Val Ile Leu Phe Ser Val Phe Asp
1               5                   10                  15

Glu Asn Arg Ser
            20

<210> 53
<211> 20
<212> PRT
<213> Homo sapiens

<400> 53
Val Ile Leu Phe Ser Val Phe Asp Glu Asn Arg Ser Trp Tyr Leu Thr
1               5                   10                  15

Glu Asn Ile Gln
            20

<210> 54
<211> 20
<212> PRT
<213> Homo sapiens

<400> 54
Glu Asn Arg Ser Trp Tyr Leu Thr Glu Asn Ile Gln Arg Phe Leu Pro
1               5                   10                  15

Asn Pro Ala Gly
            20

<210> 55
<211> 20
<212> PRT
<213> Homo sapiens

<400> 55
Glu Asn Ile Gln Arg Phe Leu Pro Asn Pro Ala Gly Val Gln Leu Glu
1               5                   10                  15

Asp Pro Glu Phe
            20

<210> 56
<211> 20
<212> PRT
<213> Homo sapiens

<400> 56

Asn Pro Ala Gly Val Gln Leu Glu Asp Pro Glu Phe Gln Ala Ser Asn
1               5                   10                  15

Ile Met His Ser
            20

<210> 57
<211> 20
<212> PRT
<213> Homo sapiens

<400> 57
Asp Pro Glu Phe Gln Ala Ser Asn Ile Met His Ser Ile Asn Gly Tyr
1               5                   10                  15

Val Phe Asp Ser
            20

<210> 58
<211> 20
<212> PRT
<213> Homo sapiens

<400> 58
Ile Met His Ser Ile Asn Gly Tyr Val Phe Asp Ser Leu Gln Leu Ser
1               5                   10                  15

Val Cys Leu His
            20

<210> 59
<211> 20
<212> PRT
<213> Homo sapiens

<400> 59
Ala Ser Asn Ile Met His Ser Ile Asn Gly Tyr Val Phe Asp Ser Leu
1               5                   10                  15

Gln Leu Ser Val
            20

<210> 60
<211> 20
<212> PRT
<213> Homo sapiens

<400> 60
Val Phe Asp Ser Leu Gln Leu Ser Val Cys Leu His Glu Val Ala Tyr
1               5                   10                  15

Trp Tyr Ile Leu
            20

<210> 61
<211> 20
<212> PRT
<213> Homo sapiens

<400> 61
Val Cys Leu His Glu Val Ala Tyr Trp Tyr Ile Leu Ser Ile Gly Ala
1               5                   10                  15

Gln Thr Asp Phe
            20

<210> 62
<211> 20
<212> PRT
<213> Homo sapiens

<400> 62
Leu His Glu Val Ala Tyr Trp Tyr Ile Leu Ser Ile Gly Ala Gln Thr
1               5                   10                  15

Asp Phe Leu Ser
            20

<210> 63
<211> 20
<212> PRT
<213> Homo sapiens

<400> 63
Trp Tyr Ile Leu Ser Ile Gly Ala Gln Thr Asp Phe Leu Ser Val Phe
1               5                   10                  15

Phe Ser Gly Tyr
            20

<210> 64
<211> 20
<212> PRT
<213> Homo sapiens

<400> 64
Gln Thr Asp Phe Leu Ser Val Phe Phe Ser Gly Tyr Thr Phe Lys His
1               5                   10                  15

Lys Met Val Tyr
            20

<210> 65
<211> 20
<212> PRT
<213> Homo sapiens

<400> 65

Phe Ser Gly Tyr Thr Phe Lys His Lys Met Val Tyr Glu Asp Thr Leu
1               5                   10                  15

Thr Leu Phe Pro
            20

<210> 66
<211> 20
<212> PRT
<213> Homo sapiens

<400> 66
Lys Met Val Tyr Glu Asp Thr Leu Thr Leu Phe Pro Phe Ser Gly Glu
1               5                   10                  15

Thr Val Phe Met
            20

<210> 67
<211> 20
<212> PRT
<213> Homo sapiens

<400> 67
Thr Leu Phe Pro Phe Ser Gly Glu Thr Val Phe Met Ser Met Glu Asn
1               5                   10                  15

Pro Gly Leu Trp
            20

<210> 68
<211> 20
<212> PRT
<213> Homo sapiens

<400> 68
Thr Val Phe Met Ser Met Glu Asn Pro Gly Leu Trp Ile Leu Gly Cys
1               5                   10                  15

His Asn Ser Asp
            20

<210> 69
<211> 20
<212> PRT
<213> Homo sapiens

<400> 69
Pro Phe Ser Gly Glu Thr Val Phe Met Ser Met Glu Asn Pro Gly Leu
1               5                   10                  15

Trp Ile Leu Gly
            20

<210> 70
<211> 20
<212> PRT
<213> Homo sapiens

<400> 70
Pro Gly Leu Trp Ile Leu Gly Cys His Asn Ser Asp Phe Arg Asn Arg
1               5                   10                  15

Gly Met Thr Ala
            20

<210> 71
<211> 20
<212> PRT
<213> Homo sapiens

<400> 71
His Asn Ser Asp Phe Arg Asn Arg Gly Met Thr Ala Leu Leu Lys Val
1               5                   10                  15

Ser Ser Cys Asp
            20

<210> 72
<211> 18
<212> PRT
<213> Homo sapiens

<400> 72
His Asn Ser Asp Phe Arg Asn Arg Gly Met Thr Ala Leu Leu Lys Val
1               5                   10                  15

Ser Ser

<210> 73
<211> 20
<212> PRT
<213> Homo sapiens

<400> 73
Gly Met Thr Ala Leu Leu Lys Val Ser Ser Cys Asp Lys Asn Thr Gly
1               5                   10                  15

Asp Tyr Tyr Glu
            20

<210> 74
<211> 20
<212> PRT
<213> Homo sapiens

<400> 74

Ser Ser Cys Asp Lys Asn Thr Gly Asp Tyr Tyr Glu Asp Ser Tyr Glu
1               5                   10                  15

Asp Ile Ser Ala
            20

<210> 75
<211> 20
<212> PRT
<213> Homo sapiens

<400> 75
Asp Lys Asn Thr Gly Asp Tyr Tyr Glu Asp Ser Tyr Glu Asp Ile Ser
1               5                   10                  15

Ala Tyr Leu Leu
            20

<210> 76
<211> 24
<212> PRT
<213> Homo sapiens

<400> 76
Asp Tyr Tyr Glu Asp Ser Tyr Glu Asp Ile Ser Ala Tyr Leu Leu Ser
1               5                   10                  15

Lys Asn Asn Ala Ile Glu Pro Arg
            20

<210> 77
<211> 20
<212> PRT
<213> Homo sapiens

<400> 77
Glu His Leu His Ala Gly Met Ser Thr Leu Phe Leu Val Tyr Ser Asn
1               5                   10                  15

Lys Cys Gln Thr
            20

<210> 78
<211> 20
<212> PRT
<213> Homo sapiens

<400> 78
Leu Ile Gly Glu His Leu His Ala Gly Met Ser Thr Leu Phe Leu Val
1               5                   10                  15

Tyr Ser Asn Lys
            20

<210> 79
<211> 20
<212> PRT
<213> Homo sapiens

<400> 79
Thr Leu Phe Leu Val Tyr Ser Asn Lys Cys Gln Thr Pro Leu Gly Met
1               5                   10                  15

Ala Ser Gly His
            20

<210> 80
<211> 20
<212> PRT
<213> Homo sapiens

<400> 80
Lys Cys Gln Thr Pro Leu Gly Met Ala Ser Gly His Ile Arg Asp Phe
1               5                   10                  15

Gln Ile Thr Ala
            20

<210> 81
<211> 20
<212> PRT
<213> Homo sapiens

<400> 81
Gln Thr Pro Leu Gly Met Ala Ser Gly His Ile Arg Asp Phe Gln Ile
1               5                   10                  15

Thr Ala Ser Gly
            20

<210> 82
<211> 20
<212> PRT
<213> Homo sapiens

<400> 82
Ala Ser Gly His Ile Arg Asp Phe Gln Ile Thr Ala Ser Gly Gln Tyr
1               5                   10                  15

Gly Gln Trp Ala
            20

<210> 83
<211> 20
<212> PRT
<213> Homo sapiens

<400> 83

Gln Ile Thr Ala Ser Gly Gln Tyr Gly Gln Trp Ala Pro Lys Leu Ala
1               5                   10                  15

Arg Leu His Tyr
            20

<210> 84
<211> 20
<212> PRT
<213> Homo sapiens

<400> 84
Gly Gln Trp Ala Pro Lys Leu Ala Arg Leu His Tyr Ser Gly Ser Ile
1               5                   10                  15

Asn Ala Trp Ser
            20

<210> 85
<211> 20
<212> PRT
<213> Homo sapiens

<400> 85
Arg Leu His Tyr Ser Gly Ser Ile Asn Ala Trp Ser Thr Lys Glu Pro
1               5                   10                  15

Phe Ser Trp Ile
            20

<210> 86
<211> 20
<212> PRT
<213> Homo sapiens

<400> 86
Asn Ala Trp Ser Thr Lys Glu Pro Phe Ser Trp Ile Lys Val Asp Leu
1               5                   10                  15

Leu Ala Pro Met
            20

<210> 87
<211> 20
<212> PRT
<213> Homo sapiens

<400> 87
Phe Ser Trp Ile Lys Val Asp Leu Leu Ala Pro Met Ile Ile His Gly
1               5                   10                  15

Ile Lys Thr Gln
            20

<210> 88
<211> 20
<212> PRT
<213> Homo sapiens

<400> 88
Leu Ala Pro Met Ile Ile His Gly Ile Lys Thr Gln Gly Ala Arg Gln
1               5                   10                  15

Lys Phe Ser Ser
            20

<210> 89
<211> 20
<212> PRT
<213> Homo sapiens

<400> 89
Ile Lys Thr Gln Gly Ala Arg Gln Lys Phe Ser Ser Leu Tyr Ile Ser
1               5                   10                  15

Gln Phe Ile Ile
            20

<210> 90
<211> 20
<212> PRT
<213> Homo sapiens

<400> 90
Lys Phe Ser Ser Leu Tyr Ile Ser Gln Phe Ile Ile Met Tyr Ser Leu
1               5                   10                  15

Asp Gly Lys Lys
            20

<210> 91
<211> 20
<212> PRT
<213> Homo sapiens

<400> 91
Gln Phe Ile Ile Met Tyr Ser Leu Asp Gly Lys Lys Trp Gln Thr Tyr
1               5                   10                  15

Arg Gly Asn Ser
            20

<210> 92
<211> 20
<212> PRT
<213> Homo sapiens

<400> 92

Asp Gly Lys Lys Trp Gln Thr Tyr Arg Gly Asn Ser Thr Gly Thr Leu
1               5                   10                  15

Met Val Phe Phe
            20

<210> 93
<211> 20
<212> PRT
<213> Homo sapiens

<400> 93
Arg Gly Asn Ser Thr Gly Thr Leu Met Val Phe Phe Gly Asn Val Asp
1               5                   10                  15

Ser Ser Gly Ile
            20

<210> 94
<211> 20
<212> PRT
<213> Homo sapiens

<400> 94
Met Val Phe Phe Gly Asn Val Asp Ser Ser Gly Ile Lys His Asn Ile
1               5                   10                  15

Phe Asn Pro Pro
            20

<210> 95
<211> 20
<212> PRT
<213> Homo sapiens

<400> 95
Ser Ser Gly Ile Lys His Asn Ile Phe Asn Pro Pro Ile Ile Ala Arg
1               5                   10                  15

Tyr Ile Arg Leu
            20

<210> 96
<211> 20
<212> PRT
<213> Homo sapiens

<400> 96
Phe Asn Pro Pro Ile Ile Ala Arg Tyr Ile Arg Leu His Pro Thr His
1               5                   10                  15

Tyr Ser Ile Arg
            20

<210> 97
<211> 20
<212> PRT
<213> Homo sapiens

<400> 97
Tyr Ile Arg Leu His Pro Thr His Tyr Ser Ile Arg Ser Thr Leu Arg
1               5                   10                  15

Met Glu Leu Met
            20

<210> 98
<211> 20
<212> PRT
<213> Homo sapiens

<400> 98
Thr His Tyr Ser Ile Arg Ser Thr Leu Arg Met Glu Leu Met Gly Cys
1               5                   10                  15

Asp Leu Asn Ser
            20

<210> 99
<211> 33
<212> DNA
<213> Artificial Sequence

<220>
<223> Description of Artificial Sequence: Synthetic
      primer

<400> 99
ggcgcgcata tggatttaaa tagttgcagc atg                                33

<210> 100
<211> 30
<212> DNA
<213> Artificial Sequence

<220>
<223> Description of Artificial Sequence: Synthetic
      primer

<400> 100
ggcgcgggat ccctagtaga ggtcctgtgc                                    30

<210> 101
<211> 44
<212> DNA
<213> Artificial Sequence

<220>
<223> Description of Artificial Sequence: Synthetic
      primer

<400> 101
ctcgagaaaa gagtggattt aaatgcttgc agcatgccat tggg                44

<210> 102
<211> 40
<212> DNA
<213> Artificial Sequence

<220>
<223> Description of Artificial Sequence: Synthetic
      primer

<400> 102
gcatgccatt gggaatggcg agtaaagcaa tatcagatgc                     40

<210> 103
<211> 45
<212> DNA
<213> Artificial Sequence

<220>
<223> Description of Artificial Sequence: Synthetic
      primer

<400> 103
gcacagatta ctgcttcatc ctacgctacc aatatgtttg ccacc               45

<210> 104
<211> 36
<212> DNA
<213> Artificial Sequence

<220>
<223> Description of Artificial Sequence: Synthetic
      primer

<400> 104
gcttcatcct actttgccaa tatgtttgcc acctgg                         36

<210> 105
<211> 45
<212> DNA
<213> Artificial Sequence

<220>
<223> Description of Artificial Sequence: Synthetic
      primer

<400> 105
cagattactg cttcatccta ctttaccgct atgtttgcca cctgg               45

<210> 106
<211> 42
<212> DNA
<213> Artificial Sequence

<220>

<223> Description of Artificial Sequence: Synthetic
      primer

<400> 106
cttcatccta ctttaccaat gcgtttgcca cctggtctcc tt                          42


<210> 107
<211> 40
<212> DNA
<213> Artificial Sequence

<220>
<223> Description of Artificial Sequence: Synthetic
      primer

<400> 107
cttcatccta ctttaccaat atggctgcca cctggtctcc                             40


<210> 108
<211> 43
<212> DNA
<213> Artificial Sequence

<220>
<223> Description of Artificial Sequence: Synthetic
      primer

<400> 108
cctactttac caatatgtgc gccacctggt ctccttcaaa agc                         43


<210> 109
<211> 43
<212> DNA
<213> Artificial Sequence

<220>
<223> Description of Artificial Sequence: Synthetic
      primer

<400> 109
cctactttac caatatgttt gccgcctggt ctccttcaaa agc                         43


<210> 110
<211> 35
<212> DNA
<213> Artificial Sequence

<220>
<223> Description of Artificial Sequence: Synthetic
      primer

<400> 110
ggtctccttc agcagctcga cttcacctcc aaggg                                  35


<210> 111
<211> 42
<212> DNA
<213> Artificial Sequence

<220>
<223> Description of Artificial Sequence: Synthetic
      primer

<400> 111
ccttcaaaag ctcgacttgc cctccaaggg aggagtaatg cc                          42


<210> 112
<211> 42
<212> DNA
<213> Artificial Sequence

<220>
<223> Description of Artificial Sequence: Synthetic
      primer

<400> 112
ccttcaaaag ctcgacttca cgcccaaggg aggagtaatg cc                          42


<210> 113
<211> 42
<212> DNA
<213> Artificial Sequence

<220>
<223> Description of Artificial Sequence: Synthetic
      primer

<400> 113
ccttcaaaag ctcgacttca cctcgcaggg aggagtaatg cc                          42


<210> 114
<211> 38
<212> DNA
<213> Artificial Sequence

<220>
<223> Description of Artificial Sequence: Synthetic
      primer

<400> 114
cgacttcacc tccaaggggc gagtaatgcc tggagacc                               38


<210> 115
<211> 33
<212> DNA
<213> Artificial Sequence

<220>
<223> Description of Artificial Sequence: Synthetic
      primer

<400> 115
ccaagggagg agtaatgcct gggcacctca ggt                                    33


<210> 116
<211> 38

<212> DNA
<213> Artificial Sequence

<220>
<223> Description of Artificial Sequence: Synthetic
      primer

<400> 116
gggaggagta atgcctggca acctcaggtg aataatcc                                38


<210> 117
<211> 44
<212> DNA
<213> Artificial Sequence

<220>
<223> Description of Artificial Sequence: Synthetic
      primer

<400> 117
ggagtaatgc ctggagacct gcggtgaata atccaaaaga gtgg                         44


<210> 118
<211> 36
<212> DNA
<213> Artificial Sequence

<220>
<223> Description of Artificial Sequence: Synthetic
      primer

<400> 118
gcctggagac ctcagatgaa taatccaaaa gagtgg                                  36


<210> 119
<211> 45
<212> DNA
<213> Artificial Sequence

<220>
<223> Description of Artificial Sequence: Synthetic
      primer

<400> 119
ggagtaatgc ctggagacct caggtggcta atccaaaaga gtggc                        45


<210> 120
<211> 39
<212> DNA
<213> Artificial Sequence

<220>
<223> Description of Artificial Sequence: Synthetic
      primer

<400> 120
cctggagacc tcaggtgaat gctccaaaag agtggctgc                               39

<210> 121
<211> 37
<212> DNA
<213> Artificial Sequence

<220>
<223> Description of Artificial Sequence: Synthetic
      primer

<400> 121
cctcaggtga ataatccagc agagtggctg caagtgg                                37


<210> 122
<211> 41
<212> DNA
<213> Artificial Sequence

<220>
<223> Description of Artificial Sequence: Synthetic
      primer

<400> 122
ggagacctca ggtgaataat ccacaagagt ggtgcaagtg g                           41


<210> 123
<211> 45
<212> DNA
<213> Artificial Sequence

<220>
<223> Description of Artificial Sequence: Synthetic
      primer

<400> 123
gtaactactc agggagtagc atctctgctt accagcatgt atgtg                       45


<210> 124
<211> 36
<212> DNA
<213> Artificial Sequence

<220>
<223> Description of Artificial Sequence: Synthetic
      primer

<400> 124
cagggagtaa aagctctgct taccagcatg tatgtg                                 36


<210> 125
<211> 29
<212> DNA
<213> Artificial Sequence

<220>
<223> Description of Artificial Sequence: Synthetic
      primer

<400> 125
gagtaaaatc tgcgcttacc agcatgtat                                        29

<210> 126
<211> 41
<212> DNA
<213> Artificial Sequence

<220>
<223> Description of Artificial Sequence: Synthetic
      primer

<400> 126
ctcaggagta aaatctctgg ctaccagcat gtatgtgaag g                          41


<210> 127
<211> 39
<212> DNA
<213> Artificial Sequence

<220>
<223> Description of Artificial Sequence: Synthetic
      primer

<400> 127
ggagtaaaat ctctgcttgc cagcatgtat gtgaaggag                             39


<210> 128
<211> 37
<212> DNA
<213> Artificial Sequence

<220>
<223> Description of Artificial Sequence: Synthetic
      primer

<400> 128
catctccagc agtcaagatg gcgctcagtg gactctc                               37


<210> 129
<211> 44
<212> DNA
<213> Artificial Sequence

<220>
<223> Description of Artificial Sequence: Synthetic
      primer

<400> 129
gcagtcaaga tggccatgcg tggactctct tttttcagaa tgcc                       44


<210> 130
<211> 33
<212> DNA
<213> Artificial Sequence

<220>
<223> Description of Artificial Sequence: Synthetic
      primer

<400> 130
ggccatcagt gggctctctt ttttcagaat ggc                                    33


<210> 131
<211> 45
<212> DNA
<213> Artificial Sequence

<220>
<223> Description of Artificial Sequence: Synthetic
      primer

<400> 131
gatggccatc agtggactgc cttttttcag aatggcaaag taaag                      45


<210> 132
<211> 45
<212> DNA
<213> Artificial Sequence

<220>
<223> Description of Artificial Sequence: Synthetic
      primer

<400> 132
cagtggactc tcttttttca ggctggcaaa gtaaaggttt ttcag                       45


<210> 133
<211> 46
<212> DNA
<213> Artificial Sequence

<220>
<223> Description of Artificial Sequence: Synthetic
      primer

<400> 133
ggactctctt ttttcagaat ggcgcagtaa aggtttttca gaatgg                      46


<210> 134
<211> 34
<212> DNA
<213> Artificial Sequence

<220>
<223> Description of Artificial Sequence: Synthetic
      primer

<400> 134
ggtgaactct ctagacccac tgttactgac tcgc                                   34


<210> 135
<211> 37
<212> DNA
<213> Artificial Sequence

<220>
<223> Description of Artificial Sequence: Synthetic

primer

<400> 135
gacccaccgt tagcgactcg ctaccttcga attcacc                                37


<210> 136
<211> 34
<212> DNA
<213> Artificial Sequence

<220>
<223> Description of Artificial Sequence: Synthetic
      primer

<400> 136
ccaccgttac tgactcacta ccttcgaatt cacc                                   34


<210> 137
<211> 36
<212> DNA
<213> Artificial Sequence

<220>
<223> Description of Artificial Sequence: Synthetic
      primer

<400> 137
ctgactcgct accttcaaat tcacccccag agttgg                                 36


<210> 138
<211> 34
<212> DNA
<213> Artificial Sequence

<220>
<223> Description of Artificial Sequence: Synthetic
      primer

<400> 138
cgctaccttc gaattgcccc ccagagttgg gtgc                                   34


<210> 139
<211> 34
<212> DNA
<213> Artificial Sequence

<220>
<223> Description of Artificial Sequence: Synthetic
      primer

<400> 139
ccttcgaatt caccccgcga gttgggtgca ccag                                   34


<210> 140
<211> 37
<212> DNA
<213> Artificial Sequence

<220>
<223> Description of Artificial Sequence: Synthetic
      primer

<400> 140
cgaattcacc cccagagtta cgtgcaccag attgccc                                37


<210> 141
<211> 35
<212> DNA
<213> Artificial Sequence

<220>
<223> Description of Artificial Sequence: Synthetic
      primer

<400> 141
ccagagttgg gtggcccaga ttgccctgag gatgg                                  35


<210> 142
<211> 37
<212> DNA
<213> Artificial Sequence

<220>
<223> Description of Artificial Sequence: Synthetic
      primer

<400> 142
ccccagagtt gggtgcacgc cattgccctg aggatgg                                37


<210> 143
<211> 26
<212> DNA
<213> Artificial Sequence

<220>
<223> Description of Artificial Sequence: Synthetic
      primer

<400> 143
ggttctgggc tgcgcggcac aggacc                                            26


<210> 144
<211> 44
<212> DNA
<213> Artificial Sequence

<220>
<223> Description of Artificial Sequence: Synthetic
      primer

<400> 144
cccaatggca tgctgcaagc atttaaatcc actcttttct cgag                        44


<210> 145
<211> 40
<212> DNA

<213> Artificial Sequence

<220>
<223> Description of Artificial Sequence: Synthetic
      primer

<400> 145
gcatctgata ttgctttact cgccattccc aatggcatgc                              40


<210> 146
<211> 45
<212> DNA
<213> Artificial Sequence

<220>
<223> Description of Artificial Sequence: Synthetic
      primer

<400> 146
ggtggcaaac atattggtag cgtaggatga agcagtaatc tgtgc                        45


<210> 147
<211> 36
<212> DNA
<213> Artificial Sequence

<220>
<223> Description of Artificial Sequence: Synthetic
      primer

<400> 147
ccaggtggca aacatattgg caaagtagga tgaagc                                  36


<210> 148
<211> 45
<212> DNA
<213> Artificial Sequence

<220>
<223> Description of Artificial Sequence: Synthetic
      primer

<400> 148
ccaggtggca aacatagcgg taaagtagga tgaagcagta atctg                        45


<210> 149
<211> 42
<212> DNA
<213> Artificial Sequence

<220>
<223> Description of Artificial Sequence: Synthetic
      primer

<400> 149
aaggagacca ggtggcaaac gcattggtaa agtaggatga ag                           42


<210> 150

<211> 40
<212> DNA
<213> Artificial Sequence

<220>
<223> Description of Artificial Sequence: Synthetic
      primer

<400> 150
ggagaccagg tggcagccat attggtaaag taggatgaag                              40


<210> 151
<211> 43
<212> DNA
<213> Artificial Sequence

<220>
<223> Description of Artificial Sequence: Synthetic
      primer

<400> 151
gcttttgaag gagaccaggt ggcgcacata ttggtaaagt agg                          43


<210> 152
<211> 43
<212> DNA
<213> Artificial Sequence

<220>
<223> Description of Artificial Sequence: Synthetic
      primer

<400> 152
gcttttgaag gagaccaggc ggcaaacata ttggtaaagt agg                          43


<210> 153
<211> 35
<212> DNA
<213> Artificial Sequence

<220>
<223> Description of Artificial Sequence: Synthetic
      primer

<400> 153
cccttggagg tgaagtcgag ctgctgaagg agacc                                   35


<210> 154
<211> 42
<212> DNA
<213> Artificial Sequence

<220>
<223> Description of Artificial Sequence: Synthetic
      primer

<400> 154
ggcattactc ctcccttgga gggcaagtcg agcttttgaa gg                           42

<210> 155
<211> 42
<212> DNA
<213> Artificial Sequence

<220>
<223> Description of Artificial Sequence: Synthetic
      primer

<400> 155
ggcattactc ctcccttggg cgtgaagtcg agcttttgaa gg                          42


<210> 156
<211> 42
<212> DNA
<213> Artificial Sequence

<220>
<223> Description of Artificial Sequence: Synthetic
      primer

<400> 156
ggcattactc ctccctgcga ggtgaagtcg agcttttgaa gg                          42


<210> 157
<211> 38
<212> DNA
<213> Artificial Sequence

<220>
<223> Description of Artificial Sequence: Synthetic
      primer

<400> 157
ggtctccagg cattactcgc cccttggagg tgaagtcg                               38


<210> 158
<211> 33
<212> DNA
<213> Artificial Sequence

<220>
<223> Description of Artificial Sequence: Synthetic
      primer

<400> 158
acctgaggtg cccaggcatt actcctccct tgg                                    33


<210> 159
<211> 38
<212> DNA
<213> Artificial Sequence

<220>
<223> Description of Artificial Sequence: Synthetic
      primer

<400> 159

ggattattca cctgaggttg ccaggcatta ctcctccc                                38

<210> 160
<211> 44
<212> DNA
<213> Artificial Sequence

<220>
<223> Description of Artificial Sequence: Synthetic
      primer

<400> 160
ccactctttt ggattattca ccgcaggtct ccaggcatta ctcc                        44

<210> 161
<211> 36
<212> DNA
<213> Artificial Sequence

<220>
<223> Description of Artificial Sequence: Synthetic
      primer

<400> 161
ccactctttt ggattattca tctgaggtct ccaggc                                 36

<210> 162
<211> 45
<212> DNA
<213> Artificial Sequence

<220>
<223> Description of Artificial Sequence: Synthetic
      primer

<400> 162
gccactcttt tggattagcc acctgaggtc tccaggcatt actcc                       45

<210> 163
<211> 39
<212> DNA
<213> Artificial Sequence

<220>
<223> Description of Artificial Sequence: Synthetic
      primer

<400> 163
gcagccactc ttttggagca ttcacctgag gtctccagg                              39

<210> 164
<211> 37
<212> DNA
<213> Artificial Sequence

<220>
<223> Description of Artificial Sequence: Synthetic
      primer

<400> 164
ccacttgcag ccactctgct ggattattca cctgagg                                37

<210> 165
<211> 41
<212> DNA
<213> Artificial Sequence

<220>
<223> Description of Artificial Sequence: Synthetic
      primer

<400> 165
ccacttgcac cactcttgtg gattattcac ctgaggtctc c                           41

<210> 166
<211> 45
<212> DNA
<213> Artificial Sequence

<220>
<223> Description of Artificial Sequence: Synthetic
      primer

<400> 166
cacatacatg ctggtaagca gagatgctac tccctgagta gttac                       45

<210> 167
<211> 36
<212> DNA
<213> Artificial Sequence

<220>
<223> Description of Artificial Sequence: Synthetic
      primer

<400> 167
cacatacatg ctggtaagca gagcttttac tccctg                                 36

<210> 168
<211> 29
<212> DNA
<213> Artificial Sequence

<220>
<223> Description of Artificial Sequence: Synthetic
      primer

<400> 168
atacatgctg gtaagcgcag attttactc                                         29

<210> 169
<211> 41
<212> DNA
<213> Artificial Sequence

<220>

<223> Description of Artificial Sequence: Synthetic
      primer

<400> 169
ccttcacata catgctggta gccagagatt ttactcctga g                41


<210> 170
<211> 39
<212> DNA
<213> Artificial Sequence

<220>
<223> Description of Artificial Sequence: Synthetic
      primer

<400> 170
ctccttcaca tacatgctgg caagcagaga ttttactcc                39


<210> 171
<211> 37
<212> DNA
<213> Artificial Sequence

<220>
<223> Description of Artificial Sequence: Synthetic
      primer

<400> 171
gagagtccac tgagcgccat cttgactgct ggagatg                37


<210> 172
<211> 44
<212> DNA
<213> Artificial Sequence

<220>
<223> Description of Artificial Sequence: Synthetic
      primer

<400> 172
ggcattctga aaaaagagag tccacgcatg gccatcttga ctgc                44


<210> 173
<211> 33
<212> DNA
<213> Artificial Sequence

<220>
<223> Description of Artificial Sequence: Synthetic
      primer

<400> 173
gccattctga aaaaagagag cccactgatg gcc                33


<210> 174
<211> 45
<212> DNA
<213> Artificial Sequence

<220>
<223> Description of Artificial Sequence: Synthetic
      primer

<400> 174
ctttactttg ccattctgaa aaaaggcagt ccactgatgg ccatc                    45


<210> 175
<211> 45
<212> DNA
<213> Artificial Sequence

<220>
<223> Description of Artificial Sequence: Synthetic
      primer

<400> 175
ctgaaaaacc tttactttgc cagcctgaaa aaagagagtc cactg                    45


<210> 176
<211> 46
<212> DNA
<213> Artificial Sequence

<220>
<223> Description of Artificial Sequence: Synthetic
      primer

<400> 176
ccattctgaa aaacctttac tgcgccattc tgaaaaaaga gagtcc                   46


<210> 177
<211> 34
<212> DNA
<213> Artificial Sequence

<220>
<223> Description of Artificial Sequence: Synthetic
      primer

<400> 177
gcgagtcagt aacagtgggt ctagagagtt cacc                                34


<210> 178
<211> 37
<212> DNA
<213> Artificial Sequence

<220>
<223> Description of Artificial Sequence: Synthetic
      primer

<400> 178
ggtgaattcg aaggtagcga gtcgctaacg gtgggtc                             37


<210> 179
<211> 34

<212> DNA
<213> Artificial Sequence

<220>
<223> Description of Artificial Sequence: Synthetic
      primer

<400> 179
ggtgaattcg aaggtagtga gtcagtaacg gtgg                                    34


<210> 180
<211> 36
<212> DNA
<213> Artificial Sequence

<220>
<223> Description of Artificial Sequence: Synthetic
      primer

<400> 180
ccaactctgg gggtgaattt gaaggtagcg agtcag                                  36


<210> 181
<211> 34
<212> DNA
<213> Artificial Sequence

<220>
<223> Description of Artificial Sequence: Synthetic
      primer

<400> 181
gcacccaact ctggggggca attcgaaggt agcg                                     34


<210> 182

<400> 182
000


<210> 183

<400> 183
000


<210> 184

<400> 184
000


<210> 185

<400> 185
000


<210> 186

<400> 186
000


<210> 187

<400> 187
000


<210> 188

<400> 188
000


<210> 189
<211> 34
<212> DNA
<213> Artificial Sequence

<220>
<223> Description of Artificial Sequence: Synthetic
      primer

<400> 189
ctggtgcacc caactcgcgg ggtgaattcg aagg                                34


<210> 190
<211> 37
<212> DNA
<213> Artificial Sequence

<220>
<223> Description of Artificial Sequence: Synthetic
      primer

<400> 190
gggcaatctg gtgcacgtaa ctctgggggt gaattcg                             37


<210> 191
<211> 35
<212> DNA
<213> Artificial Sequence

<220>
<223> Description of Artificial Sequence: Synthetic
      primer

<400> 191
ccatcctcag ggcaatctgg gccacccaac tctgg                               35


<210> 192
<211> 37
<212> DNA
<213> Artificial Sequence

<220>
<223> Description of Artificial Sequence: Synthetic
      primer

<400> 192
ccatcctcag ggcaatggcg tgcacccaac tctgggg                                37

<210> 193
<211> 26
<212> DNA
<213> Artificial Sequence

<220>
<223> Description of Artificial Sequence: Synthetic
      primer

<400> 193
ggtcctgtgc cgcgcagccc agaacc                                            26

<210> 194
<211> 20
<212> PRT
<213> Homo sapiens

<400> 194
Met Ala Tyr Thr Asp Glu Thr Phe Lys Thr Arg Glu Ala Ile Gln His
1               5                   10                  15

Glu Ser Gly Ile
            20

<210> 195
<211> 20
<212> PRT
<213> Homo sapiens

<400> 195
Leu Tyr Gly Glu Val Gly Asp Thr Leu Leu Ile Ile Phe Lys Asn Gln
1               5                   10                  15

Ala Ser Arg Pro
            20

<210> 196
<211> 20
<212> PRT
<213> Homo sapiens

<400> 196
Ala Ser Arg Pro Tyr Asn Ile Tyr Pro His Gly Ile Thr Asp Val Arg
1               5                   10                  15

Pro Leu Tyr Ser
            20

<210> 197
<211> 20
<212> PRT

<213> Homo sapiens

<400> 197
Phe Pro Ile Leu Pro Gly Glu Ile Phe Lys Tyr Lys Trp Thr Val Thr
1               5                   10                  15

Val Glu Asp Gly
            20

<210> 198
<211> 20
<212> PRT
<213> Homo sapiens

<400> 198
Leu Thr Arg Tyr Tyr Ser Ser Phe Val Asn Met Glu Arg Asp Leu Ala
1               5                   10                  15

Ser Gly Leu Ile
            20

<210> 199
<211> 20
<212> PRT
<213> Homo sapiens

<400> 199
Arg Asp Leu Ala Ser Gly Leu Ile Gly Pro Leu Leu Ile Cys Tyr Lys
1               5                   10                  15

Glu Ser Val Asp
            20

<210> 200
<211> 20
<212> PRT
<213> Homo sapiens

<400> 200
Glu Asn Arg Ser Trp Tyr Leu Thr Glu Asn Ile Gln Arg Phe Leu Pro
1               5                   10                  15

Asn Pro Ala Gly
            20

<210> 201
<211> 20
<212> PRT
<213> Homo sapiens

<400> 201
Glu Asn Arg Ser Trp Tyr Leu Thr Glu Asn Ile Gln Cys Phe Leu Pro
1               5                   10                  15

Asn Pro Ala Gly
            20

<210> 202
<211> 20
<212> PRT
<213> Homo sapiens

<400> 202
Glu Asn Ile Gln Arg Phe Leu Pro Asn Pro Ala Gly Val Gln Leu Glu
1               5                   10                  15

Asp Pro Glu Phe
            20

<210> 203
<211> 20
<212> PRT
<213> Homo sapiens

<400> 203
Glu Asn Ile Gln Cys Phe Leu Pro Asn Pro Ala Gly Val Gln Leu Glu
1               5                   10                  15

Asp Pro Glu Phe
            20

<210> 204
<211> 20
<212> PRT
<213> Homo sapiens

<400> 204
Asp Pro Glu Phe Gln Ala Ser Asn Ile Met His Ser Ile Asn Gly Tyr
1               5                   10                  15

Val Phe Asp Ser
            20

<210> 205
<211> 20
<212> PRT
<213> Homo sapiens

<400> 205
Ala Ser Asn Ile Met His Ser Ile Asn Gly Tyr Val Phe Asp Ser Leu
1               5                   10                  15

Gln Leu Ser Val
            20

<210> 206
<211> 20
<212> PRT

<213> Homo sapiens

<400> 206
Leu His Glu Val Ala Tyr Trp Tyr Ile Leu Ser Ile Gly Ala Gln Thr
1               5                   10                  15

Asp Phe Leu Ser
            20

<210> 207
<211> 20
<212> PRT
<213> Homo sapiens

<400> 207
Phe Ser Gly Tyr Thr Phe Lys His Lys Met Val Tyr Glu Asp Thr Leu
1               5                   10                  15

Thr Leu Phe Pro
            20

<210> 208
<211> 20
<212> PRT
<213> Homo sapiens

<400> 208
Phe Ser Gly Tyr Thr Phe Lys His Lys Met Val Tyr Glu Asp Thr Leu
1               5                   10                  15

Thr Leu Phe Pro
            20

<210> 209
<211> 20
<212> PRT
<213> Homo sapiens

<400> 209
Thr Val Phe Met Ser Met Glu Asn Pro Gly Leu Trp Ile Leu Gly Cys
1               5                   10                  15

His Asn Ser Asp
            20

<210> 210
<211> 20
<212> PRT
<213> Homo sapiens

<400> 210
Thr Val Phe Met Ser Met Glu Asn Pro Gly Leu Trp Ile Leu Gly Cys
1               5                   10                  15

His Asn Ser Asp
            20

<210> 211
<211> 20
<212> PRT
<213> Homo sapiens

<400> 211
Ser Val Ala Lys Lys His Pro Lys Thr Trp Val His Tyr Ile Ala Ala
1               5                   10                  15

Glu Glu Glu Asp
            20

<210> 212
<211> 20
<212> PRT
<213> Homo sapiens

<400> 212
Thr Trp Val His Tyr Ile Ala Ala Glu Glu Glu Asp Trp Asp Tyr Ala
1               5                   10                  15

Pro Leu Val Leu
            20

<210> 213
<211> 20
<212> PRT
<213> Homo sapiens

<400> 213
Glu Glu Glu Asp Trp Asp Tyr Ala Pro Leu Val Leu Ala Pro Asp Asp
1               5                   10                  15

Arg Ser Tyr Lys
            20

<210> 214
<211> 20
<212> PRT
<213> Homo sapiens

<400> 214
Pro Leu Val Leu Ala Pro Asp Asp Arg Ser Tyr Lys Ser Gln Tyr Leu
1               5                   10                  15

Asn Asn Gly Pro
            20

<210> 215
<211> 20
<212> PRT

<213> Homo sapiens

<400> 215
Arg Ser Tyr Lys Ser Gln Tyr Leu Asn Asn Gly Pro Gln Arg Ile Gly
1               5                   10                  15

Arg Lys Tyr Lys
            20

<210> 216
<211> 20
<212> PRT
<213> Homo sapiens

<400> 216
Asn Asn Gly Pro Gln Arg Ile Gly Arg Lys Tyr Lys Lys Val Arg Phe
1               5                   10                  15

Met Ala Tyr Thr
            20

<210> 217
<211> 20
<212> PRT
<213> Homo sapiens

<400> 217
Arg Lys Tyr Lys Lys Val Arg Phe Met Ala Tyr Thr Asp Glu Thr Phe
1               5                   10                  15

Lys Thr Arg Glu
            20

<210> 218
<211> 20
<212> PRT
<213> Homo sapiens

<400> 218
Met Ala Tyr Thr Asp Glu Thr Phe Lys Thr Arg Glu Ala Ile Gln His
1               5                   10                  15

Glu Ser Gly Ile
            20

<210> 219
<211> 20
<212> PRT
<213> Homo sapiens

<400> 219
Lys Thr Arg Glu Ala Ile Gln His Glu Ser Gly Ile Leu Gly Pro Leu
1               5                   10                  15

Leu Tyr Gly Glu
            20

<210> 220
<211> 20
<212> PRT
<213> Homo sapiens

<400> 220
Glu Ser Gly Ile Leu Gly Pro Leu Leu Tyr Gly Glu Val Gly Asp Thr
1               5                   10                  15

Leu Leu Ile Ile
            20

<210> 221
<211> 20
<212> PRT
<213> Homo sapiens

<400> 221
Leu Tyr Gly Glu Val Gly Asp Thr Leu Leu Ile Ile Phe Lys Asn Gln
1               5                   10                  15

Ala Ser Arg Pro
            20

<210> 222
<211> 20
<212> PRT
<213> Homo sapiens

<400> 222
Leu Leu Ile Ile Phe Lys Asn Gln Ala Ser Arg Pro Tyr Asn Ile Tyr
1               5                   10                  15

Pro His Gly Ile
            20

<210> 223
<211> 20
<212> PRT
<213> Homo sapiens

<400> 223
Ala Ser Arg Pro Tyr Asn Ile Tyr Pro His Gly Ile Thr Asp Val Arg
1               5                   10                  15

Pro Leu Tyr Ser
            20

<210> 224
<211> 20
<212> PRT

<213> Homo sapiens

<400> 224
Pro His Gly Ile Thr Asp Val Arg Pro Leu Tyr Ser Arg Arg Leu Pro
1               5                   10                  15

Lys Gly Val Lys
            20

<210> 225
<211> 20
<212> PRT
<213> Homo sapiens

<400> 225
Pro Leu Tyr Ser Arg Arg Leu Pro Lys Gly Val Lys His Leu Lys Asp
1               5                   10                  15

Phe Pro Ile Leu
            20

<210> 226
<211> 20
<212> PRT
<213> Homo sapiens

<400> 226
Lys Gly Val Lys His Leu Lys Asp Phe Pro Ile Leu Pro Gly Glu Ile
1               5                   10                  15

Phe Lys Tyr Lys
            20

<210> 227
<211> 20
<212> PRT
<213> Homo sapiens

<400> 227
Phe Pro Ile Leu Pro Gly Glu Ile Phe Lys Tyr Lys Trp Thr Val Thr
1               5                   10                  15

Val Glu Asp Gly
            20

<210> 228
<211> 20
<212> PRT
<213> Homo sapiens

<400> 228
Ile Phe Lys Tyr Lys Trp Thr Val Thr Val Glu Asp Gly Pro Thr Lys
1               5                   10                  15

Ser Asp Pro Arg
            20

<210> 229
<211> 20
<212> PRT
<213> Homo sapiens

<400> 229
Val Glu Asp Gly Pro Thr Lys Ser Asp Pro Arg Cys Leu Thr Arg Tyr
1               5                   10                  15

Tyr Ser Ser Phe
            20

<210> 230
<211> 20
<212> PRT
<213> Homo sapiens

<400> 230
Asp Pro Arg Cys Leu Thr Arg Tyr Tyr Ser Ser Phe Val Asn Met Glu
1               5                   10                  15

Arg Asp Leu Ala
            20

<210> 231
<211> 20
<212> PRT
<213> Homo sapiens

<400> 231
Leu Thr Arg Tyr Tyr Ser Ser Phe Val Asn Met Glu Arg Asp Leu Ala
1               5                   10                  15

Ser Gly Leu Ile
            20

<210> 232
<211> 20
<212> PRT
<213> Homo sapiens

<400> 232
Tyr Ser Ser Phe Val Asn Met Glu Arg Asp Leu Ala Ser Gly Leu Ile
1               5                   10                  15

Gly Pro Leu Leu
            20

<210> 233
<211> 20
<212> PRT

<213> Homo sapiens

<400> 233
Arg Asp Leu Ala Ser Gly Leu Ile Gly Pro Leu Leu Ile Cys Tyr Lys
1               5                   10                  15

Glu Ser Val Asp
            20

<210> 234
<211> 20
<212> PRT
<213> Homo sapiens

<400> 234
Gly Pro Leu Leu Ile Cys Tyr Lys Glu Ser Val Asp Gln Arg Gly Asn
1               5                   10                  15

Gln Ile Met Ser
            20

<210> 235
<211> 20
<212> PRT
<213> Homo sapiens

<400> 235
Glu Ser Val Asp Gln Arg Gly Asn Gln Ile Met Ser Asp Lys Arg Asn
1               5                   10                  15

Val Ile Leu Phe
            20

<210> 236
<211> 20
<212> PRT
<213> Homo sapiens

<400> 236
Gln Ile Met Ser Asp Lys Arg Asn Val Ile Leu Phe Ser Val Phe Asp
1               5                   10                  15

Glu Asn Arg Ser
            20

<210> 237
<211> 20
<212> PRT
<213> Homo sapiens

<400> 237
Val Ile Leu Phe Ser Val Phe Asp Glu Asn Arg Ser Trp Tyr Leu Thr
1               5                   10                  15

Glu Asn Ile Gln
            20

<210> 238
<211> 20
<212> PRT
<213> Homo sapiens

<400> 238
Glu Asn Arg Ser Trp Tyr Leu Thr Glu Asn Ile Gln Arg Phe Leu Pro
1               5                   10                  15

Asn Pro Ala Gly
            20

<210> 239
<211> 20
<212> PRT
<213> Homo sapiens

<400> 239
Glu Asn Ile Gln Arg Phe Leu Pro Asn Pro Ala Gly Val Gln Leu Glu
1               5                   10                  15

Asp Pro Glu Phe
            20

<210> 240
<211> 20
<212> PRT
<213> Homo sapiens

<400> 240
Asn Pro Ala Gly Val Gln Leu Glu Asp Pro Glu Phe Gln Ala Ser Asn
1               5                   10                  15

Ile Met His Ser
            20

<210> 241
<211> 20
<212> PRT
<213> Homo sapiens

<400> 241
Asp Pro Glu Phe Gln Ala Ser Asn Ile Met His Ser Ile Asn Gly Tyr
1               5                   10                  15

Val Phe Asp Ser
            20

<210> 242
<211> 20
<212> PRT

<213> Homo sapiens

<400> 242
Ile Met His Ser Ile Asn Gly Tyr Val Phe Asp Ser Leu Gln Leu Ser
1               5                   10                  15

Val Cys Leu His
            20

<210> 243
<211> 20
<212> PRT
<213> Homo sapiens

<400> 243
Ala Ser Asn Ile Met His Ser Ile Asn Gly Tyr Val Phe Asp Ser Leu
1               5                   10                  15

Gln Leu Ser Val
            20

<210> 244
<211> 20
<212> PRT
<213> Homo sapiens

<400> 244
Val Phe Asp Ser Leu Gln Leu Ser Val Cys Leu His Glu Val Ala Tyr
1               5                   10                  15

Trp Tyr Ile Leu
            20

<210> 245
<211> 20
<212> PRT
<213> Homo sapiens

<400> 245
Val Cys Leu His Glu Val Ala Tyr Trp Tyr Ile Leu Ser Ile Gly Ala
1               5                   10                  15

Gln Thr Asp Phe
            20

<210> 246
<211> 20
<212> PRT
<213> Homo sapiens

<400> 246
Leu His Glu Val Ala Tyr Trp Tyr Ile Leu Ser Ile Gly Ala Gln Thr
1               5                   10                  15

Asp Phe Leu Ser
            20

<210> 247
<211> 20
<212> PRT
<213> Homo sapiens

<400> 247
Trp Tyr Ile Leu Ser Ile Gly Ala Gln Thr Asp Phe Leu Ser Val Phe
1               5                   10                  15

Phe Ser Gly Tyr
            20

<210> 248
<211> 20
<212> PRT
<213> Homo sapiens

<400> 248
Gln Thr Asp Phe Leu Ser Val Phe Phe Ser Gly Tyr Thr Phe Lys His
1               5                   10                  15

Lys Met Val Tyr
            20

<210> 249
<211> 20
<212> PRT
<213> Homo sapiens

<400> 249
Phe Ser Gly Tyr Thr Phe Lys His Lys Met Val Tyr Glu Asp Thr Leu
1               5                   10                  15

Thr Leu Phe Pro
            20

<210> 250
<211> 20
<212> PRT
<213> Homo sapiens

<400> 250
Lys Met Val Tyr Glu Asp Thr Leu Thr Leu Phe Pro Phe Ser Gly Glu
1               5                   10                  15

Thr Val Phe Met
            20

<210> 251
<211> 20
<212> PRT

<213> Homo sapiens

<400> 251
Thr Leu Phe Pro Phe Ser Gly Glu Thr Val Phe Met Ser Met Glu Asn
1               5                   10                  15

Pro Gly Leu Trp
            20

<210> 252
<211> 20
<212> PRT
<213> Homo sapiens

<400> 252
Thr Val Phe Met Ser Met Glu Asn Pro Gly Leu Trp Ile Leu Gly Cys
1               5                   10                  15

His Asn Ser Asp
            20

<210> 253
<211> 20
<212> PRT
<213> Homo sapiens

<400> 253
Pro Phe Ser Gly Glu Thr Val Phe Met Ser Met Glu Asn Pro Gly Leu
1               5                   10                  15

Trp Ile Leu Gly
            20

<210> 254
<211> 20
<212> PRT
<213> Homo sapiens

<400> 254
Pro Gly Leu Trp Ile Leu Gly Cys His Asn Ser Asp Phe Arg Asn Arg
1               5                   10                  15

Gly Met Thr Ala
            20

<210> 255
<211> 20
<212> PRT
<213> Homo sapiens

<400> 255
His Asn Ser Asp Phe Arg Asn Arg Gly Met Thr Ala Leu Leu Lys Val
1               5                   10                  15

Ser Ser Cys Asp
            20

<210> 256
<211> 18
<212> PRT
<213> Homo sapiens

<400> 256
His Asn Ser Asp Phe Arg Asn Arg Gly Met Thr Ala Leu Leu Lys Val
1               5                   10                  15

Ser Ser

<210> 257
<211> 20
<212> PRT
<213> Homo sapiens

<400> 257
Gly Met Thr Ala Leu Leu Lys Val Ser Ser Cys Asp Lys Asn Thr Gly
1               5                   10                  15

Asp Tyr Tyr Glu
            20

<210> 258
<211> 20
<212> PRT
<213> Homo sapiens

<400> 258
Ser Ser Cys Asp Lys Asn Thr Gly Asp Tyr Tyr Glu Asp Ser Tyr Glu
1               5                   10                  15

Asp Ile Ser Ala
            20

<210> 259
<211> 20
<212> PRT
<213> Homo sapiens

<400> 259
Asp Lys Asn Thr Gly Asp Tyr Tyr Glu Asp Ser Tyr Glu Asp Ile Ser
1               5                   10                  15

Ala Tyr Leu Leu
            20

<210> 260
<211> 24
<212> PRT

<213> Homo sapiens

<400> 260
Asp Tyr Tyr Glu Asp Ser Tyr Glu Asp Ile Ser Ala Tyr Leu Leu Ser
1               5                   10                  15

Lys Asn Asn Ala Ile Glu Pro Arg
            20

<210> 261
<211> 20
<212> PRT
<213> Homo sapiens

<400> 261
Glu Asn Arg Ser Trp Tyr Leu Thr Glu Asn Ile Gln Cys Phe Leu Pro
1               5                   10                  15

Asn Pro Ala Gly
            20

<210> 262
<211> 20
<212> PRT
<213> Homo sapiens

<400> 262
Glu Asn Ile Gln Cys Phe Leu Pro Asn Pro Ala Gly Val Gln Leu Glu
1               5                   10                  15

Asp Pro Glu Phe
            20

<210> 263
<211> 20
<212> PRT
<213> Homo sapiens

<400> 263
Glu His Leu His Ala Gly Met Ser Thr Leu Phe Leu Val Tyr Ser Asn
1               5                   10                  15

Lys Cys Gln Thr
            20

<210> 264
<211> 20
<212> PRT
<213> Homo sapiens

<400> 264
Leu Ile Gly Glu His Leu His Ala Gly Met Ser Thr Leu Phe Leu Val
1               5                   10                  15

Tyr Ser Asn Lys
            20

<210> 265
<211> 20
<212> PRT
<213> Homo sapiens

<400> 265
Thr Leu Phe Leu Val Tyr Ser Asn Lys Cys Gln Thr Pro Leu Gly Met
1               5                   10                  15

Ala Ser Gly His
            20

<210> 266
<211> 20
<212> PRT
<213> Homo sapiens

<400> 266
Lys Cys Gln Thr Pro Leu Gly Met Ala Ser Gly His Ile Arg Asp Phe
1               5                   10                  15

Gln Ile Thr Ala
            20

<210> 267
<211> 20
<212> PRT
<213> Homo sapiens

<400> 267
Gln Thr Pro Leu Gly Met Ala Ser Gly His Ile Arg Asp Phe Gln Ile
1               5                   10                  15

Thr Ala Ser Gly
            20

<210> 268
<211> 20
<212> PRT
<213> Homo sapiens

<400> 268
Ala Ser Gly His Ile Arg Asp Phe Gln Ile Thr Ala Ser Gly Gln Tyr
1               5                   10                  15

Gly Gln Trp Ala
            20

<210> 269
<211> 20
<212> PRT

<213> Homo sapiens

<400> 269
Gln Ile Thr Ala Ser Gly Gln Tyr Gly Gln Trp Ala Pro Lys Leu Ala
1               5                   10                  15

Arg Leu His Tyr
            20

<210> 270
<211> 20
<212> PRT
<213> Homo sapiens

<400> 270
Gly Gln Trp Ala Pro Lys Leu Ala Arg Leu His Tyr Ser Gly Ser Ile
1               5                   10                  15

Asn Ala Trp Ser
            20

<210> 271
<211> 20
<212> PRT
<213> Homo sapiens

<400> 271
Arg Leu His Tyr Ser Gly Ser Ile Asn Ala Trp Ser Thr Lys Glu Pro
1               5                   10                  15

Phe Ser Trp Ile
            20

<210> 272
<211> 20
<212> PRT
<213> Homo sapiens

<400> 272
Asn Ala Trp Ser Thr Lys Glu Pro Phe Ser Trp Ile Lys Val Asp Leu
1               5                   10                  15

Leu Ala Pro Met
            20

<210> 273
<211> 20
<212> PRT
<213> Homo sapiens

<400> 273
Phe Ser Trp Ile Lys Val Asp Leu Leu Ala Pro Met Ile Ile His Gly
1               5                   10                  15

Ile Lys Thr Gln
            20

<210> 274
<211> 20
<212> PRT
<213> Homo sapiens

<400> 274
Leu Ala Pro Met Ile Ile His Gly Ile Lys Thr Gln Gly Ala Arg Gln
1               5                   10                  15

Lys Phe Ser Ser
            20

<210> 275
<211> 20
<212> PRT
<213> Homo sapiens

<400> 275
Ile Lys Thr Gln Gly Ala Arg Gln Lys Phe Ser Ser Leu Tyr Ile Ser
1               5                   10                  15

Gln Phe Ile Ile
            20

<210> 276
<211> 20
<212> PRT
<213> Homo sapiens

<400> 276
Lys Phe Ser Ser Leu Tyr Ile Ser Gln Phe Ile Ile Met Tyr Ser Leu
1               5                   10                  15

Asp Gly Lys Lys
            20

<210> 277
<211> 20
<212> PRT
<213> Homo sapiens

<400> 277
Gln Phe Ile Ile Met Tyr Ser Leu Asp Gly Lys Lys Trp Gln Thr Tyr
1               5                   10                  15

Arg Gly Asn Ser
            20

<210> 278
<211> 20
<212> PRT

<213> Homo sapiens

<400> 278
Asp Gly Lys Lys Trp Gln Thr Tyr Arg Gly Asn Ser Thr Gly Thr Leu
1               5                   10                  15

Met Val Phe Phe
            20

<210> 279
<211> 20
<212> PRT
<213> Homo sapiens

<400> 279
Arg Gly Asn Ser Thr Gly Thr Leu Met Val Phe Phe Gly Asn Val Asp
1               5                   10                  15

Ser Ser Gly Ile
            20

<210> 280
<211> 20
<212> PRT
<213> Homo sapiens

<400> 280
Met Val Phe Phe Gly Asn Val Asp Ser Ser Gly Ile Lys His Asn Ile
1               5                   10                  15

Phe Asn Pro Pro
            20

<210> 281
<211> 20
<212> PRT
<213> Homo sapiens

<400> 281
Ser Ser Gly Ile Lys His Asn Ile Phe Asn Pro Pro Ile Ile Ala Arg
1               5                   10                  15

Tyr Ile Arg Leu
            20

<210> 282
<211> 20
<212> PRT
<213> Homo sapiens

<400> 282
Phe Asn Pro Pro Ile Ile Ala Arg Tyr Ile Arg Leu His Pro Thr His
1               5                   10                  15

Tyr Ser Ile Arg
            20

<210> 283
<211> 20
<212> PRT
<213> Homo sapiens

<400> 283
Tyr Ile Arg Leu His Pro Thr His Tyr Ser Ile Arg Ser Thr Leu Arg
1               5                   10                  15

Met Glu Leu Met
            20

<210> 284
<211> 20
<212> PRT
<213> Homo sapiens

<400> 284
Thr His Tyr Ser Ile Arg Ser Thr Leu Arg Met Glu Leu Met Gly Cys
1               5                   10                  15

Asp Leu Asn Ser
            20

<210> 285
<211> 20
<212> PRT
<213> Homo sapiens

<400> 285
Asp Leu Asn Ser Cys Ser Met Pro Leu Gly Met Glu Ser Lys Ala Ile
1               5                   10                  15

Ser Asp Ala Gln
            20

<210> 286
<211> 20
<212> PRT
<213> Homo sapiens

<400> 286
Leu Gly Met Glu Ser Lys Ala Ile Ser Asp Ala Gln Ile Thr Ala Ser
1               5                   10                  15

Ser Tyr Phe Thr
            20

<210> 287
<211> 19
<212> PRT

<213> Homo sapiens

<400> 287
Asp Ala Gln Ile Thr Ala Ser Ser Tyr Phe Thr Asn Met Phe Ala Thr
1               5                   10                  15

Trp Ser Pro

<210> 288
<211> 20
<212> PRT
<213> Homo sapiens

<400> 288
Ser Asp Ala Gln Ile Thr Ala Ser Ser Tyr Phe Thr Asn Met Phe Ala
1               5                   10                  15

Thr Trp Ser Pro
            20

<210> 289
<211> 20
<212> PRT
<213> Homo sapiens

<400> 289
Ser Tyr Phe Thr Asn Met Phe Ala Thr Trp Ser Pro Ser Lys Ala Arg
1               5                   10                  15

Leu His Leu Gln
            20

<210> 290
<211> 20
<212> PRT
<213> Homo sapiens

<400> 290
Thr Trp Ser Pro Ser Lys Ala Arg Leu His Leu Gln Gly Arg Ser Asn
1               5                   10                  15

Ala Trp Arg Pro
            20

<210> 291
<211> 20
<212> PRT
<213> Homo sapiens

<400> 291
Leu His Leu Gln Gly Arg Ser Asn Ala Trp Arg Pro Gln Val Asn Asn
1               5                   10                  15

Pro Lys Glu Trp
            20

<210> 292
<211> 20
<212> PRT
<213> Homo sapiens

<400> 292
Ala Trp Arg Pro Gln Val Asn Asn Pro Lys Glu Trp Leu Gln Val Asp
1               5                   10                  15

Phe Gln Lys Thr
            20

<210> 293
<211> 20
<212> PRT
<213> Homo sapiens

<400> 293
Pro Lys Glu Trp Leu Gln Val Asp Phe Gln Lys Thr Met Lys Val Thr
1               5                   10                  15

Gly Val Thr Thr
            20

<210> 294
<211> 20
<212> PRT
<213> Homo sapiens

<400> 294
Phe Gln Lys Thr Met Lys Val Thr Gly Val Thr Thr Gln Gly Val Lys
1               5                   10                  15

Ser Leu Leu Thr
            20

<210> 295
<211> 20
<212> PRT
<213> Homo sapiens

<400> 295
Gly Val Thr Thr Gln Gly Val Lys Ser Leu Leu Thr Ser Met Tyr Val
1               5                   10                  15

Lys Glu Phe Leu
            20

<210> 296
<211> 20
<212> PRT

<213> Homo sapiens

<400> 296
Ser Leu Leu Thr Ser Met Tyr Val Lys Glu Phe Leu Ile Ser Ser Ser
1               5                   10                  15

Gln Asp Gly His
            20

<210> 297
<211> 20
<212> PRT
<213> Homo sapiens

<400> 297
Lys Glu Phe Leu Ile Ser Ser Ser Gln Asp Gly His Gln Trp Thr Leu
1               5                   10                  15

Phe Phe Gln Asn
            20

<210> 298
<211> 20
<212> PRT
<213> Homo sapiens

<400> 298
Ser Gln Asp Gly His Gln Trp Thr Leu Phe Phe Gln Asn Gly Lys Val
1               5                   10                  15

Lys Val Phe Gln
            20

<210> 299
<211> 20
<212> PRT
<213> Homo sapiens

<400> 299
Leu Phe Phe Gln Asn Gly Lys Val Lys Val Phe Gln Gly Asn Gln Asp
1               5                   10                  15

Ser Phe Thr Pro
            20

<210> 300
<211> 20
<212> PRT
<213> Homo sapiens

<400> 300
Lys Val Phe Gln Gly Asn Gln Asp Ser Phe Thr Pro Val Val Asn Ser
1               5                   10                  15

Leu Asp Pro Pro
            20

<210> 301
<211> 20
<212> PRT
<213> Homo sapiens

<400> 301
Ser Phe Thr Pro Val Val Asn Ser Leu Asp Pro Pro Leu Leu Thr Arg
1               5                   10                  15

Tyr Leu Arg Ile
            20

<210> 302
<211> 20
<212> PRT
<213> Homo sapiens

<400> 302
Leu Asp Pro Pro Leu Leu Thr Arg Tyr Leu Arg Ile His Pro Gln Ser
1               5                   10                  15

Trp Val His Gln
            20

<210> 303
<211> 20
<212> PRT
<213> Homo sapiens

<400> 303
Tyr Leu Arg Ile His Pro Gln Ser Trp Val His Gln Ile Ala Leu Arg
1               5                   10                  15

Met Glu Val Leu
            20

<210> 304
<211> 20
<212> PRT
<213> Homo sapiens

<400> 304
Trp Val His Gln Ile Ala Leu Arg Met Glu Val Leu Gly Cys Glu Ala
1               5                   10                  15

Gln Asp Leu Tyr
            20

<210> 305
<211> 15
<212> PRT

<213> Homo sapiens

<400> 305
Trp Val His Gln Ile Ala Leu Arg Met Glu Val Leu Gly Cys Glu
1               5                   10                  15


<210> 306
<211> 16
<212> PRT
<213> Homo sapiens

<400> 306
Ile Ala Leu Arg Met Glu Val Leu Gly Cys Glu Ala Gln Asp Leu Tyr
1               5                   10                  15


<210> 307
<211> 13
<212> PRT
<213> Influenza virus

<400> 307
Pro Lys Tyr Val Lys Gln Asn Thr Leu Lys Leu Ala Thr
1               5                   10


<210> 308
<211> 12
<212> PRT
<213> Physeter catodon

<400> 308
Leu Phe Arg Lys Asp Ile Ala Ala Lys Tyr Lys Glu
1               5                   10


<210> 309
<211> 11
<212> PRT
<213> Herpes simplex virus 2

<400> 309
Pro Leu Tyr Ala Thr Gly Arg Leu Ser Gln Ala
1               5                   10


<210> 310
<211> 19
<212> PRT
<213> Homo sapiens

<400> 310
Asn Pro Val Val His Phe Phe Lys Asn Ile Val Thr Pro Arg Thr Pro
1               5                   10                  15


Pro Pro Ser



<210> 311
<211> 10

<212> PRT
<213> Artificial Sequence

<220>
<223> Description of Artificial Sequence: Synthetic
      peptide

<400> 311
His His His His His His His His His His
1               5                   10


<210> 312
<211> 12
<212> PRT
<213> Homo sapiens

<400> 312
Gln Arg Phe Leu Pro Asn Pro Ala Gly Val Gln Leu
1               5                   10